(19) Europäisches Patentamt
European Patent Office
Office européen des brevets

(11) EP 4 417 606 A1

(12) **EUROPEAN PATENT APPLICATION**
published in accordance with Art. 153(4) EPC

(43) Date of publication:
**21.08.2024 Bulletin 2024/34**

(21) Application number: **22880399.5**

(22) Date of filing: **14.10.2022**

(51) International Patent Classification (IPC):
*C07D 471/00* (2006.01) *C07D 471/02* (2006.01)
*C07D 471/04* (2006.01) *C07D 487/04* (2006.01)
*C07D 519/00* (2006.01) *C07D 491/048* (2006.01)
*A61K 31/435* (2006.01) *A61K 31/4375* (2006.01)
*A61K 31/517* (2006.01) *A61P 35/00* (2006.01)

(52) Cooperative Patent Classification (CPC):
**A61K 31/435; A61K 31/4375; A61K 31/517; A61K 31/519; A61K 31/5377; A61P 35/00; C07D 471/00; C07D 471/02; C07D 471/04; C07D 487/04; C07D 491/048; C07D 519/00**

(86) International application number:
**PCT/CN2022/125282**

(87) International publication number:
**WO 2023/061463 (20.04.2023 Gazette 2023/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.10.2021 CN 202111200645**
**26.01.2022 CN 202210090616**
**02.06.2022 CN 202210624652**

(71) Applicant: **Sunshine Lake Pharma Co., Ltd.**
**Dongguan, Guangdong 523000 (CN)**

(72) Inventors:
- **XIE, Hongming**
  **Dongguan, Guangdong 523871 (CN)**
- **LIU, Haiwang**
  **Dongguan, Guangdong 523871 (CN)**
- **ZHANG, Yingjun**
  **Dongguan, Guangdong 523871 (CN)**
- **JIA, Jinlong**
  **Dongguan, Guangdong 523871 (CN)**
- **FENG, Xihui**
  **Dongguan, Guangdong 523871 (CN)**

(74) Representative: **Kurig, Thomas**
**Becker Kurig & Partner**
**Patentanwälte mbB**
**Bavariastraße 7**
**80336 München (DE)**

# (54) NOVEL PYRIMIDOPYRIDINE COMPOUND, PHARMACEUTICAL COMPOSITION THEREOF, AND USE THEREOF

(57) A pyrimidopyridine compound, a pharmaceutical composition thereof, and a use thereof. Specifically, the present invention relates to a compound represented by formula (I), or a stereoisomer, a tautomer, a nitrogen oxide, a solvate, a metabolite, a pharmaceutically acceptable salt or a prodrug of the compound represented by formula (I). The related compound and pharmaceutical composition thereof, as KRAS G12D inhibitors, can be used for preparing a drug for preventing or treating KRAS G12D-related diseases, especially for preparing a drug for preventing or treating cancer.

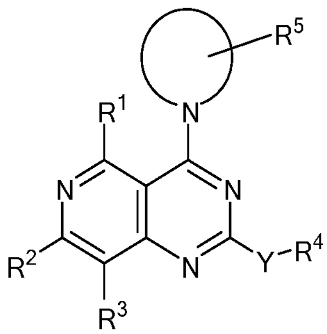

(I)

EP 4 417 606 A1

## Description

### RELATED APPLICATIONS

This application claims the priority of Chinese Patent Application No. 202111200645.6, which was filed to the State Intellectual Property Office on October 15, 2021; and claims the priority of Chinese Patent Application No. 202210090616.7, which was filed to the State Intellectual Property Office on January 26, 2022; and claims the priority of Chinese Patent Application No. 202210624652.7, which was filed to the State Intellectual Property Office on June 2, 2022, and all its contents are hereby incorporated by reference.

### FIELD OF THE INVENTION

The present invention belongs to the field of medicine. Specifically, the present invention relates to a new class of pyrimidopyridine compounds as KRAS G12D inhibitors, pharmaceutical compositions thereof, and use of the compounds and pharmaceutical compositions in the manufacture of a medicament for preventing or treating KRAS G12D related diseases.

### BACKGROUND ART

RAS gene is one of the most commonly mutated genes in cancer (20%-25%), currently known members of the RAS gene family include KRAS, NRAS and HRAS, of which KRAS mutation is the most common, accounting for approximately 85%. The mutation rate of KRAS is the highest in pancreatic ductal adenocarcinoma (PDAC), reaching 97%, followed by colorectal cancer, multiple myeloma and lung cancer, at 52%, 42% and 32% respectively. The most common way of KRAS gene mutation is point mutation, and the common mutation forms include KRAS G12D mutation (41%), KRAS G12V mutation (28%), and KRAS G12C mutation (14%). RAS gene mutations are often associated with poor prognosis of cancer, KRAS can be transiently activated by upstream growth factors or tyrosine kinases (such as EGFR), activated KRAS can activate downstream pathways, which commonly include the PI3K-AKT-mTOR signaling pathway that controls cell production, and the RAS-RAF-MEK-ERK signaling pathway that controls cell proliferation. This also lays a biological foundation for the combined use of many targets.
In recent years, people have made some progress in drug development using the allosteric sites of KRAS G12C mutants. For example, a research group reported the discovery of small molecule inhibitors of KRAS G12C (Nature, 2013, 503, 548- 551) in 2013; however, there are few studies on KRAS G12D. Compared with KRAS G12C mutations, the proportion of other mutation types such as KRAS G12D and KRAS G12V is higher in pancreatic cancer. Therefore, inhibiting KRAS G12D mutation is a potentially effective way to treat pancreatic cancer. Currently, Mirati has published a series of KRAS G12D inhibitors in patent application WO20211041671, which show specificity for KRAS G12D mutants and have anti-pancreatic cancer activity.
Therefore, it is highly attractive and urgent to develop compounds that target the KRAS G12D mutation to treat KRAS G12D-related diseases.

### SUMMARY

The present invention provides a compound, or a pharmaceutical composition thereof, which can be used as an inhibitor of KRAS, especially as an inhibitor of KRAS G12D. The invention further relates to use of said compound or pharmaceutical composition thereof in the manufacture of a medicament for treating diseases by inhibiting KRAS activity, in particular cancer.
As a type of non-covalent KRAS G12D specific inhibitor, the compound herein can effectively bind to KRAS G12D-GTP and inhibit the phosphorylation of ERK downstream of KRAS G12D.
In one aspect, the present invention provides a compound having Formula (I) or a stereoisomer, a tautomer, an N-oxide, a solvate, a metabolite, a pharmaceutically acceptable salt or a prodrug thereof,

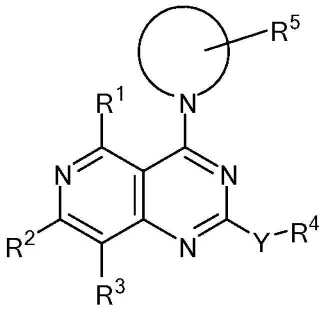

(I),

wherein,

$R^1$ is -F, -Br, -I, -CN, -SH, -C(=O)$R^{6a}$, -C(=O)O$R^{6a}$, -C(=O)N$R^6R^7$, -N$R^6$C(=O)$R^7$, -N$R^6R^7$, - N$R^6$S(=O)$_2R^7$, $C_{2-6}$ alkenyl, $C_{2-6}$ alkynyl, $C_{1-6}$ cyanoalkyl, $C_{1-6}$ hydroxyalkyl, $C_{2-6}$ haloalkenyl, $C_{2-6}$ haloalkynyl, $C_{1-6}$ alkoxy $C_{1-6}$ alkyl, $C_{1-6}$ carboxyalkyl, $C_{1-6}$ aminoalkyl, $C_{1-6}$ mercaptoalkyl or 3-6 membered heterocyclyl, wherein the $C_{2-6}$ alkenyl, $C_{2-6}$ alkynyl, $C_{1-6}$ cyanoalkyl, $C_{1-6}$ hydroxyalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl and 3-6 membered heterocyclyl are independently and optionally substituted with 1, 2, 3 or 4 substituents selected from -D, -OH, -F, -Cl, -Br, -I, -CN, -$NH_2$, -C(=O)H, -C(=O)OH, -C(=O)O$R^{6f}$, -C(=O)N$R^6R^7$, - N$R^6$C(=O)$R^7$, -Cl-6 alkyl N$R^6$C(=O)$R^7$, -N$R^{6f}$C(=O)N$R^6R^7$, -C(=O)$R^{6a}$, -N$R^6$S(=O)2$R^7$, - S(=O)$_2$N$R^6R^7$, -N$R^{6f}$S(=O)$_2$N$R^6R^7$, -N$R^{6f}R^{7f}$, -$C_{1-6}$ alkyl N$R^{6f}R^{7f}$, $C_{1-6}$ alkyl, $C_{2-6}$ alkenyl, $C_{2-6}$ alkynyl, $C_{1-6}$ alkoxy, (6-12 membered aryl)-$C_{1-6}$ alkoxy, $C_{1-6}$ cyanoalkyl, $C_{1-6}$ hydroxyalkyl, $C_{1-6}$ haloalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl, 6-12 membered aryl, 5-12 membered heteroaryl, $C_{3-6}$ cycloalkyl and 3-6 membered heterocyclyl;

$R^2$ is 6-12 membered aryl or 5-12 membered heteroaryl, wherein the 6-12 membered aryl and 5-12 membered heteroaryl are independently and optionally substituted with 1, 2, 3, 4, 5, 6 or 7 $R^8$;

each $R^8$ is independently -D, -OH, -F, -Cl, -Br, -I, -CN, -SH, -$CH_2$C(=O)N$R^{6b}R^{7b}$, -C(=O)$R^{6c}$, - C(=O)O$R^{6C}$, -C(=O)N$R^{6b}R^{7b}$, -N$R^{6b}$C(=O)$R^{7b}$, -N$R^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl and 3-6 membered heterocyclyl are independently and optionally substituted with 1, 2, 3 or 4 substituents selected from -D, -OH, -F, -Cl, -Br, -I, -CN, -$NH_2$, -C(=O)H, -C(=O)OH, $C_{1-6}$ alkyl, $C_{1-6}$ alkoxy, $C_{3-6}$ cycloalkyl or 3-6 membered heterocyclyl;

$R^3$ is -H, -D, -OH, -SH, -F, -Cl, -Br, -I, -CN or $C_{1-4}$ alkyl;

Y is a bond, O or S;

$R^4$ is -H, -D, $C_{1-6}$ alkyl, 3-10 membered heterocyclyl, -L-(3-10 membered heterocyclyl), -L-$C_{3-10}$ cycloalkyl, -L-(5-12 membered heteroaryl), -L-($C_{6-10}$ aryl), -L-N$R^6R^7$, -N$R^6R^7$, -L-NHC(=NH)$NH_2$ or -L-C(=O)N$R^6R^7$, wherein the $C_{1-6}$ alkyl, 3-10 membered heterocyclyl, -L-(3-10 membered heterocyclyl), -L-$C_{3-10}$ cycloalkyl, -L-(5-12 membered heteroaryl) and -L-($C_{6-10}$ aryl) are independently and optionally substituted with 1, 2, 3 or 4 substituents selected from -D, -OH, -F, -Cl, -Br, -I, -CN, -N$R^{6d}R^{7d}$, -C(=O)N$R^{6d}R^{7d}$, -$CH_2$N$R^{6d}R^{7d}$, -$CH_2$OC(=O)N$R^{6d}R^{7d}$, $C_{1-6}$ alkyl, $C_{1-6}$ alkoxy, $C_{1-6}$ haloalkyl, $C_{1-6}$ haloalkoxy, $C_{6-10}$ aryl $C_{1-6}$ alkyl, (5-12 membered heteroaryl)-$C_{1-6}$ alkyl, (3-6 membered heterocyclyl)-$C_{1-6}$ alkyl, $C_{3-6}$ cycloalkyl $C_{1-6}$ alkyl, $C_{3-6}$ cycloalkyl and 3-6 membered heterocyclyl, among the substituents, the $C_{1-6}$ alkyl, $C_{1-6}$ alkoxy, $C_{1-6}$ haloalkyl, $C_{1-6}$ haloalkoxy, $C_{6-10}$ aryl $C_{1-6}$ alkyl, (5-12 membered heteroaryl)-$C_{1-6}$ alkyl, (3-6 membered heterocyclyl)-$C_{1-6}$ alkyl, $C_{3-6}$ cycloalkyl $C_{1-6}$ alkyl, $C_{3-6}$ cycloalkyl and 3-6 membered heterocyclyl are independently and optionally substituted with 1, 2, 3 or 4 substituents selected from -D, -F, -Cl, -Br, -I, -OH, -CN, N$R^{6e}R^{7e}$, -C(-O)$C_{1-6}$ alkyl and $C_{1-6}$ alkyl;

L is $C_{1-6}$ alkylene;

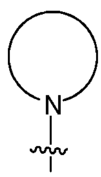

is a 5-10 membered heterocycle containing at least two ring N atoms;

$R^5$ is -H, -D, -OH, -SH, -F, -Cl, -Br, -I, -CN, -C(=O)H, -C(=O)O$R^{6a}$, -C(=O)N$R^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ haloalkoxy or 5-6 membered heteroaryl;

each $R^{6a}$ and $R^{6c}$ is independently -H, -D or $C_{1-6}$ alkyl;

each $R^6$, $R^7$, $R^{6b}$, $R^{7b}$, $R^{6d}$, $R^{7d}$, $R^{6e}$, $R^{7e}$, $R^{6f}$ and $R^{7f}$ is independently -H, -D or $C_{1-6}$ alkyl, wherein the $C_{1-6}$ alkyl is optionally substituted with 1, 2, 3 or 4 substituents selected from -D, -OH, -F, -Cl, - Br, -I, -CN, -C(=O)H, -C(=O)OH, -N$R^{6g}R^{7g}$, $C_{1-6}$ alkoxy, 6-12 membered aryl, $C_{3-6}$ cycloalkyl and 3-6 membered heterocyclyl;

or $R^6$ and $R^7$, or $R^{6b}$ and $R^{7b}$, or $R^{6d}$ and $R^{7d}$, or $R^{6e}$ and $R^{7e}$, or $R^{6f}$ and $R^{7f}$, together with the N atoms to which they are attached, form 4-6 membered heterocycle, respectively, wherein the 4-6 membered heterocycle is optionally substituted with 1, 2, 3 or 4 substituents selected from -D, -OH, -F, -Cl, -Br, -I, -CN, -$NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl;

each $R^{6g}$ and $R^{7g}$ is independently -H, -D or $C_{1-6}$ alkyl.

In some embodiments, $R^1$ is -F, -Br, -I, -CN, -SH, -C(=O)$R^{6a}$, -C(=O)O$R^{6a}$, - C(=O)N$R^6R^7$, -N$R^6$C(=O)$R^7$, -N$R^6R^7$, -N$R^6$S(=O)2$R^7$, $C_{2-4}$ alkenyl, $C_{2-4}$ alkynyl, $C_{1-4}$ cyanoalkyl, $C_{1-4}$ hydroxyalkyl, $C_{2-4}$ haloalkenyl , $C_{2-4}$ haloalkynyl, $C_{1-4}$ alkoxy $C_{1-4}$ alkyl, $C_{1-4}$ carboxyalkyl, $C_{1-4}$ aminoalkyl, $C_{1-4}$ mercaptoalkyl or 3-6 membered heterocyclyl, wherein the $C_{2-4}$ alkenyl, $C_{2-4}$ alkynyl, $C_{1-4}$ cyanoalkyl, $C_{1-4}$ hydroxyalkyl, $C_{1-4}$ alkoxy $C_{1-4}$ alkyl, $C_{1-4}$ carboxyalkyl, $C_{1-4}$ aminoalkyl, $C_{1-4}$ mercaptoalkyl and 3-6 membered heterocyclyl are independently and optionally substituted with 1, 2, 3 or 4 substituents selected from -D, -OH, -F, -Cl, -Br, -I, -CN, -$NH_2$, -C(=O)H, -C(=O)OH, - C(=O)O$R^{6f}$, -C(=O)N$R^6R^7$, -N$R^6$C(=O)$R^7$, -$C_{1-4}$ alkyl N$R^6$C(=O)$R^7$, -N$R^{6f}$C(=O)N$R^6R^7$, -C(=O)$R^{6a}$, -N$R^6$S(=O)2$R^7$, -S(=O)$_2$N$R^6R^7$, -N$R^{6f}$S(=O)$_2$N$R^6R^7$, -N$R^{6f}R^{7f}$, -$C_{1-4}$ alkyl N$R^{6f}R^{7f}$, $C_{1-4}$ alkyl, $C_{2-4}$ alkenyl, $C_{2-4}$ alkynyl, $C_{1-4}$ alkoxy, $C_{1-4}$ cyanoalkyl, $C_{1-4}$ hydroxyalkyl, $C_{1-4}$ haloalkyl, $C_{1-4}$ alkoxy $C_{1-4}$ alkyl, $C_{1-4}$ carboxyalkyl, $C_{1-4}$ aminoalkyl, $C_{1-4}$ mercaptoalkyl, 6-10 membered aryl, 5-10 membered heteroaryl, $C_{3-6}$ cycloalkyl and 3-6 membered heterocyclyl;

each $R^{6a}$ is independently -H, -D, or $C_{1-4}$ alkyl;

each $R^6$, $R^7$, $R^{6f}$ and $R^{7f}$ is independently -H, -D or $C_{1-4}$ alkyl, wherein the $C_{1-4}$ alkyl is optionally substituted with 1, 2, 3 or 4 substituents selected from -D, -OH, -F, -Cl, -Br, -I, -CN, -C(=O)H, - C(=O)OH, -N$R^{6g}R^{7g}$, $C_{1-4}$ alkoxy, 6-10 membered aryl, $C_{3-6}$ cycloalkyl and 3-6 membered heterocyclyl;

or $R^6$ and $R^7$, or $R^{6f}$ and $R^{7f}$, together with the N atom to which they are attached respectively, form 4-6 membered heterocycle, wherein the 4-6 membered heterocycle is optionally substituted with 1, 2, 3 or 4 substituents selected from -D, -OH, -F, -Cl, -Br, -I, -CN, -$NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl;

each $R^{6g}$ and $R^{7g}$ is independently -H, -D or $C_{1-4}$ alkyl.

In some embodiments, $R^1$ is -F, -Br, -I, -CN, -SH, -C(=O)$R^{6a}$, -C(=O)O$R^{6a}$, - C(=O)N$R^6R^7$, -N$R^6$C(=O)$R^7$, -N$R^6R^7$, -N$R^6$S(=O)2$R^7$, -CH=$CH_2$, -CH=CH$CH_3$, -$CH_2$CH=$CH_2$, - CHFCH=$CH_2$, -CH=CHF, -CH=CHCl, -CH=CH$CH_2$F, -CH=CH$CH_3$, -C=CH, -C≡C$CH_3$, - $CH_2$C≡CH, -C≡C$CH_2$F, -$CH_2$CN, -$(CH_2)_2$CN, -$(CH_2)_3$CN, -$CH_2$OH, -$(CH_2)_2$OH, -$(CH_2)_3$OH, - CH(OH)$CH_3$, -$CH_2$O$CH_3$, -$(CH_2)_2$O$CH_3$, -$(CH_2)_2$O$CH_2CH_3$, -$CH_2$O$CH_2CH_3$, -$CH_2$C(=O)OH, -$(CH_2)_2$C(=O)OH, -$(CH_2)_3$C(=O)OH, -$CH_2NH_2$, -$(CH_2)_2NH_2$, -$CH_2$SH, -$(CH_2)_2$SH, oxiranyl, oxetanyl or pyrrolidinyl; wherein the -CH=$CH_2$, -CH=CH$CH_3$, -$CH_2$CH=$CH_2$, -CH=CH$CH_3$, -C≡CH, - C≡C$CH_3$, -$CH_2$C≡CH, -$CH_2$CN, -$(CH_2)_2$CN, -$(CH_2)_3$CN, -$CH_2$OH, -$(CH_2)_2$OH, -$(CH_2)_3$OH, - CH(OH)$CH_3$, -$CH_2$O$CH_3$, -$(CH_2)_2$O$CH_3$, -$(CH_2)_2$O$CH_2CH_3$, -$CH_2$O$CH_2CH_3$, -$CH_2$C(=O)OH, - $(CH_2)_2$C(=O)OH, -$(CH_2)_3$C(=O)OH, -$CH_2NH_2$, -$(CH_2)_2NH_2$, -$CH_2$SH, -$(CH_2)$SH, oxiranyl, oxetanyl and pyrrolidinyl are independently and optionally substituted with 1, 2, 3 or 4 substituents selected from -D, -OH, -F, -Cl, -Br, -I, -CN, -$NH_2$, -C(=O)H, -C(=O)O$R^{6f}$, -C(=O)N$R^6R^7$, -N$R^6$C(=O)$R^7$, - $CH_2$N$R^6$C(=O)$R^7$, -N$R^{6f}$C(=O)N$R^6R^7$, -C(=O)$R^{6a}$, -N$R^6$S(=O)2$R^7$, -S(=O)$_2$N$R^6R^7$, - N$R^{6f}$S(=O)$_2$N$R^6R^7$, -N$R^{6f}R^{7f}$, -$CH_2$N$R^{6f}R^{7f}$, -CH=$CH_2$, -CH=CH$CH_3$, -$CH_2$CH=$CH_2$, -C=CH, - C≡C$CH_3$, -$CH_2$C≡CH, -C(=O)OH, methyl, ethyl, n-propyl, isopropyl, methoxy, ethoxy, n-propoxy, isopropoxy, isobutoxy, phenylmethoxy, phenylethoxy, phenylpropoxy, naphthylmethoxy, -$CH_2$CN, -$(CH_2)_2$CN, -$(CH_2)_3$CN, -$CH_2$OH, -$(CH_2)_2$OH, -$(CH_2)_3$OH, -CH(OH)$CH_3$, -$CF_3$, -$CHF_2$, -$CH_2$F, - $(CH_2)_2$F, -$(CH_2)_2$Cl, -$CH_2CF_3$, -$CH_2$O$CH_3$, -$(CH_2)_2$O$CH_3$, -$(CH_2)_2$O$CH_2CH_3$, -$CH_2$O$CH_2CH_3$, - $CH_2$C(=O)OH, -$(CH_2)_2$C(=O)OH, -$(CH_2)_3$C(=O)OH, -$CH_2NH_2$, -$(CH_2)_2NH_2$, -$CH_2$SH, -$(CH_2)_2$SH, phenyl, naphthyl, pyridyl, pyrimidinyl, cyclopropyl, cyclobutyl, cyclopentyl, cyclohexyl, oxiranyl, oxetanyl, tetrahydropyranyl, azetidinyl and pyrrolidinyl;

each $R^{6a}$ is independently -H, -D, methyl, ethyl, n-propyl, isopropyl, n-butyl, isobutyl or *tert*-butyl;

each $R^6$, $R^7$, $R^{6f}$ and $R^{7f}$ is independently -H, -D, methyl, ethyl, n-propyl, isopropyl, n-butyl, isobutyl or *tert-butyl,* wherein the methyl, ethyl, n-propyl, isopropyl, n-butyl, isobutyl and tert-butyl are independently and optionally substituted with 1, 2, 3 or 4 substituents selected from -D, -OH, -F, -Cl, - Br, -I, -CN, -C(=O)H, -C(=O)OH, -N$R^{6g}R^{7g}$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl;

or $R^6$ and $R^7$, or $R^{6f}$ and $R^{7f}$, together with the N atom to which they are attached respectively, form pyrrolidine, piperazine, piperidine, morpholinyl, oxazolidine or imidazolidine, wherein the pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, - $NH_2$, methyl, ethyl, n-propyl, isopropyl, n-butyl, isobutyl, tert-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl;

each $R^{6g}$ and $R^{7g}$ is independently -H, -D, methyl, ethyl, n-propyl, isopropyl, n-butyl, isobutyl or tert-butyl.

In some embodiments, $R^2$ is one of the following substructures:

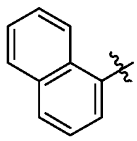, 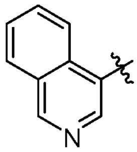, 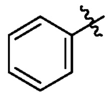, 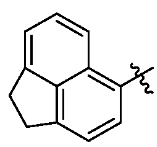, 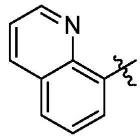, 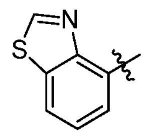, 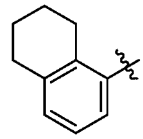,

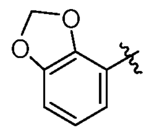, 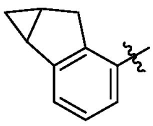, 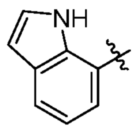, 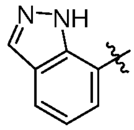, 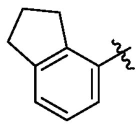, 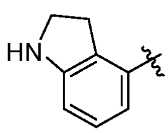, 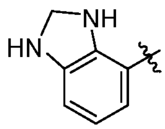,

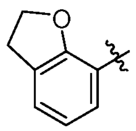, 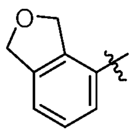, 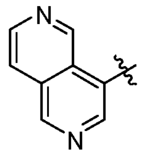, 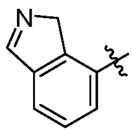, 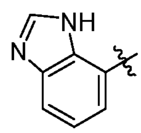, 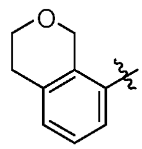, 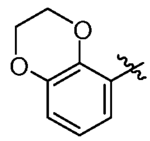 or

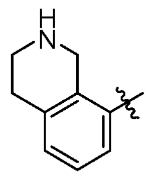,

wherein, each substructure of $R^2$ is independently and optionally substituted with 1, 2, 3, 4, 5, 6 or 7 $R^8$; wherein $R^8$ has the definition as described in the present invention.

In some embodiments, each $R^8$ is independently -D, -OH, -F, -Cl, -Br, -I, -CN, -SH, - $CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl or 3-6 membered heterocyclyl, wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl or 3-6 membered heterocyclyl;

each $R^{6c}$ is independently -H, -D or $C_{1-4}$ alkyl;

each $R^{6b}$ and $R^{7b}$ is independently -H, -D or $C_{1-4}$ alkyl, wherein the $C_{1-4}$ alkyl is independently substituted with 1, 2, 3 or 4 substituents selected from -D, -OH, -F, -Cl, -Br, -I, -CN, -C(=O)H, - C(=O)OH, $-NR^{6g}R^{7g}$, $C_{1-4}$ alkoxy, 6-10 membered aryl, $C_{3-6}$ cycloalkyl and 3-6 membered heterocyclyl; wherein the $R^{6g}$ and $R^{7g}$ have the definitions as described in the present invention;

or, $R^{6b}$ and $R^{7b}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl,

$C_{1-4}$ hydroxyalkyl, $C_{1-4}$ haloalkoxy or $C_{1-4}$ haloalkyl.

In some embodiments, each $R^8$ is independently -D, -OH, -F, -Cl, -Br, -I, -CN, -SH, - $CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, - $CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, - $CH=CHCH_3$, $-CH_2CH=CH_2$, -C=CH, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, - $CH(OH)CH_3$, $-(CH_2)_2F$, $-CH_2CHF_2$, $-CF_3$, $-CH_2CF_3$, $-CHF_2$, $-CH_2F$, -(CHz)zCl, -CH=CHF, - CH=CHC1, $-CH=CHCH_2F$, $-C=CCH_2F$, $-C\equiv C(CH_2)_2F$, -C=CF, $-OCF_3$, $-OCHF_2$, $-OCH_2CHF_2$, - $OCH_2CF_3$, $-OCHClCHCl_2$, $-OCH_2CH_2F$, $-SCF_3$, $-SCH_2CF_3$, $-SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the $-CH_3$, $-CH_2CH_3$, $-(CH_2)_2CH_3$, $-(CH_2)_3CH_3$, - $C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, - $C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, - $CH_2CF_3$, $-CHF_2$, $-CH_2F$, $-(CH_2)_2Cl$, -CH=CHF, -CH=CHCl, $-CH=CHCH_2F$, $-C=CCH_2F$, - $C\equiv C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, - $SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuryl, piperidinyl and piperazinyl are independently and optionally substituted with 1, 2, 3 or 4 substituents selected from -D, -OH, -F, -Cl, -Br, -I, -CN, $-NH_2$, -C(=O)H, -C(=O)OH, methyl, ethyl, n-propyl, isopropyl, methoxy, ethoxy, n-propoxy, isopropoxy, cyclopropyl, cyclobutyl, cyclopentyl, cyclohexyl, pyrrolidinyl, oxazolidinyl, tetrahydrofuranyl, piperidinyl and piperazinyl;

each $R^{6c}$ is independently -H, -D, methyl, ethyl, n-propyl, isopropyl, n-butyl, isobutyl or *tert*-butyl;
each $R^{6b}$ and $R^{7b}$ is independently -H, -D, methyl, ethyl, n-propyl, isopropyl, n-butyl, isobutyl or tert-butyl, wherein the methyl, ethyl, n-propyl, isopropyl, n-butyl, isobutyl and tert-butyl are independently and optionally substituted with 1, 2, 3 or 4 substituents selected from -D, -OH, -F, -Cl, -Br, -I, -CN, -C(=O)H, -C(=O)OH, $-NR^{6g}R^{7g}$, methoxy, ethoxy, n-propoxy, isopropoxy, isobutoxy, cyclopropyl, cyclobutyl, cyclopentyl, cyclohexyl, oxiranyl, oxetanyl, azetidinyl and pyrrolidinyl; wherein the $R^{6g}$ and $R^{7g}$ have the definitions as described in the present invention;
or, $R^{6b}$ and $R^{7b}$, together with the N atom to which they are attached respectively, form pyrrolidine, piperazine, piperidine, morpholinyl, oxazolidine or imidazolidine, wherein the pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, - $NH_2$, methyl, ethyl, n-propyl, isopropyl, n-butyl, isobutyl, tert-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

In some embodiments, $R^4$ is -H, -D, $C_{1-4}$ alkyl, 3-6 membered heterocyclyl, 7-10 membered heterocyclyl,

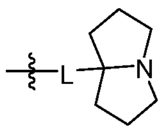,
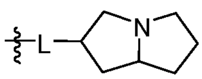,
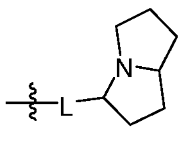,
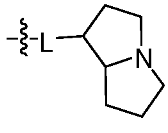,

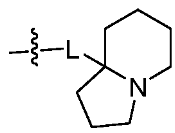,

-L-pyrrolidinyl, -L-morpholinyl, -L-oxetanyl, -L-oxypropyl, -L-tetrahydrofuranyl, -L-octahydroindolizinyl, -L -cyclopropyl, -L-cyclopentyl, -L-octahydropentadienyl, -L-octahydro-1H-indenyl, -L-decalinyl, -L-pyridyl, - L-pyrazolyl, -L-phenyl, -L-$NR^6R^7$, $-NR^6R^7$, $-L-NHC(=NH)NH_2$ or $-L-C(=O)NR^6R^7$, wherein the $C_{1-4}$ alkyl, 3-6 membered heterocyclyl, 7-10 membered heterocyclyl,

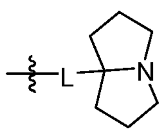,
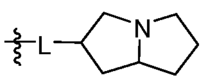,
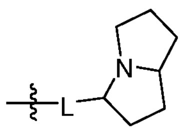,
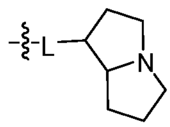,
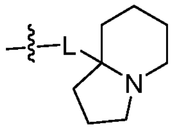,

-L-pyrrolidinyl, -L-morpholinyl, -L-oxetanyl, -L-oxypropyl, -L-tetrahydrofuranyl, -L-octahydroindolizinyl, -L-cyclopropyl, -L-cyclopentyl, -L-octahydropentadienyl, -L-octahydro-1*H*-indenyl, -L-decalinyl, -L-pyridyl, -L-pyrazolyl and -L-phenyl are independently and optionally substituted with 1, 2, 3 or 4 substituents selected from -D, -OH, -F, -Cl, -Br, -I, -CN, $-NR^{6d}R^{7d}$, $-C(=O)NR^{6d}R^{7d}$, $-CH_2NR^{6d}R^{7d}$, - $CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl $C_{1-4}$ alkyl, (5-6 membered heteroaryl)-$C_{1-4}$ alkyl, (3-6 membered heterocyclyl)-$C_{1-4}$ alkyl, $C_{3-6}$ cycloalkyl $C_{1-4}$ alkyl, $C_{3-6}$ cycloalkyl and 3-6 membered heterocyclyl, among the substituents, the $C_{1-4}$ alkyl, $C_{1-4}$ alkoxy, $C_{1-4}$ haloalkyl, $C_{1-4}$ haloalkoxy, phenyl $C_{1-4}$ alkyl, (5-6 membered heteroaryl)-$C_{1-4}$ alkyl, (3-6 membered heterocyclyl)-$C_{1-4}$ alkyl, $C_{3-6}$ cycloalkyl $C_{1-4}$ alkyl, $C_{3-6}$ cycloalkyl and 3-6 membered heterocyclyl are independently and optionally substituted with 1, 2, 3 or 4 substituents selected from -D, -F, -Cl, -Br, -I, -OH, -CN, $NR^{6e}R^{7e}$, $-C(=O)C_{1-4}$ alkyl and $C_{1-4}$ alkyl;

L is $C_{1-4}$ alkylene;
each $R^{6d}$, $R^{7d}$, $R^{6e}$ and $R^{7e}$ is independently -H, -D or $C_{1-4}$ alkyl, wherein the $C_{1-4}$ alkyl is optionally substituted with 1, 2, 3 or 4 substituents selected from -D, -OH, -F, -Cl, -Br, -I, -CN, - C(=O)H, -C(=O)OH, $-NR^{6g}R^{7g}$, $C_{1-4}$ alkoxy, 6-10 membered aryl, $C_{3-6}$ cycloalkyl and 3-6 membered heterocyclyl; wherein the $R^{6g}$ and $R^{7g}$ have the definitions as described in the present invention;
or $R^{6d}$ and $R^{7d}$, or $R^{6e}$ and $R^{7e}$, together with the N atom to which they are attached respectively, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl;
wherein $R^6$ and $R^7$ are as defined herein.

In some embodiments, $R^4$ is -H, -D, $-CH_3$, $-CH_2CH_3$, $-(CH_2)_2CH_3$, piperidinyl, piperazinyl, pyrrolidinyl, imidazolidinyl,

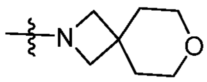 , 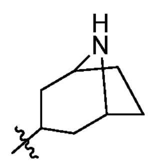 , 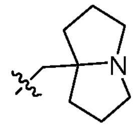 ,

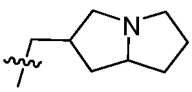 , 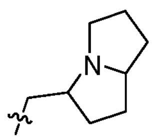 , 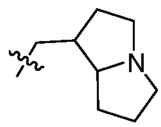 , 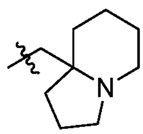 ,

$-CH_2$-pyrrolidinyl, $-CH_2$-morpholinyl, - $(CH_2)_2$-morpholinyl, $-CH_2$-oxetanyl, $-CH_2$-oxypropyl, $-CH_2$-tetrahydrofuranyl, $-CH_2$-octahydroindolizinyl, $-CH_2$-cyclopropyl, $-CH_2$-cyclopentyl, $-CH_2$-octahydropentadienyl, $-CH_2$-octahydro-1*H*-indenyl, $-CH_2$-decalinyl, $-CH_2$-pyridyl, $-(CH_2)_2$-pyridyl, $-CH_2$-pyrazolyl, $-(CH_2)_2$-pyrazolyl, $-CH_2$-phenyl, $-CH_2-NR^6R^7$, $-(CH_2)_2-NR^6R^7$, $-CH(CH_3)CH_2NR^6R^7$, $-NR^6R^7$, $-CH_2-NHC(=NH)NH_2$ or $-CH_2-C(=O)NR^6R^7$, wherein the $-CH_3$, $-CH_2CH_3$, $-(CH_2)_2CH_3$, piperidinyl, piperazinyl, pyrrolidinyl, imidazolidinyl,

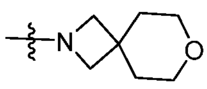 , 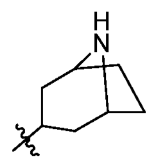 , 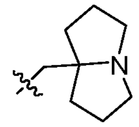 ,

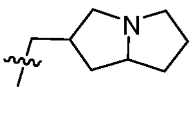 , 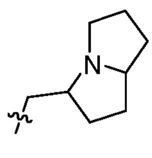 , 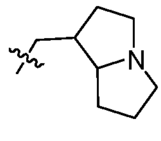 , 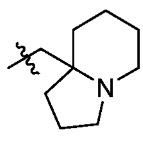 ,

$-CH_2$-pyrrolidinyl, $-CH_2$-morpholinyl, - $(CH_2)_2$-morpholinyl, $-CH_2$-oxetanyl, $-CH_2$-oxypropyl, $-CH_2$-tetrahydrofuranyl, $-CH_2$-octahydroindolizinyl, $-CH_2$-cyclopropyl, $-CH_2$-cyclopentyl, $-CH_2$-octahydropentadienyl, $-CH_2$-octahydro-1*H*-inde-

nyl, $-CH_2$-decalinyl, $-CH_2$-pyridyl, $-(CH_2)_2$-pyridyl, $-CH_2$-pyrazolyl, $-(CH_2)_2$-pyrazolyl and $-CH_2$-phenyl are independently and optionally substituted with 1, 2, 3 or 4 substituents selected from -D, -OH, -F, -Cl, -Br, -I, -CN, $-NR^{6d}R^{7d}$, $-C(=O)NR^{6d}R^{7d}$, $-CH_2NR^{6d}R^{7d}$, - $CH_2OC(=O)NR^{6d}R^{7d}$, methyl, ethyl, n-propyl, isopropyl, n-butyl, isobutyl, tert-butyl, methoxy, ethoxy, isopropoxy, $-CHF_2$, $-CF_3$, $-OCF_3$, phenylmethyl, pyridylmethyl, pyrazolylmethyl, morpholinylmethyl, pyrrolidinylmethyl, piperazinylmethyl, azetidinylmethyl, piperidylmethyl, cyclopropylmethyl, cyclopentylmethyl, cyclohexylmethyl, cyclopentyl, cyclohexyl, morpholinyl, piperidinyl, pyrrolidinyl, piperazinyl or azetidinyl, among the substituents, the methyl, ethyl, n-propyl, isopropyl, n-butyl, isobutyl, tert-butyl, methoxy, ethoxy, isopropoxy, $-CHF_2$, phenylmethyl, pyridylmethyl, pyrazolylmethyl, morpholinylmethyl, pyrrolidinylmethyl, piperazinylmethyl, azetidinylmethyl, piperidinylmethyl, cyclopropylmethyl, cyclopentylmethyl, cyclohexylmethyl, cyclopentyl, cyclohexyl, morpholinyl, piperidinyl, pyrrolidinyl, piperazinyl and azetidinyl are independently and optionally substituted with 1, 2, 3 or 4 substituents selected from -D, -F, -Cl, -Br, -I, -OH, -CN, $-NH_2$, $-NHCH_3$, $-N(CH_3)_2$, $-NHCH_2CH_3$, $-C(=O)CH_3$, $-C(=O)CH_2CH_3$, methyl, ethyl, *n*-propyl and isopropyl;

each $R^{6d}$ and $R^{7d}$ is independently -H, -D, methyl, ethyl, *n*-propyl, isopropyl, *n*-butyl, isobutyl or *tert*-butyl, wherein the methyl, ethyl, *n*-propyl, isopropyl, *n*-butyl, isobutyl and *tert*-butyl are optionally substituted with 1, 2, 3 or 4 substituents selected from -D, -OH, -F, -Cl, -Br, -I , -CN, - C(=O)H, -C(=O)OH, $-NR^{6g}R^{7g}$, methoxy, ethoxy, *n*-propoxy, isopropoxy, isobutoxy, cyclopropyl, cyclobutyl, cyclopentyl, cyclohexyl, oxiranyl, oxetanyl, azetidinyl and pyrrolidinyl; wherein the $R^{6g}$ and $R^{7g}$ are as defined herein;
or $R^{6d}$ and $R^{7d}$, together with the N atom to which they are attached, form azetidine, pyrrolidine, piperazine, piperidine, morpholinyl, oxazolidine or imidazolidine, wherein the azetidine, pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, $-NH_2$, methyl, ethyl, n-propyl, isopropyl, n-butyl, isobutyl, tert-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl; wherein $R^6$ and $R^7$ are as defined herein.

In some embodiments,

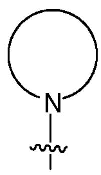

is one of the following substructures,

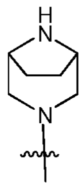 , 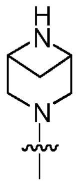 , 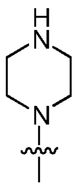 , 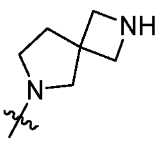 , 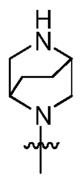 or 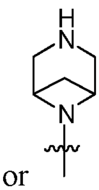 ;

$R^5$ is -H, -D, -OH, -SH, -F, -Cl, -Br, -I, -CN, -C(=O)H, $-C(=O)OCH_3$, $-C(=O)NH_2$, $-CH_3$, - $CH_2CH_3$, $C_{3-4}$ alkyl, $-CH_2OH$, $-(CH_2)_2OH$, $-CH_2CN$, $-(CH_2)_2CN$, $-CF_3$, $-CHF_2$, $-CH_2F$, $-OCF_3$, $-OCH_3$ or $-OCH_2CH_3$.

In some embodiments, $R^3$ is -H, -D, -OH, -SH, -F, -Cl, -Br, -I, -CN, methyl, ethyl, n-propyl or isopropyl.

In some embodiments, the present invention provides a compound having Formula (I-1), or a stereoisomer, a tautomer, an N-oxide, a solvate, a metabolite, a pharmaceutically acceptable salt or a prodrug thereof,

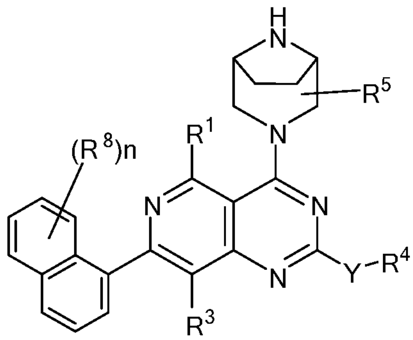

(I-1),

wherein, n is 1, 2, 3, 4, 5, 6 or 7;
wherein, $R^1$, $R^3$, $R^4$, $R^5$, Y and $R^8$ are as defined herein.

In some embodiments, the present invention provides a compound having Formula (I-2), or a stereoisomer, a tautomer, an *N*-oxide, a solvate, a metabolite, a pharmaceutically acceptable salt or a prodrug thereof,

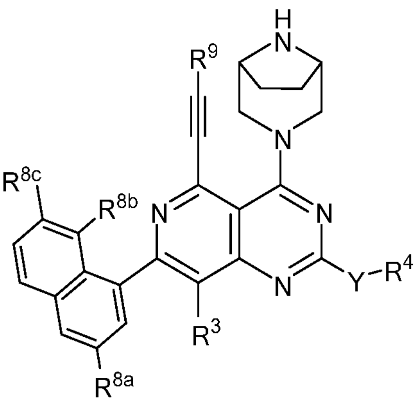

(I-2),

wherein, $R^9$ is -H, -D, CN, $-NH_2$, -C(=O)H, -C(=O)OH, $-C(=O)OR^{6f}$, $-C(=O)NR^6R^7$, - $NR^6C(=O)R^7$, $-NR^{6f}C(=O)NR^6R^7$, $-C(=O)R^{6a}$, $-C(=O)OR^{6a}$, $-NR^6S(=O)2R^7$, $-S(=O)_2NR^6R^7$, - $NR^{6f}S(=O)_2NR^6R^7$, $-NR^{6f}R^{7f}$, $-C_{1-6}$ alkyl $NR^6C(=O)R^7$, $-C_{1-6}$ alkyl $NR^{6f}R^{7f}$, $C_{1-6}$ alkyl, $C_{1-6}$ cyanoalkyl, $C_{1-6}$ hydroxyalkyl, $C_{1-6}$ haloalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl, 6-12 membered aryl, 5-12 membered heteroaryl, $C_{3-6}$ cycloalkyl and 3-6 membered heterocyclyl;
each $R^{6a}$ is independently -H, -D, or $C_{1-6}$ alkyl;
each $R^6$, $R^7$, $R^{6f}$ and $R^{7f}$ is independently -H, -D or $C_{1-6}$ alkyl, wherein the $C_{1-6}$ alkyl is optionally substituted with 1, 2, 3 or 4 substituents selected from -D, -OH, -F, -Cl, -Br, -I, -CN, -C(=O)H, - C(=O)OH, $-NR^{6g}R^{7g}$, $C_{1-6}$ alkoxy, 6-12 membered aryl, $C_{3-6}$ cycloalkyl and 3-6 membered heterocyclyl;
$R^3$, Y, $R^4$, $R^5$, $R^{6g}$ and $R^{7g}$ are as defined herein;
each $R^{8a}$, $R^{8b}$ and $R^{8c}$ has the definition as described for $R^8$ in the present invention.

In some embodiments, $R^9$ is -H, -D, CN, $-NH_2$, -C(=O)H, -C(=O)OH, $-C(=O)OR^{6f}$, - $C(=O)NR^6R^7$, $-NR^6C(=O)R^7$, $-NR^{6f}C(=O)NR^6R^7$, $-C(=O)R^{6a}$, $-C(=O)OR^{6a}$, $-NR^6S(=O)2R^7$, - $S(=O)_2NR^6R^7$, $-NR^{6f}S(=O)_2NR^6R^7$, $-NR^{6f}R^{7f}$, $-CH_2NR^6C(=O)R^7$, $-CH_2NR^{6f}R^{7f}$, $-CH_3$, $-CH_2CH_3$, - $(CH_2)_2CH_3$, $-(CH_2)_3CH_3$, $-C(CH_3)_3$, $-CH_2CH(CH_3)_2$, $-(CH_2)_2CH(CH_3)_2$, $-CH(CH_3)_2$, $-CH_2CN$, - $(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, - $CH(OH)CH_3$, $-CF_3$, $-CHF_2$, $-CH_2F$, $-(CH_2)_2F$, -(CHz)zCl, $-CH_2CF_3$, $-CH_2OCH_3$, $-(CH_2)_2OCH_3$,$-(CH_2)_2OCH_2CH_3$, $-CH_2OCH_2CH_3$, $-CH_2C(=O)OH$, $-(CH_2)_2C(=O)OH$, $-(CH_2)_3C(=O)OH$, $-CH_2NH_2$, $-(CH_2)_2NH_2$, $-CH_2SH$, $-(CH_2)_2SH$, phenyl, naphthyl, pyridyl, pyrimidinyl, cyclopropyl, cyclobutyl, cyclopentyl, cyclohexyl, oxiranyl, oxetanyl, tetrahydropyranyl, azetidinyl and pyrrolidinyl;

each $R^{6a}$ is independently -H, -D, methyl, ethyl, n-propyl, isopropyl, n-butyl, isobutyl or *tert*-butyl;
each $R^6$, $R^7$, $R^{6f}$ and $R^{7f}$ is independently -H, -D, methyl, ethyl, n-propyl, isopropyl, n-butyl, isobutyl or *tert-butyl,* wherein the methyl, ethyl, *n*-propyl, isopropyl, *n*-butyl, isobutyl and *tert*-butyl are independently and optionally substituted with 1, 2, 3 or 4 substituents selected from -D, -OH, -F, -Cl, - Br, -I, -CN, -C(=O)H, -C(=O)OH, $-NR^{6g}R^{7g}$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl;
$R^{6g}$ and $R^{7g}$ are as defined herein.

In another aspect, the invention provides a pharmaceutical composition comprising the compounds provided herein.

In some embodiments, the pharmaceutical compositions of the present invention further comprise pharmaceutically acceptable adjuvants.

In some embodiments, the adjuvants described herein include, but are not limited to, carriers, excipients, diluents, vehicles, or combinations thereof. In some embodiments, the form of the pharmaceutical composition may be liquid, solid, semi-solid, gel or spray.

In another aspect, the present invention provides use of the pharmaceutical composition disclosed herein in the manufacture of a medicament for preventing, treating or alleviating KRAS G12D related diseases.

In some embodiments, the KRAS G12D related disease is cancer.

In some embodiments, the cancer of the invention is non-small cell lung cancer, small cell lung cancer, colorectal cancer, rectal cancer, colon cancer, small intestine cancer, pancreatic cancer, uterine cancer, gastric cancer, esophageal cancer, prostate cancer, ovarian cancer, breast cancer, leukemia, melanoma, lymphoma or neuroma.

In another aspect, the present invention also provides a method for preventing or treating KRAS G12D related diseases, which includes administering to a patient a therapeutically effective amount of the compound or the pharmaceutical composition thereof disclosed herein.

In another aspect, the invention relates to methods for the preparation, isolation and purification of compounds of formula (I), (I-1) or (I-2).

Unless otherwise stated, all stereoisomers, tautomers, N-oxides, hydrates, solvates, metabolites and pharmaceutically acceptable salts and prodrugs of the compounds disclosed herein are within the scope of the invention.

The phrase "pharmaceutically acceptable" refers to that the substance or composition must be compatible chemically and/or toxicologically, with the other ingredients comprising a formulation, and/or the mammal being treated therewith.

The compounds disclosed herein also include salts of the compounds which are not necessarily pharmaceutically acceptable salts, and which may be useful as intermediates for preparing and/or purifying compounds of Formula (I), (I-1) or (I-2), and/or for separating enantiomers of compounds of Formula (I), (I-1) or (I-2).

The foregoing merely summarizes certain aspects disclosed herein and is not intended to be limiting in nature. These aspects and other aspects and embodiments are described more fully below.

## DETAILED DESCRIPTION OF THE INVENTION

### DEFINITIONS AND GENERAL TERMINOLOGY

Reference will now be made in detail to certain embodiments of the invention, examples of which are illustrated in the accompanying structures and formulas. The invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as is commonly understood by one skilled in the art to which this invention belongs. All patents and publications referred to herein are incorporated by reference in their entirety.

As used herein, the term "subject" refers to an animal. Typically the animal is a mammal. A subject also refers to for example, primates (e.g., humans, male or female), cows, sheep, goats, horses, dogs, cats, rabbits, rats, mice, fish, birds and the like. In certain embodiments, the subject is a primate. In yet other embodiments, the subject is a human.

As used herein, "patient" refers to a human (including adults and children) or other animal. In one embodiment, "patient" refers to a human.

The term "comprise" or "include" is an open expression, it means comprising the contents disclosed herein, but don't exclude other contents.

"Stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, etc. Unless otherwise indicated, all stereoisomers or mixtures of stereoisomers of the structural formulas described in the present invention belong to the scope of the present invention. Additionally, unless otherwise stated, the structural formula of compounds described in the present invention comprises an enriched isotope with one or more different atoms.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Any resulting mixtures of stereoisomers can be separated into the pure or substantially pure geometric isomers, enantiomers, diastereomers on the basis of the physicochemical differences of the constituents, for example, by chromatography and/or fractional crystallization.

The term "tautomer" or "tautomeric form" refers to structural isomers of different energies which are interconvertible via a low energy barrier. Where tautomerization is possible (e.g. in solution), a chemical equilibrium of tautomers can be reached. For example, proton tautomers (also known as prototropic tautomers) include interconversions via migration of a proton, such as keto-enol and imine-enamine isomerizations. Valence tautomers include interconversions by reorganization of some of the bonding electrons. A specific example of keto-enol tautomerization is the interconversion of pentane-2,4-dione and 4-hydroxypent-3-en-2-one tautomers. Another example of tautomerization is phenol-keto tautomerization. The specific example of phenol-keto tautomerisms is pyridin-4-ol and pyridin-4(1*H*)-one tautomerism. Unless otherwise stated, all tautomeric forms of the compounds disclosed herein are within the scope of the invention.

As described herein, compounds disclosed herein may be independently and optionally substituted with one or more substituents, such as the general formula compound above, or as exemplified by particular classes, subclasses, and species of the invention. It will be appreciated that the phrase "independently and optionally substituted with" or "optionally substituted with" is used interchangeably with the phrase "substituted or unsubstituted". In general, the term "substituted" refers to the replacement of one or more hydrogen radicals in a given structure with the radical of a specified substituent. Unless otherwise indicated, each substitutable position of the group may be substituted with an optionally substituted group. When more than one position in a given structure can be substituted with more than one substituent selected from a specified group, the substituent may be either the same or different at each position.

Furthermore, what need to be explained is that the phrase "each...is independently" and "each of...and...is independently", unless otherwise stated, should be broadly understood. The specific options expressed by the same symbol are independent of each other in different groups; or the specific options expressed by the same symbol are independent of each other in same groups.

At various places in the present specification, substituents of compounds disclosed herein are disclosed in groups or in ranges. It is specifically intended that the invention include each and every individual subcombination of the members of such groups and ranges. For example, the term "$C_{1-6}$ alkyl" is specifically intended to individually disclose methyl, ethyl, $C_3$ alkyl, $C_4$ alkyl, $C_5$ alkyl, and $C_6$ alkyl.

At various places in the present specification, linking substituents are described. Where the structure clearly requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "alkyl" or "alkyl group" refers to a saturated linear or branched-chain monovalent hydrocarbon group of 1-20 carbon atoms, wherein the alkyl group is optionally substituted with one or more substituents described herein. In one embodiment, the alkyl group contains 1-6 carbon atoms, represented by $C_{1-6}$ alkyl; in yet another embodiment, the alkyl group contains 1-4 carbon atoms, represented by $C_{1-4}$ alkyl; in yet another embodiment, the alkyl group contains 1-3 carbon atoms and is represented by $C_{1-3}$ alkyl. Some non-limiting examples of the alkyl group include, methyl (Me, $-CH_3$), ethyl (Et, $-CH_2CH_3$), n-propyl (n-Pr, $-CH_2CH_2CH_3$), isopropyl (i-Pr, $-CH(CH_3)_2$), n-butyl (n-Bu, $-CH_2CH_2CH_2CH_3$), isobutyl (i-Bu, $-CH_2CH(CH_3)_2$), sec-butyl (s-Bu, $-CH(CH_3)CH_2CH_3$), *tert*-butyl (t-Bu, $-C(CH_3)_3$), n-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-l-butyl ($-CH_2CH_2CH(CH_3)_2$), 2-methyl-l-butyl ($-CH_2CH(CH_3)CH_2CH_3$), n-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$)), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$, n-heptyl and n-octyl, etc.

The term "alkylene" refers to a saturated divalent hydrocarbon group derived from a straight or branched chain saturated hydrocarbon by the removal of two hydrogen atoms. In some embodiments, the alkylene group contains 1-6

carbon atoms, represented by $C_{1-6}$ alkylene; in other embodiments, the alkylene group contains 1-4 carbon atoms, represented by $C_{1-4}$ alkylene; in still other embodiments, the alkylene group contains 1-3 carbon atoms, represented by $C_{1-3}$ alkylene; in yet other embodiments, the alkylene group contains 1-2 carbon atoms, represented by $C_{1-2}$ alkylene. Some non-limiting examples of the alkylene group include $-CH_2-$, $-CH_2CH_2-$, $-CH(CH_3)CH_2-$, and the like.

The term "alkenyl" refers to linear or branched-chain monovalent hydrocarbon radical of 2 to 12 carbon atoms with at least one site of unsaturation, i.e., a carbon-carbon, $sp^2$ double bond, wherein the alkenyl group may be optionally substituted independently with one or more substituents described herein, and includes radicals having *"cis"* and *"trans"* orientations, or alternatively, "E" and "Z" orientations. In some embodiments, the alkenyl group contains 2 to 6 carbon atoms, represented by $C_{2-6}$ alkenyl. In other embodiments, the alkenyl group contains 2 to 4 carbon atoms, represented by $C_{2-4}$ alkenyl. Some non-limiting examples of the alkenyl group include ethenyl or vinyl ($-CH=CH_2$), allyl ($-CH_2CH=CH_2$), propenyl ($-CH=CHCH_3$), and the like.

The term "alkynyl" refers to a linear or branched monovalent hydrocarbon radical of 2 to 12 carbon atoms with at least one site of unsaturation, i.e., a carbon-carbon, sp triple bond, wherein the alkynyl radical may be optionally substituted independently with one or more substituents described herein. In some embodiments, the alkynyl group contains 2 to 6 carbon atoms, represented by $C_{2-6}$ alkynyl. In other embodiments, the alkynyl group contains 2 to 4 carbon atoms, represented by $C_{2-4}$ alkynyl. Some non-limiting examples of the alkynyl group include ethynyl ($-C\equiv CH$), propargyl ($-CH_2C\equiv CH$), 1-propynyl ($-C\equiv C-CH_3$), and the like.

The term "cyanoalkyl" refers to an alkyl group substituted with one or more cyano groups, wherein the cyano and alkyl groups have the meanings described herein. In some embodiments, "cyanoalkyl" means an alkyl group substituted with one cyano group. In some embodiments, "cyanoalkyl" is $C_{1-6}$ cyanoalkyl, i.e., $C_{1-6}$ alkyl substituted with one or more cyano groups. In some preferred embodiments, $C_{1-6}$ cyanoalkyl is $C_{1-6}$ alkyl substituted with one cyano group. In other embodiments, "cyanoalkyl" is $C_{1-4}$ cyanoalkyl, i.e., $C_{1-4}$ alkyl substituted with one or more cyano groups. Some non-limiting examples of the cyanoalkyl include $-CH_2CN$, $-CH_2CH_2CH_2CH_2CN$, $-CH_2CH_2CN$, $-CH_2CH(CN)CH_2CH_2CN$, $-CH_2CH(CN)CH_2CH(CH_3)CN$, and the like.

The term "hydroxyalkyl" refers to an alkyl group substituted with one or more hydroxyl groups, wherein the alkyl and hydroxyl groups have the meanings described herein. In some embodiments, hydroxyalkyl means an alkyl group substituted with 1, 2, 3, or 4 hydroxyl groups. In some embodiments, hydroxyalkyl means an alkyl group substituted with one or two hydroxyl groups. In some embodiments, hydroxyalkyl means $C_{1-6}$ hydroxyalkyl group, i.e., $C_{1-6}$ alkyl substituted with one or more hydroxyl groups, preferably, $C_{1-6}$ hydroxyalkyl means $C_{1-6}$ alkyl substituted with one hydroxyl group. In some embodiments, hydroxyalkyl means $C_{1-4}$ hydroxyalkyl. In some embodiments, hydroxyalkyl means $C_{1-3}$ hydroxyalkyl. Some non-limiting examples of the hydroxyalkyl include $-CH_2OH$, $-CH_2CH_2CH_2CH_2OH$, $-CH_2CH_2OH$, $-CH_2CH(OH)CH_2CH_2OH$, $-CH_2CH(OH)CH_2CH(CH_3)OH$, and the like.

The term "haloalkyl" means an alkyl group substituted with one or more halogen atoms, wherein the alkyl and halogen have the meanings described herein. In some embodiments, haloalkyl is $C_{1-6}$ haloalkyl, meaning that the $C_{1-6}$ alkyl group is substituted with one or more halogen atoms; in other embodiments, haloalkyl is $C_{1-4}$ haloalkyl, meaning that the $C_{1-4}$ alkyl group is substituted with one or more halogen atoms; in other embodiments, the haloalkyl group is $C_{1-3}$ haloalkyl, meaning that the $C_{1-3}$ alkyl group is substituted with one or more halogen atoms. Such examples include, but are not limited to, monofluoromethyl, difluoromethyl, trifluoromethyl, 1-fluoroethyl, 2-fluoroethyl, 1,2-difluoroethyl, 1,1-difluoroethyl, 2,2-difluoroethyl, monochloromethyl, dichloromethyl, trichloromethyl, 2-chloroethyl, 1-chloroethyl, 1,2-dichloroethyl, 1,1-dichloroethyl, 2,2-dichloroethyl, 1,1-dibromoethyl, etc.

The term "haloalkenyl" means that an alkenyl group is substituted with one or more halogen atoms, wherein the alkenyl group has the meaning described herein. In some embodiments, haloalkenyl is $C_{2-6}$ haloalkenyl, meaning that the $C_{2-6}$ alkenyl group is substituted with one or more halogen atoms; in other embodiments, haloalkenyl is $C_{2-4}$ haloalkenyl, meaning that the $C_{2-4}$ alkenyl group is substituted with one or more halogen atoms. Such examples include, but are not limited to, 1-chlorovinyl ($-CCl=CHz$), 2-fluorovinyl ($-CH=CHF$), 1-fluoroallyl ($-CHFCH=CH_2$), 3-fluoropropenyl (i.e., $-CH=CH-CH_2F$), 3,3-difluoropropenyl (i.e., $-CH=CH-CHF_2$).

The term "haloalkynyl" means that an alkynyl group is substituted with one or more halogen atoms, wherein the alkynyl group has the meaning described herein. In some embodiments, the haloalkynyl group is $C_{2-6}$ haloalkynyl, meaning that the $C_{2-6}$ alkynyl group is substituted with one or more halogen atoms; in other embodiments, the haloalkynyl group is $C_{2-4}$ haloalkynyl, meaning that the $C_{2-4}$ alkynyl group is substituted with one or more halogen atoms. Such examples include, but are not limited to, 2-chloroethynyl ($-C\equiv CCl$), 1-chloropropargyl ($-CHClC\equiv CH$), 3-chloropropynyl ($-C\equiv C-CH_2Cl$), etc.

The term "hydroxyalkynyl" means that an alkynyl group is substituted with one or more hydroxyl groups, wherein the hydroxyl and alkynyl groups have the meanings described herein. In some embodiments, the hydroxyalkynyl group is a $C_{2-6}$ hydroxyalkynyl group, meaning that the $C_{2-6}$ alkynyl group is substituted with one or more hydroxyl groups; in other embodiments, the hydroxyalkynyl group is a $C_{2-4}$ hydroxyalkynyl group, meaning that the $C_{2-4}$ alkynyl group is substituted with one or more hydroxyl groups. Such examples include, but are not limited to, 3-hydroxypropynyl ($-C\equiv C-CH_2OH$), 4-hydroxybutynyl ($-C\equiv C-(CH_2)_2OH$), etc.

The term "alkoxyalkyl" means an alkyl group substituted with an alkoxy group, wherein the alkoxy and alkyl groups have the meanings described herein. In some embodiments, alkoxyalkyl means $C_{1-6}$ alkoxy $C_{1-6}$ alkyl; in other embodiments, alkoxyalkyl means $C_{1-4}$ alkoxy $C_{1-4}$ alkyl; in other embodiments, alkoxyalkyl means $C_{1-4}$ alkoxy $C_{1-3}$ alkyl; in some embodiments, alkoxyalkyl means $C_{1-3}$ alkoxy $C_{1-3}$ alkyl. Examples of alkoxy groups include, but are not limited to, methoxymethyl, ethoxymethyl, n-propoxymethyl, isopropoxymethyl, methoxyethyl, methoxy n-propyl, methoxyisopropyl, ethoxyethyl, ethoxy n-propyl, ethoxy isopropyl, n-propoxyethyl, isopropoxyethyl, n-propoxyn-propyl, n-propoxyisopropyl, isopropoxyn-propyl, isopropoxyisopropyl, etc.

The term "carboxyalkyl" means an alkyl group substituted with one or more carboxyl groups, wherein the carboxyl and alkyl groups have the meanings described herein. In some embodiments, carboxyalkyl is $C_{1-6}$ carboxyalkyl, meaning that $C_{1-6}$ alkyl is substituted with one or more carboxy groups; in other embodiments, carboxyalkyl is $C_{1-4}$ carboxyalkyl, meaning that $C_{1-4}$ alkyl is substituted with one or more carboxyl groups; in other embodiments, carboxyalkyl is $C_{1-3}$ carboxyalkyl, meaning that $C_{1-3}$ alkyl is substituted with one or more carboxyl groups. Examples of carboxyalkyl groups include, but are not limited to, carboxymethyl ($-CH_2COOH$), 2-carboxyethyl ($-(CH_2)_2COOH$), 3-carboxypropyl ($-(CH_2)_3COOH$), and the like.

The term "aminoalkyl" means an alkyl group substituted with one or more amino groups, wherein the alkyl and amino groups have the meanings described herein. In some embodiments, aminoalkyl is $C_{1-6}$ aminoalkyl, meaning that $C_{1-6}$ alkyl is substituted with one or more amino groups; in other embodiments, aminoalkyl is $C_{1-4}$ aminoalkyl, meaning that $C_{1-4}$ alkyl is substituted with one or more amino groups; in other embodiments, aminoalkyl is $C_{1-3}$ aminoalkyl, meaning that $C_{1-3}$ alkyl is substituted with one or more amino groups. Examples of aminoalkyl group include, but are not limited to, aminomethyl ($-CH_2NH_2$), 2-aminoethyl ($-(CH_2)_2NH_2$), 1-aminoethyl ($-CH(NH_2)CH_3$), 1,2-diaminoethyl ($-CH(NH_2)CH_2NH_2$), 3-aminopropyl ($-(CH_2)_3NH_2$).

The term "alkylamino" or "alkylamino group" means an amino group substituted with one or two alkyl groups, including "N-alkylamino" and "*N,N*-dialkylamino", wherein the alkyl and amino groups have the meaning as described herein. In some embodiments, alkylamino means $C_{1-6}$ alkylamino, which is an alkylamino group containing 1-6 carbon atoms; in other embodiments, alkylamino means $C_{1-4}$ alkylamino, which is an alkylamino group containing 1-4 carbon atoms; alkylamino means $C_{1-3}$ alkylamino, which is an alkylamino group containing 1-3 carbon atoms. Suitable alkylamino groups may be monoalkylamino or dialkylamino, examples of which include, but are not limited to, N-methylamino ($-NHCH_3$), N-ethylamino ($-NHCH_2CH_3$), *N,N*-dimethylamino($-N(CH_3)_2$), *N,N*-diethylamino($-N(CH_2CH_3)_2$), etc.

The term "mercaptoalkyl" means an alkyl group substituted with one or more mercapto groups, wherein the alkyl group has the meaning as described herein. In some embodiments, mercaptoalkyl means $C_{1-6}$ mercaptoalkyl, which is a $C_{1-6}$ alkyl group substituted with one or more mercapto groups; preferably, $C_{1-6}$ mercaptoalkyl is a $C_{1-6}$ alkyl group substituted with one mercapto group. In other embodiments, mercaptoalkyl means $C_{1-4}$ mercaptoalkyl. In other embodiments, mercaptoalkyl means $C_{1-3}$ mercaptoalkyl. Examples of mercaptoalkyl groups include, but are not limited to, mercaptomethyl ($-CH_2SH$), 2-mercaptoethyl ($-(CH_2)_2SH$), 3-mercaptopropyl ($-(CH_2)_3SH$), 2,3-dimercaptopropyl ($-CH_2CH(SH)CH_2(SH)$), etc.

The term "alkoxy" refers to an alkyl group, as previously defined, attached to the parent molecular moiety via an oxygen atom. Unless otherwise specified, the alkoxy group contains 1-12 carbon atoms. In one embodiment, the alkoxy group contains 1-6 carbon atoms, means $C_{1-6}$ alkoxy. In other embodiment, the alkoxy group contains 1-4 carbon atoms, means $C_{1-4}$ alkoxy. In still other embodiment, the alkoxy group contains 1-3 carbon atoms, means $C_{1-3}$ alkoxy. The alkoxy group may be optionally substituted with one or more substituents disclosed herein. Some non-limiting examples of the alkoxy group include, but are not limited to, methoxy (MeO, $-OCH_3$), ethoxy (EtO, $-OCH_2CH_3$), 1-propoxy (n-PrO, n-propoxy, $-OCH_2CH_2CH_3$), 2-propoxy (i-PrO, i-propoxy, $-OCH(CH_3)_2$), 1-butoxy (n-BuO, n-butoxy, $-OCH_2CH_2CH_2CH_3$), 2-methyl-l-propoxy (i-BuO, i-butoxy, - $OCH_2CH(CH_3)_2$), 2-butoxy (s-BuO, s-butoxy, $-OCH(CH_3)CH_2CH_3$), 2-methyl-2-propoxy (t-BuO, *t*-butoxy, $-OC(CH_3)_3$), 1-pentoxy (n-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-l-butoxy ($-OCH_2CH_2CH(CH_3)_2$), 2-methyl-l-butoxy ($-OCH_2CH(CH_3)CH_2CH_3$), and the like.

The term "haloalkoxy" means an alkoxy group substituted with one or more halogens, wherein the alkoxy and halogen have the meanings as described herein. In some embodiments, haloalkoxy means a haloalkoxy group containing 1-6 carbon atoms, i.e., $C_{1-6}$ haloalkoxy; in other embodiments, haloalkoxy means a haloalkoxy group containing 1-4 carbon atoms, i.e., $C_{1-4}$ haloalkoxy; in other embodiments, haloalkoxy means a haloalkoxy group containing 1-3 carbon atoms, i.e., $C_{1-3}$ haloalkoxy. Examples of haloalkoxy include, but are not limited to, trifluoromethoxy ($-OCF_3$), monofluoromethoxy ($-OCH_2F$), 2-fluoroethoxy ($-OCH_2CH_2F$), and the like.

The term "cycloalkyl" refers to a monovalent saturated monocyclic or bicyclic carbocyclic ring system of 3-12 carbon atoms, in which the $-CH_2-$ group may optionally be substituted with -C(=O)- (or -(CO)-). In some embodiments, the cycloalkyl group contains 3-10 carbon atoms, i.e., $C_{3-10}$ cycloalkyl. In other embodiments, the cycloalkyl group contains 3-8 carbon atoms, i.e., $C_{3-}a$ cycloalkyl. In still other embodiments, the cycloalkyl group contains 3-6 carbon atoms, i.e., $C_{3-6}$ cycloalkyl. Examples of cycloalkyl groups include, but are not limited to, cyclopropyl, cyclobutyl, cyclopentyl, cyclohexyl, octahydro-1*H*-indenyl, octahydropentacyclopentadienyl, and the like. Examples of the $-CH_2-$ group in the carbocyclic ring that can be substituted by -C(=O)- include but are not limited to: cyclopentanone, cyclobutanone, etc.

The terms "heterocyclyl" or "heterocycle" refer to a saturated or partially saturated monocyclic, bicyclic or tricyclic ring system of 3-12 ring members, in which at least one ring member is selected from nitrogen, sulfur and oxygen atom; wherein, the heterocycle or heterocyclyl is nonaromatic and does not contain any aromatic ring. When a heterocycle is connected to the rest of the molecule through a linking site, it means a monovalent heterocyclyl group. Unless otherwise specified, the heterocyclyl group may be carbon or nitrogen linked, and a $-CH_2-$ group can be optionally replaced by a -C(=O)- group. In which, the sulfur can be optionally oxygenized to S-oxide and the nitrogen can be optionally oxygenized to *N*-oxide. In some embodiments, the heterocycle or heterocyclyl composed of 3-10 atoms, means 3-10 membered heterocycle or 3-10 membered heterocyclyl; in other embodiments, the heterocycle or heterocyclyl composed of 3-9 atoms, means 3-9 membered heterocycle or 3-9 membered heterocyclyl; in other embodiments, the heterocycle or heterocyclyl composed of 5-9 atoms, means 5-9 membered heterocycle or 5-9 membered heterocyclyl; in other embodiments, the heterocycle or heterocyclyl composed of 3-6 atoms, means 3-6 membered heterocycle or 3-6 membered heterocyclyl; in other embodiments, the heterocycle or heterocyclyl composed of 5-6 atoms, means 5-6 membered heterocycle or 5-6 membered heterocyclyl. Examples of the heterocycle include, but are not limited to, oxirane, aziridine, azetidine, oxetane, pyrrolidine, tetrahydrofuran, tetrahydrothiophene, thiazolidine, pyrazolidine, pyrazoline, oxazolidine, imidazolidine, piperidine, piperazine, morpholine, 3,8-diazabicyclo[3.2.1]octane, 3,6-diazabicyclo[3.1.1]heptane, 2,5-diazabicyclo[2.2.2]octane. The heterocyclyl group includes, but is not limited to, oxiranyl, aziridinyl, azetidinyl, oxetanyl, pyrrolidinyl, tetrahydrofuranyl, tetrahydrothienyl, thiazolidinyl, pyrazolidinyl, pyrazolinyl, oxazolidinyl, imidazolidinyl, piperidinyl, piperazinyl or morpholinyl, etc.

The term "aryl" refers to monovalent monocyclic, bicyclic and tricyclic carbocyclic ring systems having a total of 6-14 ring members, or 6-12 ring members, or 6-10 ring members, wherein at least one ring in the system is aromatic, wherein each ring in the system contains 3-7 ring members. In some embodiments, the aryl group contains 6-12 ring members, represented as $C_{6-12}$ aryl or 6-12 membered aryl. In some embodiments, the aryl group contains 6-10 ring members, represented as $C_{6-10}$ aryl or 6-10 membered aryl. Examples of the aryl group include phenyl, naphthyl, 1,2,3,4-tetrahydronaphthyl, and anthracene.

The term "heteroaryl" or "heteroaromatic ring" refers to a monovalent monocyclic, bicyclic, or tricyclic carbocyclic ring system having a total of 5-14 ring members, or 5-12 ring members, or 5-10 ring members, or 5-6 ring members, and wherein at least one ring in the system is aromatic, and at least one ring contains one or more ring heteroatoms selected from nitrogen, oxygen, and sulfur. The heteroaryl group is generally, but not necessarily bonded to the parent molecule through an aromatic ring of the heteroaryl group. When a $-CH_2-$ group is present in a heteroaryl group, the $-CH_2-$ group may optionally be substituted with -C(=O)-. Unless otherwise stated, the heteroaryl group can be connected to the rest of the molecule (such as the main structure in the general formula) through any reasonable positions (such as C or N). The term "heteroaryl" and "heteroaromatic ring" or "heteroaromatic compound" can be used interchangeably herein. In some embodiments, the heteroaryl group is a heteroaryl group containing 5-12 ring atoms, represented by 5-12 membered heteroaryl; in other embodiments, the heteroaryl group is a heteroaryl group containing 5-10 ring atoms, represented by 5-10 membered heteroaryl; in other embodiments, the heteroaryl group is a heteroaryl group containing 5-6 ring atoms, represented by 5-6 membered heteroaryl. Some non-limiting examples of such group include furyl, imidazolyl, isoxazolyl, oxazolyl, pyrrolyl, pyrazolyl, pyridyl, pyrimidinyl, pyridazinyl, pyrazinyl, thienyl, thiazolyl, triazolyl, tetrazolyl, benzopyridyl, benzimidazolyl, benzopyrrolyl, benzopyrazolyl, benzopyrrolidinyl, etc.

The term "alkylthio" refers to an alkyl group, as previously defined, attached to the parent molecular moiety via a sulfur atom. In some embodiments, the alkylthio is $C_{1-6}$ alkylthio, means an alkylthio group containing 1-6 carbon atoms; in other embodiments, the alkylthio is $C_{1-4}$ alkylthio, means an alkylthio group containing 1-4 carbon atoms; in other embodiments, the alkylthio is $C_{1-3}$ alkylthio, means an alkylthio group containing 1-3 carbon atoms. Examples of alkylthio groups include, but are not limited to, methylthio ($-SCH_3$), ethylthio ($-SCH_2CH_3$), and the like.

The term "haloalkylthio" means an alkylthio group substituted with one or more halogen atoms, wherein the alkylthio group has the meaning as described herein. In some embodiments, haloalkylthio is $C_{1-6}$ haloalkylthio, means $C_{1-6}$ alkylthio substituted with one or more halogens; in other embodiments, haloalkylthio is $C_{1-4}$ haloalkylthio, means $C_{1-4}$ alkylthio substituted with one or more halogens; in other embodiments, haloalkylthio is $C_{1-3}$ haloalkylthio, means $C_{1-3}$ alkylthio substituted with one or more halogens. Examples of haloalkylthio include, but are not limited to, trifluoromethylthio ($-SCF_3$), 2,2,2-trifluoroethylthio ($-SCH_2CF_3$), monofluoromethylthio ($-SCH_2F$), and the like.

The term "aralkyl" or "arylalkyl" refers to an alkyl group substituted with one aryl group, wherein the aryl and the alkyl are as defined herein. In some embodiments, arylalkyl is $C_{6-10}$ aryl $C_{1-6}$ alkyl or (6-10 membered aryl)-$C_{1-6}$ alkyl; in other embodiments, arylalkyl is $C_{6-10}$ aryl $C_{1-4}$ alkyl or (6-10 membered aryl)-$C_{1-4}$ alkyl; in other embodiments, arylalkyl is $C_{6-10}$ aryl $C_{1-3}$ alkyl or (6-10 membered aryl)-$C_{1-3}$ alkyl; in other embodiments, arylalkyl is phenyl $C_{1-6}$ alkyl; in other embodiments, arylalkyl is phenyl $C_{1-4}$ alkyl; in other embodiments, arylalkyl is phenyl $C_{1-3}$ alkyl. Examples of arylalkyl groups include, but are not limited to, phenylmethyl, phenylethyl, naphthylmethyl, and the like.

The term "heteroarylalkyl" refers to alkyl group substituted with one heteroaryl radical, wherein the heteroaryl

radical and the alkyl group are as defined herein. In some embodiments, the heteroarylalkyl is (5-12 membered heteroaryl)-$C_{1-6}$ alkyl; in other embodiments, the heteroarylalkyl is (5-12 membered heteroaryl). )-$C_{1-4}$ alkyl; in other embodiments, the heteroarylalkyl is (5-12 membered heteroaryl)-$C_{1-3}$ alkyl; in other embodiments, the heteroarylalkyl is (5-6 membered heteroaryl)-$C_{1-6}$ alkyl; in other embodiments, the heteroarylalkyl is (5-6 membered heteroaryl)-$C_{1-4}$ alkyl; in other embodiments, the heteroarylalkyl is (5-6 membered heteroaryl)-$C_{1-3}$ alkyl. Examples of heteroarylalkyl groups include, but are not limited to, pyrimidinylmethyl, pyridylmethyl, pyridylethyl, pyrazolylmethyl, and the like.

The term "heterocyclylalkyl" means an alkyl group substituted with a heterocyclyl group, wherein the heterocyclyl and alkyl groups are as defined herein. In some embodiments, heterocyclylalkyl is (3-6 membered heterocyclyl)-$C_{1-6}$ alkyl; in other embodiments, heterocyclylalkyl is (3-6 membered heterocyclyl)-$C_{1-4}$ alkyl; in other embodiments, heterocyclylalkyl is (3-6 membered heterocyclyl)-$C_{1-3}$ alkyl. Examples of heterocyclylalkyl include, but are not limited to, piperidylmethyl, piperidylethyl, pyrrolidinylmethyl, and the like.

The term "cycloalkylalkyl" means an alkyl group substituted with one cycloalkyl group. In some embodiments, cycloalkylalkyl is $C_{3-6}$ cycloalkyl $C_{1-6}$ alkyl; in other embodiments, cycloalkylalkyl is $C_{3-6}$ cycloalkyl $C_{1-4}$ alkyl; in other embodiments, cycloalkylalkyl is $C_{3-6}$ cycloalkyl $C_{1-3}$ alkyl. Examples of cycloalkylalkyl groups include, but are not limited to: cyclopropylmethyl, cyclopentylmethyl, cyclohexylmethyl, cyclohexylethyl, and the like.

The term "halogen" means F (fluorine), Cl (chlorine), Br (bromine) or I (iodine).

The term "oxo" means =O.

The term "cyano" means -CN or -C=N.

The term "mercapto" means -SH.

The term "hydroxy" means -OH.

The term "carboxy" means -C(=O)OH.

The term "amino" means $-NH_2$.

The term "composed of j-k atoms" or "j-k membered" means that the cyclic group is composed of j-k ring atoms, and the ring atoms include carbon atoms and/or heteroatoms such as O, N, S, P, etc.; each j and k is independently any non-zero natural number, and k> j; the "j-k" includes j, k and any natural number between the two. For example, "composed of 3-8 members" or "3-8 membered", "composed of 3-6 members" or "3-6 membered", "composed of 5-10 members" or "5-10 membered", "composed of 5-6 members" or "5-6 membered", means that the cyclic group consists of 3-8 (i.e., 3, 4, 5, 6, 7 or 8), 3-6 (i.e., 3, 4, 5, or 6), 5-10 (i.e., 5, 6, 7, 8, 9, or 10), or 5-6 (i.e., 5 or 6) ring atoms, the ring atoms include carbon atoms and/or O, N, S, P and other heteroatoms.

As described in the present invention, the ring system formed by the substituent $(R)_q$ connected to the central ring by a bond represents substitution of q membered substituents R at any substitutable position on the ring. For example, formula a represents that the naphthalene ring can be substituted with n $R^8$, when n is greater than 1, each $R^8$ can be independently selected from the same or different substituents.

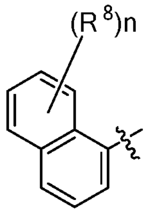

Formula a

The term "prodrug" refers to a compound that is transformed in vivo into a compound of Formula (I), (I-1) or (I-2). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_{1-24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

A "pharmaceutically acceptable salts" refers to organic or inorganic salts of a compound disclosed herein. Pharmaceutically acceptable salts are well known in the art. For example, S. M. Berge *et al.*, describe pharmaceutically

acceptable salts in detail in J. Pharmaceutical Sciences, 1977, 66:1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. This invention also envisions the quaternization of any basic nitrogen-containing groups of the compounds disclosed herein. Water or oil soluble or dispersable products may be obtained by such quaternization. Pharmaceutically acceptable salts further include, when appropriate, nontoxic ammonium, quaternary ammonium, and amine cations formed using counterions such as halide, hydroxide, carboxylate, sulfate, phosphate, nitrate, $C_{1-8}$ sulfonate or aryl sulfonate.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid, ethanolamine or mixtures thereof. The term "hydrate" refers to the complex where the solvent molecule is water.

The term "hydrate" can be used when said solvent is water. In one embodiment, one water molecule is associated with one molecule of the compounds disclosed herein, such as monohydrate. In another embodiment, more than one water molecule may be associated with one molecule of the compounds disclosed herein, such as a dihydrate. In still another embodiment, less than one water molecule may be associated with one molecule of the compounds disclosed herein, such as a hemihydrate. Furthermore, all the solvates of the invention retain the biological effectiveness of the non-hydrate form of the compounds disclosed herein.

As used herein, the term "treat", "treating" or "treatment" of any disease or disorder refers in some embodiments, to ameliorating the disease or disorder (i.e., slowing or arresting or reducing the development of the disease or at least one of the clinical symptoms thereof). In another embodiments, "treat", "treating" or "treatment" refers to alleviating or ameliorating at least one physical parameter including those which may not be discernible by the patient. In yet another embodiments, "treat", "treating" or "treatment" refers to modulating the disease or disorder, either physically, (e.g., stabilization of a discernible symptom), physiologically, (e.g., stabilization of a physical parameter), or both. In yet another embodiments, "treat", "treating" or "treatment" refers to preventing or delaying the onset or development or progression of the disease or disorder.

The term "preventing" or "prevention" refers to a reduction in risk of acquiring a disease or disorder (i.e., causing at least one of the clinical symptoms of the disease not to develop in a subject that may be exposed to or predisposed to the disease but does not yet experience or display symptoms of the disease).

The term "therapeutically effective amount" refers to an amount of a compound that, when administered to a subject to treat a disease, is an amount sufficient to be effective in treating the disease. The "therapeutically effective amount" may vary with the compound, the disease and severity, as well as the condition, age, weight, sex, etc. of the subject to be treated.

Unless otherwise stated, all suitable isotopic changes, stereoisomers, tautomers, solvates, metabolites, pharmaceutically acceptable salts and prodrugs of the compounds of the present invention are included in the scope of the present invention.

When the stereochemistry of any particular chiral atom in a structure disclosed herein is not specified, all stereoisomers of that structure are contemplated by the present invention and are included in the present invention as a compound disclosed herein. When stereochemistry is indicated by a solid wedge or a dashed line indicating a specific configuration, then the stereoisomers of that structure are clear and defined.

*N*-oxides of the compounds of the invention are also included within the scope of the invention. The *N*-oxides of the compounds of the present invention can be prepared by oxidizing the corresponding nitrogen-containing alkaline substance at elevated temperature using a customary oxidizing agent such as hydrogen peroxide in the presence of an acid such as acetic acid, or by reacting with a peracid in a suitable solvent, such as reacting with peracetic acid in dichloromethane, ethyl acetate or methyl acetate, or reacting with 3-chloroperoxybenzoic acid in chloroform or dichloromethane.

The compound of formula (I), (I-1) or (I-2) may exist in the form of a salt.

Any formula given herein is also intended to represent isotopically unenriched forms as well as isotopically enriched forms of the compounds. Any formula given herein is also intended to represent isotopically unenriched forms as well as isotopically enriched forms of the compounds. Examples of isotopes that can be incorporated into compounds of the invention include isotopes of hydrogen, carbon, nitrogen, oxygen, phosphorous, fluorine, chlorine and iodine, such as $^{2}H$, $^{3}H$, $^{11}C$, $^{13}C$, $^{14}C$, $^{15}N$, $^{17}O$, $^{18}O$, $^{18}F$, $^{31}P$, $^{32}P$, $^{35}S$, $^{36}Cl$ and $^{125}I$, respectively.

## DESCRIPTION OF COMPOUNDS OF THE INVENTION

The present invention provides a compound, or a pharmaceutical composition thereof, which can be used as an inhibitor of KRAS, especially as an inhibitor of KRAS 12D. The invention further relates to use of the compound or a

pharmaceutical composition thereof in the manufacture of a medicament for treating diseases and/or conditions by inhibiting KRAS activity by the compound.

The excellent properties of the compounds of the present invention, such as half-life, clearance rate, selectivity, bioavailability, chemical stability, metabolic stability, membrane permeability, solubility, etc., can reduce side effects, expand the therapeutic index, or improve tolerance.

In one aspect, the present invention provides a compound having Formula (I) or a stereoisomer, a tautomer, an *N*-oxide, a solvate, a metabolite, a pharmaceutically acceptable salt or a prodrug thereof,

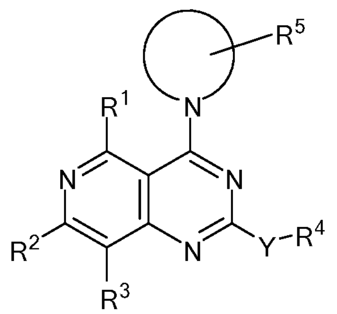

(I),

wherein $R^1$, $R^2$, $R^3$, Y, $R^4$, $R^5$ and

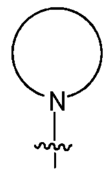

are as defined herein.

In some embodiments, $R^1$ is -F, -Br, -I, -CN, -SH, -C(=O)$R^{6a}$, -C(=O)O$R^{6a}$, - C(=O)N$R^6R^7$, -N$R^6$C(=O)$R^7$, -N$R^6R^7$, -N$R^6$S(=O)$_2R^7$, $C_{2-6}$ alkenyl, $C_{2-6}$ alkynyl, $C_{1-6}$ cyanoalkyl, $C_{1-6}$ hydroxyalkyl, $C_{2-6}$ haloalkenyl, $C_{2-6}$ haloalkynyl, $C_{1-6}$ alkoxy $C_{1-6}$ alkyl, $C_{1-6}$ carboxyalkyl, $C_{1-6}$ aminoalkyl, $C_{1-6}$ mercaptoalkyl or 3-6 membered heterocyclyl, wherein the $C_{2-6}$ alkenyl, $C_{2-6}$ alkynyl, $C_{1-6}$ cyanoalkyl, $C_{1-6}$ hydroxyalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl and 3-6 membered heterocyclyl are independently and optionally substituted with 1, 2, 3 or 4 substituents selected from -D, -OH, -F, -Cl, -Br, -I, -CN, -$NH_2$, -C(=O)H, -C(=O)OH, - C(=O)O$R^{6f}$, -C(=O)N$R^6R^7$, -N$R^6$C(=O)$R^7$, -N$R^{6f}$C(=O)N$R^6R^7$, -C(=O)$R^{6a}$, -N$R^6$S(=O)$_2R^7$, - S(=O)$_2$N$R^6R^7$, -N$R^{6f}$S(=O)$_2$N$R^6R^7$, -N$R^{6f}R^{7f}$, -$C_{1-6}$ alkyl N$R^{6f}R^{7f}$, $C_{1-6}$ alkyl, $C_{2-6}$ alkenyl, $C_{2-6}$ alkynyl, $C_{1-6}$ alkoxy, (6-12 membered aryl)-$C_{1-6}$ alkoxy, $C_{1-6}$ cyanoalkyl, $C_{1-6}$ hydroxyalkyl, $C_{1-6}$ haloalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl, 6-12 membered aryl, 5-12 membered heteroaryl, $C_{3-6}$ cycloalkyl and 3-6 membered heterocyclyl, wherein $R^{6a}$, $R^{6f}$, $R^{7f}$, $R^6$ and $R^7$ have the definitions as described herein.

In some embodiments, $R^2$ is 6-12 membered aryl or 5-12 membered heteroaryl, wherein the 6-12 membered aryl and 5-12 membered heteroaryl are independently and optionally substituted with 1, 2, 3, 4, 5, 6 or 7 $R^8$; wherein the $R^8$ has the definition as described herein.

In some embodiments, each $R^8$ is independently -D, -OH, -F, -Cl, -Br, -I, -CN, -SH, - $CH_2$C(=O)N$R^{6b}k^{7b}$, -C(=O)$R^{6c}$, -C(=O)O$R^{6c}$, -C(=O)N$R^{6b}R^{7b}$, -N$R^{6b}$C(=O)$R^{7b}$, -N$R^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{1-6}$ haloalkenyl, $C_{1-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{1-6}$ haloalkenyl, $C_{1-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl and 3-6 membered heterocyclyl are independently and optionally substituted with 1, 2, 3 or 4 substituents selected from -D, -OH, -F, -Cl, -Br, -I, -CN, -$NH_2$, -C(=O)H, -C(=O)OH, $C_{1-6}$ alkyl, $C_{1-6}$ alkoxy, $C_{3-6}$ cycloalkyl or 3-6 membered heterocyclyl, wherein $R^{6b}$, $R^{7b}$ and $R^{6c}$ have the definitions as described herein.

In some embodiments, $R^3$ is -H, -D, -OH, -SH, -F, -Cl, -Br, -I, -CN or $C_{1-4}$ alkyl.

In some embodiments, Y is a bond, O or S.

In some embodiments, $R^4$ is -H, -D, $C_{1-6}$ alkyl, 3-10 membered heterocyclyl, -L-(3-10 membered heterocyclyl), -L-$C_{3-10}$ cycloalkyl, -L-(5-12 membered heteroaryl), -L-($C_{6-10}$ aryl), -L-N$R^6R^7$, -N$R^6R^7$, -C(=O)N$R^{6d}R^{7d}$, -L-

$NHC(=NH)NH_2$ or $-L-C(=O)NR^6R^7$, wherein the $C_{1-6}$ alkyl, 3-10 membered heterocyclyl, -L-(3-10 membered heterocyclyl), -L-$C_{3-10}$ cycloalkyl, -L-(5-12 membered heteroaryl) and -L-($C_{6-10}$ aryl) are independently and optionally substituted with 1, 2, 3 or 4 substituents selected from -D, -OH, -F, -Cl, -Br, -I, -CN, $-NR^{6d}R^{7d}$, $-CH_2NR^{6d}R^{7d}$, - $CH_2OC(=O)NR^{6d}R^{7d}$, $C_{1-6}$ alkyl, $C_{1-6}$ alkoxy, $C_{1-6}$ haloalkyl, $C_{1-6}$ haloalkoxy, $C_{6-10}$ aryl $C_{1-6}$ alkyl, (5-12 membered heteroaryl)-$C_{1-6}$ alkyl, (3-6 membered heterocyclyl)-$C_{1-6}$ alkyl, $C_{3-6}$ cycloalkyl $C_{1-6}$ alkyl, $C_{3-6}$ cycloalkyl and 3-6 membered heterocyclyl, among the substituents, the $C_{1-6}$ alkyl, $C_{1-6}$ alkoxy, $C_{1-6}$ haloalkyl, $C_{1-6}$ haloalkoxy, $C_{6-10}$ aryl $C_{1-6}$ alkyl, (5-12 membered heteroaryl)-$C_{1-6}$ alkyl, (3-6 membered heterocyclyl)-$C_{1-6}$ alkyl, $C_{3-6}$ cycloalkyl $C_{1-6}$ alkyl, $C_{3-6}$ cycloalkyl and 3-6 membered heterocyclyl are independently and optionally substituted with 1, 2, 3 or 4 substituents selected from -D, -F, -Cl, -Br, -I, -OH, -CN, $NR^{6e}R^{7e}$, $-C(=O)C_{1-6}$ alkyl and $C_{1-6}$ alkyl; wherein L, $R^6$, $R^7$, $R^{6d}$, $R^{7d}$, $R^{6e}$ and $R^{7e}$ have the definitions as described herein.

In some embodiments, Lis $C_{1-6}$ alkylene.

In some embodiments,

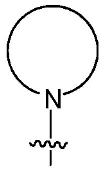

is a 5-10 membered heterocycle containing at least two ring N atoms.

In some embodiments, $R^5$ is -H, -D, -OH, -SH, -F, -Cl, -Br, -I, -CN, -C(=O)H, - $C(=O)OR^{6a}$, $-C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ haloalkoxy or 5-6 membered heteroaryl, wherein the $R^{6a}$, $R^{6a}$ and $R^7$ have the definitions as described herein.

In some embodiments, each $R^{6a}$ and $R^{6c}$ is independently -H, -D, or $C_{1-6}$ alkyl.

In some embodiments, each $R^6$, $R^7$, $R^{6b}$, $R^{7b}$, $R^{6d}$, $R^{7d}$, $R^{6e}$, $R^{7e}$, $R^{6f}$ and $R^{7f}$ is independently -H, -D or $C_{1-6}$ alkyl, wherein the $C_{1-6}$ alkyl is optionally substituted with 1, 2, 3 or 4 substituents selected from -D, -OH, -F, -Cl, -Br, -I, -CN, -C(=O)H, -C(=O)OH, $-NR^{6g}R^{7g}$, $C_{1-6}$ alkoxy, 6-12 membered aryl, $C_{3-6}$ cycloalkyl and 3-6-membered heterocyclyl; wherein, $R^{6g}$ and $R^{7g}$ have the definitions as described herein.

In some embodiments, each $R^{6g}$ and $R^{7g}$ is independently -H, -D, or $C_{1-6}$ alkyl.

In some embodiments, $R^6$ and $R^7$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^{6b}$ and $R^{7b}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^{6d}$ and $R^{7d}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^{6e}$ and $R^{7e}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^{6f}$ and $R^{7f}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl.

In some embodiments, $R^1$ is -F, -Br, -I, -CN, -SH, $-C(=O)R^{6a}$, $-C(=O)OR^{6a}$, - $C(=O)NR^6R^7$, $-NR^6C(=O)R^7$, $-NR^6R^7$, $-NR^6S(=O)_2R^7$, $C_{2-4}$ alkenyl, $C_{2-4}$ alkynyl, $C_{1-4}$ cyanoalkyl, $C_{1-4}$ hydroxyalkyl, $C_{2-4}$ haloalkenyl, $C_{2-4}$ haloalkynyl, $C_{1-4}$ alkoxy $C_{1-4}$ alkyl, $C_{1-4}$ carboxyalkyl, $C_{1-4}$ aminoalkyl, $C_{1-4}$ mercaptoalkyl or 3-6 membered heterocyclyl, wherein the $C_{2-4}$ alkenyl, $C_{2-4}$ alkynyl, $C_{1-4}$ cyanoalkyl, $C_{1-4}$ hydroxyalkyl, $C_{1-4}$ alkoxy $C_{1-4}$ alkyl, $C_{1-4}$ carboxyalkyl, $C_{1-4}$ aminoalkyl, $C_{1-4}$ mercaptoalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, - $C(=O)OR^{6f}$, , $-C(=O)NR^6R^7$, $-NR^6C(=O)R^7$, $-C_{1-4}$ alkyl $NR^6C(=O)R^7$, $-NR^{6f}C(=O)NR^6R^7$, $-C(=O)R^{6a}$, $-NR^6S(=O)_2R^7$, $-S(=O)_2NR^6R^7$, $-NR^{6f}S(=O)_2NR^6R^7$, $-NR^{6f}R^{7f}$, $-C_{1-4}$ alkyl $NR^{6f}R^{7f}$, $C_{1-4}$ alkyl, $C_{2-4}$ alkenyl, $C_{2-4}$ alkynyl, $C_{1-4}$ alkoxy, (6-10 membered

aryl)-$C_{1-4}$ alkoxy, $C_{1-4}$ cyanoalkyl, $C_{1-4}$ hydroxyalkyl, $C_{1-4}$ haloalkyl, $C_{1-4}$ alkoxy $C_{1-4}$ alkyl, $C_{1-4}$ carboxyalkyl, $C_{1-4}$ aminoalkyl, $C_{1-4}$ mercaptoalkyl, 6-10 membered aryl, 5-10 membered heteroaryl, $C_{3-6}$ cycloalkyl and 3-6 membered heterocyclyl, wherein $R^{6a}$, $R^{6f}$, $R^{7f}$, $R^6$ and $R^7$ have the definitions as described herein.

In some embodiments, each $R^{6a}$ is independently -H, -D, or $C_{1-4}$ alkyl.

In some embodiments, each $R^6$ and $R^7$ is independently -H, -D or $C_{1-4}$ alkyl, wherein the $C_{1-4}$ alkyl is optionally substituted with 1, 2, 3 or 4 substituents selected from -D, -OH, -F, -Cl, -Br, - I, -CN, -C(=O)H, -C(=O)OH, $-NR^{6g}R^{7g}$, $C_{1-4}$ alkoxy, 6-10 membered aryl, $C_{3-6}$ cycloalkyl and 3-6 membered heterocyclyl, wherein $R^{6g}$ and $R^{7g}$ have the definitions as described herein.

In some embodiments, $R^6$ and $R^7$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, each $R^{6f}$ and $R^{7f}$ is independently -H, -D or $C_{1-4}$ alkyl, wherein the $C_{1-4}$ alkyl is optionally substituted with 1, 2, 3 or 4 substituents selected from -D, -OH, -F, -Cl, - Br, -I, -CN, -C(=O)H, -C(=O)OH, $-NR^{6g}R^{7g}$, $C_{1-4}$ alkoxy, 6-10 membered aryl, $C_{3-6}$ cycloalkyl and 3-6 membered heterocyclyl, wherein $R^{6g}$ and $R^{7g}$ have the definitions as described herein.

In some embodiments, $R^{6f}$ and $R^{7f}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, each $R^{6g}$ and $R^{7g}$ is independently -H, -D, or $C_{1-4}$ alkyl.

In some embodiments, $R^1$ is -F, -Br, -I, -CN, -SH, $-C(=O)R^{6a}$, $-C(=O)OR^{6a}$, $- C(=O)NR^6R^7$, $-NR^6C(=O)R^7$, $-NR^6R^7$, $-NR^6S(=O)_2R^7$, $-CH=CH_2$, $-CH=CHCH_3$, $-CH_2CH=CH_2$, $- CHFCH=CH_2$, -CH=CHF, -CH=CHCl, $-CH=CHCH_2F$, $-CH=CHCH_3$, -C=CH, $-C\equiv CCH_3$, $- CH_2C\equiv CH$, $-C\equiv CCH_2F$, $-CH_2CN$, $-(CH_2)_2CN$, $-(CH_2)_3CN$, $-CH_2OH$, $-(CH_2)_2OH$, $-(CH_2)_3OH$, $- CH(OH)CH_3$, $-CH_2OCH_3$, $-(CH_2)_2OCH_3$, $-(CH_2)_2OCH_2CH_3$, $-CH_2OCH_2CH_3$, $-CH_2C(=O)OH$, $-(CH_2)_2C(=O)OH$, $-(CH_2)_3C(=O)OH$, $-CH_2NH_2$, $-(CH_2)_2NH_2$, $-CH_2SH$, $-(CH_2)_2SH$, oxiranyl, oxetanyl or pyrrolidinyl, wherein the $-CH=CH_2$, $-CH=CHCH_3$, $-CH_2CH=CH_2$, $-CH=CHCH_3$, $-C\equiv CH$, $- C\equiv CCH_3$, $-CH_2C\equiv CH$, $-CH_2CN$, $-(CH_2)_2CN$, $-(CH_2)_3CN$, $-CH_2OH$, $-(CH_2)_2OH$, $-(CH_2)_3OH$, $- CH(OH)CH_3$, $-CH_2OCH_3$, $-(CH_2)_2OCH_3$, $-(CH_2)_2OCH_2CH_3$, $-CH_2OCH_2CH_3$, $-CH_2C(=O)OH$, $- (CH_2)_2C(=O)OH$, $-(CH_2)_3C(=O)OH$, $-CH_2NH_2$, $-(CH_2)_2NH_2$, $-CH_2SH$, oxiranyl, oxetanyl and pyrrolidinyl are independently and optionally substituted with 1, 2, 3 or 4 substituents selected from -D, -OH, -F, -Cl, -Br, -I, -CN, $-NH_2$, -C(=O)H, -C(=O)OH, $-C(=O)OR^{6f}$, $-C(=O)NR^6R^7$, $- NR^6C(=O)R^7$, $-CH_2NR^6C(=O)R^7$, $-NR^{6f}C(=O)NR^6R^7$, $-C(=O)R^{6a}$, $-NR^6S(=O)_2R^7$, $-S(=O)_2NR^6R^7$, $- NR^{6f}S(=O)_2NR^6R^7$, $-NR^{6f}R^{7f}$, $-CH_2NR^{6f}R^{7f}$, $-CH=CH_2$, $-CH=CHCH_3$, $-CH_2CH=CH_2$, -C=CH, $- C\equiv CCH_3$, $-CH_2C\equiv CH$, methyl, ethyl, n-propyl, isopropyl, methoxy, ethoxy, n-propoxy, isopropoxy, isobutoxy, phenylmethoxy, phenylethoxy, phenylpropoxy, naphthylmethoxy, $-CH_2CN$, $-(CH_2)_2CN$, $- (CH_2)_3CN$, $-CH_2OH$, $-(CH_2)_2OH$, $-(CH_2)_3OH$, $-CH(OH)CH_3$, $-CF_3$, $-CHF_2$, $-CH_2F$, $-(CH_2)_2F$, $- (CH_2)_2Cl$, $-CH_2CF_3$, $-CH_2OCH_3$, $-(CH_2)_2OCH_3$, $-(CH_2)_2OCH_2CH_3$, $-CH_2OCH_2CH_3$, $-CH_2C(=O)OH$, $-(CH_2)_2C(=O)OH$, $-(CH_2)_3C(=O)OH$, $-CH_2NH_2$, $-(CH_2)_2NH_2$, $-CH_2SH$, $-(CH_2)_2SH$, phenyl, naphthyl, pyridyl, pyrimidinyl, cyclopropyl, cyclobutyl, cyclopentyl, cyclohexyl, oxiranyl, oxetanyl, tetrahydropyranyl, azetidinyl and pyrrolidinyl, wherein $R^{6a}$, $R^{6f}$, $R^{7f}$, $R^6$ and $R^7$ have the definitions as described herein.

In some embodiments, each $R^{6a}$ is independently -H, -D, methyl, ethyl, n-propyl, isopropyl, n-butyl, isobutyl, or *tert*-butyl.

In some embodiments, each $R^6$ and $R^7$ is independently -H, -D, methyl, ethyl, *n*-propyl, isopropyl, *n*-butyl, isobutyl or *tert*-butyl, wherein the methyl, ethyl, *n*-propyl, isopropyl, *n*-butyl, isobutyl and *tert*-butyl are optionally substituted with 1, 2, 3 or 4 substituents selected from -D, -OH, -F, -Cl , -Br, -I, -CN, -C(=O)H, -C(=O)OH, $-NR^{6g}R^{7g}$, methoxy, ethoxy, *n*-propoxy, isopropoxy, isobutoxy, cyclopropyl, cyclobutyl, cyclopentyl, cyclohexyl, phenyl, oxiranyl, oxetanyl, azetidinyl and pyrrolidinyl; wherein $R^{6g}$ and $R^{7g}$ have the definitions as described herein.

In some embodiments, each $R^{6f}$ and $R^{7f}$ is independently -H, -D, methyl, ethyl, n-propyl, isopropyl, *n*-butyl, isobutyl or *tert*-butyl, wherein the methyl, ethyl, *n*-propyl, isopropyl, *n*-butyl, isobutyl and *tert*-butyl are optionally substituted with 1, 2, 3 or 4 substituents selected from -D, -OH, -F, -Cl , -Br, -I, -CN, -C(=O)H, -C(=O)OH, $-NR^{6g}R^{7g}$, methoxy, ethoxy, *n*-propoxy, isopropoxy, isobutoxy, cyclopropyl, cyclobutyl, cyclopentyl, cyclohexyl, phenyl, oxiranyl, oxetanyl, azetidinyl and pyrrolidinyl; wherein $R^{6g}$ and $R^{7g}$ have the definitions as described herein.

In some embodiments, $R^6$ and $R^7$, together with the N atom to which they are attached, form pyrrolidine, piperazine, piperidine, morpholinyl, oxazolidine or imidazolidine, wherein the pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, $- NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

In some embodiments, $R^{6f}$ and $R^{7f}$, together with the N atom to which they are attached, form pyrrolidine,

piperazine, piperidine, morpholinyl, oxazolidine or imidazolidine, wherein the pyrrolidine, piperazine, piperidine,morpholinyl, oxazolidine and imidazolidine are independently and optionally substituted with 1, 2, 3 or 4 substituents selected from -D, -OH, -F, -Cl, -Br, -I, -CN, $-NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

In some embodiments, each $R^{6g}$ and $R^{7g}$ is independently -H, -D, methyl, ethyl, n-propyl, isopropyl, *n*-butyl, isobutyl, or *tert*-butyl.

In some embodiments, $R^2$ is one of the following substructures,

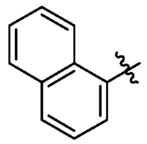, 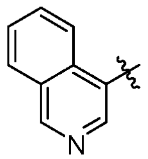, 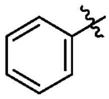, 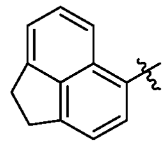, 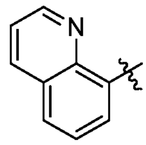, 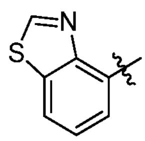, 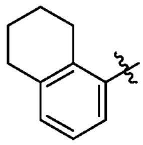,

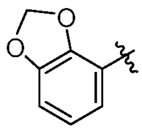, 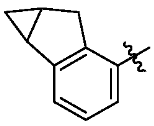, 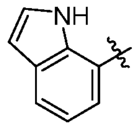, 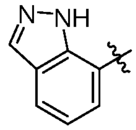, 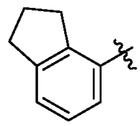, 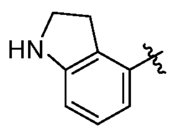, 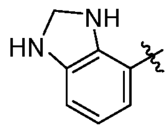,

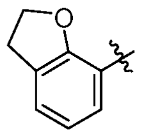, 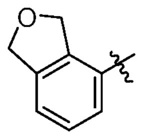, 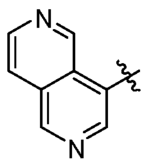, 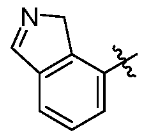, 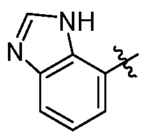, 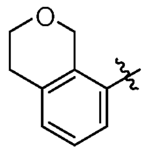, 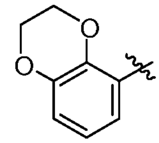

or

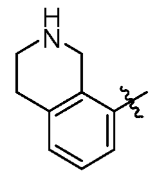,

wherein, each substructure of $R^2$ is independently and optionally substituted with 1, 2, 3, 4, 5, 6 or 7 $R^8$; wherein $R^8$ has the definition as described in the present invention.

In some embodiments, each $R^8$ is independently -D, -OH, -F, -Cl, -Br, -I, -CN, -SH, - $CH_2C(=O)NR^{6b}k^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl or 3-6 membered heterocyclyl, wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl or 3-6 membered heterocyclyl, wherein $R^{6b}$, $R^{7b}$ and $R^{6c}$ have the definitions as described herein.

In some embodiments, each $R^{6c}$ is independently -H, -D, or $C_{1-4}$ alkyl.

In some embodiments, each $R^{6b}$ and $R^{7b}$ is independently -H, -D or $C_{1-4}$ alkyl, wherein the $C_{1-4}$ alkyl is optionally substituted with 1, 2, 3 or 4 substituents selected from -D, -OH, -F, -Cl, - Br, -I, -CN, -C(=O)H, -C(=O)OH, $-NR^{6g}R^{7g}$, $C_{1-4}$ alkoxy, 6-10 membered aryl, $C_{3-6}$ cycloalkyl and 3-6 membered heterocyclyl, wherein $R^{6g}$ and $R^{7g}$ have the definitions as described herein.

In some embodiments, $R^{6b}$ and $R^{7b}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy,

$C_{1-4}$ cyanoalkyl, $C_{1-4}$ hydroxyalkyl, $C_{1-4}$ haloalkoxy or $C_{1-4}$ haloalkyl.

In some embodiments, each $R^8$ is independently -D, -OH, -F, -Cl, -Br, -I, -CN, -SH, - $CH_2C(=O)NR^{6b}k^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, - $CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, - $CH=CHCH_3$, $-CH_2CH=CH_2$, -C=CH, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, - $CH(OH)CH_3$, $-(CH_2)_2F$, $-CH_2CHF_2$, $-CF_3$, $-CH_2CF_3$, $-CHF_2$, $-CH_2F$, -(CHz)zCl, -CH=CHF, - CH=CHCl, $-CH=CHCH_2F$, $-C\equiv CCH_2F$, $-C\equiv C(CH_2)_2F$, -C=CF, $-OCF_3$, $-OCHF_2$, $-OCH_2CHF_2$, - $OCH_2CF_3$, $-OCHClCHCl_2$, $-OCH_2CH_2F$, $-SCF_3$, $-SCH_2CF_3$, $-SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the $-CH_3$, $-CH_2CH_3$, $-(CH_2)_2CH_3$, $-(CH_2)_3CH_3$, - $C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, - $C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_30H$, $-CH(OH)CH_3$, $-(CH_2)_2F$, $-CH_2CHF_2$, - $CH_2CF_3$, $-CHF_2$, $-CH_2F$, $-(CH_2)_2Cl$, -CH=CHF, -CH=CHCl, $-CH=CHCH_2F$, $-C\equiv CCH_2F$, - $C\equiv C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, - $SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuryl, piperidinyl and piperazinyl are independently and optionally substituted with 1, 2, 3 or 4 substituents selected from -D, -OH, -F, -Cl, -Br, -I, -CN, $-NH_2$, -C(=O)H, -C(=O)OH, methyl, ethyl, n-propyl, isopropyl, methoxy, ethoxy, *n*-propoxy, isopropoxy, cyclopropyl, cyclobutyl, cyclopentyl, cyclohexyl, pyrrolidinyl, oxazolidinyl, tetrahydrofuranyl, piperidinyl and piperazinyl; wherein $R^{6c}$, $R^{6b}$ and $R^{7b}$ have the definitions as described herein.

In some embodiments, each $R^{6c}$ is independently -H, -D, methyl, ethyl, *n*-propyl, isopropyl, *n*-butyl, isobutyl, or *tert*-butyl.

In some embodiments, each $R^{6b}$ and $R^{7b}$ is independently -H, -D, methyl, ethyl, *n*-propyl, isopropyl, *n*-butyl, isobutyl, or *tert-butyl,* wherein the methyl, ethyl, *n*-propyl, isopropyl, *n*-butyl, isobutyl and *tert*-butyl are optionally substituted with 1, 2, 3 or 4 substituents selected from -D, -OH, -F, -Cl, -Br, -I, -CN, -C(=O)H, -C(=O)OH, $-NR^{6g}R^{7g}$, methoxy, ethoxy, *n*-propoxy, isopropoxy, isobutoxy, phenyl, cyclopropyl, cyclobutyl, cyclopentyl, cyclohexyl, oxiranyl, oxetanyl, azetidinyl and pyrrolidinyl; wherein $R^{6g}$ and $R^{7g}$ have the definitions as described herein;

or, $R^{6b}$ and $R^{7b}$, together with the N atom to which they are attached respectively, form pyrrolidine, piperazine, piperidine, morpholinyl, oxazolidine or imidazolidine, wherein the pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, - $NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

In some embodiments, $R^4$ is -H, -D, $C_{1-4}$ alkyl, 3-6 membered heterocyclyl, 7-10 membered heterocyclyl,

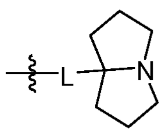,
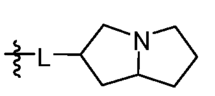,
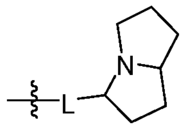,
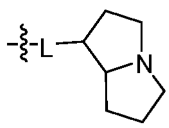,
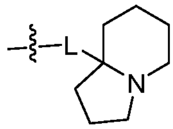,

-L-pyrrolidinyl, -L-morpholinyl, -L-oxetanyl, -L-oxypropyl, -L-tetrahydrofuranyl, -L-octahydroindolizinyl, -L-cyclopropyl, -L-cyclopentyl, -L-octahydropentadienyl, -L-octahydro-1H-indenyl, -L-decalinyl, -L-pyridyl, -L-pyrazolyl, -L-phenyl, -L-$NR^6R^7$, $-NR^6R^7$, $-L-NHC(=NH)NH_2$ or $-L-C(=O)NR^6R^7$, wherein the $C_{1-4}$ alkyl, 3-6 membered heterocyclyl, 7-10 membered heterocyclyl,

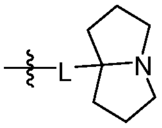,
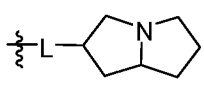,
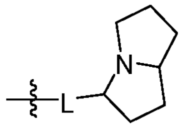,
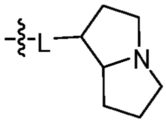,
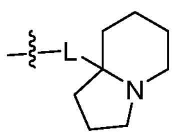,

-L-pyrrolidinyl, -L-morpholinyl, -L-oxetanyl, -L-oxypropyl, -L-tetrahydrofuranyl, -L-octahydroindolizinyl, -L-cyclopropyl, -L-cyclopentyl, -L-octahydropentadienyl, -L-octahydro-1*H*-indenyl, -L-decalinyl, -L-pyridyl, -L-pyrazolyl and -L-phenyl are

independently and optionally substituted with 1, 2, 3 or 4 substituents selected from -D, -OH, -F, -Cl, -Br, -I, -CN, $-NR^{6d}R^{7d}$, $-C(=O)NR^{6d}R^{7d}$, $-CH_2NR^{6d}R^{7d}$, - $CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl $C_{1-4}$ alkyl, (5-6 membered heteroaryl)-$C_{1-4}$ alkyl, (3-6 membered heterocyclyl)-$C_{1-4}$ alkyl, $C_{3-6}$ cycloalkyl $C_{1-4}$ alkyl, $C_{3-6}$ cycloalkyl and 3-6 membered heterocyclyl, among the substituents, the $C_{1-4}$ alkyl, $C_{1-4}$ alkoxy, $C_{1-4}$ haloalkyl, $C_{1-4}$ haloalkoxy, phenyl $C_{1-4}$ alkyl, (5-6 membered heteroaryl)-$C_{1-4}$ alkyl, (3-6 membered heterocyclyl)-$C_{1-4}$ alkyl, $C_{3-6}$ cycloalkyl $C_{1-4}$ alkyl, $C_{3-6}$ cycloalkyl and 3-6 membered heterocyclyl are independently and optionally substituted with 1, 2, 3 or 4 substituents selected from -D, -F, -Cl, -Br, -I, -OH, -CN, $-NR^{6e}R^{7e}$, $-C(=O)C_{1-4}$ alkyl and $C_{1-4}$ alkyl; wherein L, $R^6$, $R^7$, $R^{6d}$, $R^{7d}$, $R^{6e}$ and $R^{7e}$ have the definitions as described herein.

In some embodiments, L is $C_{1-4}$ alkylene.

In some embodiments, L is $C_{1-2}$ alkylene.

In some embodiments, L is $-CH_2-$, $-CH(CH_3)-$, $-(CH_2)_2-$, $-(CH_2)_3-$ or $-(CH_2)_4-$.

In some embodiments, each $R^{6d}$, $R^{7d}$, $R^{6e}$ and $R^{7e}$ is independently -H, -D or $C_{1-4}$ alkyl, wherein the $C_{1-4}$ alkyl is optionally substituted with 1, 2, 3 or 4 substituents selected from -D, -OH, - F, -Cl, -Br, -I, -CN, -C(=O)H, -C(=O)OH, $-NR^{6g}R^{7g}$, $C_{1-4}$ alkoxy, 6-10 membered aryl, $C_{3-6}$ cycloalkyl and 3-6 membered heterocyclyl; wherein $R^{6g}$ and $R^{7g}$ have the definitions as described herein.

In some embodiments, $R^{6d}$ and $R^{7d}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, $R^{6e}$ and $R^{7e}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

In some embodiments, $R^4$ is -H, -D, $-CH_3$, $-CH_2CH_3$, $-(CH_2)_2CH_3$, piperidinyl, piperazinyl, pyrrolidinyl, imidazolidinyl,

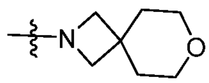 , 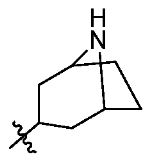 , 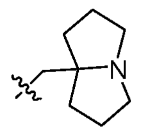 ,

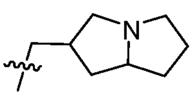 , 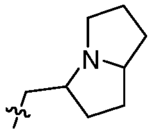 , 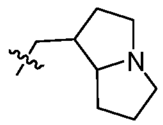 , 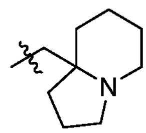 ,

$-CH_2$-pyrrolidinyl, $-CH_2$-morpholinyl, - $(CH_2)_2$-morpholinyl, $-CH_2$-oxetanyl, $-CH_2$-oxypropyl, $-CH_2$-tetrahydrofuranyl, $-CH_2$-octahydroindolizinyl, $-CH_2$-cyclopropyl, $-CH_2$-cyclopentyl, $-CH_2$-octahydropentadienyl, $-CH_2$-octahydro-1*H*-indenyl, $-CH_2$-decalinyl, $-CH_2$-pyridyl, $-(CH_2)_2$-pyridyl, $-CH_2$-pyrazolyl, $-(CH_2)_2$-pyrazolyl, $-CH_2$-phenyl, $-CH_2-NR^6R^7$, $-(CH_2)_2-NR^6R^7$, $-CH(CH_3)CH_2NR^6R^7$, $-NR^6R^7$, $-CH_2-NHC(=NH)NH_2$ or $-CH_2-C(=O)NR^6R^7$, wherein the $-CH_3$, $-CH_2CH_3$, $-(CH_2)_2CH_3$, piperidinyl, piperazinyl, pyrrolidinyl, imidazolidinyl,

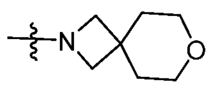 , 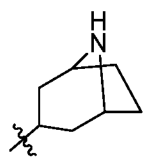 , 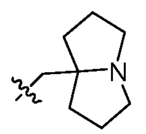 ,

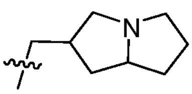 , 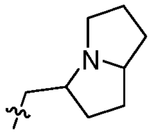 , 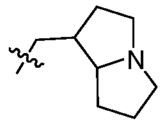 , 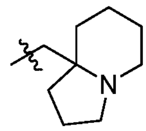 ,

$-CH_2$-pyrrolidinyl, $-CH_2$-morpholinyl, - $(CH_2)_2$-morpholinyl, $-CH_2$-oxetanyl, $-CH_2$-oxypropyl, $-CH_2$-tetrahydrofuranyl, $-CH_2$-octahydroindolizinyl, $-CH_2$-cyclopropyl, $-CH_2$-cyclopentyl, $-CH_2$-octahydropentadienyl, $-CH_2$-octahydro-1*H*-indenyl, $-CH_2$-decalinyl, $-CH_2$-pyridyl, $-(CH_2)_2$-pyridyl, $-CH_2$-pyrazolyl, $-(CH_2)_2$-pyrazolyl and $-CH_2$-phenyl are independently and optionally substituted with 1, 2, 3 or 4 substituents selected from -D, -OH, -F, -Cl, -Br, -I, -CN, $-NR^{6d}R^{7d}$, $-C(=O)NR^{6d}R^{7d}$, $-CH_2NR^{6d}R^{7d}$, - $CH_2OC(=O)NR^{6d}R^{7d}$, methyl, ethyl, *n*-propyl, isopropyl, *n*-butyl, isobutyl, *tert*-butyl, methoxy, ethoxy, isopropoxy, $-CHF_2$, $-CF_3$, $-OCF_3$, phenylmethyl, pyridylmethyl, pyrazolylmethyl, morpholinylmethyl, pyrrolidinylmethyl, piperazinylmethyl, azetidinylmethyl, piperidylmethyl, cyclopropylmethyl, cyclopentylmethyl, cyclohexylmethyl, cyclopentyl, cyclohexyl, morpholinyl, piperidinyl, pyrrolidinyl, piperazinyl or azetidinyl, among the substituents, the methyl, ethyl, n-propyl, isopropyl, *n*-butyl, isobutyl, *tert*-butyl, methoxy, ethoxy, isopropoxy, $-CHF_2$, phenylmethyl, pyridylmethyl, pyrazolylmethyl, morpholinylmethyl, pyrrolidinylmethyl, piperazinylmethyl, azetidinylmethyl, piperidinylmethyl, cyclopropylmethyl, cyclopentylmethyl, cyclohexylmethyl, cyclopentyl, cyclohexyl, morpholinyl, piperidinyl, pyrrolidinyl, piperazinyl and azetidinyl are independently and optionally substituted with 1, 2, 3 or 4 substituents selected from -D, -F, -Cl, -Br, -I, -OH, -CN, $-NH_2$, $-NHCH_3$, $-N(CH_3)_2$, $-NHCH_2CH_3$, $-C(=O)CH_3$, $-C(=O)CH_2CH_3$, methyl, ethyl, n-propyl and isopropyl; wherein $R^6$, $R^7$, $R^{6d}$ and $R^{7d}$ have the definitions as described herein.

In some embodiments, each $R^{6d}$ and $R^{7d}$ is independently -H, -D, methyl, ethyl, *n*-propyl, isopropyl, *n*-butyl, isobutyl, or *tert-butyl,* wherein the methyl, ethyl, *n*-propyl, isopropyl, *n*-butyl, isobutyl and *tert*-butyl are optionally substituted with 1, 2, 3 or 4 substituents selected from -D, -OH, -F, -Cl, -Br, -I, -CN, -C(=O)H, -C(=O)OH, $-NR^{6g}R^{7g}$, methoxy, ethoxy, *n*-propoxy, isopropoxy, isobutoxy, phenyl, cyclopropyl, cyclobutyl, cyclopentyl, cyclohexyl, oxiranyl, oxetanyl, azetidinyl and pyrrolidinyl; wherein $R^{6g}$ and $R^{7g}$ have the definitions as described herein.

Or $R^{6d}$ and $R^{7d}$, together with the N atom to which they are attached, form azetidine, pyrrolidine, piperazine, piperidine, morpholinyl, oxazolidine or imidazolidine, wherein the azetidine, pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, - $NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

In some embodiments,

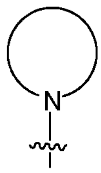

is one of the following substructures:

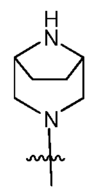 , 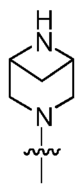 , 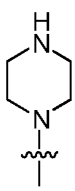 , 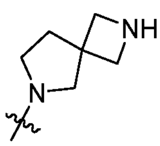 , 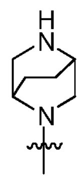 or 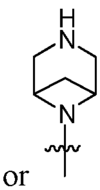 .

In some embodiments, $R^5$ is -H, -D, -OH, -SH, -F, -Cl, -Br, -I, -CN, -C(=O)H, - $C(=O)OCH_3$, $-C(=O)NH_2$, $-CH_3$, $-CH_2CH_3$, $C_{3-4}$ alkyl, $-CH_2OH$, $-(CH_2)_2OH$, $-CH_2CN$, $-(CH_2)_2CN$, - $CF_3$, $-CHF_2$, $-CH_2F$, $-OCF_3$, $-OCH_3$ or $-OCH_2CH_3$.

In some embodiments, $R^3$ is -H, -D, -OH, -SH, -F, -Cl, -Br, -I, -CN, methyl, ethyl, n-propyl or isopropyl.

In some embodiments, the present invention provides a compound having Formula (I-1), or a stereoisomer, a tautomer, an *N*-oxide, a solvate, a metabolite, a pharmaceutically acceptable salt or a prodrug thereof,

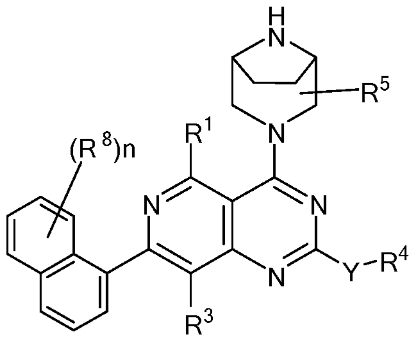

(I-1),

wherein, n is 1, 2, 3, 4, 5, 6 or 7;
$R^1$, $R^3$, $R^4$, $R^5$, Y and $R^8$ are as defined herein.

In some embodiments, the present invention provides a compound having Formula (I-2), or a stereoisomer, a tautomer, an *N*-oxide, a solvate, a metabolite, a pharmaceutically acceptable salt or a prodrug thereof,

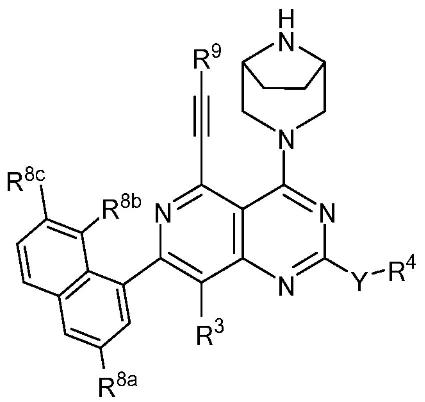

(I-2),

wherein, $R^9$ is -H, -D, CN, $-NH_2$, -C(=O)H, -C(=O)OH, $-C(=O)OR^{6f}$, $-C(=O)NR^6R^7$, - $NR^6C(=O)R^7$, $-NR^{6f}C(=O)NR^6R^7$, $-C(=O)R^{6a}$, $-C(=O)OR^{6a}$, $-NR^6S(=O)_2R^7$, $-S(=O)_2NR^6R^7$, - $NR^{6f}S(=O)_2NR^6R^7$, $-NR^{6f}R^{7f}$, $-C_{1-6}$ alkyl $NR^6C(=O)R^7$, $-C_{1-6}$ alkyl $NR^{6f}R^{7f}$, $C_{1-6}$ alkyl, $C_{1-6}$ cyanoalkyl, $C_{1-6}$ hydroxyalkyl, $C_{1-6}$ haloalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl, 6-12 membered aryl, 5-12 membered heteroaryl, $C_{3-6}$ cycloalkyl and 3-6 membered heterocyclyl;
each $R^{6a}$ is independently -H, -D, or $C_{1-6}$ alkyl;
each $R^6$, $R^7$, $R^{6f}$ and $R^{7f}$ is independently -H, -D or $C_{1-6}$ alkyl, wherein the $C_{1-6}$ alkyl is optionally substituted with 1, 2, 3 or 4 substituents selected from -D, -OH, -F, -Cl, -Br, -I, -CN, -C(=O)H, - C(=O)OH, $-NR^{6g}R^{7g}$, $C_{1-6}$ alkoxy, 6-12 membered aryl, $C_{3-6}$ cycloalkyl and 3-6 membered heterocyclyl;
$R^3$, Y, $R^4$, $R^5$, $R^8$, $R^{6g}$ and $R^{7g}$ are as defined herein;
$R^{8a}$, $R^{8b}$ and $R^{8c}$ are as defined herein.

In some embodiments, $R^9$ is -H, -D, CN, $-NH_2$, -C(=O)H, -C(=O)OH, $-C(=O)OR^{6f}$, - $C(=O)NR^6R^7$, $-NR^6C(=O)R^7$, $-NR^{6f}C(=O)NR^6R^7$, $-C(=O)R^{6a}$, $-C(=O)OR^{6a}$, $-NR^6S(=O)_2R^7$, - $S(=O)_2NR^6R^7$, $-NR^{6f}S(=O)_2NR^6R^7$, $-NR^{6f}R^{7f}$, $-CH_2NR^6C(=O)R^7$, $-CH_2NR^{6f}R^{7f}$, $-CH_3$, $-CH_2CH_3$, - $(CH_2)_2CH_3$, $-(CH_2)_3CH_3$, $-C(CH_3)_3$, $-CH_2CH(CH_3)_2$, $-(CH_2)_2CH(CH_3)_2$, $-CH(CH_3)_2$, $-CH_2CN$, - $(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, - $CH(OH)CH_3$, $-CF_3$, $-CHF_2$, $-CH_2F$, $-(CH_2)_2F$, $-(CH_2)_2Cl$, $-CH_2CF_3$, $-CH_2OCH_3$, $-(CH_2)_2OCH_3$, - $(CH_2)_2OCH_2CH_3$, $-CH_2OCH_2CH_3$, $-CH_2C(=O)OH$, $-(CH_2)_2C(=O)OH$, $-(CH_2)_3C(=O)OH$, $-CH_2NH_2$, $-(CH_2)_2NH_2$, $-CH_2SH$, $-(CH_2)_2SH$, phenyl, naphthyl, pyridyl, pyrimidinyl, cyclopropyl, cyclobutyl, cyclopentyl, cyclohexyl, oxiranyl, oxetanyl, tetrahydropyranyl, azetidinyl and pyrrolidinyl;

each $R^{6a}$ is independently -H, -D, methyl, ethyl, n-propyl, isopropyl, n-butyl, isobutyl or *tert*-butyl;
each $R^6$, $R^7$, $R^{6f}$ and $R^{7f}$ is independently -H, -D, methyl, ethyl, n-propyl, isopropyl, n-butyl, isobutyl or *tert*-butyl, wherein the methyl, ethyl, n-propyl, isopropyl, n-butyl, isobutyl and tert-butyl are independently and optionally substituted with 1, 2, 3 or 4 substituents selected from -D, -OH, -F, -Cl, - Br, -I, -CN, -C(=O)H, -C(=O)OH, $-NR^{6g}R^{7g}$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl;
$R^{6g}$ and $R^{7g}$ are as defined herein.

In some embodiments, each $R^{8a}$, $R^{8b}$ and $R^{8c}$ is independently -D, -OH, -F, -Cl, -Br, -I, - CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, $C_{1-6}$ alkyl, $C_{1-6}$ alkoxy, $C_{3-6}$ cycloalkyl or 3-6 membered heterocyclyl; wherein $R^{6b}$, $R^{7b}$ and $R^{6c}$ are as defined herein.

In some embodiments, each $R^{8a}$, $R^{8b}$ and $R^{8c}$ is independently -D, -OH, -F, -Cl, -Br, -I, - CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl or 3-6 membered heterocyclyl, wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-6}$ haloalkoxy, $C_{1-4}$ haloalkylthio, 6-10 membered aryl, 5-10 membered heteroaryl, $C_{3-4}$ cycloalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-4}$ cycloalkyl or 3-6 membered heterocyclyl; wherein $R^{6b}$, $R^{7b}$ and $R^{6c}$ are as defined herein.

In some embodiments, each $R^{8a}$, $R^{8b}$ and $R^{8c}$ is independently -D, -OH, -F, -Cl, -Br, -I, - CN, -SH, $-CH_2C(=O)NR^{6b}R^{7b}$, $-C(=O)R^{6c}$, $-C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, - $CH=CHCH_3$, $-CH_2CH=CH_2$, -C=CH, $-C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, - $CH(OH)CH_3$, $-(CH_2)_2F$, $-CH_2CHF_2$, $-CF_3$, $-CH_2CF_3$, $-CHF_2$, $-CH_2F$, $-(CH_2)_2Cl$, -CH=CHF, - CH=CHCl, $-CH=CHCH_2F$, $-C\equiv CCH_2F$, $-C\equiv C(CH_2)_2F$, -C=CF, $-OCF_3$, $-OCHF_2$, $-OCH_2CHF_2$, - $OCH_2CF_3$, $-OCHClCHCl_2$, $-OCH_2CH_2F$, $-SCF_3$, $-SCH_2CF_3$, $-SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the $-CH_3$, $-CH_2CH_3$, $-(CH_2)_2CH_3$, $-(CH_2)_3CH_3$, - $C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, - $C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, - $CH_2CF_3$, $-CHF_2$, $-CH_2F$, $-(CH_2)_2Cl$, -CH=CHF, -CH=CHCl, $-CH=CHCH_2F$, $-C\equiv CCH_2F$, - $C\equiv C(CH_2)_2F$, $-OCHF_2$, $-OCH_2CHF_2$, $-OCH_2CF_3$, $-OCHClCHCl_2$, $-OCH_2CH_2F$, $-SCH_2CF_3$, - $SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuranyl, piperidyl and piperazinyl are independently and optionally substituted with 1, 2, 3 or 4 substituents selected from -D, -OH, -F, -Cl, -Br, -I, -CN, $-NH_2$, -C(=O)H, -C(=O)OH, methyl, ethyl, n-propyl, isopropyl, methoxy, ethoxy, n-propoxy, isopropoxy, cyclopropyl, cyclobutyl, cyclopentyl, cyclohexyl, pyrrolidinyl, oxazolidinyl, tetrahydrofuranyl, piperidinyl and piperazinyl; wherein $R^{6b}$, $R^{7b}$ and $R^{6c}$ are as defined herein.

In some embodiments, the present invention provides a compound having Formula (I-3), or a stereoisomer, a tautomer, an *N*-oxide, a solvate, a metabolite, a pharmaceutically acceptable salt or a prodrug thereof,

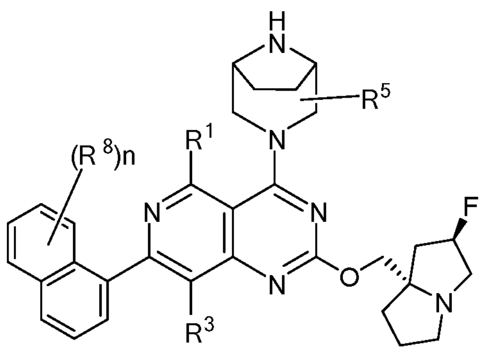

(I-3),

wherein, n is 1, 2, 3, 4, 5, 6 or 7;

$R^1$, $R^3$, $R^5$ and $R^8$ are as defined herein.

In other embodiments, the present invention provides a compound having Formula (I-4), or a stereoisomer, a tautomer, an N-oxide, a solvate, a metabolite, a pharmaceutically acceptable salt or a prodrug thereof,

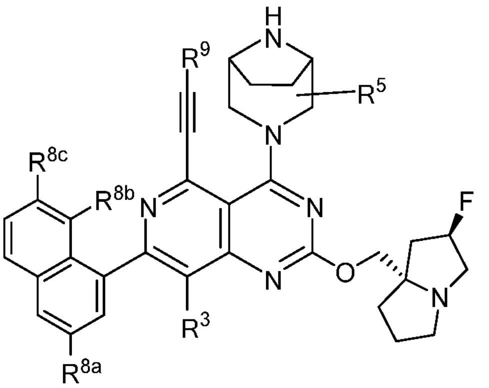

(I-4),

wherein $R^1$, $R^3$, $R^5$, $R^{8a}$, $R^{8b}$, $R^{8c}$ and $R^9$ are as defined herein.

In other aspect, the compounds provided by the present invention are compounds with one of the following structures, or a stereoisomer, a tautomer, an N-oxide, a solvate, a metabolite, a pharmaceutically acceptable salt or a prodrug thereof,

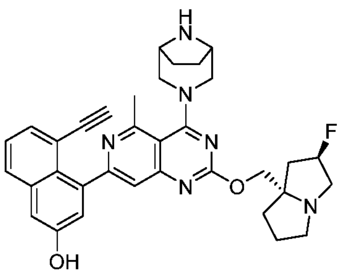
1,
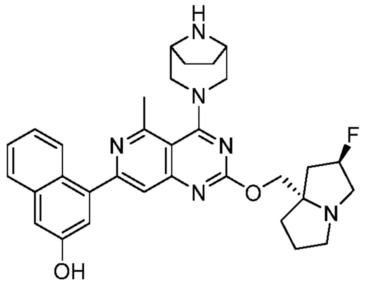
2,
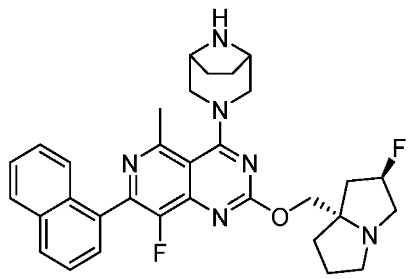
3,
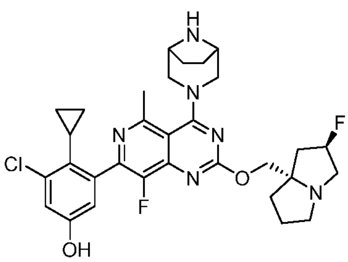
4,
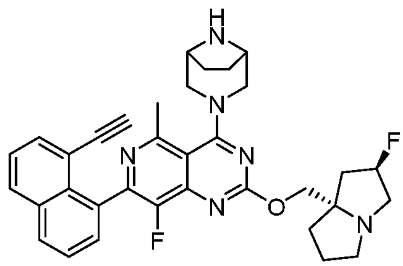
5,
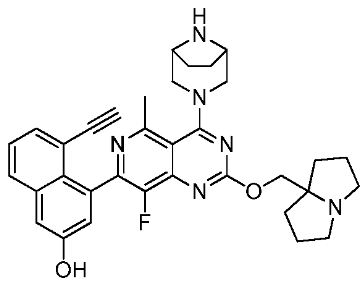
6,
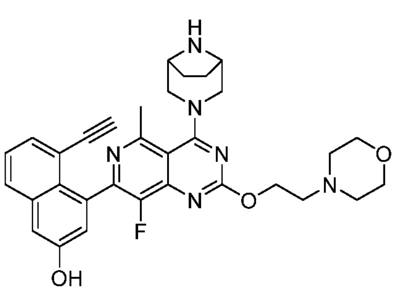
7,
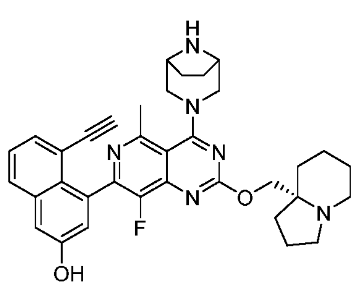
8,
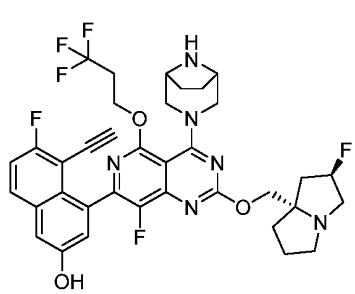
9,

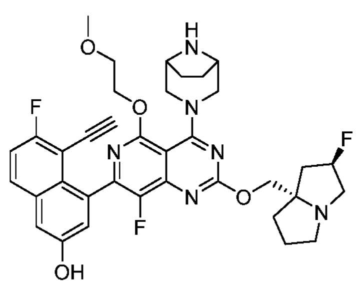 10,

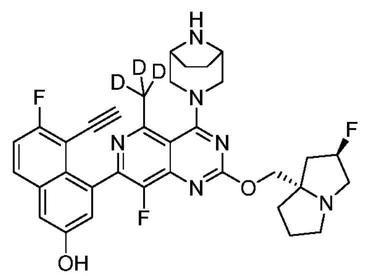 11,

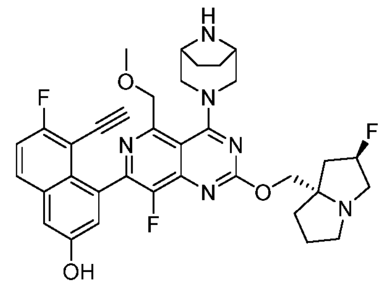 12,

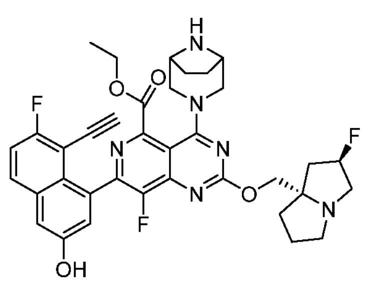 13,

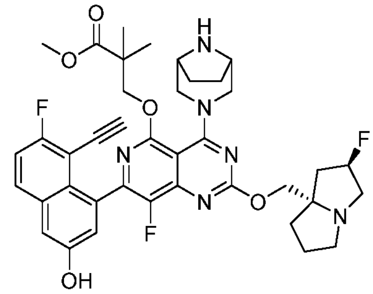 14,

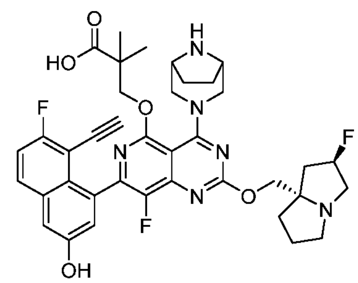 15,

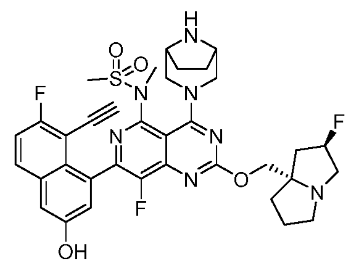 16,

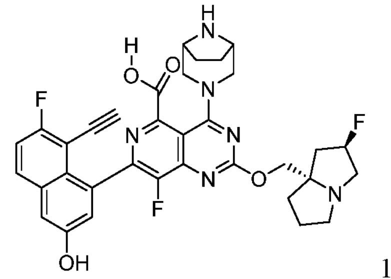 17,

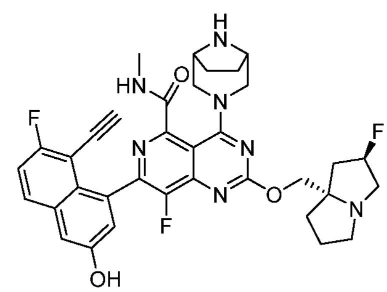 18,

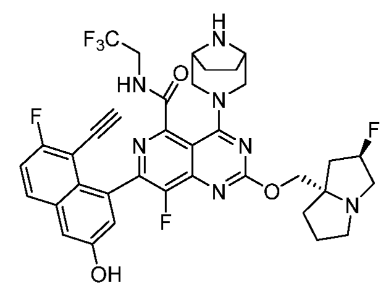 19,

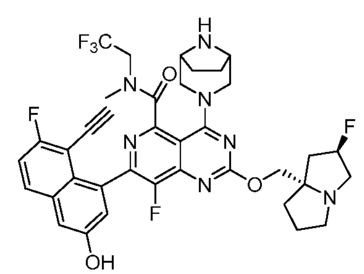 20,

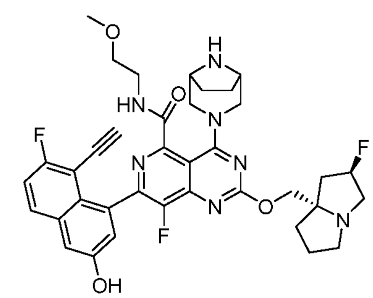 21,

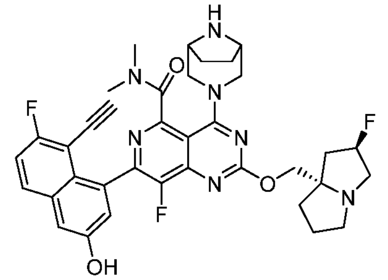 22,

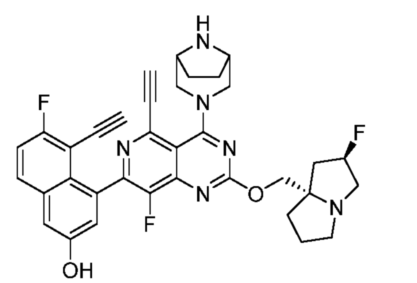 23,

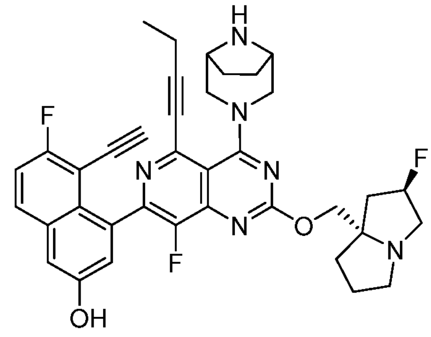 24, 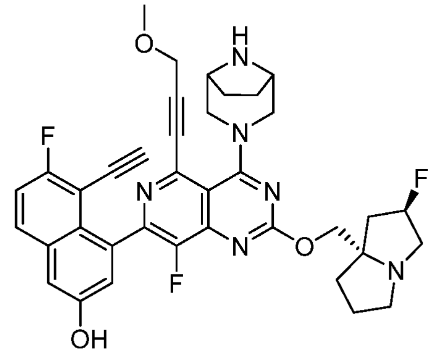 25,

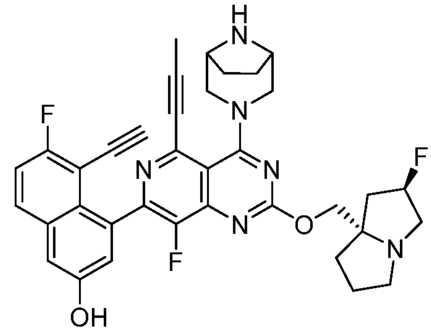 26, 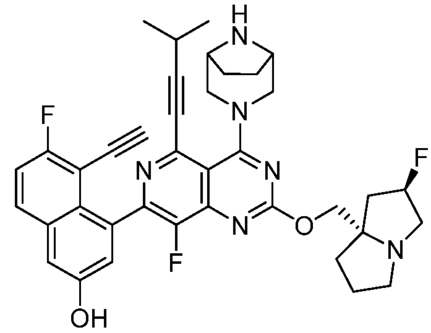 27,

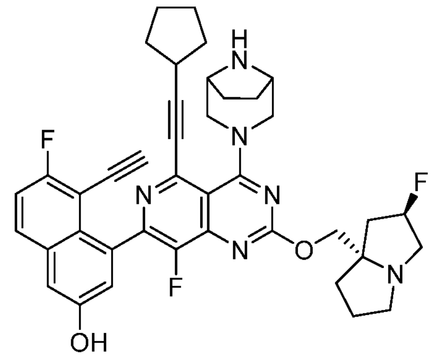 28, 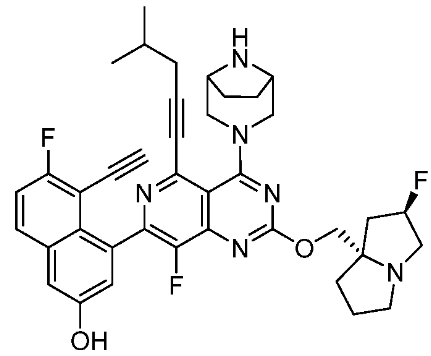 29,

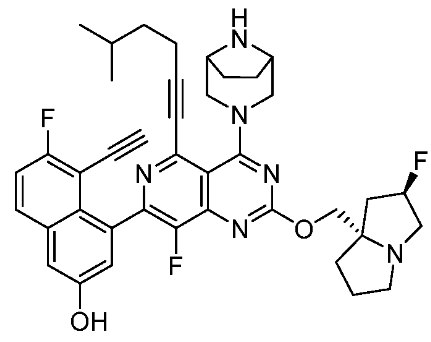 30, 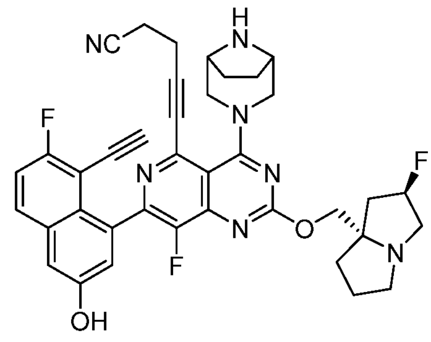 31,

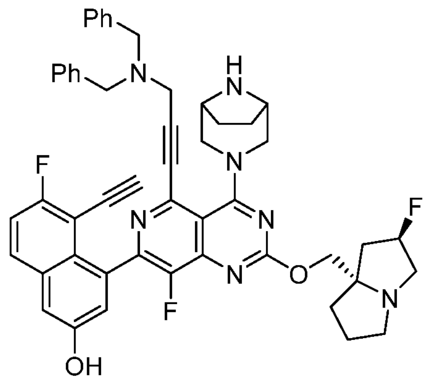
32,

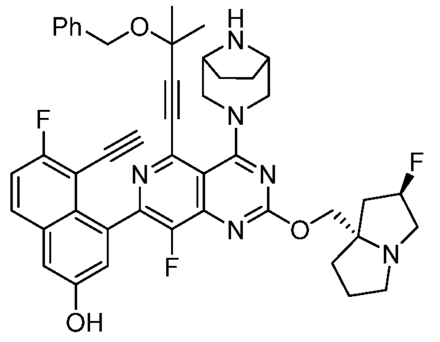
33,

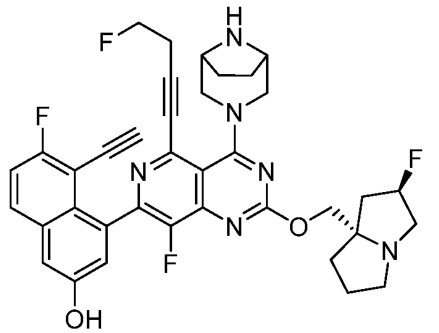
34,

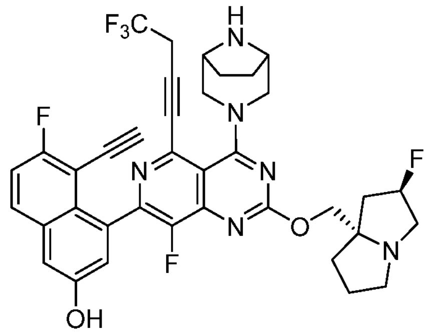
35,

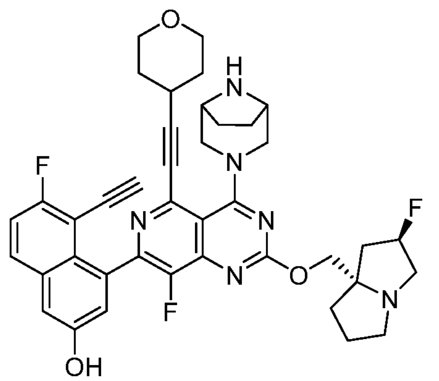
36,

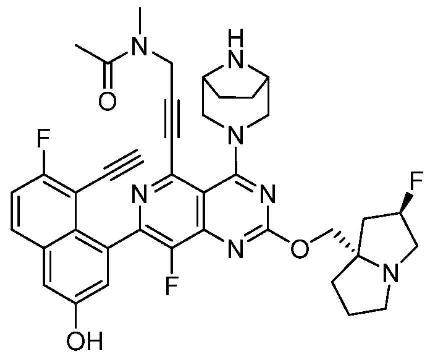
37,

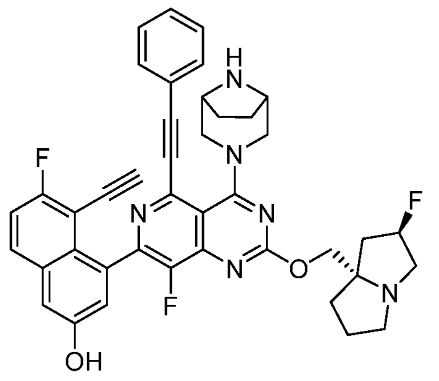
38,

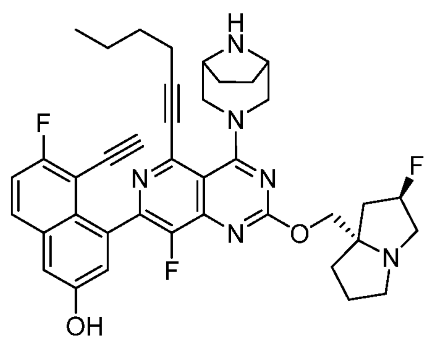
39,

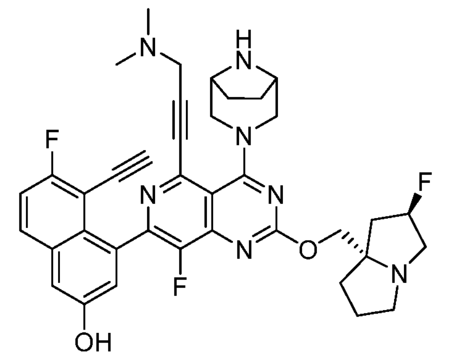
40,

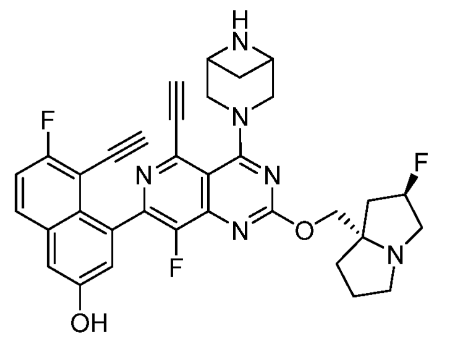
41,

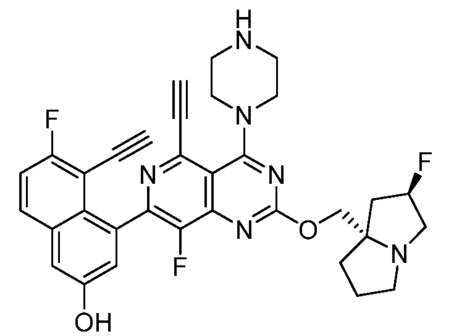
42,

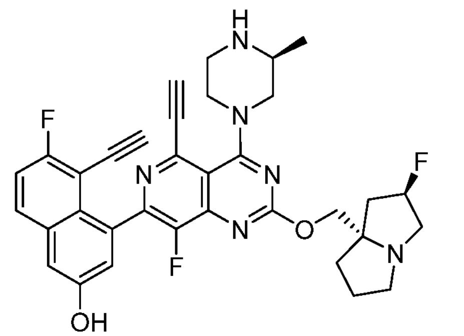
43,

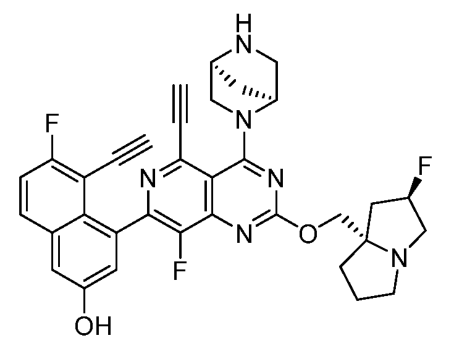
44,

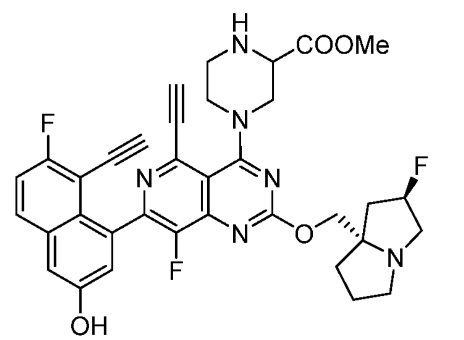
45,

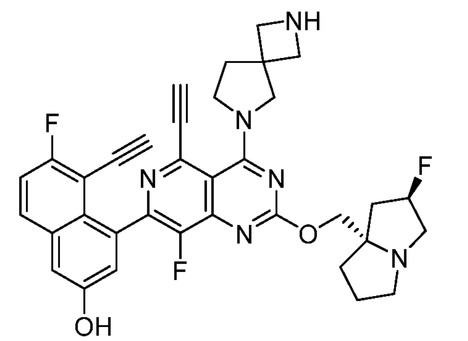
46,

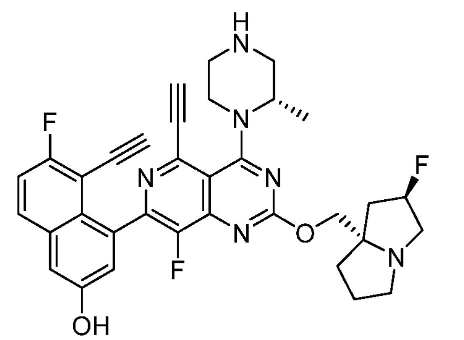
47,

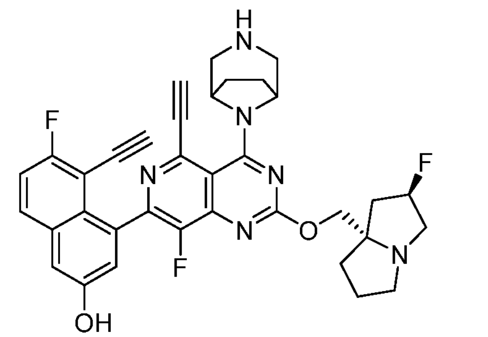

48,

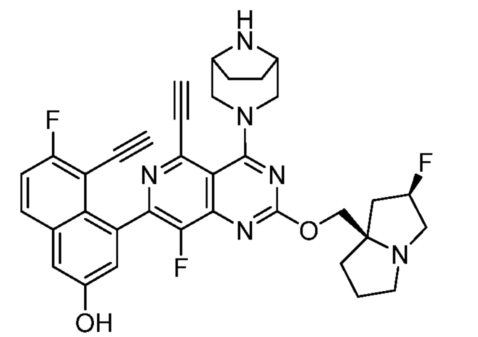

49,

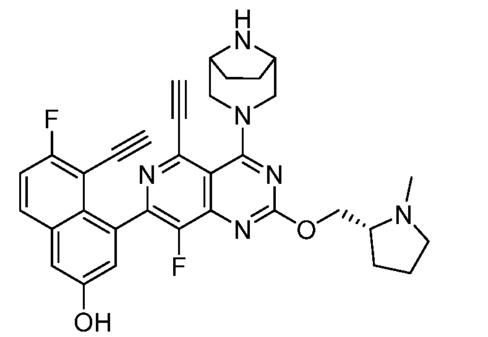

50,

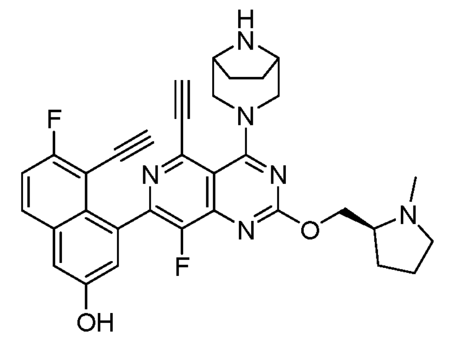

51,

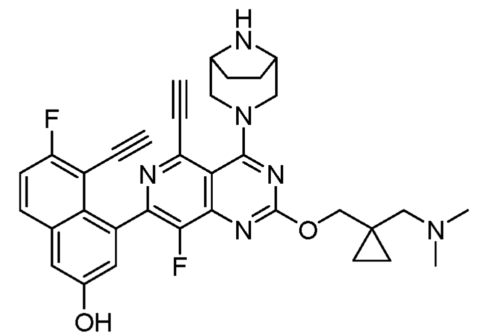

52,

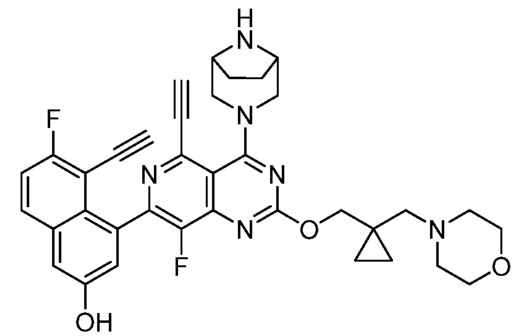

53,

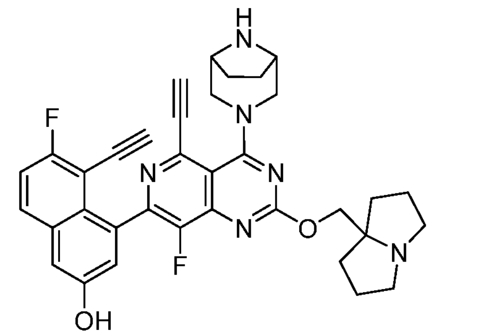

54,

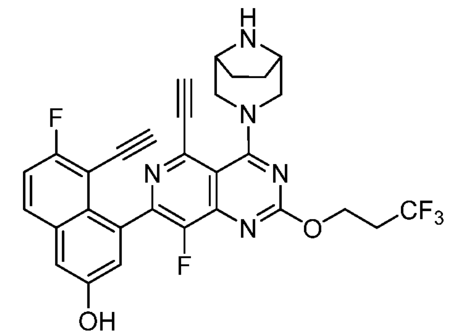

55,

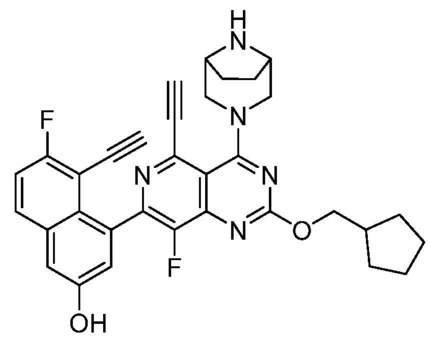

56,

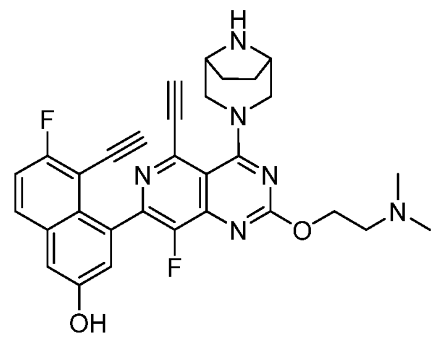

57,

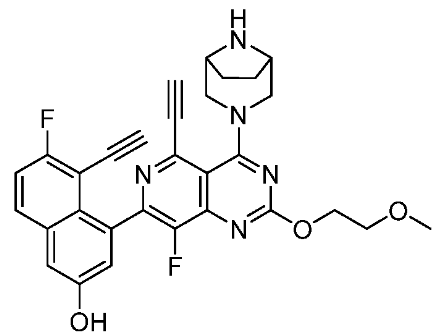

58,

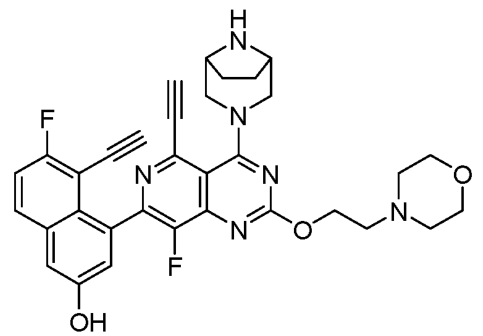

59,

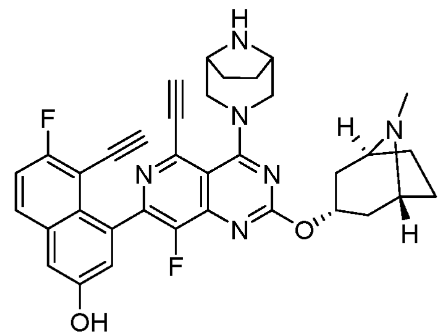

60,

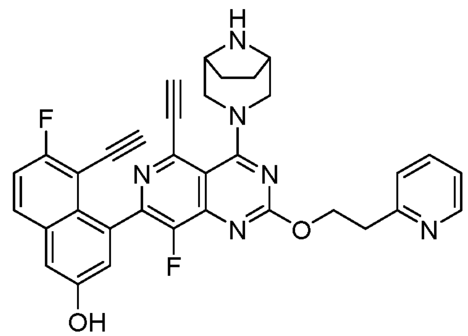

61,

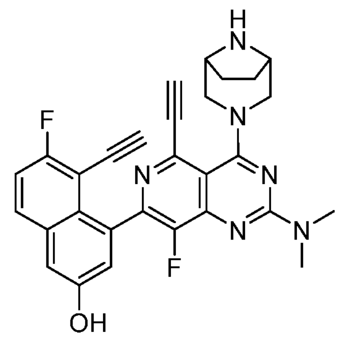

62,

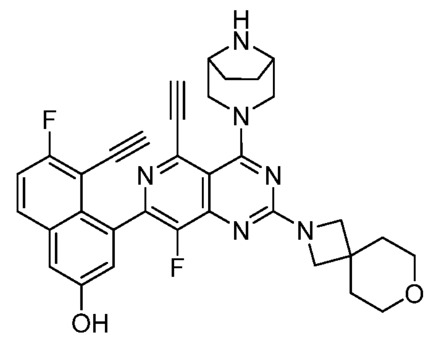

63,

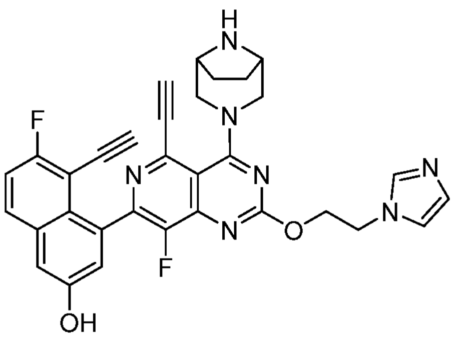
64,

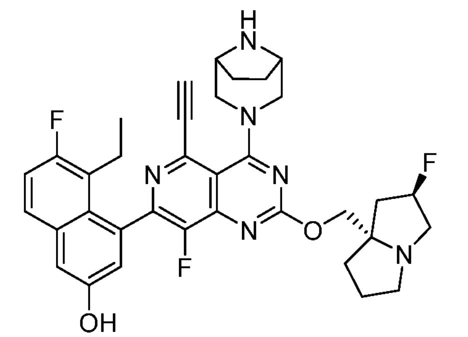
65,

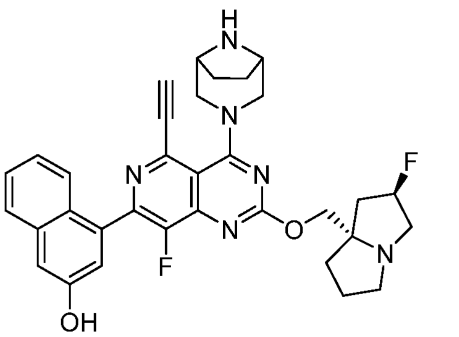
66,

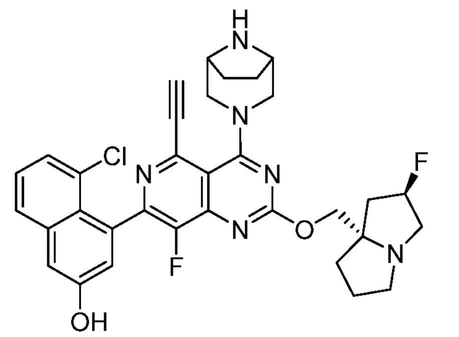
67,

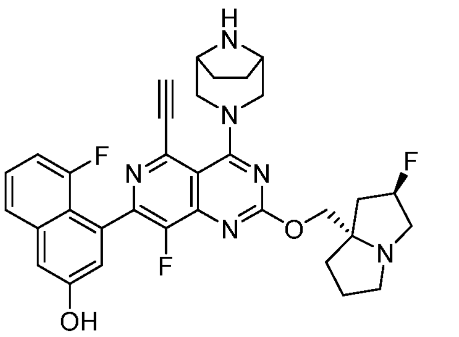
68,

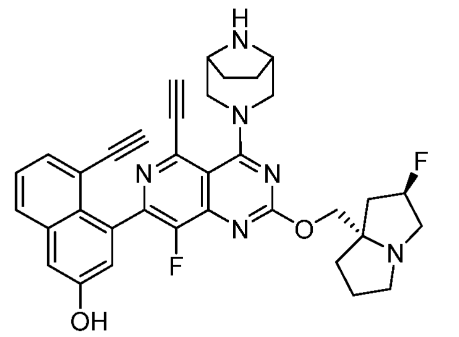
69,

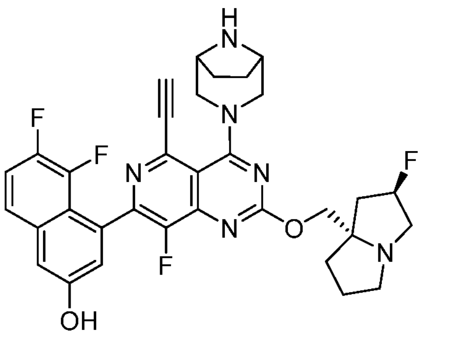
70,

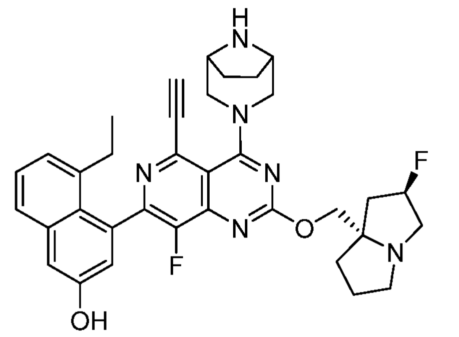
71,

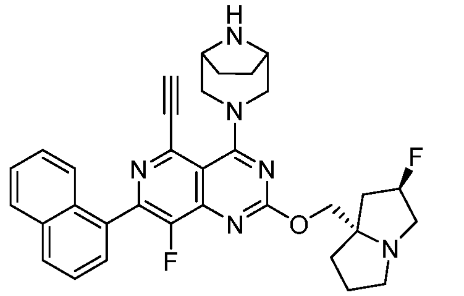
72,
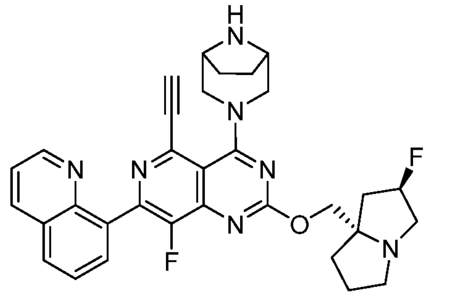
73,
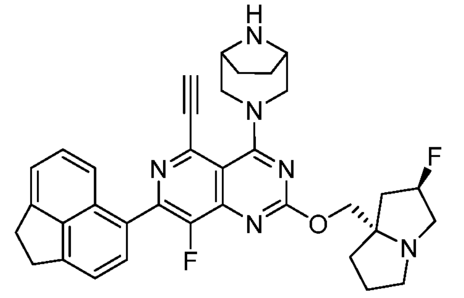
74,
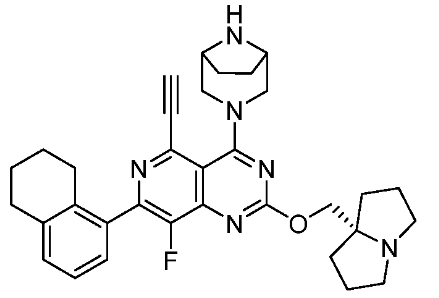
75,
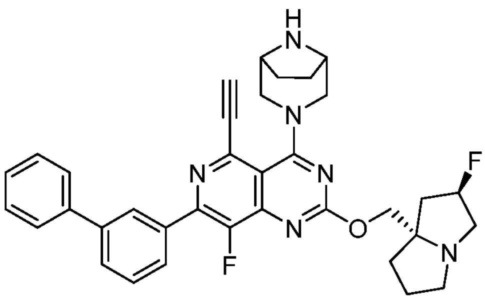
76,
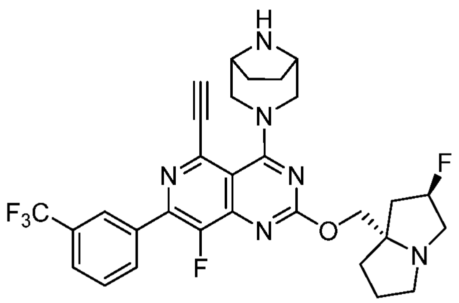
77,
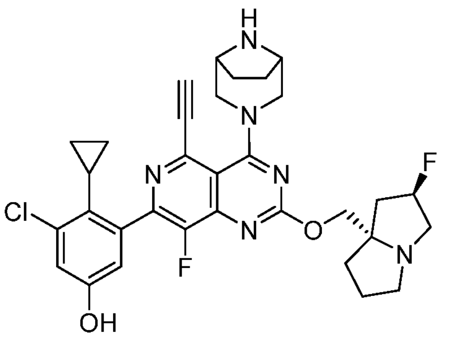
78,
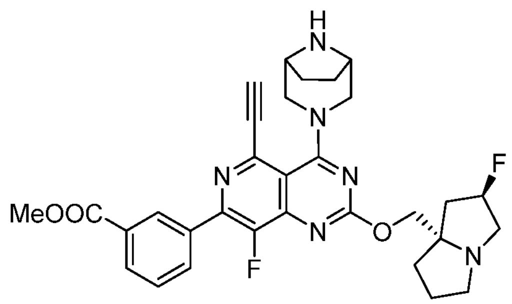
79,
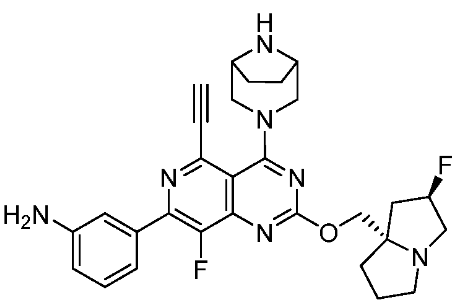
80,
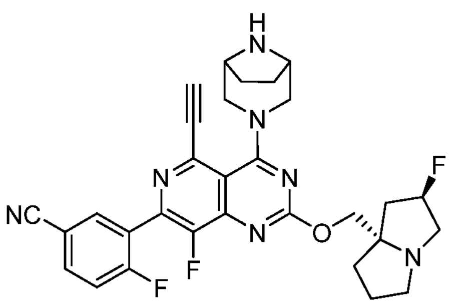
81,

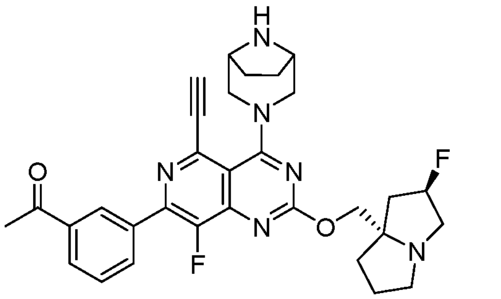
82,

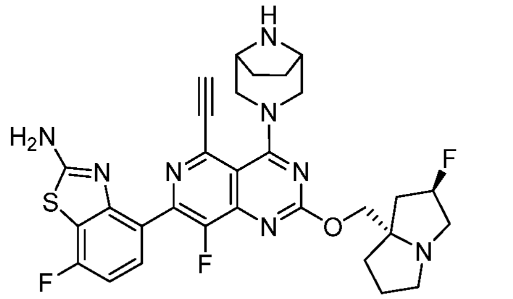
83,

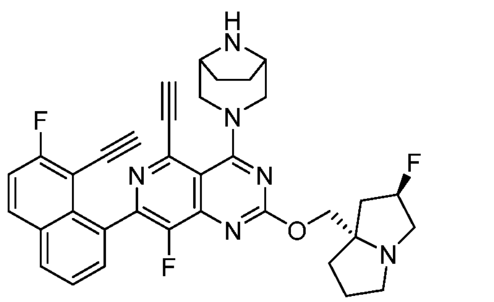
84,

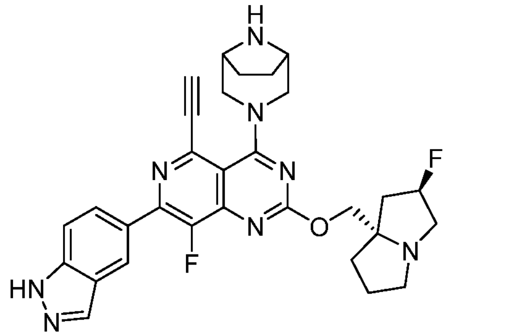
85,

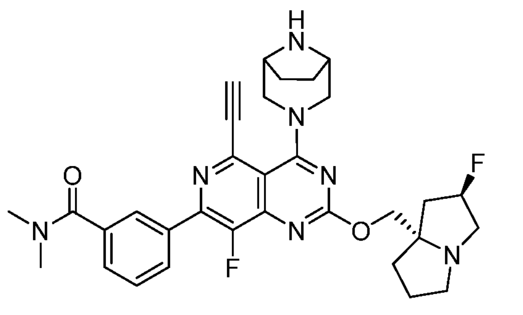
86,

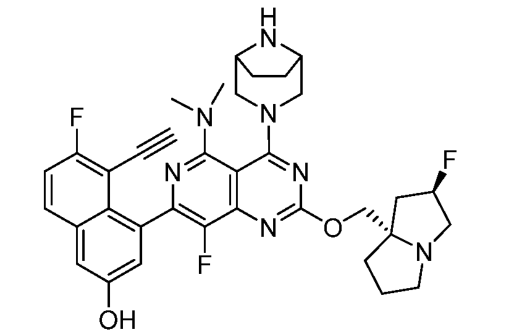
87,

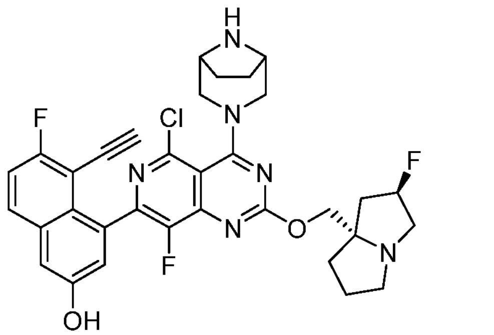
88,

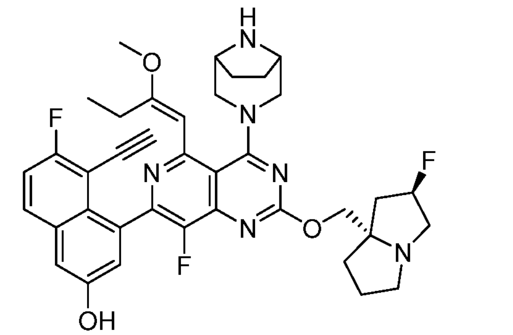
89,

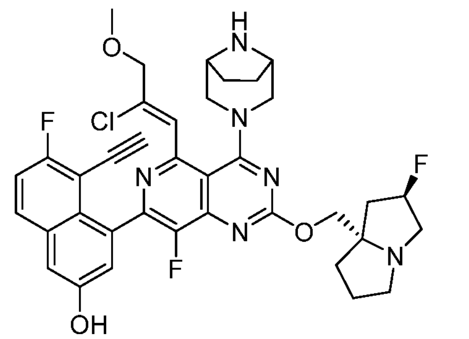

90,

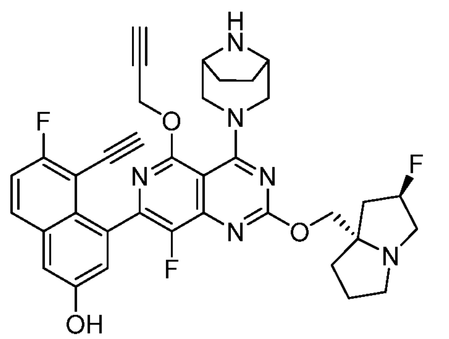

91,

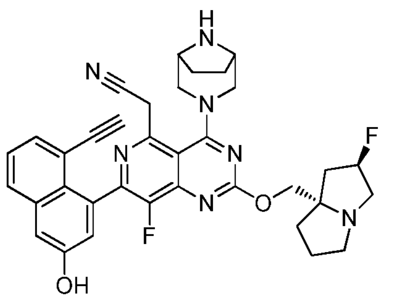

92,

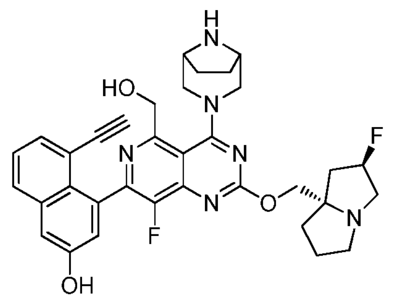

93,

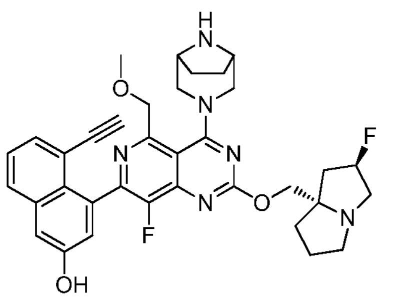

94,

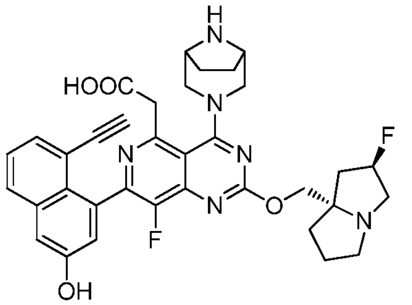

95,

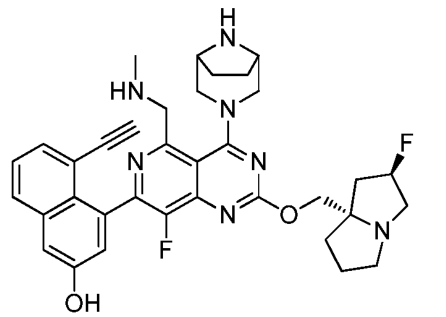

96,

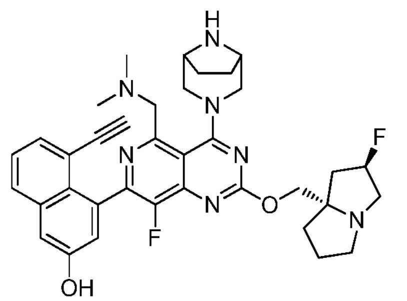

97,

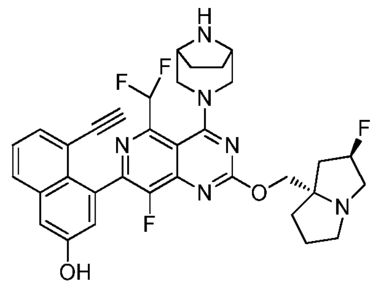

98,

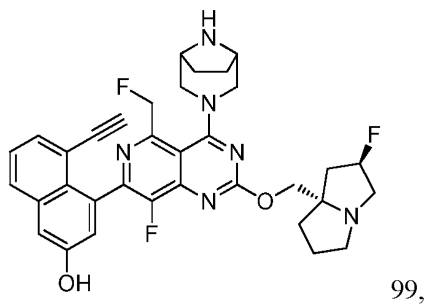

99,

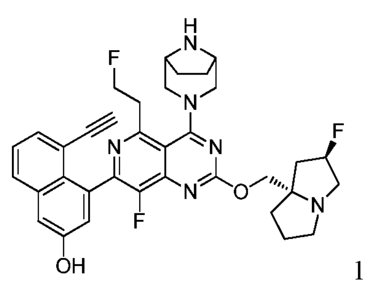

100,

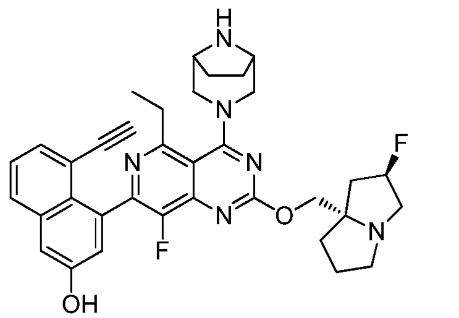 101, 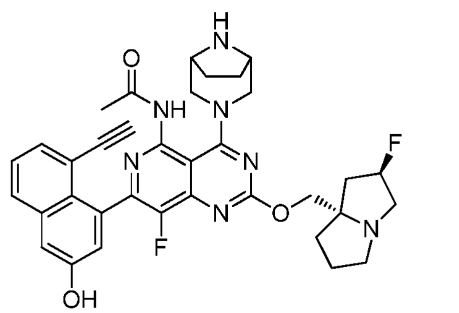 102,

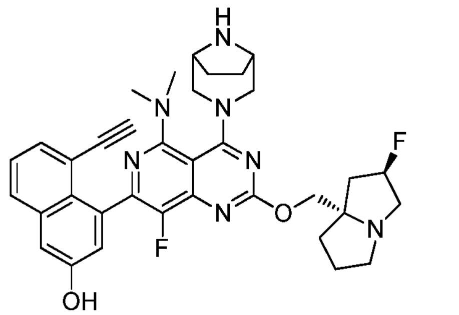 103, 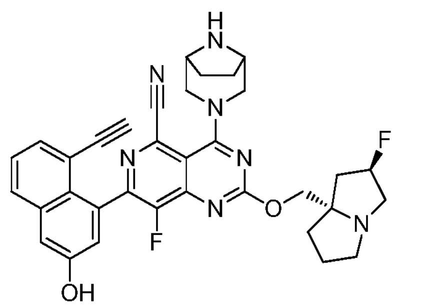 104,

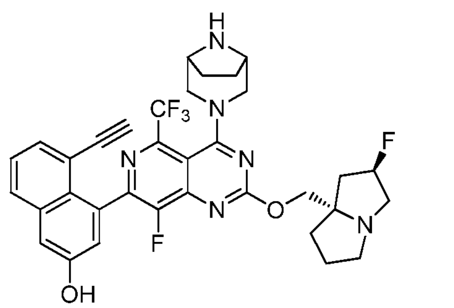 105, 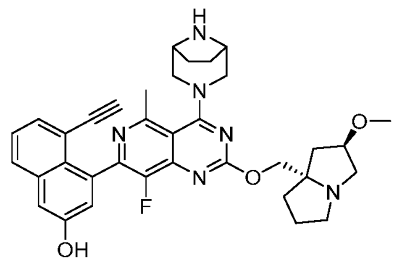 106,

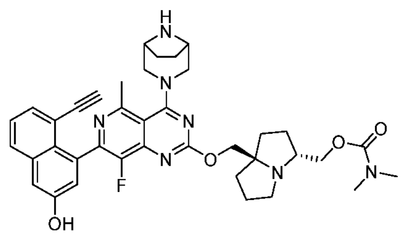 107, 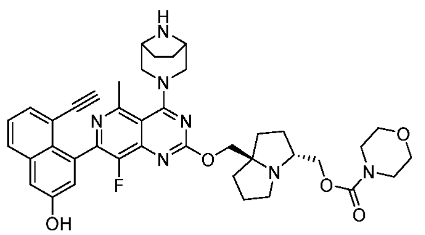 108,

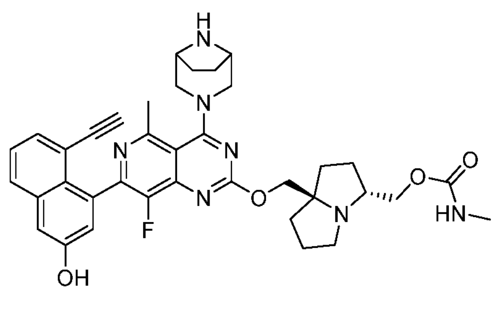 109, 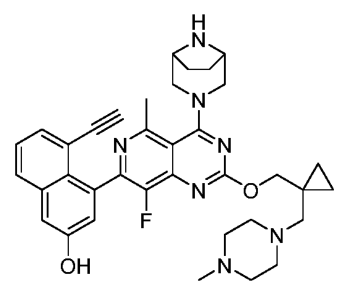 110,

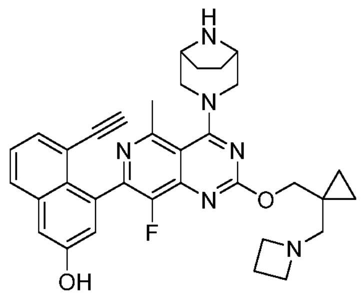 111, 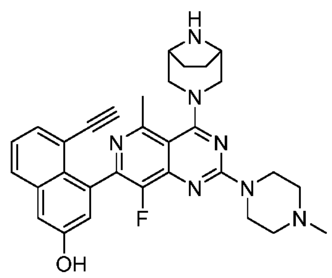 112, 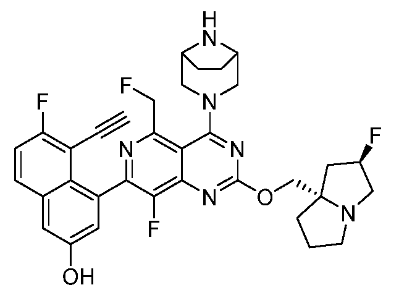 113,

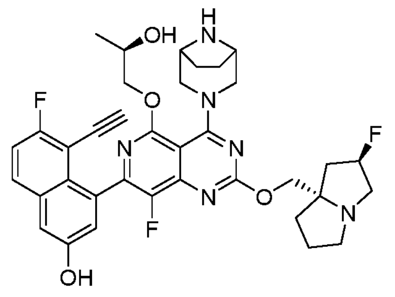 114, 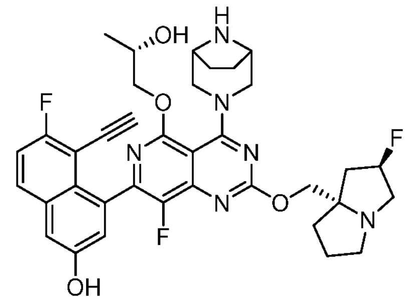 115,

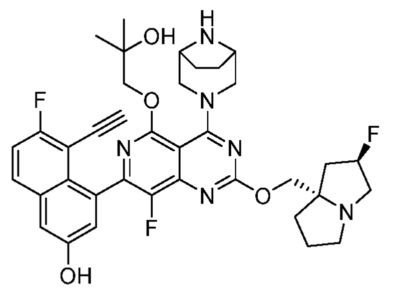 116, 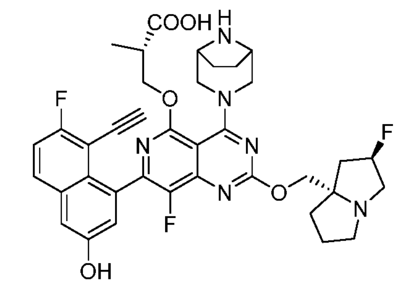 117,

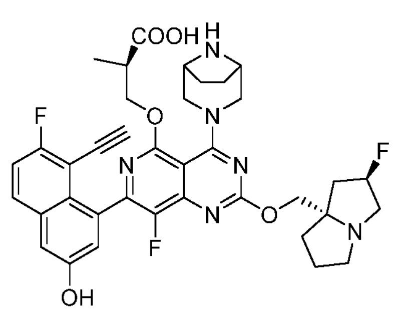 118, 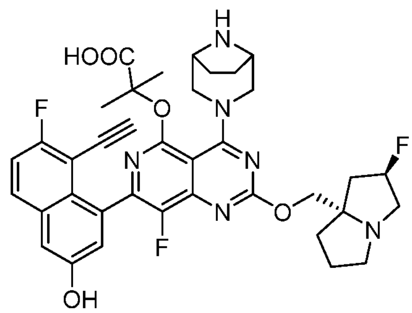 119,

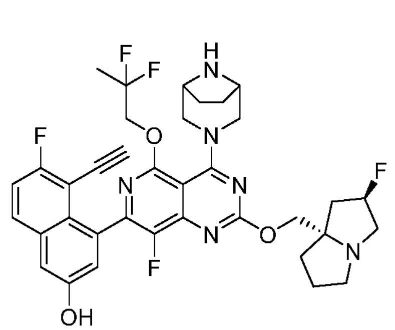 120, 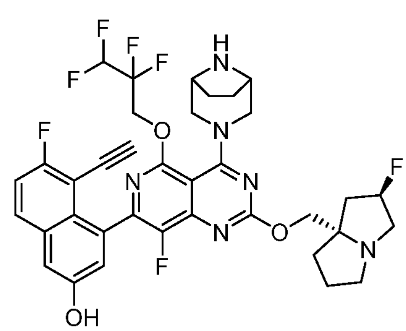 121,

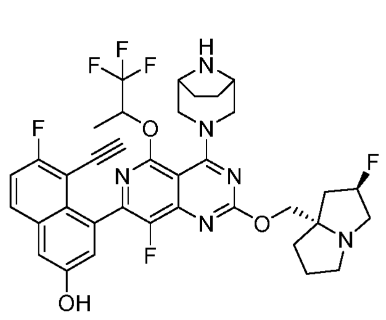

122,

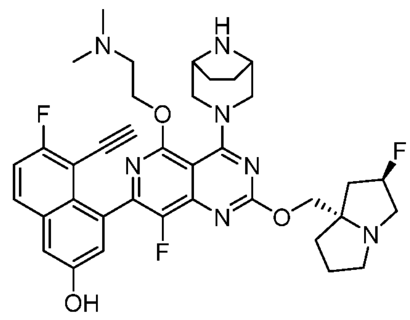

123,

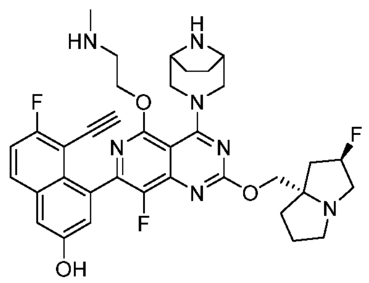

124,

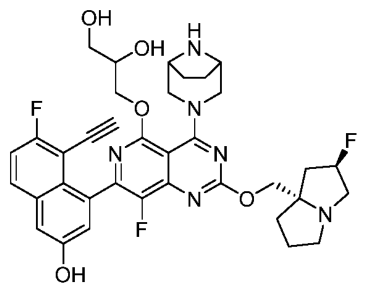

125,

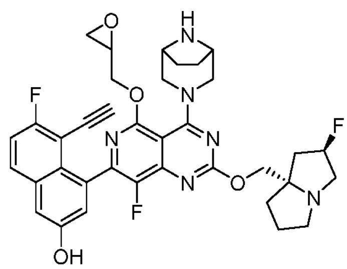

126,

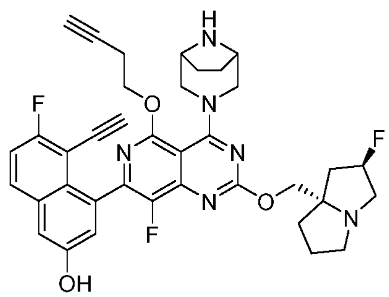

127,

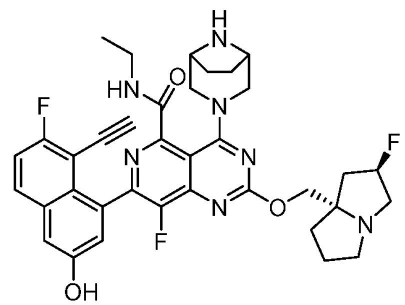

128,

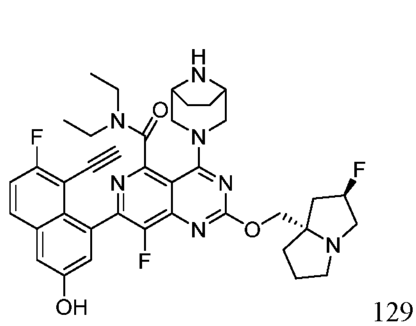

129 or

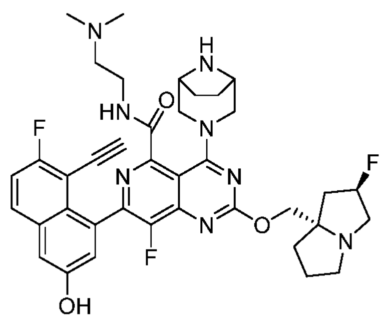

130.

In another aspect, the invention provides a pharmaceutical composition comprising the compounds provided herein.

In some embodiments, the pharmaceutical composition of the present invention further comprises pharmaceutically acceptable adjuvants.

In some embodiments, the adjuvants described herein include, but are not limited to, carriers, excipients, diluents, vehicles, or combinations thereof. In some embodiments, the form of the pharmaceutical composition may be liquid, solid, semi-solid, gel or spray.

In another aspect, the present invention provides use of the pharmaceutical composition disclosed herein in the manufacture of a medicament for preventing, treating or alleviating KRAS G12D related diseases.

In some embodiments, the KRAS G12D related disease is cancer.

In some embodiments, the compounds of the invention or pharmaceutical compositions thereof are effective in preventing, treating or alleviating cancer in patients, including, but not limited to: cardiac cancer: sarcoma (angiosarcoma, fibrosarcoma, rhabdomyosarcoma, liposarcoma ), myxoma, rhabdomyomas, fibromas, lipomas and teratomas; lung cancer: bronchial carcinoma (squamous cell, undifferentiated small cell, undifferentiated large cell, adenocarcinoma), non-small cell lung cancer, small cell lung cancer, alveolar (bronchioles) cancer, bronchial adenoma, sarcoma, lymphoma, enchondromatous hamartoma, mesothelioma; gastrointestinal cancer: esophagus (squamous cell carcinoma, adenocarcinoma, leiomyosarcoma, lymphoma) cancer, stomach (cancer, lymphoma, leiomyosarcoma) cancer, pancreas (ductal adenocarcinoma, insulinoma, glucagonoma, gastrinoma, carcinoid tumor, vipoma) cancer, small intestine (adenocarcinoma, lymphoma, carcinoid tumor, Kaposi's sarcoma, leiomyoma, hemangioma, lipoma, neurofibroma, fibroma), colon (adenocarcinoma, tubular adenoma, villous adenoma, hamartoma, leiomyoma) cancer, colorectal cancer; genitourinary tract cancers: kidney (adenocarcinoma, nephroblastoma (nephroblastoma), lymphoma, leukemia) cancer, bladder and urethra cancer (squamous cell carcinoma, transitional cell carcinoma, adenocarcinoma) , prostate (adenocarcinoma, sarcoma) cancer, testis (seminoma, teratoma, embryonal carcinoma, teratoma, choriocarcinoma, sarcoma, stromal cell carcinoma, fibroma, fibroadenoma, adenomatoid tumor , lipoma) cancer; liver cancer: liver cancer (hepatocellular carcinoma), cholangiocarcinoma, hepatoblastoma, angiosarcoma, hepatocellular adenoma, hemangioma; biliary tract cancer: gallbladder cancer, ampullary cancer, cholangiocarcinoma; bone cancer: osteosarcoma, fibrosarcoma, malignant fibrous histiocytoma, chondrosarcoma, Ewing's sarcoma, malignant lymphoma (reticulum cell sarcoma), multiple myeloma, malignant giant cell tumor, chordoma, osteochronfroma (osteochondral exostosema), benign enchondroma, chondroblastoma, chondromyxofibroma, osteoid osteoma and giant cell tumor; nervous system cancers: neuroma, skull (osteoma, hemangioma, granuloma, xanthomas tumors, osteitis deformans), meningeal cancers (meningiomas, meningiosarcomas, gliomas), brain cancers (astrocytoma, medulloblastoma, glioma, ependymoma, germ cell (pineal tumor), glioblastoma multiforme, oligodendroglioma, schwannoma, retinoblastoma, congenital tumors), spinal neurofibroma, meningioma, glioma , sarcoma); gynecological cancers: uterus (endometrial carcinoma (serous cystadenocarcinoma, mucinous cystadenocarcinoma, unclassified) carcinoma), granulosa cell tumor, Leydig cell tumor, dysgerminoma, malignant teratoma ), vulvar (squamous cell carcinoma, intraepithelial carcinoma, adenocarcinoma) cancer, fibrosarcoma, melanoma), vaginal cancer (clear cell carcinoma, squamous cell carcinoma, botryoid sarcoma (embryonal rhabdomyosarcoma), fallopian tube (carcinoma) , ovarian cancer, breast cancer; hematological cancers: leukemia (myeloid leukemia (acute and chronic), acute lymphoblastic leukemia, chronic lymphocytic leukemia, myeloproliferative leukemia) diseases, multiple myeloma, myelodysplastic syndromes) , Hodgkin's disease, non-Hodgkin's lymphoma (malignant lymphoma); skin cancers: melanoma, basal cell carcinoma, squamous cell carcinoma, Kaposi's sarcoma, nevus dysplasia, lipoma, hemangioma, skin Fibroids, keloids, psoriasis; and cancer of the adrenal gland: neuroblastoma.

In some embodiments, the cancer of the invention is non-small cell lung cancer, small cell lung cancer, colorectal cancer, rectal cancer, colon cancer, small intestine cancer, pancreatic cancer, uterine cancer, gastric cancer, esophageal cancer, prostate cancer, ovarian cancer, breast cancer, leukemia, melanoma, lymphoma or neuroma.

In another aspect, the present invention also provides a method for preventing or treating KRAS G12D related diseases, which includes administering to a patient a therapeutically effective amount of the compound or the pharmaceutical composition thereof disclosed herein.

In another aspect, provided herein are methods for preparing, separating, and purifying the compounds represented by Formula (I), (I-1), (I-2), (I-3) or (I-4).

Unless otherwise stated, all stereoisomers, tautomers, N oxides, hydrates, solvates, metabolites and pharmaceutically acceptable salts and prodrugs of the compounds disclosed herein are within the scope of the invention.

The phrase "pharmaceutically acceptable" refers to that the substance or composition must be compatible chemically and/or toxicologically, with the other ingredients comprising a formulation, and/or the mammal being treated therewith.

The salts of the compounds of the present invention also include salts of intermediates used in the preparation or purification of the compounds represented by formula (I), (I-1), (I-2), (I-3) or (I-4) or the separated enantiomers of the compounds represented by formula (I), (I-1), (I-2), (I-3) or (I-4), but not have to be a pharmaceutically acceptable salt.

## PHARMACEUTICAL COMPOSITIONS, FORMULATIONS, ADMINISTRATION AND USES OF THE COMPOUNDS OF THE PRESENT INVENTION

The characteristics of the pharmaceutical composition of the present invention include compounds represented by formula (I), (I-1), (I-2), (I-3) or (I-4), compounds of the present invention, or compounds of the embodiments, and pharmaceutically acceptable carriers. The pharmaceutical compositions of the present invention contain compounds in an amount effective to treat or alleviate KRAS G12D mediated disease in patients.

It will also be appreciated that certain of the compounds disclosed herein can exist in free form for treatment, or where appropriate, as a pharmaceutically acceptable derivative thereof. Some non-limiting examples of the pharmaceutically acceptable derivative include pharmaceutically acceptable prodrugs, salts, esters, salts of such esters, or any other adducts or derivatives which upon administration to a patient in need is capable of providing, directly or indirectly, a compound as otherwise described herein, or a metabolite or residue thereof.

As described herein, the pharmaceutically acceptable compositions of the present invention further comprise pharmaceutically acceptable adjuvants, which, as used in the present invention, include any solvents, diluents, or other liquid excipients, dispersants or suspending agent, surfactant, isotonicity agent, thickener, emulsifier, preservative, solid binder or lubricant, etc., suitable for the unique target dosage form. As described in the following: In Remington: Troy et al., Remington: The Science and Practice of Pharmacy, 21st ed., 2005, Lippincott Williams & Wilkins, Philadelphia, and Swarbrick et al., Encyclopedia of Pharmaceutical Technology, eds. 19881999, Marcel Dekker, New York, both of which are herein incorporated by reference in their entireties, discloses various carriers used in formulating pharmaceutically acceptable compositions and known techniques for the preparation thereof. Except insofar as any conventional adjuvants incompatible with the compounds disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable carriers include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants.

In preparing the pharmaceutical compositions provided by the invention, the active ingredients are usually mixed with excipients, diluted with excipients or enclosed in such vehicles in the form of, for example, capsules, sachets, paper or other containers. If the excipient is used as a diluent, it can be a solid, semi-solid or liquid material which serves as a vehicle, carrier or medium for the active ingredient. Suitable carriers include, but are not limited to, magnesium carbonate, magnesium stearate, talc, sugar, lactose, pectin, dextrin, starch, gelatin, tragacanth, methylcellulose, sodium carboxymethylcellulose, low melting point wax, cocoa butter, etc. Thus, the composition may be a tablet, pill, powder, lozenge, sachet, cachet, elixir, suspension, emulsion, solution, syrup, aerosol (in solid form or in a liquid medium), for example ointments, soft and hard gelatin capsules, suppositories, sterile injectable solutions and sterile packaged powders containing up to 10% by weight of the active compound. In one embodiment, the composition is formulated for oral administration. In one embodiment, the composition is formulated as a tablet or capsule.

The compounds or the pharmaceutical compositions of the present invention may be administered in the form of oral dosage forms such as tablets, capsules each of which includes a sustained-release or time-release formula, pills, powders, granules, elixirs, tinctures, suspensions, syrups and emulsifiers. They may also be administered intravenously (bolus or infusion), intraperitoneally, subcutaneously or intramuscularly, all using dosage forms well known to those of ordinary skill in the pharmaceutical arts. They can be administered alone, but will generally be administered together with a single pharmaceutical carrier based on the chosen mode of administration and standard pharmaceutical practice.

The compounds or the pharmaceutical compositions of the present invention may be administered intranasally, for topical use with a suitable intranasal vehicle, or transdermally by the use of a transdermal patch. When administered in the form of a transdermal delivery system, the dosage administered throughout the dosage is continuous rather than intermittent.

The compounds or the pharmaceutical compositions of the invention may also be administered in the form of liposomal delivery systems, such as small, monolayer, large, single-layered vesicles and multilamellar vesicles. Liposomes can be formed by different phospholipids, such as cholesterol, stearylamine, or phosphatidylcholine.

The compounds or the pharmaceutical compositions of the present invention are also conjugated to soluble polymers that serve as targeted drug carriers. Such polymers may encompass polyvinylpyrrolidone, pyran copolymer, polyhydroxypropylmethacrylamide-phenol, polyhydroxyethylaspartamide phenol, or polyethylene oxide-polylysine substituted with palmitoyl residues. Moreover, the compounds of the invention can be coupled to a class of biodegradable polymers, used to complete controlled release of drugs. For example, polylactic acid, polyglycolic acid, copolymers of polylactic acid and polyglycolic acid, polyepsilon caprolactone, polyhydroxybutyric acid, polyorthoesters, polyacetals, polydihydropyrans, polycyanoacrylates, and crosslinked or amphipathic blocking copolymers of hydrogels.

The dosage regimen of the compounds or the pharmaceutical compositions of this invention will vary with known factors such as the pharmacokinetic profile of the particular agent and its mode and route of administration; the race, age, sex, health status, medical condition and weight of the recipient; The nature and extent of symptoms; the

type of concurrent treatment; the frequency of treatment; the route of administration; the renal and hepatic function of the patient; and the effect desired. A physician or veterinarian can make a decision and prescribe an effective amount of medication to prevent, counteract or prevent the development of a thromboembolic disorder.

In accordance with the general guidelines, the daily oral dose of each active ingredient employed will range from about 0.001 to 1000 mg/kg of body weight, preferably from about 0.01 to 100 mg/kg of body weight, in order to achieve the indicated effect between. The compounds of the present invention may be administered once daily, or they may be administered in two, three or four times daily.

Each unit dose of dosage form (pharmaceutical composition) suitable for administration may contain from about 1 mg to about 100 mg of active ingredient. In these pharmaceutical compositions, the weight of the active ingredient will generally be about 0.5-95% of the total weight of the composition.

The compounds and compositions described herein can be administered alone or in combination with other compounds or other therapeutic agents. The compounds or compositions of the present invention may be administered simultaneously or sequentially with other therapeutic agents by the same or different routes of administration. The compounds of the present invention may be included in a single formulation with other therapeutic agents or in separate formulations.

When the compounds of the present invention are administered with other therapeutic agents, in general, in view of the additive or synergistic effect of the therapeutic agent at the time of co-administration, in each of the typical daily doses and typical dosage forms The amount of one component may be reduced relative to the usual dose when administered alone.

The compounds involved in the present invention or pharmaceutically acceptable salts or hydrates or pharmaceutical compositions thereof can be effectively used to prevent, treat or alleviate KRAS mediated diseases in patients, especially KRAS G12D mediated diseases, especially cancer.

In some embodiments, the compounds of the invention or pharmaceutical compositions thereof are effective in preventing, treating or alleviating cancer in patients, including, but not limited to: cardiac cancer: sarcoma (angiosarcoma, fibrosarcoma, rhabdomyosarcoma, liposarcoma ), myxoma, rhabdomyomas, fibromas, lipomas and teratomas; lung cancer: bronchial carcinoma (squamous cell, undifferentiated small cell, undifferentiated large cell, adenocarcinoma), non-small cell lung cancer, small cell lung cancer, alveolar (bronchioles) cancer, bronchial adenoma, sarcoma, lymphoma, enchondromatous hamartoma, mesothelioma; gastrointestinal cancer: esophagus (squamous cell carcinoma, adenocarcinoma, leiomyosarcoma, lymphoma) cancer, stomach (cancer, lymphoma, leiomyosarcoma) cancer, pancreas (ductal adenocarcinoma, insulinoma, glucagonoma, gastrinoma, carcinoid tumor, vipoma) cancer, small intestine (adenocarcinoma, lymphoma, carcinoid tumor, Kaposi's sarcoma, leiomyoma, hemangioma, lipoma, neurofibroma, fibroma), colon (adenocarcinoma, tubular adenoma, villous adenoma, hamartoma, leiomyoma) cancer, colorectal cancer; genitourinary tract cancers: kidney (adenocarcinoma, nephroblastoma (nephroblastoma), lymphoma, leukemia) cancer, bladder and urethra cancer (squamous cell carcinoma, transitional cell carcinoma, adenocarcinoma) , prostate (adenocarcinoma, sarcoma) cancer, testis (seminoma, teratoma, embryonal carcinoma, teratoma, choriocarcinoma, sarcoma, stromal cell carcinoma, fibroma, fibroadenoma, adenomatoid tumor, lipoma) cancer; liver cancer: liver cancer (hepatocellular carcinoma), cholangiocarcinoma, hepatoblastoma, angiosarcoma, hepatocellular adenoma, hemangioma; biliary tract cancer: gallbladder cancer, ampullary cancer, cholangiocarcinoma; bone cancer: osteosarcoma, fibrosarcoma, malignant fibrous histiocytoma, chondrosarcoma, Ewing's sarcoma, malignant lymphoma (reticulum cell sarcoma), multiple myeloma, malignant giant cell tumor, chordoma, osteochronfroma (osteochondral exostosema), benign enchondroma, chondroblastoma, chondromyxofibroma, osteoid osteoma and giant cell tumor; nervous system cancers: neuroma, skull (osteoma, hemangioma, granuloma, xanthomas tumors, osteitis deformans), meningeal cancers (meningiomas, meningiosarcomas, gliomas), brain cancers (astrocytoma, medulloblastoma, glioma, ependymoma, germ cell (pineal tumor), glioblastoma multiforme, oligodendroglioma, schwannoma, retinoblastoma, congenital tumors), spinal neurofibroma, meningioma, glioma, sarcoma); gynecological cancers: uterus (endometrial carcinoma (serous cystadenocarcinoma, mucinous cystadenocarcinoma, unclassified) carcinoma), granulosa cell tumor, Leydig cell tumor, dysgerminoma, malignant teratoma ), vulvar (squamous cell carcinoma, intraepithelial carcinoma, adenocarcinoma) cancer, fibrosarcoma, melanoma), vaginal cancer (clear cell carcinoma, squamous cell carcinoma, botryoid sarcoma (embryonal rhabdomyosarcoma), fallopian tube (carcinoma), ovarian cancer, breast cancer; hematological cancers: leukemia (myeloid leukemia (acute and chronic), acute lymphoblastic leukemia, chronic lymphocytic leukemia, myeloproliferative leukemia) diseases, multiple myeloma, myelodysplastic syndromes) , Hodgkin's disease, non-Hodgkin's lymphoma (malignant lymphoma); skin cancers: melanoma, basal cell carcinoma, squamous cell carcinoma, Kaposi's sarcoma, nevus dysplasia, lipoma, hemangioma, skin Fibroids, keloids, psoriasis; and cancer of the adrenal gland: neuroblastoma.

In particular, the compounds of the present invention or pharmaceutical compositions thereof can be effectively used to prevent, treat or alleviate cancer in patients, including non-small cell lung cancer, small cell lung cancer, colorectal cancer, rectal cancer, colon cancer, small intestine cancer, pancreatic cancer, uterus cancer, stomach cancer, esophageal cancer, prostate cancer, ovarian cancer, breast cancer, leukemia, melanoma, lymphoma or neuroma.

GENERAL SYNTHETIC PROCEDURES

In order to describe the present invention, the following examples will be used to further illustrate the technical solution of the present invention. The following examples are only used to illustrate the specific implementation methods of the present invention so that those skilled in the art can understand the present invention, but are not used to limit the protection scope of the present invention. In the specific implementation method of the present invention, technical means or methods that are not specifically described are conventional technical means or methods in the art.

Unless further stated, the substituents are defined as described in the present invention. The following non-limiting schemes and examples are presented to further exemplify the invention.

Persons skilled in the art will recognize that the chemical reactions described may be readily adapted to prepare other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius (°C), the room temperature in the examples represents 15 °C - 30 °C; in some embodiments, the room temperature is 20 °C - 30 °C. Reagents were purchased from commercial suppliers such as Aldrich Chemical Company, Arco Chemical Company and Alfa Chemical Company, and were used without further purification. Unless otherwise indicated, common solvents were purchased from commercial suppliers such as Shantou XiLong Chemical Factory, Guangdong Guanghua Reagent Chemical Factory Co. Ltd., Guangzhou Reagent Chemical Factory, Tianjin Hao YuYu Chemical Ltd., Tianjin Fuchen Chemical Reagent Factory, Wuhan Xinhuayuan Technology Development Co., Ltd., Qingdao Tenglong Reagent Chemical Ltd., and Qingdao Ocean Chemical Factory.

Anhydrous THF, dioxane, toluene, and ether were obtained by refluxing the solvent with sodium. Anhydrous $CH_2Cl_2$ and $CHCl_3$ were obtained by refluxing the solvent with $CaH_2$. EtOAc, PE, hexane, DMAC and DMF were treated with anhydrous $Na_2SO_4$ prior use.

The reactions set forth below were done generally under a positive pressure of nitrogen or argon or with a drying tube (unless otherwise stated) in anhydrous solvents, and the reaction bottles were typically fitted with rubber septa for the introduction of substrates and reagents via syringe. Glassware was oven dried and/or heat dried.

Column chromatography was conducted using a silica gel column. Silica gel (300-400 mesh) was purchased from Qingdao Ocean Chemical Factory.

$^1H$ NMR spectra were recorded using a Bruker 400 MHz, 600 MHz or 599 MHz nuclear magnetic resonance spectrometer. $^1H$ NMR spectra were obtained by using $CDCl_3$, DMSO-$d_6$, $CD_3OD$ or acetone-$d_6$ as solvent (reported in ppm), with TMS (0 ppm) or chloroform (7.26 ppm) as the reference standard. When peak multiplicities are reported, the following abbreviations are used: s (singlet), d (doublet), t (triplet), q (quartet), m (multiplet), br (broadened), br s (broadened singlet, br.s), dd (doublet of doublets), dt (doublet of triplets), qt (quartet of triplets).

Coupling constants J, when given, were reported in Hertz (Hz).

The measurement conditions for low-resolution mass spectrometry (MS) data were: Agilent 6120 quadrupole HPLC-MS, column model: Zorbax SB-C18, 2.1 × 30 mm, 3.5 micron, 6 min, flow rate was 0.6 mL/min. Mobile phase: 5% - 95% (0.1% formic acid in $CH_3CN$) in (0.1% formic acid in $H_2O$), by using electrospray ionization (ESI) at 210 nm/254 nm with UV detection.

Pure compounds were detected by using Agilent 1260 pre-HPLC or Calesep pump 250 pre-HPLC (column model: NOVASEP 50/80 mm DAC) at 210 nm/254 nm with UV detection.

The following abbreviations or English words are used throughout the specification:

| | | | |
|---|---|---|---|
| EtOH | Ethyl alcohol or ethanol | DIPEA | *N,N*-diisopropylethylamine |
| $I_2$ | Iodine | $POCl_3$ | Phosphorus oxychloride |
| $H_5IO_6$ | Periodic acid | toluene | Methylbenzene |
| CO | Carbon monoxide | OMOM | Methoxymethyloxy, $-OCH_2OCH_3$ |
| EtOAc, EA | Ethyl acetate | TIPS | Triisopropylsilyl |
| $PdCl_2(PPh_3)_2$, $Pd(PPh_3)_2Cl_2$ | Bistriphenylphosphine palladium dichloride | XPhos Pd G2 | Chloro(2-dicyclohexylphosphino-2',4',6'-triisopropyl-1,1'-biphenyl)[2-(2'-amino-1,1'-biphenyl)]palladium(II) |
| TEA, $Et_3N$ | Triethylamine | $K_3PO_4$ | Potassium phosphate |
| PE | Petroleum ether | ice-bath | Ice bath |
| THF | Tetrahydrofuran | CsF | Cesium fluoride |
| DMSO | Dimethylsulfoxide | EtzO | Ether |
| DMSO-$d_6$ | Dimethylsulfoxide-$d_6$ | DCE | 1,2-Dichloroethane |
| LDA | Lithium diisopropylamide | MTBE | Methyl tert-butyl ether |
| $Pd_2(dba)_3$ | Tris(dibenzylideneacetone)dipalladium | DCM | Dichloromethane |
| XantPhos | 9,9-Dimethyl-4,5-bis(diphenylphosphino) xanthene | DMF | *N,N*-dimethylfortnamide |
| dioxane | 1,4-Dioxane | Pre-TLC | Preparative thin layer chromatography |
| $CS_2CO_3$ | Cesium carbonate | h | Hour, hours |
| HCl/dioxane | Hydrogenchloride in 1,4-dioxane solution | min | Minute, minutes |
| MeCN | $CH_3CN$, Acetonitrile | MPa | Mega pascal |
| PTSA | *p*-Toluenesulfonic acid | °C | Degrees celsius |
| $Na_2SO_3$ | Sodium sulfate | g | Gram |
| $NaHCO_3$ | Sodium bicarbonate | mmol | Millimole |
| MeOH | $CH_3OH$, Methanol | mL | Milliliter |
| $NH_3$ | Ammonia | mg | Milligram |

| | | | |
|---|---|---|---|
| $NH_3$/MeOH,$NH_3$.MeOH | Methanol solution of ammonia | rt | Room temperature |
| $NH_4HCO_3$ | Ammonia bicarbonate | NIS | *N*-iodosuccinimide |
| M, mol/L | mol/liter | HTRF | Homogeneous time-resolved method fluorescence |
| $NH_2$Boc | *Tert*-butyl carbamate | | |
| *t*-BuBrettPhos | 2-Di-*t*-butylphosphino-2',4',6'-tri-i-propyl-3,6-dimethoxy-1,1'-biphenyl | *t*-BuBrettPhos G3 Pd | Methanesulfonato(2-(di-t-butylphosphino)-3,6-dimethoxy-2',4',6'-tri-i-propyl-1,1'-biphenyl)(2-amino-1,1'-biphenyl-2-yl)palladium(II) |
| MeCN $CH_3CN$, | Acetonitrile | $CD_3MgI$ | $d_3$-Methyl magnesium iodide |
| TFA, $CF_3COOH$ | Trifluoroacetate | AIBN | Azobisisobutyronitrile |
| DIAD | Diisopropyl azodicarboxylate | KOH | Potassium hydroxide |
| $PPh_3$ | Triphenylphosphine | LiOH | Lithium hydroxide |
| CsF | Cesium fluoride | HATU | 2-(7-aza-1*H*-benzotriazole-1-,3,3-tetramethyluroniumyl)-1,1hexafluorophosphate |
| $CD_3OD$ | Methyl alcohol-$d_4$ | DMAP | *N,N*-Dimethylformamide |
| THF/$H_2O$ | Mixed solution of tetrahydrofuran and water | TMSOTf | Trimethylsilyl triflate |
| DCM/MeOH/ammonia | Mixed solution of methylenechloride, methanol and ammonia | TMPMgCl-LiCl | 2,2,6,6-Tetramethylpiperidinylmagnesium chloride lithium chloride complex |
| BoczO | Di-*tert*-butyl dicarbonate | Boc | Tert-butoxycarbonyl |
| CuI | Cuprous iodide | TBAF | Tetrabutylammonium fluoride |
| XPhos Pd G3 | Methanesulfonato(2-dicyclohexylphosphino-2', 4',6'-tri-*i*-propyl-1,1'-biphenyl)(2'-amino-1,1 '-biphenyl-2-yl)palladium(II) | HRMS | High-resolution mass spectrometry |

Synthesis Scheme 1

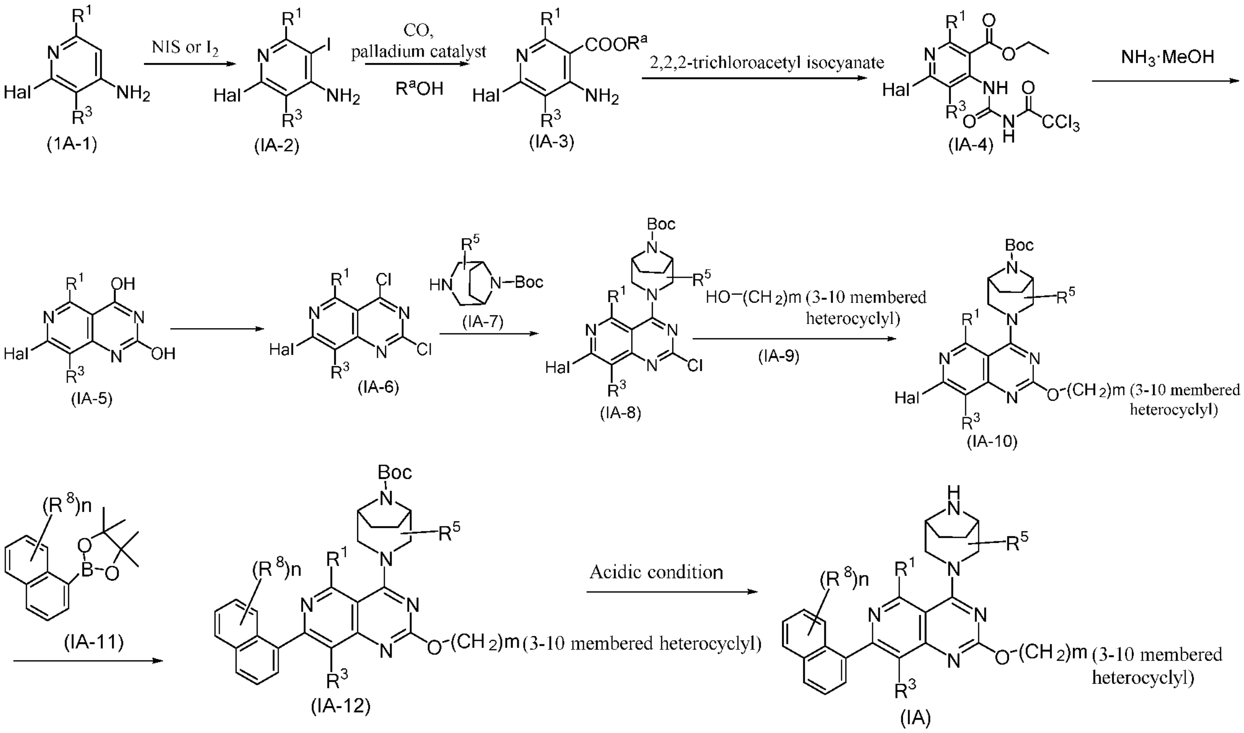

(1A-1) (IA-2) (IA-3) (IA-4) (IA-5) (IA-6) (IA-8) (IA-10) (IA-12) (IA)

Compound (IA) can be synthesized by referring to the method of Synthesis Scheme 1. Wherein $R^1$, $R^3$, $R^5$, $R^8$ and n have the definitions as described herein; Hal is halogen, preferably Cl or Br; $R^a$ is $C_{1-4}$ alkyl, preferably methyl or ethyl; m is a natural number from 1 to 6, preferably 1 or 2; 3-10 membered heterocyclyl has the definition as described herein, and can be optionally substituted by the substituents as described in the present invention. Compound (IA-1) reacts under the action of NIS or $I_2$ and under the conditions of suitable acid (such as periodic acid or *p*-toluenesulfonic acid) and suitable solvents (such as ethanol or acetonitrile) to obtain compound (IA-2); compound (IA-2) reacts with CO and alcohol $R^aOH$ (such as ethanol or methanol) under the action of a palladium catalyst (such as $Pd(PPh_3)_2Cl_2$) and under appropriate conditions (such as alkaline condition TEA) to obtain compound (IA-3); compound (IA-3) reacts with 2,2,2-trichloroacetyl isocyanate in a suitable solvent (such as tetrahydrofuran) to obtain compound (IA-4); compound (IA-4) reacts in a methanol solution of ammonia to obtain compound (IA-5); compound (IA-5) reacts with $POCl_3$ under appropriate conditions (such as heating) to obtain compound (IA-6); compound (IA-6) and compound (IA-7) react under appropriate conditions (such as the presence of DIPEA) to obtain compound (IA-8); compound (IA-8) and compound (IA-9) react under appropriate conditions (such as heating, under the action of DIPEA) to obtain compound (IA-10); compound (IA-10) and compound (IA-11) react under the action of a suitable catalyst (such as XPhos Pd G2) to obtain compound (IA-12); compound (IA-12) reacts under acidic conditions (such as HCl) to obtain compound (IA).

Synthesis Scheme 2

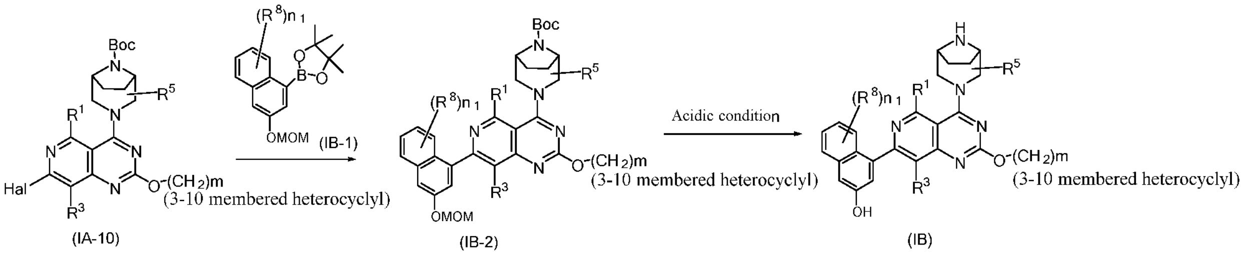

(IA-10) (IB-2) (IB)

Compound (IB) can be synthesized by referring to the method of Synthesis Scheme 2. Wherein $R^1$, $R^3$, $R^5$ and $R^8$ have the definitions as described herein; n1 is a natural number from 1 to 6; m is a natural number from 1 to 6, preferably 1 or 2; the 3-10 membered heterocyclyl has the definition as described herein, and can be optionally substituted by the substituents as described in the present invention. Compound (IA-10) and compound (IB-1) react under appropriate catalysts (such as XPhos Pd G2) to obtain compound (IB-2); compound (IB-2) reacts under acidic conditions (such as HCl) to obtain compound (IB).

Synthesis Scheme 3

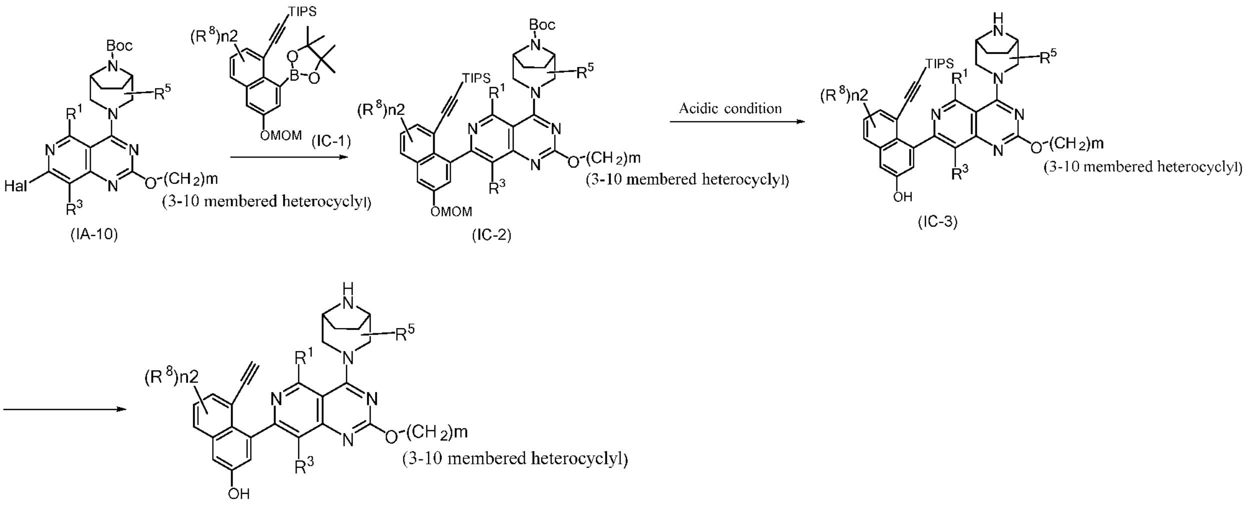


(IA-10) (IC-1) (IC-2) (IC-3)

(IC)

Compound (IC) can be synthesized by referring to the method in Synthesis Scheme 3. Wherein $R^1$, $R^3$, $R^5$ and $R^8$ have the definitions as described herein; n2 is a natural number from 1 to 5; m is a natural number from 1 to 6, preferably 1 or 2; the 3-10 membered heterocyclyl has the definition as described herein, and can be optionally substituted by the substituents as described in the present invention. Compound (IA-10) reacts with compound (IC-1) under the action of a suitable catalyst (such as XPhos Pd G2 or XPhos Pd G3) to obtain compound (IC-2); compound (IC-2) reacts under acidic conditions (such as HCl) to obtain compound (IC-3) or the acid addition salt of compound (IC-3); compound (IC-3) or the acid addition salt of compound (IC-3) reacts under appropriate conditions (such as under the action of CsF, in DMF solvent) to remove the TIPS group to obtain compound (IC).

Synthesis Scheme 4

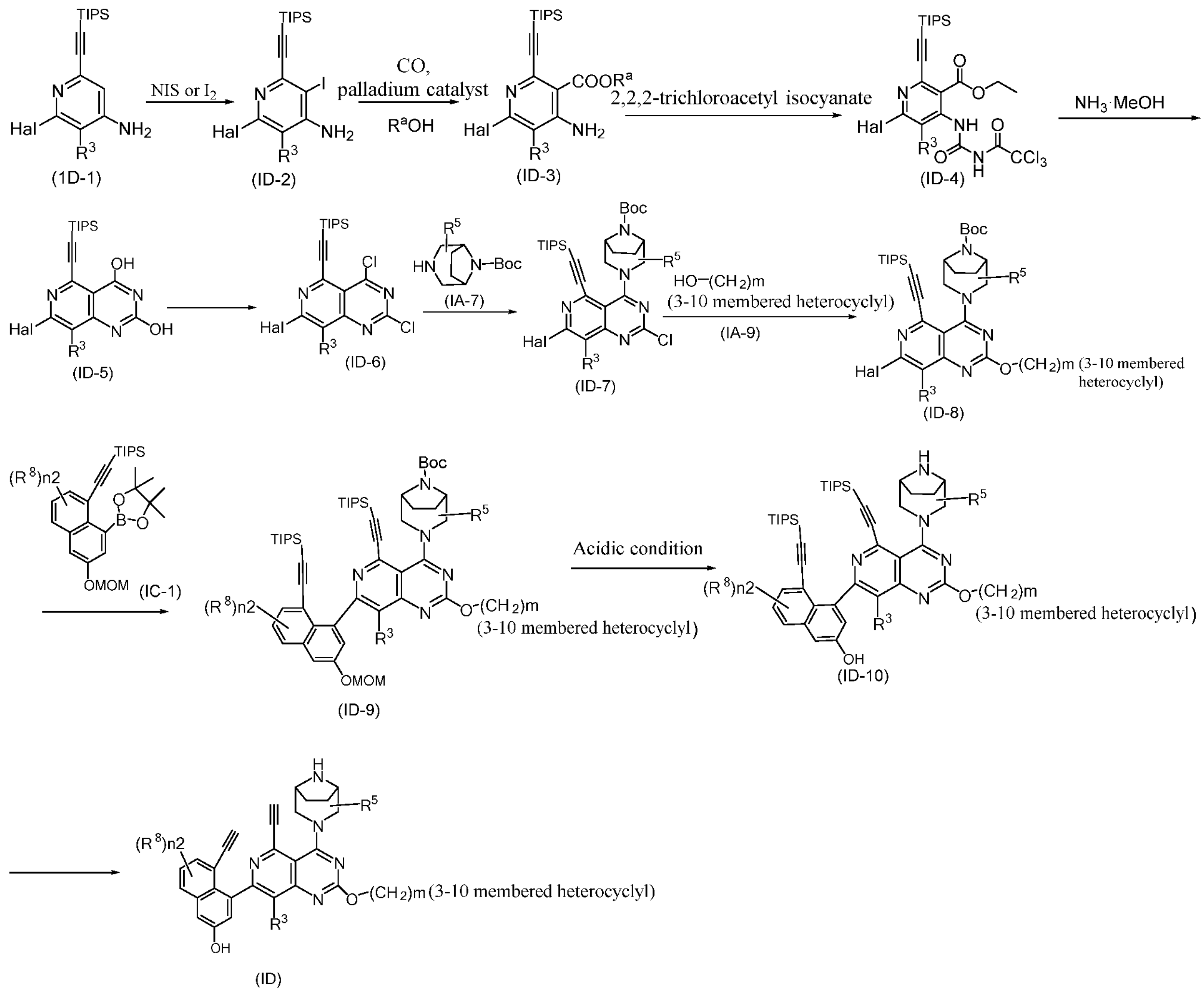


Compound (IA) can be synthesized by referring to the method in Synthesis Scheme 4. Wherein $R^3$, $R^5$ and $R^8$ have the definitions as described in the present invention; n2 is a natural number from 1 to 5; Hal is halogen, preferably Cl or Br; $R^a$ is $C_{1-4}$ alkyl, preferably methyl or ethyl; m is a natural number from 1 to 6 is, preferably 1 or 2; the 3-10 membered heterocyclyl has the definition as described herein, and can be optionally substituted by the substituents as described in the present invention. Compound (ID-1) reacts under the action of NIS or $I_2$ and under the conditions of suitable acid (such as periodic acid or *p*-toluenesulfonic acid) and suitable solvents (such as ethanol or acetonitrile) to obtain compound (ID-2); compound (ID-2) reacts with CO and alcohol $R^aOH$ (such as ethanol or methanol) under the action of a palladium catalyst (such as $Pd(PPh_3)_2Cl_2$) and under appropriate conditions (such as alkaline condition TEA) to obtain compound (ID-3); compound (ID-3) reacts with 2,2,2-trichloroacetyl isocyanate in a suitable solvent (such as tetrahydrofuran) to obtain compound (ID-4); compound (ID-4) reacts in a methanol solution of ammonia to obtain compound (ID-5); compound (ID-5) reacts with $POCl_3$ under appropriate conditions (such as heating) to obtain compound (ID-6); compound (ID-6) reacts with compound (IA-7) under appropriate conditions (such as the presence of DIPEA) to obtain compound (ID-7); compound (ID-7) and compound (IA-9) react under appropriate conditions (such as heating, in the presence of DIPEA) to obtain compound (ID-8); compound (ID-8) reacts with compound (IC-1) under the action of a suitable catalyst (such as XPhos Pd G2) to obtain compound (ID-9); compound (ID-9) reacts under acidic condition (such as HCl) to obtain compound (ID-10) or the acid addition salt of compound (ID-10); compound (ID-10) or the acid addition salt of compound (ID-10) reacts under appropriate conditions (such as the action of CsF, in DMF solvent) to remove the TIPS group to obtain compound (ID).

Synthesis Scheme 5

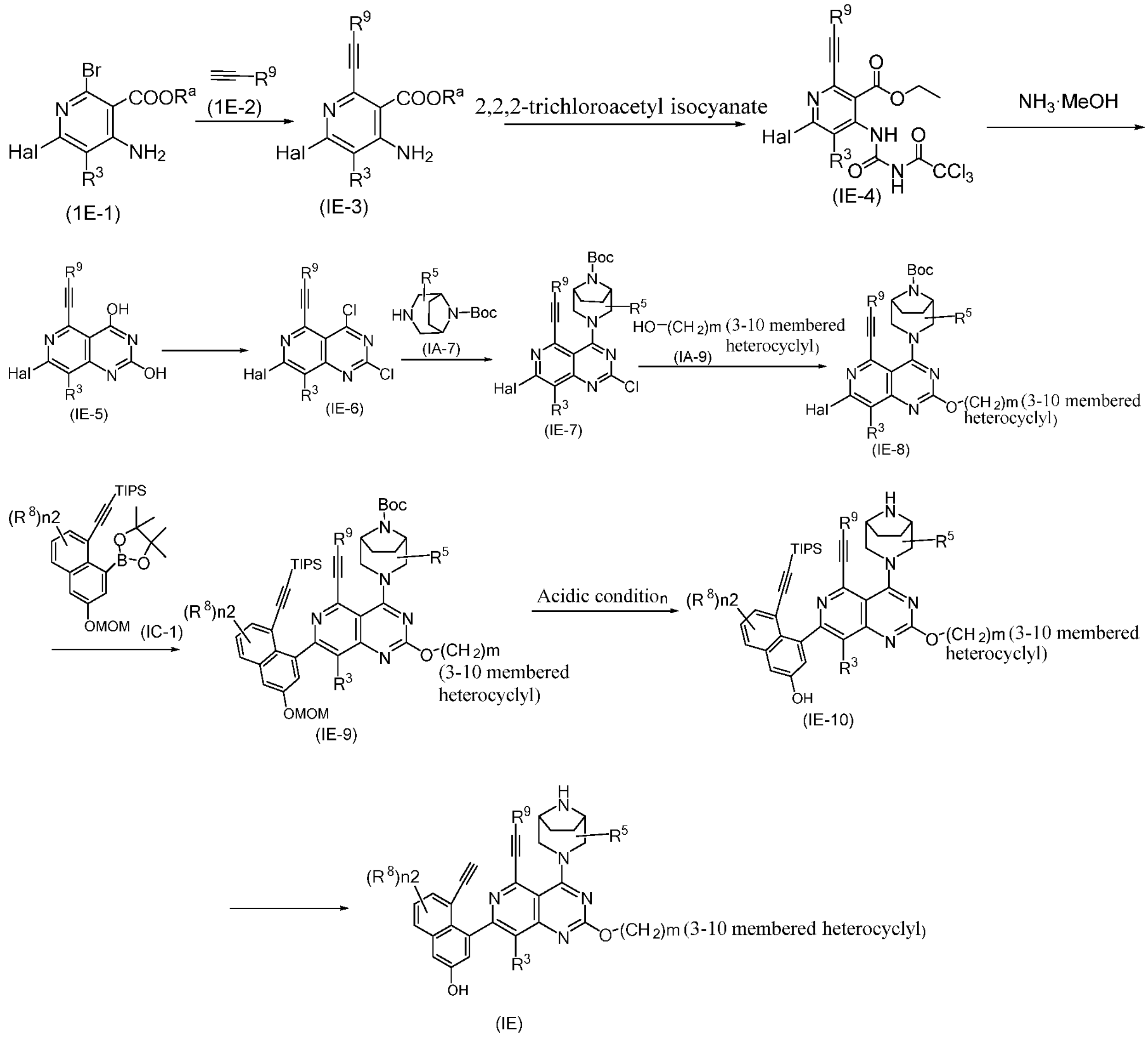


Compound (IE) can be synthesized by referring to the method of Synthesis Scheme 5. Wherein $R^3$, $R^5$, $R^8$ and $R^9$ have the definitions as described in the present invention; Hal is halogen, preferably Cl or Br; $R^a$ is $C_{1-4}$ alkyl, preferably methyl or ethyl; m is a natural number from 1 to 6, preferably 1 or 2; n2 is a natural number from 1 to 5; the 3-10 membered heterocyclyl has the definition as described herein, and can be optionally substituted by the substituents as described in the present invention. Compound (IE-1) and compound (IE-2) undergo a coupling reaction under appropriate conditions (such as under the action of $Pd(PPh_3)_2Cl_2$, CuI and TEA, in THF solvent) to obtain compound (IE-2); compound (IE-2) reacts with 2,2,2-trichloroacetyl isocyanate in a suitable solvent (such as tetrahydrofuran) to obtain compound (IE-4); compound (IE-4) reacts in a methanol solution of ammonia to obtain compound (IE-5); compound (IE-5) reacts with $POCl_3$ under appropriate conditions (such as heating) to obtain compound (IE-6); compound (IE-6) reacts with compound (IA-7) under appropriate conditions (such as the presence of DIPEA) to obtain compound (IE-7); compound (IE-7) reacts with compound (IA-9) under appropriate conditions (such as heating, in the presence of DIPEA) to obtain compound (IE-8); compound (IE-8) reacts with compound (IC-1) under the action of a suitable catalyst (such as XPhos Pd G3) to obtain compound (IE-9); compound (IE-9) reacts under acidic conditions (such as HCl or TMSOTf) to obtain compound (IE-10) or the acid addition salt of compound (IE-10); compound (IE-10) or the acid addition salt of compound (IE-10) reacts under appropriate conditions (such as the action of CsF, in DMF solvent) to remove the TIPS group to obtain compound (IE).

The compounds, pharmaceutical compositions and uses thereof provided by the present invention will be further described below in conjunction with the examples.

## EXAMPLE

### Synthesis of intermediate compound M1

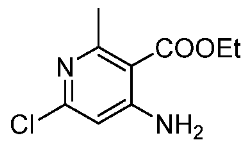

**M1**

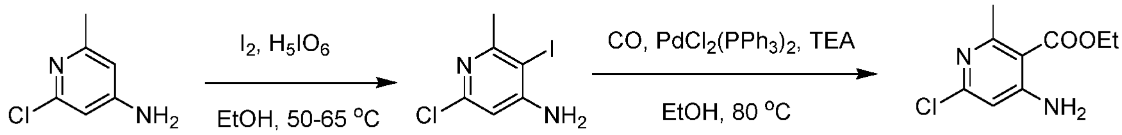


**M1-1** **M1-2** **M1**

Step 1: Synthesis of Compound **M1-2**

**M1-1** (2.50 g, 17.50 mmol) and ethanol (50 mL) were added to a 50 mL single-neck bottle, then periodic acid (1.20 g, 5.26 mmol) and $I_2$ (3.60 g, 14.2 mmol) were added, the mixture was heated to 50°C and stirred for 10 hours. The reaction solution was poured into water (30 mL), then saturated sodium sulfite (30 mL) was added, the mixture was stirred for 10 minutes, then extracted with EtOAc (50 mL), and washed with saturated brine (30 mL). The organic phase was dried over anhydrous sodium sulfate, filtered, and concentrated to obtain a residue, the residue was purified by column chromatography with PE/THF (v/v = 7.5/1) eluent, and concentrated to obtain a crude product, the crude product was purified again by column chromatography with PE/EtOAc (v/v=11/1) eluent, then 1.62 g of white solid was obtained with a yield of 34.40%. $^1H$ NMR (400MHz, DMSO-$d_6$): δ 6.46 (s, 1H), 6.41 (br s, 2H), 2.48 (s, 3H).

Step 2: Synthesis of Compound **M1**

**M1-2** (1.62 g, 6.03 mmol), triethylamine (3.40 mL, 24.00 mmol), ethanol (20 mL) and $Pd(PPh_3)_2Cl_2$ (0.45 g, 0.64 mmol) were added to a 250 mL autoclave in sequence, then the autoclave was replaced with $N_2$, and filled with CO (3 MPa), then the mixture was heated to 80 °C and stirred for 24 hours. The autoclave was cooled to room temperature, excess CO was slowly released, then the reaction solution was concentrated, and the remaining residue was directly purified by column chromatography (PE/EtOAc (v/v=11/1) to obtain 1.08 g of yellow solid, with a yield of 83.40%. LC-MS (ESI, pos. ion) m/z: 215.1 $[M+H]^+$; $^1H$ NMR (400MHz, DMSO-$d_6$): δ 6.92 (br s, 2H), 6.59 (s, 1H), 4.29 (q, J=7.2Hz, 2H), 2.40 (s, 3H), 1.29 (t, J=7.2Hz, 3H).

### Synthesis of intermediate compound M2

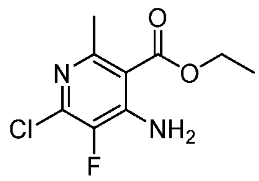

**M2**

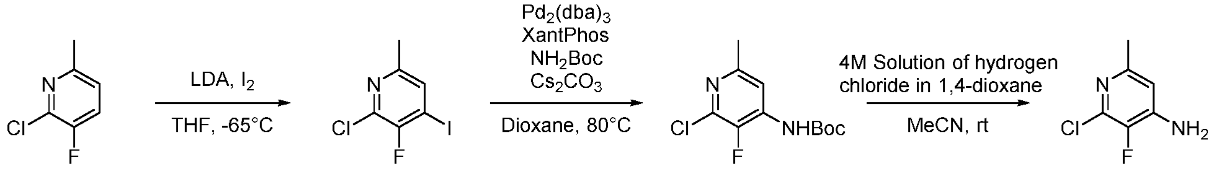


**M2-1** **M2-2** **M2-3** **M2-4**

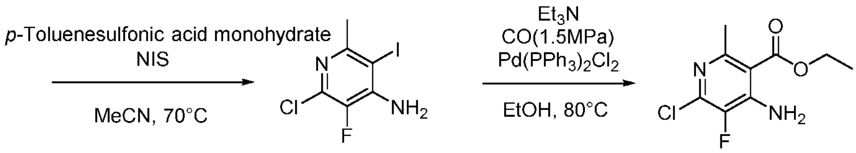


**M2-5** **M2**

Step 1: Synthesis of Compound **M2-2**

**M2-1** was added to a 250 mL three-neck bottle, then the bottle was replaced with $N_2$ three times, then anhydrous THF (50 mL) was added, the mixture was cooled to -65°C and a solution of LDA in THF was added slowly (7.36 g, 68.7 mmol, 2 mol/ L), the resulting mixture was stirred at -65 °C for 45 min, then a solution of iodine (17.44g, 68.7 mmol) in THF (50 mL) was added dropwise, and the mixture was stirred at -65 °C for 1 h. Then stirring was stopped, saturated ammonium chloride solution (50 mL) was added to quench the reaction, then separated and extracted with EA (100 mL × 3), the organic phase was washed with saturated sodium thiosulfate solution (100 mL) and saturated sodium chloride solution (80 mL) in sequence, then dried over anhydrous sodium sulfate and spin-dried to obtain 9.90 g of crude product, with a yield of 99%. $^1H$ NMR (400 MHz, $CDCl_3$): $\delta$ 7.48 (d, *J* = 3.6 Hz, 1H), 2.47 (s, 3H).

Step 2: Synthesis of Compound **M2-3**

**M2-2** (9.00 g, 33.15 mmol), $Pd_2(dba)_3$ (1.52 g, 1.66 mmol), XantPhos (1.92 g, 3.31 mmol), tert-butyl carbamate (4.66 g, 39.78 mmol) and cesium carbonate (27.00 g, 82.88 mmol) were added to a 250 mL three-neck bottle, then the bottle was replaced with $N_2$ three times, then anhydrous 1,4-dioxane (100 mL) was added, and the bottle was replaced with $N_2$ three times again, the resulting mixture was heated to 80°C and stirred for 2 h. After the reaction was stopped, the reaction solution was filtered through a celite pad, and concentrated, then water (100 mL) was added to dilute, extracted with EA (100 mL × 3), the combined organic phase was washed with saturated sodium chloride solution, dried over anhydrous sodium sulfate, concentrated, separated by column chromatography (PE/EA (v/v = 98/2) to obtain 6.00 g of yellow solid, with a yield of 69%. LC-MS (ESI, pos. ion) m/z:261.2 $[M+H]^+$; $^1H$ NMR (400 MHz, $CDCl_3$): $\delta$ 7.94 (d, *J* = 5.0 Hz, 1H), 6.91 (s, 1H), 2.46 (s, 3H), 1.54 (s, 9H).

Step 3: Synthesis of Compound **M2-4**

**M2-3** (5.00g, 19.18mmol) and acetonitrile (50 mL) were added to a 250 mL single-neck bottle, and then a solution of hydrogen chloride in 1,4-dioxane (38.36 mL, 153.44 mmol, 4 M) was added dropwise at 0°C, then the mixture was heated to room temperature and stirred for 2 h. Then stirring was stopped, the resulting mixture was concentrated and slurried by adding EA (20mL × 2), then filtered, the filter cake was dissolved by water (30 mL), saturated $NaHCO_3$ solution was added dropwise to pH = 8, then the mixture was extracted with EA (40 mL × 3), the combined organic phase was washed with saturated sodium chloride solution (50 mL), dried over anhydrous sodium sulfate, concentrated, and dried under vacuum to obtain 3.02 g of yellow solid, with a yield of 98%. LC-MS (ESI, pos. ion) m/z: 161.0 [M+H] ;$^1H$

NMR (400 MHz, $CDCl_3$): δ 6.44 (d, *J* = 5.8 Hz, 1H), 4.30 (s, 2H), 2.35 (s, 3H).

Step 4: Synthesis of Compound **M2-5**

**M2-4** (2.515 g, 15.66 mmol), NIS (4.22g, 18.76 mmol), acetonitrile (50 mL) and *p*-toluenesulfonic acid monohydrate (0.21 g, 1.10 mmol) were added to a 250 mL single-neck bottle, the mixture was heated to 70°C and stirred for 1 h. Then stop heating and stirring, the reaction solution was concentrated, EA (100 mL) was added to dissolve and water (20 mL) was added to dilute, and the organic phase was washed with saturated NaHCOs solution (100 mL), saturated $Na_2SO_3$ solution (100 mL) and saturated sodium chloride solution (100 mL) in sequence, then dried over anhydrous sodium sulfate, concentrated under vacuum to obtain 4.48 g of yellow solid, with a yield of 99%. LC-MS (ESI, pos. ion) m/z: 286.9[M+H]$^+$; $^1$H NMR (400 MHz, $CDCl_3$): δ 4.86 (s, 2H), 2.64 (d, *J* = 0.9 Hz, 3H).

Step 4: Synthesis of Compound **M2**

Compound **M2-5** (4.47 g, 15.60 mmol), $Pd(PPh_3)_2Cl_2$ (1.09 g, 1.56 mmol), EtOH (85 mL) and triethylamine (5.70 g, 56.34 mmol) were added to a 200 mL autoclave, the autoclave was replaced with $N_2$ and CO in sequence, then filled with CO to make the pressure inside the autoclave at 1.5 MPa, and then the mixture was heated to 80 °C and stirred overnight. After the reaction was stopped, the resulting mixture was cooled, filtered through a celite pad and concentrated, and eluted with column chromatography (PE/EA(v/v)=97/3) to obtain 3.38 g of yellow solid, with a yield of 93%. LC-MS (ESI, pos. ion) m/z: 233.0 [M+H]$^+$; $^1$H NMR (400 MHz, $CDCl_3$): δ 6.19 (s, 2H), 4.40 (q, *J* = 7.1 Hz, 2H), 2.64 (d, *J* = 0.8 Hz, 3H), 1.41 (t, *J* = 7.1 Hz, 3H).

**Synthesis of intermediate compound M6**

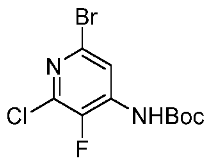

**M6**

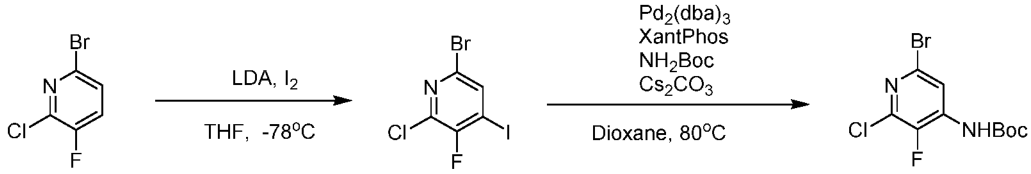


**M6-1** **M6-2** **M6**

Step 1: Synthesis of Compound **M6-2**

**M6-1** (503.0 mg, 2.39 mmol) was added to a 100 mL three-neck bottle, and the bottle was replaced with $N_2$ three times, then anhydrous THF (3 mL) was added and the mixture was cooled to -78°C, then a solution of LDA in THF (2.39 mL, 4.78mmol, 2 mol/L) was slowly added dropwise, then the mixture was stirred at -78 °C for 45 min, and a solution of iodine (1.22g, 4.78 mmol) in THF (3 mL) was added, and the mixture was stirred at -78 °C for 0.5 h. Then stirring was stopped, saturated ammonium chloride solution (10 mL) was added to quench the reaction, separated and extracted with EA (20 mL × 3), and the organic phase was washed with saturated sodium thiosulfate solution (20 mL) and saturated sodium chloride solution (40 mL) in sequence, then dried over anhydrous sodium sulfate, spin-dried to obtain 740.0 mg of crude product, with a yield of 92.1%. $^1$H NMR (400 MHz, $CDCl_3$): δ 7.82 (d, *J* = 3.5 Hz, 1H).

Step 2: Synthesis of Compound **M6**

**M6-2** (2.40 g, 7.14 mmol), $Pd_2(dba)_3$ (0.13 g, 0.14 mmol), XantPhos (0.21 g, 0.36 mmol), tert-butyl carbamate (1.00 g, 8.57 mmol) and cesium carbonate (5.82 g, 17.85 mmol) were added to a 250 mL three-neck bottle, then the bottle was replaced with $N_2$ three times, then anhydrous 1,4-dioxane (40 mL) was added, and the bottle was replaced with $N_2$ three times again, the resulting mixture was heated to 80°C and stirred for 2 h. After the reaction was stopped,

the reaction solution was filtered through a celite pad, and concentrated, then water (20 mL) was added to dilute, extracted with EA (50 mL × 3), the combined organic phase was washed with saturated sodium chloride solution, dried over anhydrous sodium sulfate, concentrated, separated by column chromatography (PE/EA (v/v = 98/2) to obtain 1.68 g of yellow solid, with a yield of 72.3%. LC-MS (ESI, neg. ion) m/z: 323.0 [M-H]; $^1H$ NMR (400 MHz, $CDCl_3$): δ 8.32 (d, *J* = 4.7 Hz, 1H), 6.98 (s, 1H), 1.54 (s, 9H).

**Synthesis of intermediate compound M7**

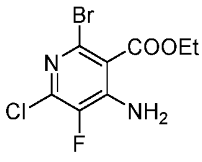

**M7**

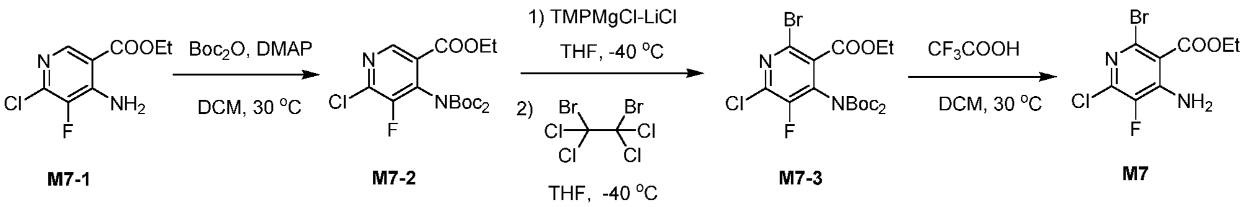


**M7-1** **M7-2** **M7-3** **M7**

Step 1: Synthesis of Compound **M7-2**

**M7-1** (10 g, 45.74 mmol), DMAP (1.12 g, 9.15 mmol) and DCM (200 mL) were added to a 1000 mL single-neck bottle, the mixture was stirred at room temperature, and then di-tert-butyl dicarbonate (21.96 g, 100.63 mmol) was added dropwise, then the resulting mixture was stirred at 30°C for 18 h. After the reaction was stopped, imidazole (3.11 g, 45.74 mmol) was added to the solution, the mixture was stirred for 0.5 h, then washed with saturated ammonium chloride solution (100 mL × 3) and separated, and the organic phase was washed with saturated sodium chloride solution (100 mL × 2), the combined organic phase was dried over anhydrous sodium sulfate, and concentrated to obtain 17.4 g of yellow product with a yield of 90.82%, which was directly used for the next step. $^1H$ NMR (599 MHz, $CDCl_3$) δ 8.79 (s, 1H), 4.37 (q, *J* = 7.1 Hz, 2H), 1.41 (s, 18H), 1.38 (t, *J*= 7.1 Hz, 3H).

Step 2: Synthesis of Compound **M7-3**

**M7-2** (10 g, 23.88 mmol) and anhydrous THF (100 mL) were added to a 500 mL three-neck bottle, the mixture was stirred and cooled to -40°C, then the solution of TMPMgCl-LiCl in THF (35.82 mL, 35.82 mmol, 1 M) was added dropwise at -40 °C. The reaction solution was continuously stirred at -40°C for 4 h, and a solution of diabrotetlchloroethaline (9.33g, 28.66 mmol) in THF (30 mL) was added dropwise. Then the mixture was continuously stirred at -40°C for 4 h. After the reaction was stopped, 100 mL saturated ammonium chloride solution was added to quench the reaction, and then extracted with EA (100 mL × 2). The combined organic phase was dried over anhydrous sodium sulfate and concentrated, and purified by column chromatography with PE/DCM (v/v = 100/1-1/1) eluent to obtain 8.5 g of yellow solid with a yield of 71.5%. $^1H$ NMR (599 MHz, $CDCl_3$): δ 4.42 - 4.38 (q, *J* = 7.2 Hz, 2H), 1.42 (s, 18H), 1.37 (t, *J* = 7.2 Hz, 3H).

Step 3: Synthesis of Compound **M7**

**M7-3** (5 g, 10.05 mmol), DCM (50 mL) and TFA (14.97 mL, 200.1 mmol) were added to a 250 mL three-neck bottle, and the mixture was stirred at 30°C for 5 h. After the reaction was stopped, saturated sodium carbonate solution was added to the mixture until the pH is neutral, and then extracted with DCM (30 mL × 3). The combined organic phase was dried over anhydrous sodium sulfate, and concentrated to obtain 2.9 g of light brown solid with a yield of 97%. LC-MS (ESI, pos. ion) m/z: 297.1 $[M+H]^+$; $^1H$ NMR (599 MHz, $CDCl_3$): δ 6.07 (s, 2H), 4.41 (q, *J* = 7.1 Hz, 2H), 1.41 (t, *J* = 7.1 Hz, 3H).

**Synthesis of intermediate compound M8**

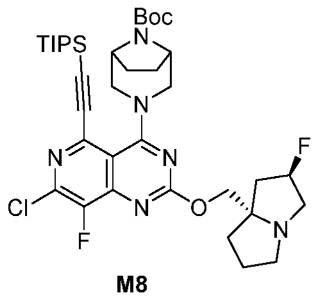

**M8**

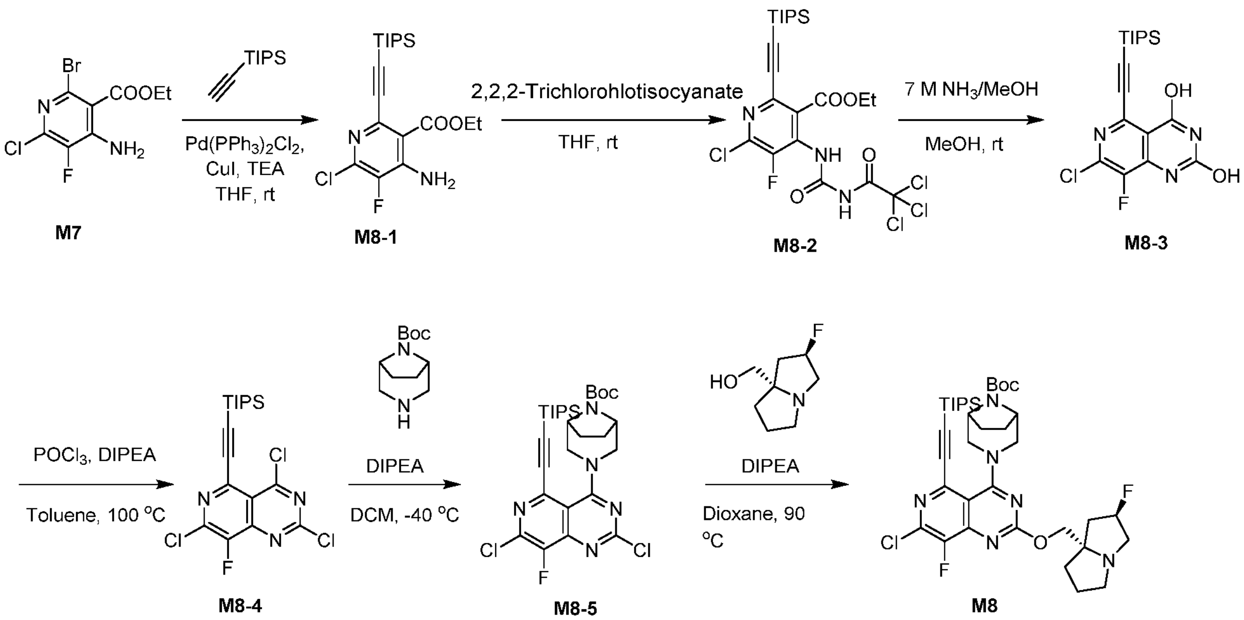


M7 M8-1 M8-2 M8-3 M8-4 M8-5 M8

Step 1: Synthesis of Compound **M8-1**

**M7** (9 g, 30.25 mmol), $Pd(PPh_3)_2Cl_2$ (2.17 g, 3.03 mmol) and CuI (0.58 g, 3.03 mmol) were added to a 500 mL double-neck bottle, then the bottle was replaced with $N_2$ three times, and triisopropylsilylacetylene (7.32g, 39.33 mmol), TEA (12.58 ml, 90.75 mmol), and anhydrous THF (180 mL) were added, then the bottle was replaced with $N_2$ three times again, the mixture was stirred at 30°C for 2 h. After the reaction was stopped, the resulting mixture was filtered, the filtrate was spin-dried and purified by column chromatography with PE/EA (v/v = 80/1-30/1) eluent to obtain 10.45 g yellow solid M8-1 with a yield of 86.6%. $^1H$ NMR (400 MHz, $CDCl_3$): δ 6.14 (s, 2H), 4.44 (q, *J*= 7.1 Hz, 2H), 1.40 (t, *J* = 7.1 Hz, 3H), 1.19-1.06 (m, 21H).

Step 2: Synthesis of Compound **M8-2**

**M8-1** (10.34 g, 25.92 mmol) and anhydrous THF (200 mL) were added to a 500 mL single-neck bottle, the mixture was stirred to dissolve, and then 2,2,2-trichlorohlotisocyanate (6.17 g, 31.10 mmol) was added, the mixture was stirred at 30 °C for 0.5 h. After the reaction was stopped, the resulting mixture was used at the next step directly after spin-drying, and the yield was calculated at 100%.

Step 3: Synthesis of Compound **M8-3**

Methanol (200 mL) was added to **M8-2** 15.22 g, 25.92 mmol) obtained in the previous step, the mixture was stirred to dissolve, and then methanol solution of ammonia (18.51 mL, 129.55 mmol, 7 M) was added, then the mixture was stirred at 30°C for 0.5 h. After the reaction was stopped, the resulting mixture was concentrated and the obtained

rough product was slurried with PE/EA (100 ml/10 mL) for 0.5 h, then filtered to obtain 8.72 g white solid **M8-3,** and the yield of step 2 and step 3 was 85%. LC-MS (ESI, pos. ion) m/z: 396.4 $[M+H]^+$.

Step 4: Synthesis of Compound **M8-4**

**M8-3** (5 g, 12.63 mmol) and anhydrous toluene (100 mL) were added to a 250 mL single-neck bottle, and then phosphorus oxychloride (4.61 mL, 50.52 mmol) and DIPEA (8.37 mL, 50.52 mmol) were added, the mixture was heated at 100°C and stirred for 2 h. After the reaction was stopped, the resulting mixture was cooled and used at the next step directly after concentration, the yield was calculated at 100%.

Step 5: Synthesis of Compound **M8-5**

Anhydrous methylene chloride (100 mL) was added to the **M8-4** (5.47 g, 12.63 mmol) obtained in the previous step, the bottle was replaced with $N_2$ three times, the mixture was stirred to dissolve, and cooled to -40 °C, and then DIPEA (6.27 mL, 37.83 mmol) was added, then a solution of tert-butyl 3,8-diazabicyclo[3.2.1]octane-8-carboxylate (2.76 g, 12.63 mmol) in DCM (10 mL) was added to the mixture. The resulting mixture was stirred at -40°C for 0.5 h, then saturated ammonium chloride solution (20 mL) was added to quench the reaction, then the reaction solution was returned to room temperature and the combined organic phase was concentrated, and purified by column chromatography with PE/EA (v/v = 50/1-10/1) eluent to obtain 3.3 g of yellow solid **M8-5,** and the yield of step 4 and step 5 was 43%. LC-MS (ESI, pos. ion) m/z: 608.3 $[M+H]^+$.

Step 6: Synthesis of Compound **M8**

**M8-5** (3 g, 4.93 mmol), ((2R, 7a*S*)-2-fluorohexahydro-1*H*-pyrrolizin-7a-yl)methanol (1.57 g, 9.86 mmol), DIPEA (2.45 mL, 14.79 mmol) and anhydrous dioxane (30 mL) were added to a 100 mL single-neck bottle, then the bottle was replaced with $N_2$ three times, and the mixture was heated to 90 °C and stirred for 20 h. After the reaction was stopped, the mixture was cooled to room temperature, then saturated ammonium chloride solution (30 mL) was added, extracted with EA (30 mL × 3), the combined organic phase was dried over anhydrous sodium sulfate, concentrated and purified by column chromatography with DCM/EMeOH (v/v = 100/0-90/1) eluent to obtain 2.1 g of yellow solid M8, with a yield of 58.2%. LC-MS (ESI, pos. ion) m/z: 731.3 $[M+H]^+$.

**Example 1: Synthesis of Compound 1**

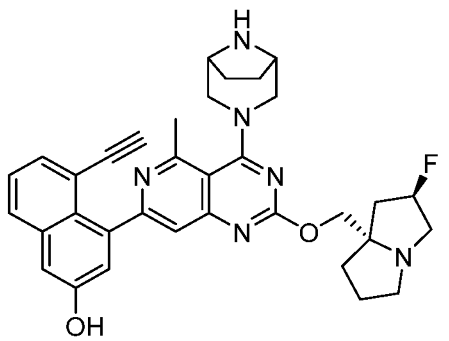

**1**

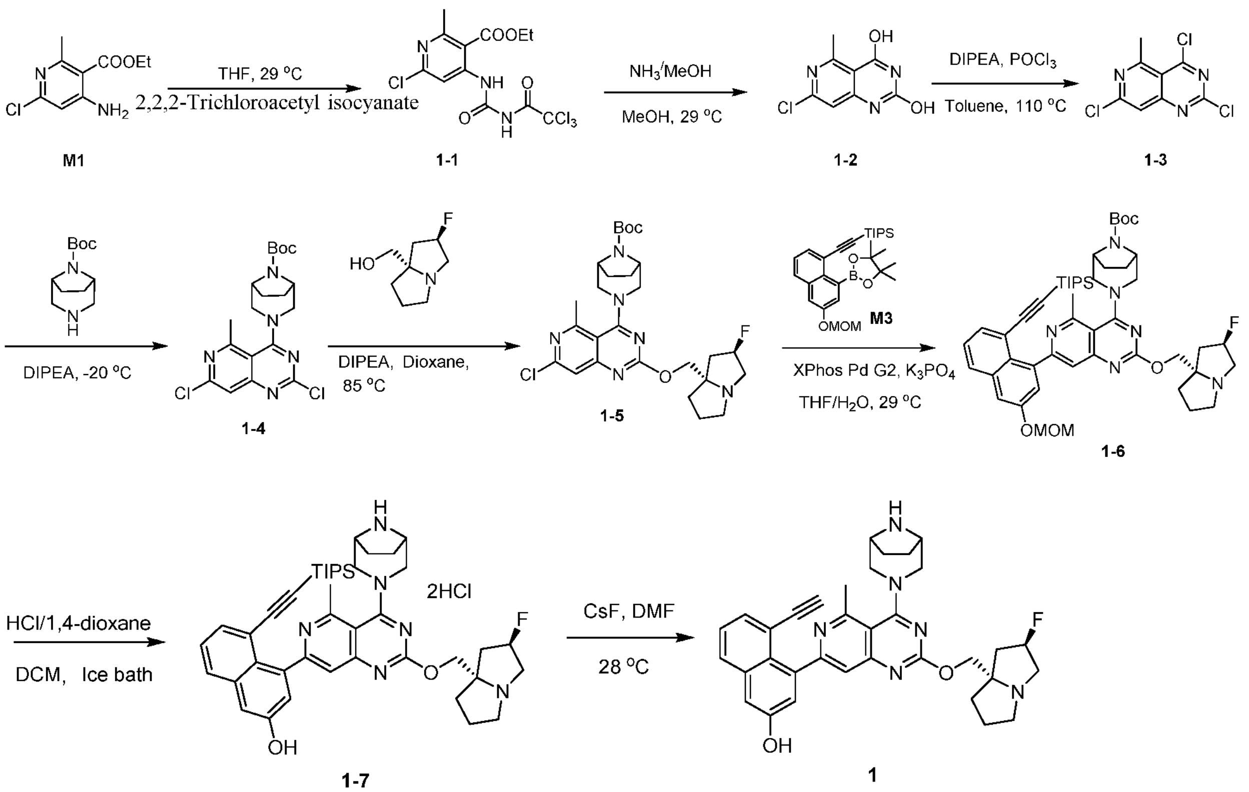


Step 1: Synthesis of Compound **1-1**

**M1** (1.05 g, 4.89 mmol) and tetrahydrofuran (8 mL) were added to a 50 mL single-neck bottle, then 2,2,2-trichloroacetyl isocyanate (1.38 g, 7.33 mmol) was added dropwise under $N_2$, then the mixture was stirred at 29 °C for 2 hours. The reaction solution was concentrated directly, and $Et_2O$ (15 mL) was added to the residue, the mixture was stirred at room temperature and filtered, the filter cake was the target product, and 1.82 g of white solid was obtained with a yield of 92.34%. $^1H$ NMR (400MHz, DMSO-$d_6$): δ 12.07 (br s, 1H), 10.79 (s, 1H), 8.08 (s, 1H), 4.41 (q, *J*=7.2Hz, 2H), 2.54 (s, 3H), 1.33 (t, J=7.2Hz, 3H).

Step 2: Synthesis of Compound **1-2**

**1-1** (1.82 g, 4. 52 mmol) and methanol (10 mL) were added to a 50 mL single-neck bottle, then methanol solution of ammonia (3.3 mL, 22.60 mmol, 7 mol/L) were added, the mixture was stirred at 29 °C for 2 hours. The reaction solution was directly filtered, and the filter cake was collected to obtain 1.02 g of white solid, with a yield of 106.64%. $^1H$ NMR (400MHz, DMSO-$d_6$): δ 6.87(s, 1H), 2.72(s, 3H).

Step 3: Synthesis of Compound **1-3**

Compound **1-2** (810.0 mg, 3.83 mmol), toluene (10 mL), *N,N*-diisopropylethylamine (3.17 mL, 19.15 mmol) and phosphorus oxychloride (1.75 mL, 19.15 mmol) were added to a 50 mL single-neck bottle, the mixture was heated to 110 °C and reacted for 2 hours. The reaction solution was directly concentrated to remove phosphorus oxychloride and toluene, and the residue was drained to obtain a brown oily substance. The yield was calculated as 100%, the resulting product was directly used for the next step without further purification.

Step 4: Synthesis of Compound **1-4**

Compound **1-3** (950.0 mg, 3.82 mmol) obtained in the previous step, *N,N*-diisopropylethylamine (0.77 mL, 4.64 mmol) and DCE (10 mL) were added to a 50 mL single-neck bottle, and the bottle was placed at -20 °C, to the bottle was slowly added dropwise a solution *of tert*-butyl 3,8-diazabicyclo[3.2.1]octane-8-carboxylate (650.0 mg, 3.06 mmol) in DCE (5 mL), and the mixture was continuously reacted at -20 °C for 0.5 h. The reaction solution was directly poured into saturated ammonium chloride solution (20 mL), the aqueous phase was extracted with DCM (20 mL × 2), the organic

phase was concentrated, and purified by column chromatography with PE/EtOAc (v/v =8/1) eluent to obtain 490.0 mg of yellow solid, with a yield of 30.23%. LC-MS (ESI, pos. ion) m/z: 424.0 $[M+H]^+$; $^1H$ NMR (400MHz, $CDCl_3$): δ 7.38 (s, 1H), 4.28 (br s, 3H), 3.55 (br s, 4H), 2.63 (s, 3H), 1.83 (br s, 3H), 1.50 (s, 9H).

Step 5: Synthesis of Compound **1-5**

Compound **1-4** (490.0 mg, 1.15 mmol), 1,4-dioxane (5 mL), *N,N*-diisopropylethylamine (0.57 mL, 3.45 mmol) and [(2*R*,7a*S*)-2-fluoro-tetrahydro-1*H*-pyrrolidin-7a(5*H*)-yl]methanol (0.31 mL, 2.3 mmol) were added to a 25 mL single-neck bottle, and the mixtire was heated to 85 °C for 22 h. Saturated ammonium chloride solution (30 mL) and EtOAc (30 mL) were directly add to the reaction solution to dilute, the organic phase was washed with saturated sodium chloride solution (40 mL), dried with anhydrous sodium sulfate, and filtered, and concentrated, then the obtained residue was purified by by column chromatography with DCM/MeOH (v/v=9/1) to obtain 140.0 mg of yellow foamy solid, with a yield of 22.25%. LC-MS (ESI, pos. ion) m/z: 547.2 $[M+H]^+$, $^1H$ NMR(400MHz, $CDCl_3$): δ 7.23 (s, 1H), 5.32 (d, *J*=52.0,1H), 4.31-4.25 (m, 5H), 3.69-3.24 (m, 7H), 3.08-3.00 (m, 2H), 2.59 (s, 3H), 2.37-2.59 (m, 4H), 2.09-1.92 (m, 4H), 1.50 (s, 9H).

Step 6: Synthesis of Compound **1-6**

Compound **1-5** (100.0 mg, 0.18 mmol), Compound **M3** (143.0 mg, 0.29 mmol, Anaiji Chemical), potassium phosphate (120.0 mg, 0.57 mmol) and Xphos Pd G2 (80.0 mg, 0.10 mmol) were added to a 10 mL two-neck bottle, the bottle was replaced with $N_2$ three times, then tetrahydrofuran (3 mL) and water (0.6 mL) were added, and the mixture was stirred at 29 °C for 16 h. The reaction solution was directly poured into DCM (10 mL) and water (5 mL) for extraction, the organic phase was dried with anhydrous sodium sulfate, and concentrated, and purified by column chromatography with DCM/MeOH (v/v =15/1), the resulting mixture was concentrated to obtain 110.0 mg of a brown solid, with a yield of 69.51%. LC-MS (ESI, pos. ion) m/z: 879.6 $[M+H]^+$.

Step 7: Synthesis of Compound **1-7**

Compound **1-6** (57.0 mg, 0.065 mmol) and dichloromethane (2 mL) were added to a 25 mL single-neck bottle, the mixture was stirred to dissolve, and then HCl/1,4-dioxane (0.25 mL, 0.99 mmol) was added under ice bath, the solution was stirred in ice bath for 2 h. The reaction solution was directly drained to obtain 48.0 mg of yellow solid, the yield was calculated as 100.00% and the resulting mixture was directly used for the next step. LC-MS (ESI, pos. ion) m/z: 735.6$[M+H]^+$.

Step 6: Synthesis of Compound **1**

Compound **1-7** (48.0 mg, 0.07 mmol) and DMF (2 mL) were added to a 10 mL single-neck bottle, then cesium fluoride (150.0 mg, 0.99 mmol) was added, and the mixture was stirred at 28°C for 15 h. The reaction solution was directly purified by a reverse silica gel column (mobile phase: [water (0.01%NH4HCO3)/acetonitrile; v/v=1/1]) to obtain 18.0 mg of brown solid, with a yield of 40.15%. LC-MS (ESI, pos. ion) m/z: 579.5 [M+H] ;$^1H$ NMR (400MHz, $CD_3OD$) δ 7.79 (d, J=8.0Hz, 1H), 7.50 (d, J=6.8Hz, 1H), 7.39-7.35 (m, 1H), 7. 30-7.23 (m, 2H), 7.15 (s, 1H), 5.37-5.23 (m, 1H), 4.30-4.19 (m, 2H), 3.80 (br s, 1H), 3.55 (br s, 3H), 3.24-3.19 (m, 3H), 3.05-2.99 (m, 1H), 2.86 (br s, 1H), 2.61 (s, 3H), 2.35-1.60 (m, 12H); $^{19}F$ NMR (400MHz, $CDCl_3$): δ -173.66(s, 1F).

**Example 2: Synthesis of Compound 2**

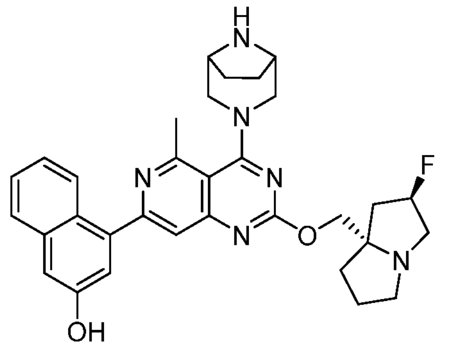

**2**

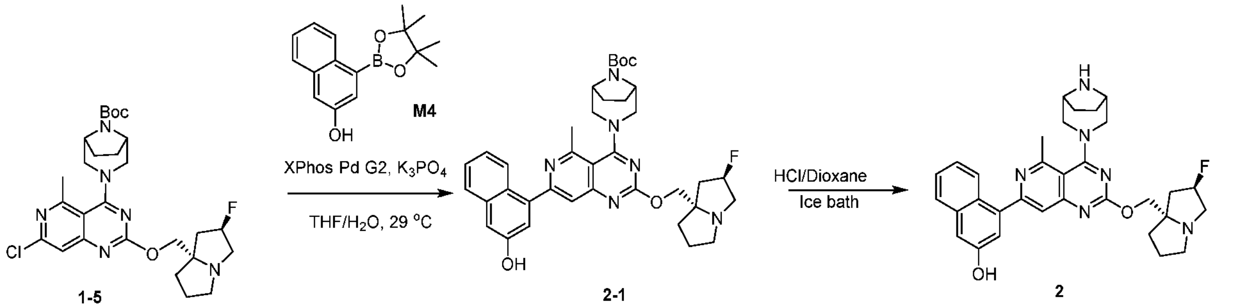


Step 1: Synthesis of Compound **2-1**

Compound **1-5** was synthesized according to steps 1-5 in Example 1.

Compound **1-5** (100.0 mg, 0.18 mmol), compound **M4** (146.0 mg, 0.54 mmol, Anaiji Chemical), potassium phosphate (120.0 mg, 0.57 mmol) and Xphos Pd G2 (80.0 mg, 0.10 mmol) were added to a 10 mL two-neck bottle, and the bottle was replaced with $N_2$ three times, then THF (4 mL) and water (0.6 mL) was added, and the mixture was stirred at 29 °C for 17 h. The reaction solution was directly poured into EtOAc (20 mL) and saturated ammonium chloride solution (15 mL), the aqueous phase was extracted with EtOAc (15 mL), the combined organic phase was dried over anhydrous sodium sulfate, then filtered and concentrated, and purified by column chromatography with DCM/MeOH (v/v =9/1) eluent to obtain 80.0 mg of yellow solid, with a yield of 91.81%. LC-MS (ESI, pos. ion) m/z: 655.2 $[M+H]^+$.

Step 2: Synthesis of Compound **2**

Compound **2-1** (80.0 mg, 0.10 mmol) and DCM (2 mL) were added to a 25 mL single-neck bottle, the mixture was stirred to dissolve, then a solution of hydrogen chloride in 1,4-dioxane (0.60 mL, 2.40 mmol, 4 M) was added dropwise in ice bath, the resulting mixture was stirred in ice bath for 2 h. The reaction solution was directly drained, then ammonia water was added to adjust the pH to 7-8, and silica gel was added and mixed, and the resulting mixture was purified by column chromatography with DCM/MeOH (v/v = 24/1) to obtain 25.0 mg of a yellow solid with a yield of 31.95%. LC-MS (ESI, pos. ion) m/z: 555.2 $[M+H]^+$; $^1$H NMR (400MHz, DMSO-$d_6$): δ 10.05 (s, 1H), 8.02 (d, J=8.4Hz, 1H), 7.76 (d, J=8.4Hz, 1H), 7.44-7.24 (m, 5H), 5.62-5.49 (m, 1H), 4.57 (br s, 2H), 4.37 (br s, 1H), 4.18-3.85(m, 5H), 3.78-3.63 (m, 5H), 2.68 (s, 3H), 2.59-2.55 (m, 1H), 2.33-2.26 (m, 2H), 2.18-2.12 (m, 2H), 2.09-1.95 (m, 2H), 1.90-1.87 (m, 2H); $^{19}$F NMR (400MHz, DMSO-$d_6$): δ - 172.353.

Examples 3-8: Synthesis of Compound 3-8

| Example | Compound | Synthesis process and structural characterization |
|---|---|---|
| Example 3 | 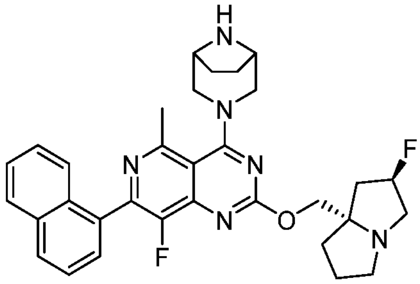 Compound 3 | Referring to the preparation process of compound 2 in Example 2. LC-MS (ESI, pos. ion) m/z: 557.4 [M+H]+. |
| Example 4 | 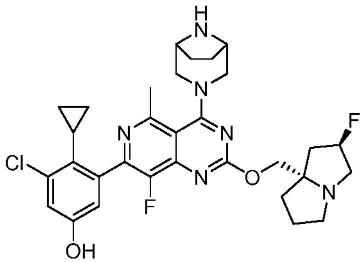 Compound 4 | Referring to the preparation process of compound 2 in Example 2. LC-MS (ESI, pos. ion) m/z: 597.3 [M+H]+. |
| Example 5 | 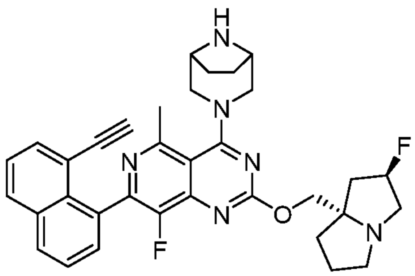 Compound 5 | Referring to the preparation process of compound 1 in Example 1. LC-MS (ESI, pos. ion) m/z: 581.5 [M+H]+. |
| Example 6 | 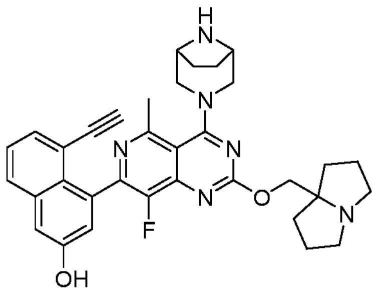 Compound 6 | Referring to the preparation process of compound 1 in Example 1. LC-MS (ESI, pos. ion) m/z: 579.3 [M+H]+. |

(continued)

| Example | Compound | Synthesis process and structural characterization |
|---|---|---|
| Example 7 | 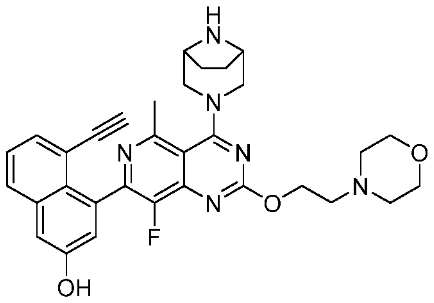 Compound 7 | Referring to the preparation process of compound 1 in Example 1. LC-MS (ESI, pos. ion) m/z: 569.3 $[M+H]^+$. |
| Example 8 | 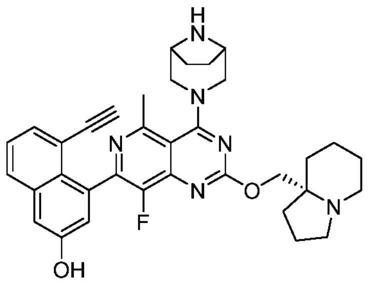 Compound 8 | Referring to the preparation process of compound 1 in Example 1. LC-MS (ESI, pos. ion) m/z: 593.3 $[M+H]^+$. |

**Example 9: Synthesis of Compound 9**

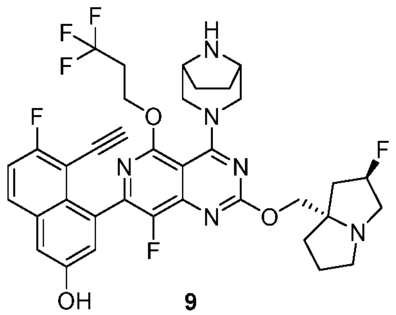

9

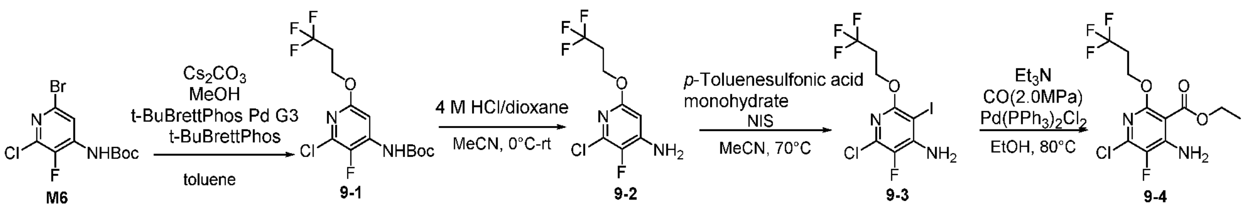


M6 9-1 9-2 9-3 9-4

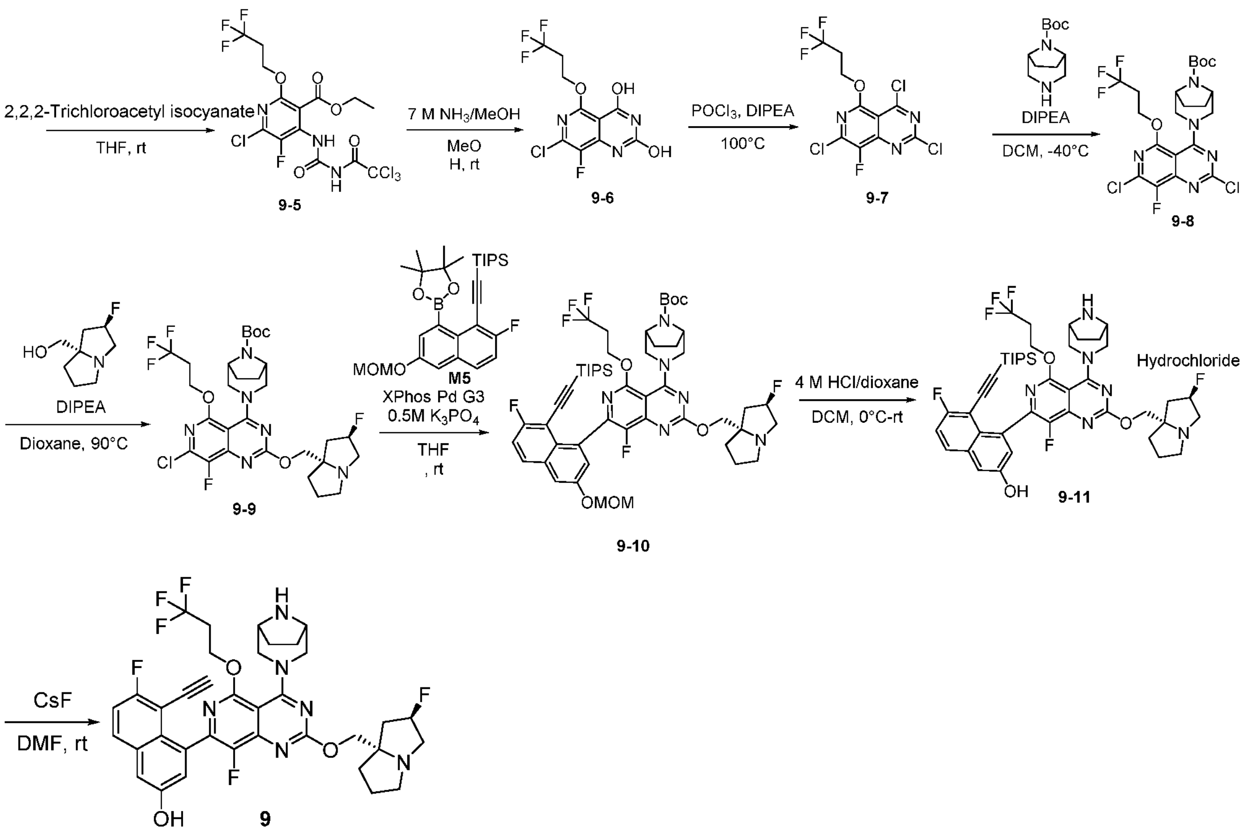


Step 1: Synthesis of Compound **9-1**

Compound M6 (3.00 g, 9.21 mmol), cesium carbonate (9.00 g, 27.63 mmol), *t*-BuBrettPhos G3 Pd (0.79 g, 0.92 mmol) and *t*-BuBrettPhos (0.22 g, 0.46 mmol) were added to a 50 mL two-neck bottle, then the bottle was replaced with $N_2$ three times, and to the mixture were added 3,3,3-trifluoropropan-1-ol (2.10 g, 18.42 mmol) and toluene (30 mL), the bottle was replaced with $N_2$ three times again, the resulting mixture was stirred and reacted for 17.5 h at 35°C, then the mixture was heated to 45°C and stirred for 1 h. Then stirring was stopped, the reaction solution was filtered through a celite pad and concentrated, and mixed with silica gel, then the mixture was purified by column chromatography with PE/EA (v/v = 99.5/0.5) eluent to obtain 1100 mg of yellow oil, with a yield of 33.29%. LC-MS (ESI, pos. ion) m/z: 359.3 $[M+H]^+$; $^1H$ NMR (400 MHz, $CDCl_3$): δ 7.49 (d, *J* = 4.4 Hz, 1H), 6.94 (s, 1H), 4.45 (t, *J* = 6.4 Hz, 2H), 2.56 (qt, *J* = 10.6, 6.4 Hz, 2H), 1.52 (s, 9H).

Step 2&3: Synthesis of Compounds **9-3**

The synthesis of compounds **9-3** can refer to the synthesis of **M2-3** to **M2-5** in intermediate M2, and 0.84 g of yellow oily crude product was obtained, which was directly used for the next step. LC-MS (ESI, neg. ion) m/z: 382.9 $[M-H]^-$.

Step 4: Synthesis of Compound **9-4**

Compound **9-3** (0.84 g, 2.55 mmol), $Pd(PPh_3)_2Cl_2$ (0.39g, 0.56 mmol), ethanol (20 mL) and triethylamine (1.01 g, 10.01 mmol) were added to a 250 mL autoclave, the autoclave was replaced with $N_2$ and CO in sequence, then filled with CO to make the pressure inside the autoclave at 2.0 MPa, and then the mixture was heated to 80°C and stirred for 14.5 h. After the reaction was stopped, the resulting mixture was cooled, filtered through a celite pad and concentrated, and eluted with column chromatography (PE/EA(v/v) = 20/1) to obtain 700 mg of a yellow solid, with a yield of 76.15%. LC-MS (ESI, pos. ion) m/z: 331.1 $[M+H]^+$; $^1H$ NMR (400 MHz, $CDCl_3$): δ 6.45 (s, 2H), 4.51 (q, *J* = 6.3 Hz, 2H), 4.33 (q, *J*= 7.1 Hz, 2H), 2.65-2.53 (m, 2H), 1.36 (t, *J* = 7.2 Hz, 3H).

Step 5: Synthesis of Compound **9-5**

Compound **9-4** (700 mg, 2.12 mmol), anhydrous THF (3 mL) and 2,2,2-trichloroacetyl isocyanate (1.2 g, 6.36

mmol) were added to a 50 mL single-neck bottle, and the mixture was stirred at room temperature for 0.5 h, then stirring was stopped, the resulting mixture was concentrated and vacuumed to obtain a light yellow solid, the yield was calculated as 100%.

Step 6: Synthesis of Compound **9-6**

Compound **9-5** (1.1 g, 2.12 mmol), methanol (5 mL) and methanol solution of ammonia (6.05 mL, 42.4 mmol, 7 mol/L) were added to a 50 mL single-neck bottle, the mixture was stirred and reacted at room temperature for 1 h, then stirring was stopped, the resulting mixture was concentrated and slurried with MTBE (20 mL) for 30 min, then filtered and vacuumed to obtain 0.69 g of a white solid, with a yield of 99%. LC-MS (ESI, neg. ion): m/z: 326.0 $[M-H]^-$.

Step 7: Synthesis of Compound **9-7**

Compound **9-6** (630 mg, 1.92 mmol), phosphorus oxychloride (4.38 mL, 48 mmol) and DIPEA (1.59 mL, 9.6 mmol) were added to a 50 mL single-neck bottle, the mixture was heated to 100°C and stirred for 4.5 h under $N_2$, then stirring was stopped, the reaction solution was concentrated, the yield was calculated as 100%, and the resulting mixture was used directly for the next step.

Step 8: Synthesis of Compound **9-8**

Compound **9-7** (700 mg, 1.92 mmol) and DCM (7 mL) were added to a 50 mL single-neck bottle, the bottle was replaced with $N_2$ three times, then to the mixture were added DIPEA (1.59 mL, 9.6 mmol) and a solution of *tert*-butyl 3,8-diazabicyclo[3.2.1]octane-8-carboxylate (0.37 g, 1.75 mmol) in DCM (2.5 mL), the resulting mixture was stirred for 2 h, then stopped stirring, and saturated ammonium chloride solution (30 mL) was added, the mixture was separated, extracted with DCM (20 mL × 2), the combined organic phase was dried over anhydrous sodium sulfate, concentrated and mixed with silica gel, and purified by column chromatography with PE/EA (v/v = 20/1) eluent to obtain 490 mg of a yellow solid, with a yield of 47%. LC-MS (ESI, pos. ion) m/z: 540.2 $[M+H]^+$.

Step 9: Synthesis of Compound **9-9**

Compound **9-8** (300 mg, 0.56 mmol), 1,4-dioxane (5 mL), [(2*R*, 7a*S*)-2-fluoro-hexahydro-1*H*-pyrrolizin-7*a*-yl]methanol (80 mg, 1.12 mmol) and DIPEA (43 g, 3.36 mmol) were added to a 100 mL single-neck bottle, and the mixture was heated to 90 °C and stirred for 24 h, then stopped stirring, the resulting mixture was concentrated, to the mixture were added ethyl acetate (20 mL) and saturated sodium chloride solution (20 mL) and then separated, the aqueous phase was extracted with ethyl acetate (20 mL × 2), the combined organic phase was dried over anhydrous sodium sulfate, then concentrated and purified by column chromatography with DCM/MeOH (v/v = 10/1) eluent to obtain 290 mg of a yellow solid, with a yield of 78.1%. LC-MS (ESI, pos. ion) m/z: 663.5 $[M+H]^+$.

Step 10: Synthesis of Compound **9-10**

Compound **9-9** (120 mg, 0.18 mmol), compound **M5** (140 mg, 0.27 mmol), anhydrous potassium phosphate (76 mg, 0.36 mmol) and XPhos Pd G3 (34 mg, 0.040 mmol) were added to a 25 mL two-neck bottle, the bottle was replaced with $N_2$ three times, then water (0.5 mL) and THF (5 mL) were added to the mixture, the bottle was replaced with $N_2$ three times again, the mixture was stirred at 25°C for 16.5 h. Then stirring was stopped, the mixture was filtered through a celite pad and concentrated, then DCM (20 mL) and saturated ammonium chloride solution (20 mL) were added in sequence and separated, and the aqueous phase was extracted with DCM (20 mL), the combined organic phase was dried over anhydrous sodium sulfate and concentrated, and the residue was purified by thin layer chromatography DCM/MeOH (v/v = 97.5/2.5 - 95/5) to obtain 120 mg of brown oil, with a yield of 65.8%. LC-MS (ESI, pos. ion) m/z: 507.4 $[M+2H]^{2+}$.

Step 11: Synthesis of Compound **9-11**

Compound **9-10** (120 mg, 0.12 mmol) and dichloromethane (3 mL) were added to a 25 mL single-neck bottle, and a solution of hydrogen chloride in 1,4-dioxane (1.2 mL, 4.8 mmol, 4 M) was added dropwise at 0°C, the mixture was the heated to 25°C and stirred for 3 h. Then stirring was stopped, the reaction solution was concentrated, the obtained solid was slurried with DCM and filtered to obtain 87 mg of a yellow solid, with a yield of 84.53%. LC-MS (ESI, pos. ion) m/z: 870.4$[M+H]^+$.

Step 12: Synthesis of Compound **9**

Compound **9-11** (87 mg, 0.10 mmol), DMF (1 mL) and cesium fluoride (230 mg, 1.5 mmol) were added to a 25 mL single-neck bottle, the bottle was replaced with $N_2$ three times, and the mixture was stirred at 25°C for 17 h. Then stirring was stopped, the reaction solution was purified by reverse phase column with $H_2O$/MeCN (v/v = 0-1/1) eluent to obtain 16 mg of a yellow solid, with a yield of 22.45%. LC-MS (ESI, pos. ion) m/z: 713.3 $[M+H]^+$; $^1H$ NMR (400 MHz, DMSO-$d_6$): δ 10.23 (s, 1H), 7.97 (dd, *J* = 9.2, 5.9 Hz, 1H), 7.46 (t, *J* = 9.0 Hz, 1H), 7.38 (d, *J* = 2.6 Hz, 1H), 7.23 (s, 1H), 5.27 (d, *J* = 54.2 Hz, 1H), 4.50 (t, *J* = 6.2 Hz, 2H), 4.15 - 3.85 (m, 5H), 3.45 (d, *J* = 11.5 Hz, 4H), 3.14 - 3.05 (m, 2H), 3.01 (s, 1H), 2.85 - 2.65 (m, 3H), 2.15 - 1.92 (m, 3H), 1.90 - 1.64 (m, 3H), 1.61 - 1.35 (m, 4H), 0.85 (t, *J* = 6.6 Hz, 1H).

**Example 10: Synthesis of Compound 10**

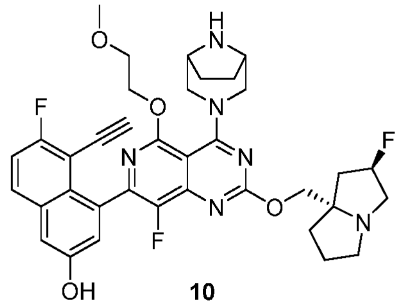

**10**

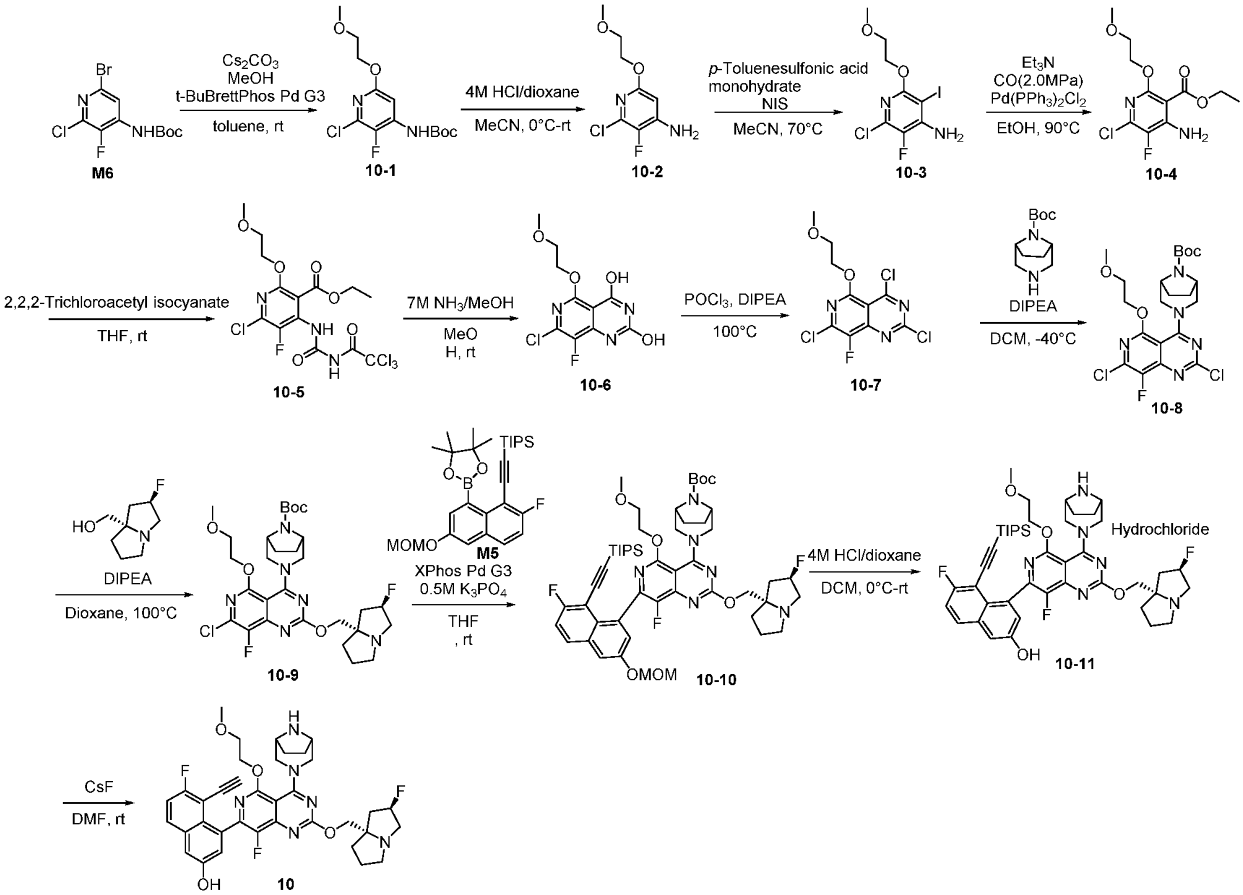


The synthesis of compound 10 can refer to the synthesis of compound 9 in Example 9. In step 1, the initial raw material 3,3,3-trifluoropropan-1-ol was replaced with ethylene glycol methyl ether, and finally a yellow solid of 45 mg was obtained, the yield was 47.64%. LC-MS (ESI, pos. ion) m/z: 675.35 $[M+H]^+$; $^1H$ NMR (400 MHz, $CD_3OD$): δ 7.84

(dd, $J$= 9.0, 5.7 Hz, 1H), 7.40 - 7.27 (m, 2H), 7.26 - 7.17 (m, 1H), 5.36 (d, $J$ = 53.9 Hz, 1H), 4.72 - 4.59 (m, 1H), 4.58 - 4.19 (m, 5H), 3.95 - 3.78 (m, 3H), 3.77 - 3.55 (m, 3H), 3.51 - 3.34 (m, 6H), 3.16 - 3.05 (m, 1H), 2.46 - 2.15 (m, 3H), 2.10 - 2.02 (m, 2H), 1.95 (s, 4H), 1.89 - 1.72 (m, 1H), 1.45 - 1.25 (m, 1H); $^{19}F$ NMR (376 MHz, $CD_3OD$) δ -111.86 (s), -132.26 (s), -150.80 (d, $J$ = 8.9 Hz).

**Example 11: Synthesis of Compound 11**

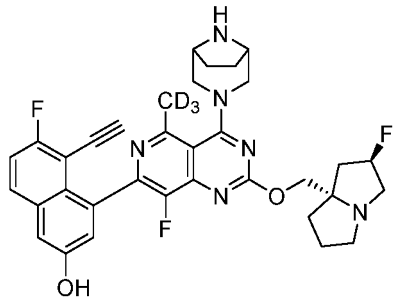

**11**

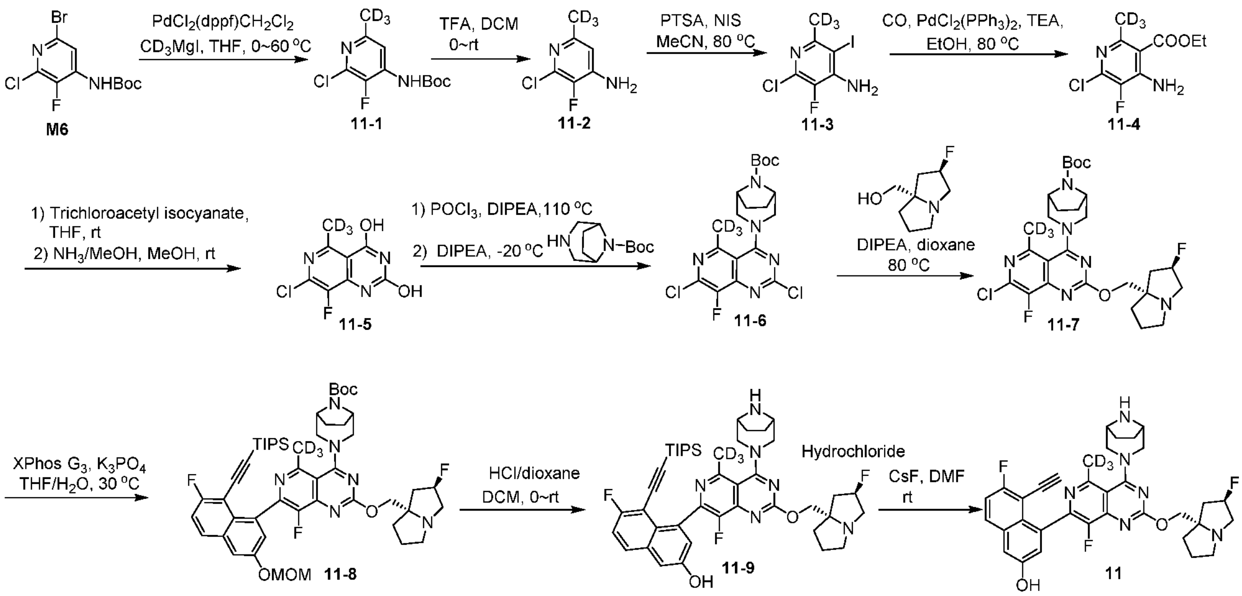


Step 1: Synthesis of Compound **11-1**

Compound **M6** (1.48 g, 4.55 mmol), THF (15 mL) and 1,1'-Bis(diphenylphosphino)ferrocene-palladium(II)dichloride dichloromethane complex (0.56 g, 0.69 mmol) were added to a 50 mL two-neck bottle, the bottle was replaced with $N_2$ three times, the mixture was cooled to 0 °C, then $d_3$-methyl-magnesium iodide (9.1 mL, 9.1 mmol, 1mol/L) was added dropwise, the mixture was continuously stirred for 30 minutes, then the reaction solution was heated to 60 °C and stirred for 3 h. The reaction solution was poured directly into saturated ammonium chloride solution (30 mL), hydrochloric acid (5 mL, 0.5 M) and EtOAc (40 mL), the aqueous phase was extracted with EtOAc (30 mL) once again, and then washed directly with saturated brine (40 mL) once. The organic phase was directly dried over anhydrous sodium sulfate, then filtered and concentrated, the obtained residue was directly purified by column chromatography with PE/EtOAc (v/v = 40/1), the resulting mixture was concentrated to obtain 450.0 mg of a yellow solid, with a yield of 37.50%. LC-MS (ESI, pos. ion) m/z: 264.2 $[M+H]^+$; $^1H$ NMR (400 MHz, $CDCl_3$): δ 7.94 (d, $J$ = 4.8Hz, 1H), 6.90( br.s, 1H), 1.53 (s, 9H).

Step 2: Synthesis of Compound 11-2

Compound 11-1 (550.0 mg, 2.09 mmol) and DCM (5 mL) were added to a 50 mL single-neck bottle, the bottle was cooled in ice bath at 0 °C under $N_2$, then TFA (7.8 mL, 104.7 mmol) was added dropwise and the mixture was

stirred at 20°C for 2 h. The reaction solution was poured directly into saturated sodium carbonate solution (30 mL) and DCM (20 mL), the organic phase was directly dried over anhydrous sodium sulfate, concentrated under vacuum and purified by column chromatography with PE/EtOAc (v/v = 3.5/1) to obtain 302.0 mg of a white solid, with a yield of 88.32%. LC-MS (ESI, pos. ion) m/z:164.3 $[M+H]^+$; $^1H$ NMR (400MHz, $CDCl_3$): δ 6.44 (d, *J* = 5.6 Hz, 1H), 4.28 (br.s, 2H).

Step 3: Synthesis of Compound **11-3**

Compound **11-2** (340.0 mg, 2.08 mmol), acetonitrile (5 mL), *p*-toluenesulfonic acid monohydrate (123.0 mg, 0.65 mmol) and NIS (716.0 mg, 3.18 mmol) were added to a 50 mL single-neck bottle, the bottle was replaced with $N_2$ three times, then the mixture was heated to 80 °C and stirred for 4 h. The reaction solution was directly poured into saturated sodium bicarbonate solution (20 mL) and EtOAc (30 mL), the organic phase was washed with saturated sodium sulfite solution (30 mL) and saturated sodium chloride solution (30 mL) in sequence, and then the resulting mixture was dried with sodium sulfate, filtered and concentrated, the obtained residue was directly purified by silica gel column chromatography with PE/EtOAc(v/v = 24/1), then the mixture was concentrated to obtain 0.59 g of light brown solid, with a yield of 97.98%. LC-MS (ESI, pos. ion) m/z: 290.1$[M+H]^+$; $^1H$ NMR (400MHz, $CDCl_3$): δ 4.86 (br.s, 2H). $^{19}F$ NMR (400MHz, $CDCl_3$): δ -142.32.

Step 4: Synthesis of Compound **11-4**

Compound **11-3** (590.0 mg, 2.04 mmol), ethanol (10 mL), triethylamine (1.14 mL, 8.20 mmol) and bistriphenylphosphine palladium dichloride (170.0 mg, 0.24 mmol) were added to a 250 mL autoclave, the autoclave was replaced with $N_2$ three times and filled with CO to 2.2 Mpa, then the mixture was heated to 80 °C and stirred for 18 h. The reaction solution was directly concentrated, the obtained residue was directly purified by column chromatography with PE/EtOAc (v/v = 21/1), then concentrated to obtain 438.0 mg of a light brown solid, with a yield of 91.11%. LC-MS (ESI, pos. ion) m/z: 236.2$[M+H]^+$; $^1H$ NMR (400MHz, $CDCl_3$): δ 6.20 (br.s, 2H), 4.40 (q, *J* = 7.2Hz, 2H), 1.41 (t, *J* = 7.2Hz, 3H); $^{19}F$ NMR (400MHz, $CDCl_3$): δ -147.86.

Step 5: Synthesis of Compound **11-5**

Compound **11-4** (438.0 mg, 1.86 mmol) and tetrahydrofuran (3 mL) were added to a 50 mL single-neck bottle, then 2,2,2-trichloroacetyl isocyanate (0.55 mL, 4.65 mmol) was added dropwise in ice bath under $N_2$, the mixture was stirred at 22 °C for 2 h. The reaction solution was directly concentrated, then to the obtained residue was added methanol (10 mL) to dissolve, and then methanol solution of ammonia (2.7 mL, 18.90 mmol) was added and the resulting mixture was stirred at 22 °C for 1 h. The reaction solution was filtered directly, the filter cake was rinsed with methanol (10 mL), and the filter cake was further dried to obtain 0.43 g of a white solid, with a yield of 99.39%. LC-MS(ESI, neg. ion) m/z: 231.2 $[M-H]^-$; $^1H$ NMR (400MHz, DMSO-$d_6$): δ 7.86 (br.s, 2H); $^{19}F$ NMR (400MHz, DMSO-$d_6$): δ -141.56.

Step 6: Synthesis of Compound **11-6**

Compound **11-5** (0.43 g, 1.85 mmol), phosphorus oxychloride (15.0 mL, 164.4mmol) and DIPEA (1.25 mL, 7.54 mmol) were added to a 50 mL single-neck bottle, then the mixture was heated to 110 °C and reacted for 40 min. The reaction solution was concentrateed directly, to the obtained brown oily substance was added DCE (4 mL) to dissolve, the mixture was placed at -20 °C, then DIPEA (2.45 mL, 14.8 mmol) and a solution of 8-*tert*-butoxycarbonyl-3,8-diazabicyclo[3.2.1]octane (0.39 g, 1.84 mmol) in DCE (1 mL) were added dropwise, and the resulting mixture was stirred at -20 °C for 10 min. The reaction solution was directly poured into saturated ammonium chloride solution (10 mL) and DCM (10 mL) for extraction, the organic phase was directly dried over anhydrous sodium sulfate, then filtered and concentrated, the obtained residue was directly purified by silica gel column chromatography with PE/EtOAc (v/v = 4/1), then concentrated to obtain 0.60 g of a yellow foamy solid, with a yield of 72.83%. LC-MS (ESI, pos. ion) m/z: 445.3$[M+H]^+$; $^1H$ NMR (400 MHz, $CDCl_3$): δ 4.29 (br.s, 2H), 3.58 (br.s, 3H), 1.84 (br.s, 2H), 1.66-1.56 (m, 3H), 1.50 (s, 9H). $^{19}F$ NMR (400MHz, $CDCl_3$): δ -138.17.

Step 7: Synthesis of Compound **11-7**

Compound **11-6** (600.0 mg, 1.35 mmol), 1,4-dioxane (5 mL), DIPEA (0.89 mL, 5.40 mmol) and ((2*R*,7a*S*)-2-fluorotetrahydro-1*H*-pyrrolizin-7a(5*H*)-yl)methanol (0.36 mL, 2.70 mmol) were added to a 50 mL single-neck bottle, the mixture was heated to 80 °C and stirred for 12 h. The reaction solution was directly cooled to room temperature, then poured into saturated ammonium chloride solution (20 mL) and EtOAc (20 mL), the organic phase was washed with saturated sodium chloride solution (20 mL), and dried over anhydrous sodium sulfate, then filtered and concentrated,

the obtained residue was directly purified by silica gel column chromatography with DCM/MeOH (v/v = 32/1), and then concentrated to obtain 300.0 mg of a pink solid, with a yield of 39.12%. $^1$H NMR (400 MHz, $CDCl_3$): δ5.33-5.20 (m, 1H), 4.27-4.13 (m, 5H), 3.59 (br.s, 3H), 3.28-3.17 (m, 3H), 3.01-2.94 (m, 1H), 2.54 (br.s, 1H), 2.32-2.07 (m, 3H), 1.95-1.79 (m, 6H), 1.50 (s, 9H). $^{19}$F NMR (400MHz, $CDCl_3$): δ -140.37, -173.20.

Step 8: Synthesis of Compound **11-8**

Compound **11-7** (162.0 mg, 0.32 mmol), potassium phosphate (133.0 mg, 0.63 mmol) and XPhos G3 Pd (45.0 mg, 0.05 mmol) were added to a 10 mL two-neck bottle, the bottle was replaced with $N_2$ three times, and then tetrahydrofuran (3 mL) and water (0.6 mL) were added, the resulting mixture was stirred at 30 °C. The reaction solution was directly poured into saturated ammonium chloride solution (10 mL), and then extracted with EtOAc (10 mL), the organic phase was washed once with saturated brine (10 mL), and dried over anhydrous sodium sulfate, then filtered and concentrated, the obtained residue was directly purified by column chromatography with DCM/MeOH(v/v = 24/1), and then concentrated to obtain 160.0 mg of a brown solid, with a yield of 82.98%. LC-MS (ESI, pos. ion) m/z: 918.5[M+H]$^+$; $^1$H NMR (400MHz, $CDCl_3$): δ 7.71 (br.s, 2H), 7.53-7.51 (m, 2H), 4.35-3.90 (m, 6H), 3.48 (d, J=4.0Hz, 1H), 2.31-2.19 (m, 1H), 1.05-2.04 (m, 28H), 0.74-0.97 (m, 22H).

Step 9: Synthesis of Compound **11-9**

Compound **11-8** (160.0 mg, 0.17 mmol) and DCM (4.0 mL) were added to a 50 mL single-neck bottle, the mixture was stirred to dissolve and cooled at 0 °C under $N_2$, then a solution of hydrogen chloride in 1,4-dioxane (2.98 mL, 11.9 mmol) was added dropwise, then the resulting mixture was heated to 22 °C and stirred for 40 min. The reaction solution was directly concentrated to obtain 144.0 mg of a dark yellow solid with a yield of 100.00%, the resulting mixture was directly used for the next step without further purification. LC-MS (ESI, pos. ion) m/z: 774.4[M+H]$^+$.

Step 10: Synthesis of Compound **11**

Compound **11-9** (0.14 g, 0.17 mmol), DMF (2 mL) and cesium fluoride (388.0 mg, 2.55 mmol) were added to a 50 mL single-neck bottle, the bottle was replaced with $N_2$ three times, and the mixture was stirred at 22 °C for 24 h. The reaction solution was directly poured into saturated sodium bicarbonate solution (10 mL), then DCM (20 mL × 2) was added for extraction, the combined organic phase was washed with saturated brine (40 mL × 2), and dried directly over anhydrous sodium sulfate, then filtered and concentrated, the obtained residue was directly removed DMF by PE, then purified by column chromatography with DCM/MeOH (v/v = 2.2/1), and concentrated to obtain 60.0 mg of a yellow solid, with a yield of 57.14%. LC-MS (ESI, pos. ion) m/z: 618.5[M+H]$^+$; $^1$H NMR(400MHz, CD3OD): δ7.87-7.84 (m, 1H), 7.29-7.39 (m, 2H), 7.21 (br.s, 1H), 5.42-5.28 (m, 1H), 4.59-4.30 (m, 4H), 3.88-3.59 (m, 4H), 3.46-3.34 (m, 3H), 3.12-3.06 (m, 1H), 2.60 (br.s, 1H), 2.44-2.16 (m, 3H), 2.09-1.91 (m, 3H), 1.81 (br.s, 2H), 1.60 (br.s, 2H). $^{19}$F NMR(400MHz, CD3OD): δ -111.58, -145.15, -173.63.

**Example 12: Synthesis of Compound 12**

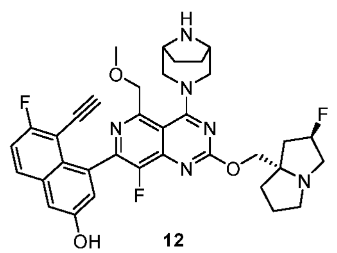

**12**

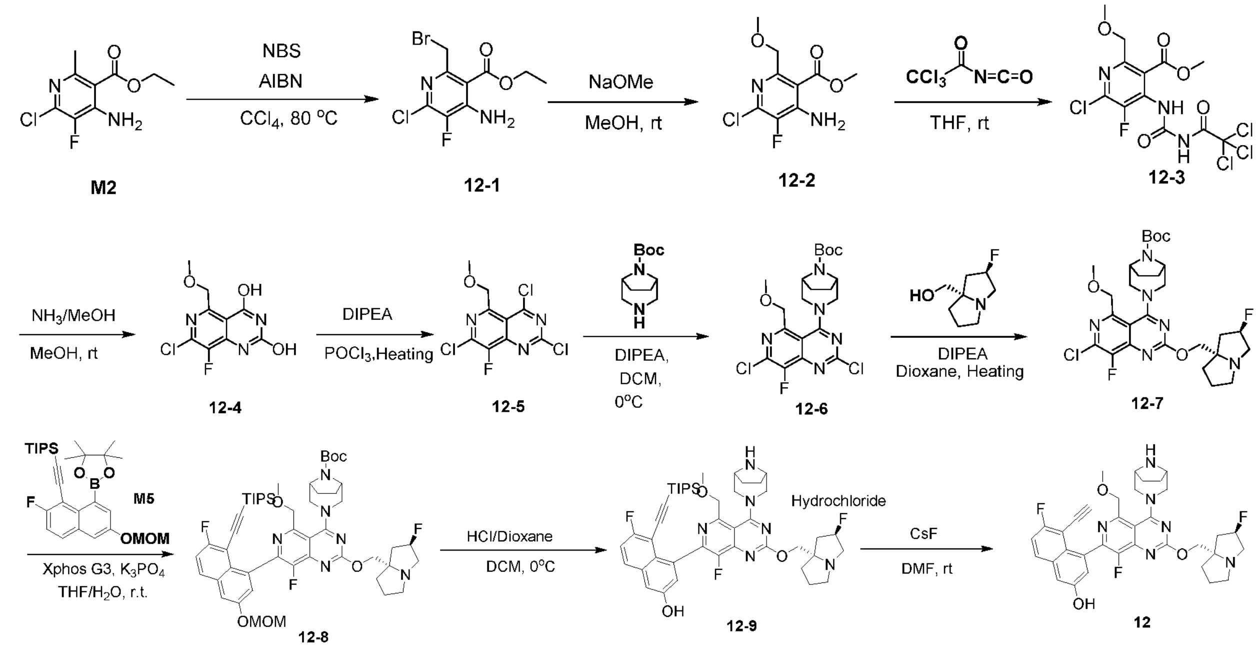


Step 1: Synthesis of Compound **12-1**

**M2** (14.4 g, 61.90 mmol), NBS (14.32 g, 80.47 mmol), AIBN (12.38 mmol) and carbon tetrachloride (140 mL) were added to a 1 L single-neck bottle, the mixture was stirred at 80°C for 13 h under $N_2$ replacement protection. Then stirring was stopped, the reaction solution was concentrated under vacuum, then water (100 mL) was added and extracted with EA (200 mL), then washed once with saturated brine (100 mL), the organic phase was dried over anhydrous sodium sulfate, and filtered and concentrated, then purified by silica gel column chromatography (DCM/PE (v/v) = 10/90 ~ 50/50) to obtain 12.7 g of a gray solid, with a yield of 65.86%. LC-MS (ESI, pos. ion) m/z: 310.9 $[M+H]^+$ ; $^1H$ NMR (400 MHz, $CDCl_3$): δ 6.30 (s, 2H), 4.77 (s,2H), 4.46 (q, *J* = 7.2 Hz, 2H), 1.48 (t, *J* = 7.1 Hz, 3H).

Step 2: Synthesis of Compound **12-2**

Compound **12-1** (500 mg, 1.60 mmol), methanol (2 mL) and sodium methoxide (173 mg, 3.20 mmol) were added to a 25 mL single-neck bottle, then the mixture was stirred at 27 °C for 1 h. The reaction solution was poured directly into saturated ammonium chloride (20 mL) and EtOAc (20 mL) for extraction, the organic phase was washed once with saturated brine (20 mL), and dried over anhydrous sodium sulfate, then filtered and concentrated, the obtained residue was directly purified by column chromatography with PE/EtOAc (v/v = 8.8/1), and concentrated to obtain 230.0 mg of yellow oily liquid, with a yield of 57.81%. LC-MS (ESI, pos. ion) m/z: 249.2 $[M+H]^+$; $^1H$ NMR (400MHz, $CDCl_3$): δ 5.96 (br.s, 2H), 4.63 (s, 2H), 3.93 (s, 3H), 3.35 (s, 3H).

Step 3: Synthesis of Compound **12-3**

Compound **12-2** (210.0 mg, 0.84 mmol) and tetrahydrofuran (2 mL) were added to a 25 mL single-neck bottle, then 2,2,2-trichloroacetyl isocyanate (0.26 mL, 2.20 mmol) was added dropwise in ice bath under $N_2$, the mixture was stirred at 25 °C for 1 h. The reaction solution was concentrated and the resulting mixture was used directly for the next step.

Step 4: Synthesis of Compound **12-4**

The concentrated Compound **12-3** in the previous step was dissolved with methanol (2 mL), then mthanol solution of ammonia (1.2 mL, 8.4 mmol, 7 mol/L) was added, and the mixture was stirred at 25 °C for 3 h. The reaction solution was directly filtered, and the filter cake was concentrated to obtain 0.21 g of a white solid, with a yield of 96.29%. LC-MS (ESI, pos. ion) m/z:258.1 $[M-H]^-$; $^1H$ NMR (400MHz, DMSO-d6): δ 7.45 (br.s, 2H), 4.77 (s, 2H), 3.30 (s, 3H).

Step 5: Synthesis of Compound **12-5**

Compound **12-4** (180 mg, 0.69 mmol), phosphorus oxychloride (3 mL, 32.87 mmol) and DIPEA (0.5 mL, 3.02

mmol) were added to a 25 mL single-neck bottle, the mixture was heated to 110 °C and reacted for 50 min. The reaction solution was directly concentrated to obtain a brown oily substance, then drained, and the resulting mixture was used directly for the next step.

Step 6: Synthesis of Compound **12-6**

To the brown oily Compound **12-5** in the previous step was added DCE (2 mL) to dissolve, the mixture was placed in ice bath and then DIPEA (0.91 mL, 5.52 mmol) was added, a solution of 8-tert-butoxycarbonyl-3,8-diazabicyclo[3.2.1]octane (150 mg, 0.71 mmol) in DCE (1 mL) was added dropwise, the resulting mixture was continuously stirred for 30 min. Then the reaction solution was directly poured into saturated ammonium chloride (10 mL) and DCM (10 mL) for extraction, the organic phase was directly dried over anhydrous sodium sulfate, then filtered and concentrated, the obtained residue was directly purified by silica gel column chromatography with PE/EtOAc(v/v = 7.5/1), and concentrated to obtain 250.0 mg of a yellow foamy solid, with a yield of 76.71%. LC-MS (ESI, pos. ion) m/z: 472.3[M+H]$^+$; $^1$H NMR (400MHz, $CD_3COCD_3$): $\delta$ 4.70 (br.s, 2H), 4.29 (br.s, 2H), 3.66 (m, 2H), 3.32 (s, 3H), 2.77 (s, 2H), 1.78 (br.s, 2H), 1.49 (s, 9H), 1.27-1.33 (m, 2H).

Step 7: Synthesis of Compound **12-7**

Compound **12-6** (0.35 mL, 2.12 mmol) and ((2*R*,7a*S*)-2-fluorotetrahydro-1*H*-pyrrolizin-7a(5*H*)-yl)methanol (0.14 mL, 1.06 mmol) were added to a 50 mL two-neck bottle, the mixture was heated to 80 °C and reacted for 12 h. The reaction solution was directly cooled to room temperature, then poured into saturated ammonium chloride (20 mL) and EtOAc (20 mL), the organic phase was washed with saturated sodium chloride (20 mL), dried over anhydrous sodium sulfate, then filtered and concentrated, the obtained residue was directly purified by silica gel column chromatography with DCM/MeOH (v/v = 24/1), and concentrated to obtain 190.0 mg of a yellow foamy solid, with a yield of 60.24%. LC-MS (ESI, pos. ion) m/z: 595.5 [M+H]$^+$; $^1$H NMR (400MHz, DMSO-d6): $\delta$5.34-5.20 (m, 1H), 4.53 (s, 2H), 4.26-4.09 (m, 5H), 3.56-3.17 (m, 9H), 3.01-2.95 (m, 1H), 2.32-1.79 (m, 10H), 1.50 (s, 9H).

Step 8: Synthesis of Compound **12-8**

Compound **12-7** (120.0 mg, 0.20 mmol), **M5** (205.0 mg, 0.40 mmol), potassium phosphate (126.0 mg, 0.59 mmol) and XPhos Pd G3 (94.0 mg, 0.11 mmol) were added to a 10 mL two-neck bottle, then the bottle was replaced with $N_2$ three times, and tetrahydrofuran (2 mL) and water (0.4 mL) were added, and the mixture was stirred at 30 °C for 16 h. The reaction solution was poured directly into saturated ammonium chloride (10 mL), and then extracted with EtOAc (10 mL), the organic phase was washed once with saturated brine (10 mL), and directly dried over anhydrous sodium sulfate, then filtered and concentrated, the obtained residue was purified by column chromatography with DCM/MeOH (v/v = 24/1), and concentrated to obtain 100.0 mg of a brown solid, with a yield of 52.90%. LC-MS (ESI, pos. ion) m/z: 945.8[M+H]$^+$.

Step 9: Synthesis of Compound **12-9**

Compound **12-8** (100.0 mg, 0.11 mmol) and dichloromethane (4 mL) were added to a 50 mL single-neck bottle, the mixture was stirred to dissolve, and a solution of hydrogen chloride in dioxane (0.85 mL, 3.30 mmol) was added dropwise in ice bath at 0°C, then the mixture was heated to 26 °C and stirred for 1 h. The reaction solution was directly concentrated at room temperature to obtain 97.0 mg of a dark yellow solid with a yield of 100.00%, the resulting mixture was directly used for the next step without further purification. LC-MS (ESI, pos. ion) m/z : 801.7.

Step 10: Synthesis of Compound **12**

Compound **12-9** (97.0 mg, 0.11mmol), DMF (2 mL), and cesium fluoride (251.0 mg, 1.65 mmol) were added to a 25 mL single-neck bottle, then the bottle was replaced with $N_2$ three times, and the mixture was stirred at 26 °C for 24 h. The reaction solution was directly poured into saturated sodium bicarbonate (10 mL), then DCM (20 mL $\times$ 2) was added for extraction, the combined organic phase was washed with saturated brine (40 mL $\times$ 2), and dried over anhydrous sodium sulfate, then filtered and concentrated, the obtained residue was directly purified by column chromatography with DCM/MeOH (v/v = 6/1), and concentrated to obtain 40 mg of a light brown solid, with a yield of 55.38%. LC-MS (ESI, pos. ion) m/z: 645.5[M+H]$^+$; $^1$H NMR(400MHz, $CD_3OD$): $\delta$7.87-7.84 (m, 1H), 7.34-7.29 (m, 2H), 7.24 (br.s, 1H), 5.38-5.24 (m, 1H), 4.95-4.92 (m, 2H), 4.74-4.58 (m, 4H), 4.33-4.22 (m, 2H), 3.63-3.47 (m, 4H), 3.33(s, 3H), 3.26-3.19 (m, 3H), 3.06-3.00 (m, 1H), 2.37-1.77 (m, 9H); $^{19}$F NMR(400MHz, $CD_3OD$): $\delta$ 111.65, 173.61, 173.64.

**Example 13: Synthesis of Compound 13**

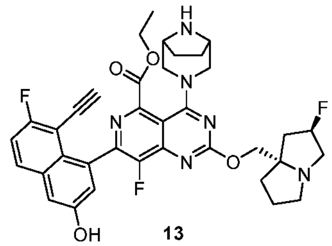

13

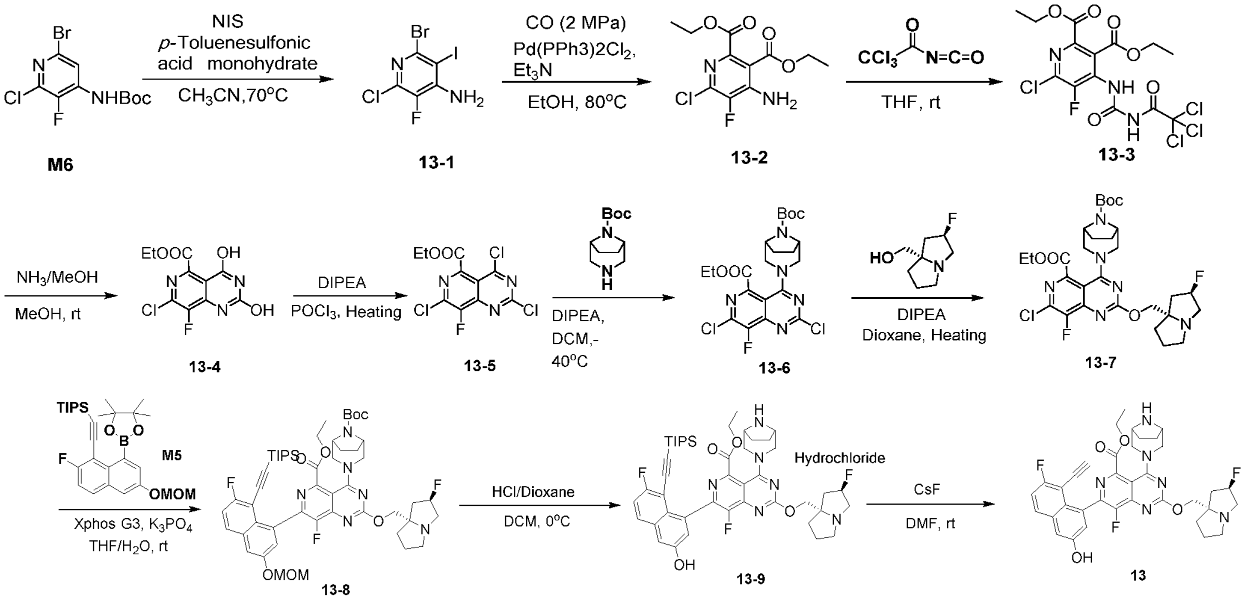


M6, 13-1, 13-2, 13-3, 13-4, 13-5, 13-6, 13-7, M5, 13-8, 13-9, 13

Step 1: Synthesis of Compound **13-1**

**M6** (200.0 mg, 0.61 mmol), NIS (164.69 mg, 0.73 mmol), acetonitrile (10 mL) and *p*-toluenesulfonic acid monohydrate (232.0 mg, 0.122 mmol) were added to a 50 mL single-neck bottle, the mixture was heated to 70°C and stirred for 3 h. Then stop heating and stirring, the reaction solution was concentrated, and EA (50 mL) was added to dissolve, and the mixture was diluted with water (20 mL), the organic phase was washed with saturated $NaHCO_3$ solution (30 mL), saturated $Na_2SO_3$ solution (30 mL) and saturated sodium chloride solution (30 mL) in sequence, then dried over anhydrous sodium sulfate and concentrated under vacuum to obtain 214.0 mg of a light yellow solid, with a yield of 99.8%. LC-MS (ESI, pos. ion) m/z: 351.0 $[M-H]^+$.

Step 2: Synthesis of Compound **13-2**

Compound **13-1** (100.0 mg, 0.28 mmol), $Pd(PPh_3)_2Cl_2$ (39.3 mg, 0.06 mmol), EtOH (20 mL) and triethylamine (0.2 mL, 1.40 mmol) were added to a 200 mL autoclave, the autoclave was replaced with $N_2$ and CO in sequence, then filled with CO to make the pressure inside the autoclave at 2 MPa, and the mixture was stirred and reacted overnight at 80°C. Then the reaction was stopped, the reaction solution was cooled and filtered through a celite pad, then concentrated, the resulting mixture was eluted with column chromatography (PE/EA (v/v) = 97/3) to obtain 40.0 mg of brown oil, with a yield of 49.2%. LC-MS (ESI, pos. ion) m/z: 291.3 $[M+H]^+$; $^1H$ NMR (400 MHz, $CDCl_3$): δ 6.38 (s, 2H), 4.36 (m, 4H), 1.37 (m, 6H).

Step 3: Synthesis of Compound **13-3**

Compound **13-2** (645.0 mg, 2.22 mmol) and tetrahydrofuran (6 mL) were added to a 50 mL single-neck bottle, then 2,2,2-trichloroacetyl isocyanate (0.4 mL, 3.33 mmol) was added dropwise under $N_2$, then the mixture was stirred

at 25°C for 20 min. The reaction solution was directly concentrated to obtain 923.0 mg of a brown solid, with a yield of 86.8%. LC-MS (ESI, pos. ion) m/z: 480.1 $[M+H]^+$.

Step 4: Synthesis of Compound **13-4**

Compound **13-3** (923.0 mg, 1.93 mmol), methanol (4 mL) and mthanol solution of ammonia (4.2 mL, 28.95 mmol, 7 mol/L) were added to a 50 mL single-neck bottle, the mixture was stirred at 25 °C for 3 h. The reaction solution was directly filtered, and the filter cake was collected to obtain 540.0 mg of a white solid, with a yield of 97.28%. LC-MS (ESI, pos. ion) m/z: 288.1 $[M+H]^+$; $^1H$ NMR (400 MHz, $CD_3OD$): δ 4.42 (q, *J* = 7.2 Hz, 2H), 1.39 (t, *J* = 7.1 Hz, 3H).

Step 5: Synthesis of Compound **13-5**

Compound **13-4** (60.0 mg, 0.21 mmol), *N,N*-diisopropylethylamine (0.3 mL, 1.68 mmol) and phosphorus oxychloride (0.96 mL, 10.5 mmol) were added to a 25 mL single-neck bottle, the mixture was heated to 110 °C and stirred for 4 h. The reaction solution was directly concentrated to remove phosphorus oxychloride, and the residue was drained to obtain a brown oily substance, the yield was calculated as 100% and the resulting mixture was directly used for the next step.

Step 6: Synthesis of Compound **13-6**

Compound **13-5** (68.0 mg, 0.21 mmol) obtained in the previous step, *N,N*-diisopropylethylamine (0.17 mL, 1.05 mmol) and DCM (4 mL) were added to a 25 mL single-neck bottle, the bottle was cooled at -40 °C, then a solution of tert-butyl 3,8-diazabicyclo[3.2.1]octane-8-carboxylate (44.6 mg, 0.21 mmol) in DCE (2 mL) was slowly added dropwise to the reaction solution and the resulting mixture was reacted at -40 °C for 0.5 h. The reaction solution was directly poured into saturated ammonium chloride (20 mL), the aqueous phase was extracted with DCM (10 mL × 2), the organic phase was concentrated and then purified by column chromatography with PE/EtOAc (v/v = 8/1) as eluent, then the resulting mixture was concentrated to obtain 35.0 mg of a yellow solid, with a yield of 33.3%. LC-MS (ESI, pos. ion) m/z: 500.1 $[M+H]^+$; $^1H$ NMR (400 MHz, $CDCl_3$): δ 4.47 (m, 2H), 4.27 (s, 2H), 3.43 (m, 2H), 1.89 - 1.82 (m, 2H), 1.50 (s, 9H), 1.47 (m, 2H), 1.46 - 1.42 (m, 3H), 0.93 - 0.78 (m, 2H).

Step 7: Synthesis of Compound **13-7**

Compound **13-6** (400.0 mg, 0.80 mmol), 1,4-dioxane (4 mL), *N,N*-diisopropylethylamine (0.66 mL, 4.0 mmol) and [(2*R*,7a*S*)-2-fluoro-tetrahydro-1*H*-pyrrolidin-7a(5*H*)-yl]methanol (379.7 mg, 2.40 mmol) were added to a 25 mL single-neck bottle, the mixture was heated to 80 °C and reacted for 24 h. To the reaction solution were directly added saturated ammonium chloride solution (30 mL) and EtOAc (50 mL) to dilute, the organic phase was washed with saturated sodium chloride (40 mL) again, then dried over anhydrous sodium sulfate, filtered and concentrated, the obtained residue was purified by column chromatography with DCM/MeOH (v/v = 6/1) to obtain 240.0 mg of a yellow foamy solid, with a yield of 48.2%. LC-MS (ESI, pos. ion) m/z: 623.5 $[M+H]^+$.

Step 8: Synthesis of Compound **13-8**

Compound **13-7** (10.0 mg, 0.016 mmol), **M5** (12.3 mg, 0.024 mmol, Anaiji Chemical), potassium phosphate (10.2 mg, 0.048 mmol) and Xphos G3 (4.1 mg, 0.0048 mmol) were added to a 10 mL two-neck bottle, the bottle was replaced with $N_2$ three times, then tetrahydrofuran (2 mL) and water (0.5 mL) were added, and the mixture was stirred at 30 °C for 24 h. The reaction solution was directly poured into DCM (10 mL) and water (5 mL) for extraction, the organic phase was dried with anhydrous sodium sulfate, then concentrated and purified by column chromatography with DCM/MeOH (v/v = 15/1), and concentrated to obtain 5.0 mg of a brown solid, with a yield of 32.11%. LC-MS (ESI, pos. ion) m/z: 973.7 $[M+H]^+$.

Step 9: Synthesis of Compound **13-9**

Compound **13-8** (73.0 mg, 0.075 mmol) and dichloromethane (2 mL) were added to a 25 mL single-neck bottle, the mixture was stirred to dissolve, then HCl/1,4-dioxane (1.3 mL, 5.25 mmol, 4 mol/L) solution was added and the mixture was stirred for 4 h under ice bath. The reaction solution was directly drained to obtain 58.0 mg of a yellow solid, with a yield of 93.28%. LC-MS (ESI, pos. ion) m/z: 829.4.

Step 10: Synthesis of Compound **13**

Compound **13-9** (62.2 mg, 0.075 mmol), DMF (2 mL) and cesium fluoride (0.23 g, 1.49 mmol) were added to a 10 mL single-neck bottle, the mixture was stirred at room temperature 25 °C for 12 h. The reaction solution was directly purified by reverse silica gel column (mobile phase: water/acetonitrile (v/v=1/1)) to obtain 24.0 mg of a light yellow solid, with a yield of 47.57%. LC-MS (ESI, pos. ion) m/z: 673.1 $[M+H]^+$; $^1H$ NMR (400 MHz, $CD_3OD$): δ 7.87 (dd, *J* = 9.1, 5.8 Hz, 1H), 7.38 - 7.29 (m, 2H), 7.25 (s, 1H), 5.40 (d, *J* = 53.2 Hz, 1H), 4.56 - 4.37 (m, 5H), 3.78 (m, 2H), 3.61 (m, 2H), 3.44 (m, 7.6 Hz, 3H), 3.27 (d, *J* = 5.5 Hz, 1H), 3.17 (m, 9.4 Hz, 1H), 2.54 - 2.21 (m, 3H), 2.17 - 1.96 (m, 4H), 1.95 - 1.79 (m, 2H), 1.62 (dt, *J* = 11.6, 5.5 Hz, 2H), 1.39 (t, *J* = 6.9 Hz, 3H);$^{19}F$ NMR (376 MHz, $CD_3OD$): δ -111.39 - -111.49 (m, 1F), -131.93 (s, 1F), -135.72 (d, *J*= 14.9 Hz, 1F).

**Example 14: Synthesis of Compound 14**

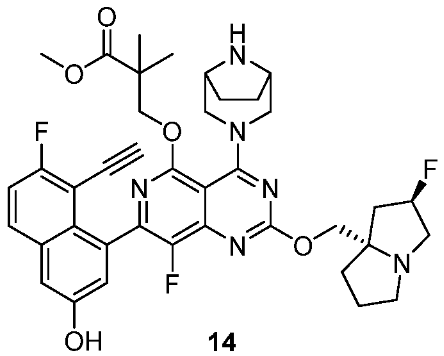

**14**

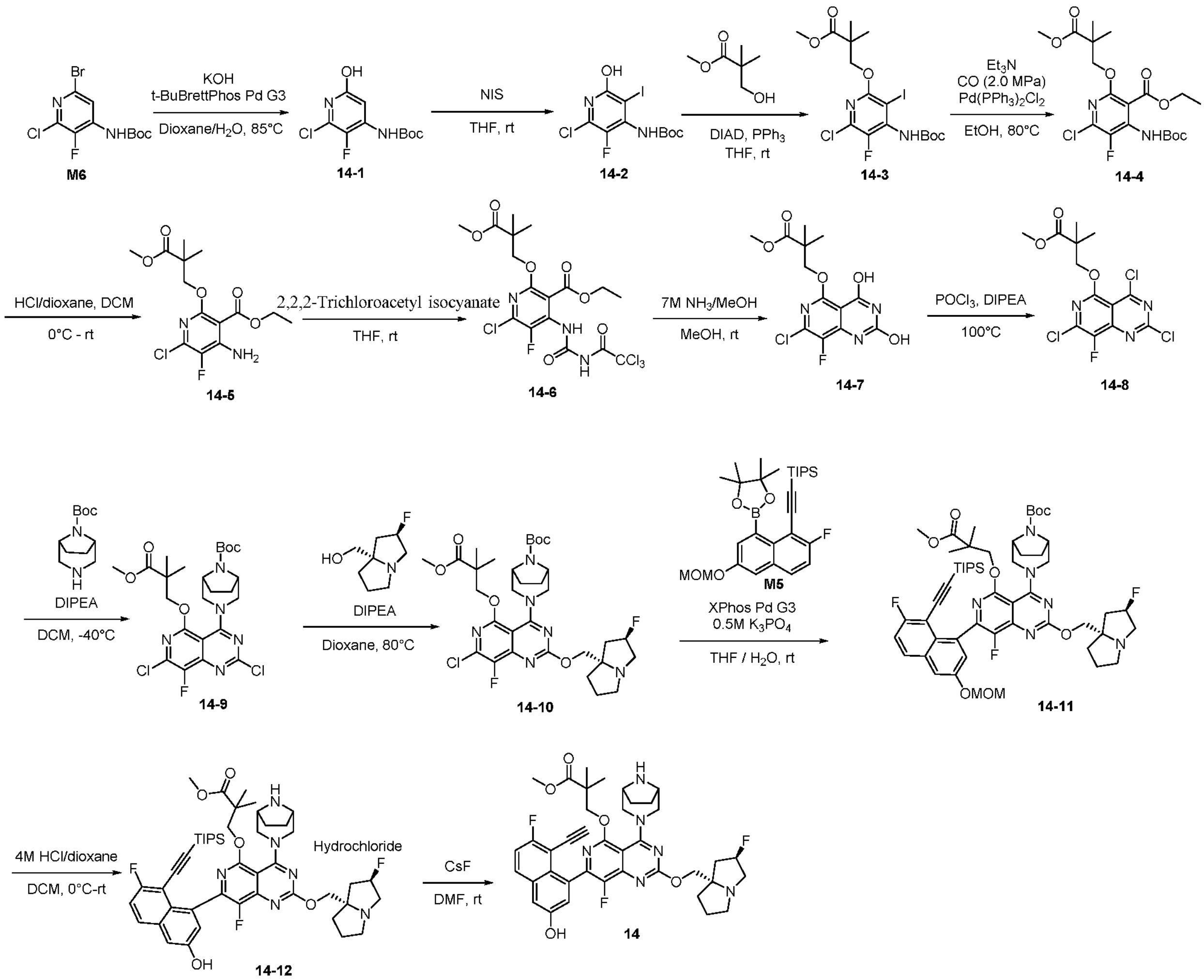


Step 1: Synthesis of Compound **14-1**

**M6** (5.0 g, 15.36 mmol), potassium hydroxide (2.59 g, 46.08 mmol), t-BuBrettPhos G3 Pd (1.31 g, 1.54 mmol), 1,4-dioxane (50 mL) and water (4 mL) were added to a 100 mL two-neck bottle, the bottle was replaced with $N_2$ three times, the mixture was heated to 85 °C and stirred for 1 h. The reaction solution was directly poured into saturated ammonium chloride (30 mL) to quench the reaction, then hydrochloric acid (30 mL, 0.5 M) was added to quench, the resulting mixture was extracted twice with EA (30 mL), and the organic phase was washed with saturated brine (50 mL), then dried with anhydrous sodium sulfate, filtered and concentrated, the obtained residue was purified by silica gel column chromatography with PE/EA (v/v = 100/0 - 60/40) as eluent, then concentrated to obtain 2.7 g of a brown solid, which was directly used for the next step. LC-MS (ESI, pos. ion) m/z: 263.2 $[M+H]^+$; $^1H$ NMR (400 MHz, $CDCl_3$): δ 7.51(d, *J*=4.8Hz, 1H), 6.92(br.s, 1H), 1.25(s, 9H).

Step 2: Synthesis of Compound **14-2**

Compound **14-1** (1.3 g, 4.95 mmol), THF (5 mL) and NIS (1.67 g, 7.47 mmol) were added to a 50 mL single-neck bottle, the bottle was replaced with $N_2$ three times, and the mixture was stirred at room temperature for 17 h. The reaction solution was directly poured into saturated sodium sulfite solution (10 mL) and EA (20 mL), the mixture was stirred and separated, the organic phase was washed with saturated sodium chloride solution (10 mL) again, dried over anhydrous sodium sulfate, then filtered and concentrated, to the obtained residue was added *n*-heptane (1 mL) to slurry, then filtered, the filter cake was washed with n-heptane (1 mL) and collected and dried to obtain 850.0 mg solid, which was directly used for the next step. LC-MS (ESI, pos. ion) m/z: 389.1 $[M+H]^+$.

Step 3: Synthesis of Compound **14-3**

Compound **14-2** (800.0 mg, 2.06 mmol), 2, 2-dimethyl-3-hydroxypropionic acid methyl ester (0.35 mL, 2.76 mmol) and triphenylphosphine (827.0 mg, 3.15 mmol) were added to a 10 mL two-neck bottle, the bottle was replaced with $N_2$ three times, then diisopropyl azodicarboxylate (0.61 mL, 3.13 mmol) was added under an ice bath at 0 °C, and the mixture was stirred at room temperature for 17 h. The reaction solution was directly poured into saturated ammonium chloride (20 mL) and EA (20 mL), the organic phase was washed with saturated brine (15 mL), then dried over anhydrous sodium sulfate, filtered and concentrated, the obtained residue was purified by silica gel column chromatography with PE /EA (v/v = 100/0 - 97/3) to obtain 0.33 g of a light brown oily product. LC-MS (ESI, pos. ion) m/z: 503.0 $[M+H]^+$.

Step 4: Synthesis of Compound **14-4**

Compound **14-3** (840.0 mg, 1.67 mmol), EtOH (10 mL), TEA (0.94 mL, 6.75 mmol) and $Pd(PPh_3)_2Cl_2$ (176.0 mg, 0.25 mmol) were added to a 250 mL autoclave, the autoclave was replaced with $N_2$ three times, then filled with CO to 2.2 Mpa, then the mixture was heated to 80 °C and stirred for 23 h. The reaction solution was concentrated, the obtained residue was purified by silica gel column chromatography with PE/EA (v/v = 96/4 - 90/10) to obtain 220.0 mg of a white solid. LC-MS (ESI, pos. ion) m/z: 449.2 $[M+H]^+$.

Step 5: Synthesis of Compound **14-5**

Compound **14-4** (0.22 g, 0.49 mmol) and DCM (2 mL) were added to a 25 mL single-neck bottle, the mixture was stirred to dissolve, then HCl/dioxane (4 M, 3.67 mL, 14.7 mmol) was added dropwise at 0 °C, and the resulting mixture was stirred at room temperature for 2.5 h. The reaction solution was directly concentrated, to the obtained residue was added saturated sodium bicarbonate (8 mL) to free product, then EtOAc (10 mL) was added to extract twice. The combined organic phase was washed twice with saturated brine (10 mL × 2), and dried with anhydrous sodium sulfate, then filtered and concentrated, the resulting mixture was purified by silica gel column chromatography with PE/EA (v/v = 96/4 - 94/6) to obtain 0.14 g of colorless oil. LC-MS (ESI, pos. ion) m/z: 349.1 $[M+H]^+$; $^1H$ NMR (400 MHz, $CDCl_3$): δ 6.46(br.s, 2H), 4.29-4.35(m, 4H), 3.67(s, 3H), 1.36(t, *J*=7.2Hz, 3H), 1.31(s, 6H).

Step 6 to Step 13: Synthesis of Compound **14**

For the synthesis steps from step 6 to step 13, refer to the synthesis steps 5 to 12 in compound 7 to obtain 45.0 mg of yellow solid **14.** LC-MS (ESI, pos. ion) m/z: 731.3 $[M+H]^+$; $^1H$ NMR(400MHz, $CD_3OD$): δ 7.86-7.82 (m, 1H), 7.33-7.29 (m, 2H), 7.21 (s, 1H), 5.43-5.29 (m, 1H), 4.60-4.50 (m, 2H), 4.39-4.30 (m, 4H), 3.77-3.71 (m, 2H), 3.64 (s, 3H), 3.52-3.35 (m, 4H), 3.15-3.09 (m, 1H), 2.46-2.18 (m, 4H), 2.11-1.92 (m, 4H), 1.87-1.84 (m, 2H), 1.73-1.68 (m, 2H), 1.27-1.22 (m, 6H). $^{19}F$ NMR(400MHz, $CD_3OD$): δ -76.94, -111.82, -150.24.

**Example 15: Synthesis of Compound 15**

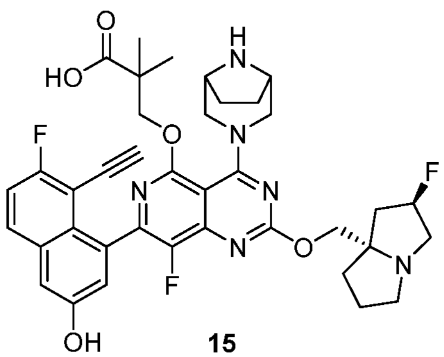

**15**

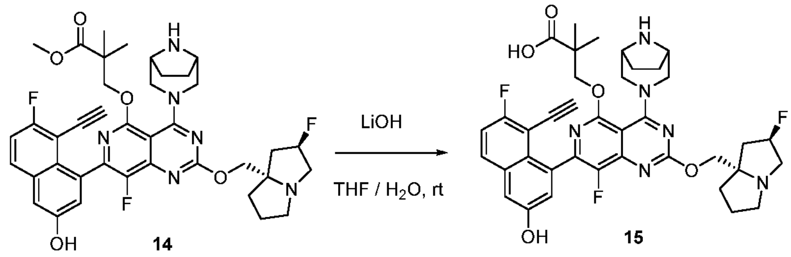


Compound **14** (40.0 mg, 0.06 mmol), THF (2 mL), water (0.5 mL) and lithium hydroxide monohydrate (34.0 mg, 0.80 mmol) were added to a 25 mL single-neck bottle, the mixture was stirred at room temperature for 20 h. The reaction solution was directly concentrated to remove THF, the residue was placed to an ice bath, then 1 M HCl was added dropwise to adjust pH at 5, and then concentrated, the resulting mixture was directly purified by reverse silica gel column purification with $H_2O/CH_3CN$ (v/v=100/0-80/20), then concentrated to obtain 22.0 mg of a yellow solid. LC-MS (ESI, pos. ion) m/z: 717.6 $[M+H]^+$; $^1H$ NMR(400MHz, $CD_3OD$): δ7.88-7.84 (m, 1H), 7.34-7.29 (m, 2H), 7.21 (br.s, 1H), 5.62-5.49 (m, 1H), 4.67-4.64 (m, 2H), 4.50-4.45 (m, 2H), 4.17-3.77 (m, 8H), 3.70-3.67 (m, 4H), 3.61-3.56 (m, 1H), 3.44-3.39 (m, 2H), 2.33-2.26 (m, 2H), 2.22-2.00 (m, 4H), 1.21-1.12 (m, 6H); $^{19}F$ NMR(400MHz, $CD_3OD$): δ -76.95, -111.85, -150.99.

**Example 16: Synthesis of Compound 16**

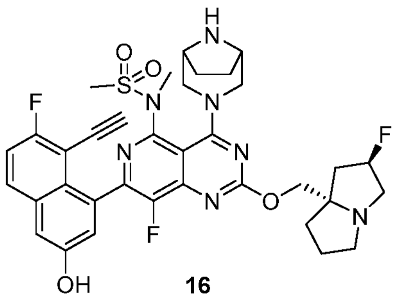

**16**

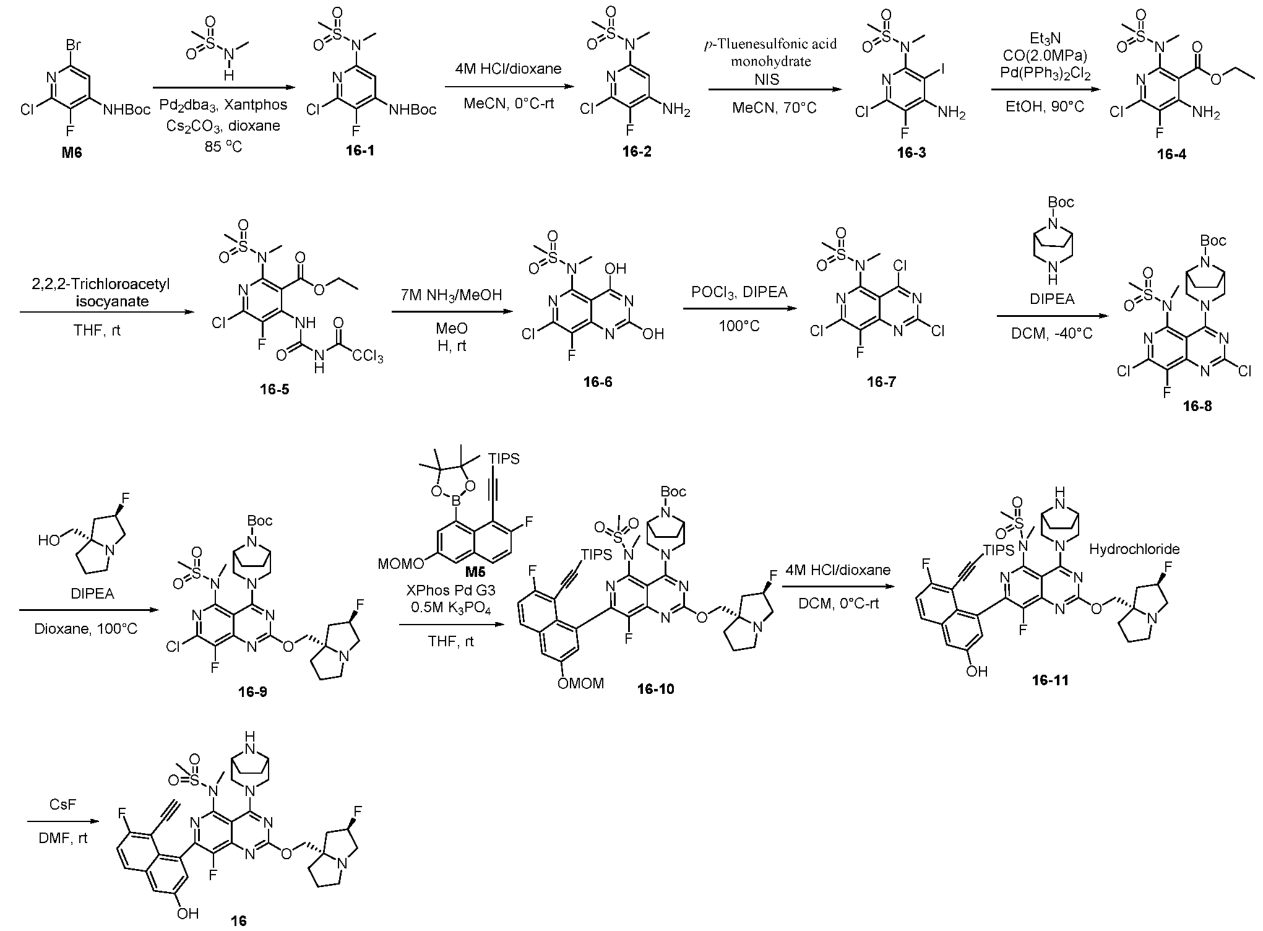


Step 1: Synthesis of Compound **16-1**

Compound **M6** (1.00 g, 3.07 mmol), cesium carbonate (2.40 g, 7.37 mmol), $Pd_2(dba)_3$ (206.0 mg, 0.22 mmol), Xantphos (302.0 mg, 0.52 mmol), *N*-methylmethanesulfonamide (0.40 mL, 4.24 mmol) were added to a 50 mL two-neck bottle, the bottle was replaced with $N_2$ three times, then 1,4-dioxane (10 mL) was added, and the mixture was heated to 85 °C and stirred for 15 h. The reaction solution was directly quenched by adding EtOAc (25 mL) and saturated ammonium chloride (30 mL), the organic phase was directly washed with saturated brine (20 mL), then dried over anhydrous sodium sulfate, filtered and concentrated, the obtained residue was purified by silica gel column chromatography with PE/EA (v/v = 87/17) eluent, the resulting mixture was concentrated to obtain 0.86 g of a yellow solid, with a yield of 79.14%. $^1$H NMR (400MHz, $CDCl_3$): δ 8.17 (d, *J*=4.4Hz, 1H), 6.95 (s, 1H), 3.34 (s, 3H), 3.08 (s, 3H), 1.53 (s, 9H).

Step 2 to Step 12: Synthesis of Compound **16**

For the synthesis steps from step 2 to step 12, refer to the synthesis steps 2 to step 12 of compound **9** in Example 9. Finally, 25 mg of a yellow solid **16** was obtained. LC-MS (ESI, pos. ion) m/z: 708.5 $[M+H]^+$; $^1$H NMR(400MHz, CD3OD): δ7.89-7.85 (m, 1H), 7.35-7.30 (m, 2H), 7.16 (br.s, 1H), 5.43-5.29 (m, 1H), 4.58 (br.s, 3H), 4.42-4.29 (m, 2H), 3.84-3.54 (m, 4H), 3.44-3.37 (m, 5H), 3.16-3.07 (m, 2H), 2.93-2.93 (m, 3H), 2.46-2.18 (m, 4H), 2.08-1.85 (m, 6H).

## Example 17: Synthesis of Compound 17

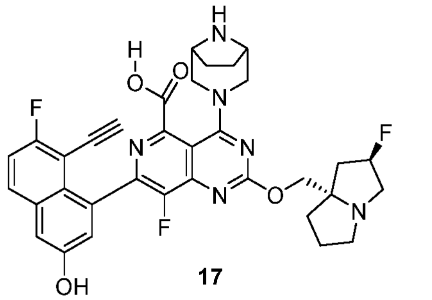

17

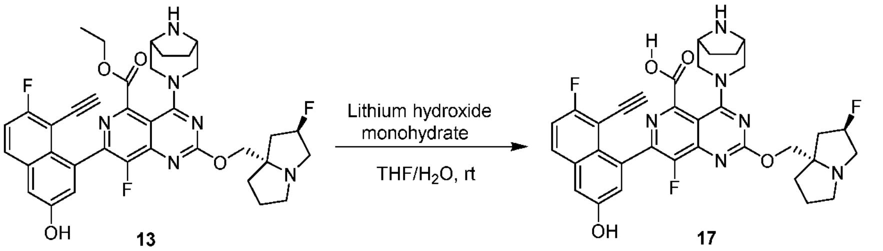


13

17

Compound **13** (65.0 mg, 0.097mmol) and lithium hydroxide monohydrate (16.28mg, 0.39mmol) were dissolved in THF (4mL) and $H_2O$ (0.5 mL), and the mixture was stirred at 25 °C for 24 h. Then the reaction was stopped, the reaction solution was concentrated and added with water (2 mL), saturated ammonium chloride solution was added to adjust the pH to 6.8, then a large amount of solid was precipitated, the mixture was filtered with suction, the filter cake was washed with 2 mL water, and dried to obtain 16.0 mg of a light yellow solid, with a yield of 25.6%. LC-MS (ESI, neg. ion) m/z: 643.1 $[M-H]^-$; $^1H$ NMR (400 MHz, $CD_3OD$): δ 7.90 - 7.83 (m, 1H), 7.40 - 7.29 (m, 2H), 7.25 (d, *J* = 4.8 Hz, 1H), 5.36 (d, *J* = 53.1 Hz, 1H), 4.74 - 4.56 (m, 2H), 4.43 - 4.27 (m, 2H), 3.86 - 3.67 (m, 3H), 3.44 - 3.36 (m, 2H), 3.30 - 3.24 (m, 1H), 3.05-3.15 (m, 1H), 2.39 - 2.02 (m, 6H), 1.99 - 1.58 (m, 6H); $^{19}F$ NMR (376 MHz, $CD_3OD$): δ -111.90, -127.14, -149.36.

**Example 18: Synthesis of Compound 18**

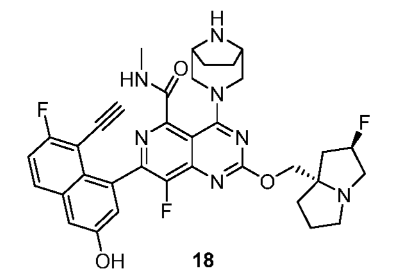

18

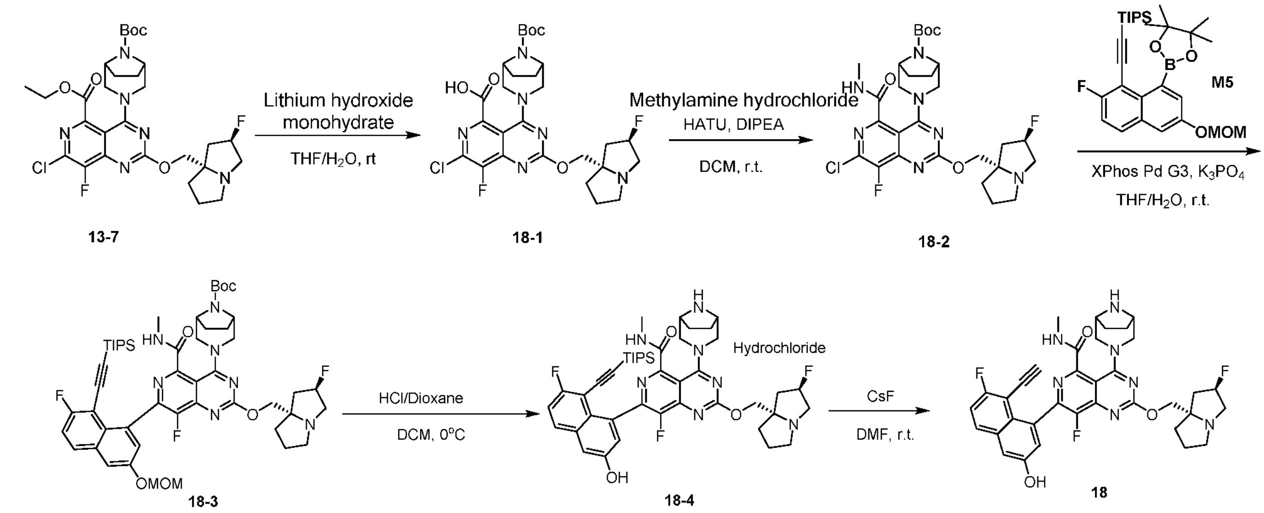


Step 1: Synthesis of Compound **18-1**

Compound **13-7** (400mg, 0.64mmol) and lithium hydroxide monohydrate (107.42mg, 2.56mmol) were dissolved in THF (6mL) and $H_2O$ (2mL), and the mixture was stirred at 30°C for 5 h. 2 M hydrochloric acid was added to adjust the pH to 4, then extracted with DCM (20 mL × 3), the combined organic phase was dried over anhydrous sodium sulfate, then filtered and concentrated to obtain 280 mg of a light yellow solid, which was used directly for the next step. LC-MS (ESI, pos. ion) m/z: 595.3 $[M+H]^+$.

Step 2: Synthesis of Compound **18-2**

HATU (228.14 mg, 0.60 mmol) and DIPEA (0.25 mL, 1.5 mmol) were added to a solution of Compound **18-1** in DCM (6 mL), the mixture was stirred at 25 °C for 5 min, then methylamine hydrochloride (21.88 mg, 0.60 mmol) was added and the resulting mixture was stirred at 25 °C for 8 h. The reaction solution was diluted with DCM (20 mL), then washed with saturated ammonium chloride solution (10 mL × 2) and saturated brine (10 mL × 2) in sequence, the resulting mixture was dried over anhydrous sodium sulfate, then filtered and concentrated, and then the mixture was separated by silica gel column chromatography (DCM/MeOH (v/v = 9/1)) to obtain 110 mg of a yellow-white solid, with a two-step yield of 21.5%. LC-MS (ESI, pos. ion) m/z: 608.3 $[M+H]^+$.

Step 3: Synthesis of Compound **18-3**

Compound **18-2** (110 mg, 0.18 mmol), **M5** (184.51 mg, 0.36 mmol, Anaiji Chemical), potassium phosphate (114.63 mg, 0.54 mmol) and Xphos G3 (76.18 mg, 0.090 mmol) were added to a 10 mL two-neck bottle, the bottle was replaced with $N_2$ three times, then THF (4 mL) and $H_2O$ (0.5 mL) were added, and the mixture was stirred at 30°C for 24 h. The reaction solution was directly poured into DCM (20 mL) and water (10 mL) for extraction, the organic phase was dried with anhydrous sodium sulfate, then concentrated and separated by silica gel column chromatography (DCM/MeOH (v/v =15/1)) to obtain 120.0 mg of a brown solid, with a yield of 69.23%. LC-MS (ESI, pos. ion) m/z: 958.8 $[M+H]^+$.

Step 4: Synthesis of Compound **18-4**

Compound **18-3** (80 mg, 0.083 mmol) and dichloromethane (2 mL) were added to a 25 mL single-neck bottle, the mixture was stirred to dissolve, then hydrochloric acid/1,4-dioxane (0.32 mL, 10.51 mmol, 4mol/L) was added and the mixture was stirred under ice bath for 2 h. The reaction solution was directly drained to obtain 62.0 mg of a yellow solid, with a yield of 91.22%. LC-MS (ESI, pos. ion) m/z: 814.7 $[M+H]^+$.

Step 5: Synthesis of Compound **18**

Compound **18-4** (62 mg, 0.076 mmol), DMF (2 mL) and cesium fluoride (115.44 mg, 0.76 mmol) were added to a 10 mL single-neck bottle, the mixture was stirred at 25°C for 12 h. Then dichloromethane (30 mL) and methanol (2 mL) were added, the reaction solution was washed with saturated brine (10 mL × 3) and separated by column chromatography (DCM/MeOH (v/v =3/1)) to obtain 15 mg of a light yellow solid, with a yield of 29.96%. LC-MS (ESI, pos. ion) m/z: 658.3 $[M+H]^+$; $^1H$ NMR (400 MHz, $CD_3OD$): δ 7.87 (dd, *J* = 9.1, 5.8 Hz, 1H), 7.34 (dd, *J* = 14.3, 5.6 Hz, 2H), 7.27 (s, 1H), 5.41 (d, *J* = 54.3 Hz, 1H), 4.43 (m, 2H), 3.77 - 3.40 (m, 6H), 3.20 (m, 2H), 2.93 (s, 3H), 2.54 - 2.32 (m, 2H), 2.29 - 1.77 (m, 9H), 1.60 (br. s, 2H); $^{19}F$ NMR (376 MHz, $CD_3OD$): δ -111.45 (s, 1F), -137.71 (s, 1F), -173.75 (s, 1F).

**Example 19: Synthesis of Compound 19**

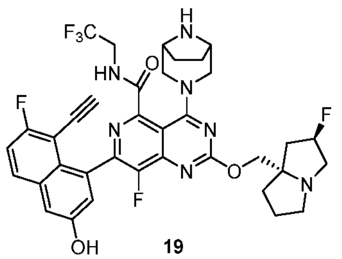

19

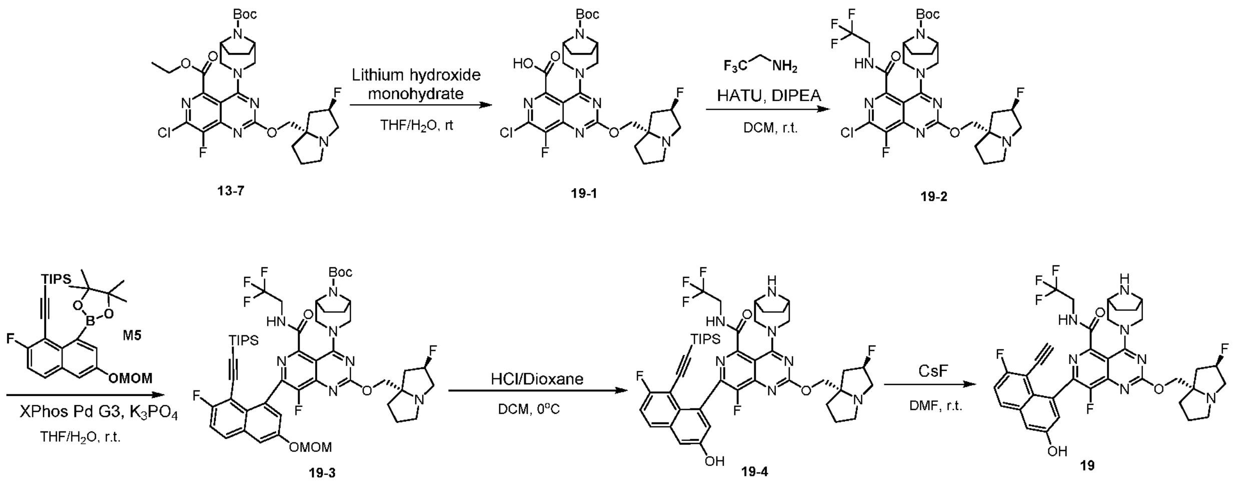


13-7 19-1 19-2 19-3 19-4 19

The synthesis of compound **19** can refer to the synthesis of compound **18** in Example 18, methylamine hydrochloride, the initial raw material in step 2, was replaced with 2,2,2-trifluoroethylamine, and finally 4 mg of a light yellow solid was obtained, the yield of the last step was 45.48%. LC-MS (ESI, pos. ion) m/z: 726.3 $[M+H]^+$; $^1H$ NMR (400 MHz, $CD_3OD$): δ 7.87 (dd, *J* = 9.2, 5.8 Hz, 1H), 7.34 (dd, *J* = 14.6, 5.7 Hz, 2H), 7.29 (d, *J* = 1.1 Hz, 1H), 5.48 - 5.43 (d, *J* = 52.3 Hz, 1H), 5.33 (s, 1H), 4.59 (s, 2H), 4.46 - 4.35 (m, 2H), 4.25-3.81 (m, 3H), 3.51 (m, 4H), 3.14 (m, 2H), 2.40 - 2.18 (m, 4H), 2.08 (m, 4H), 1.81 (m, 2H), 1.60 (m, 2H).

**Example 20: Synthesis of Compound 20**

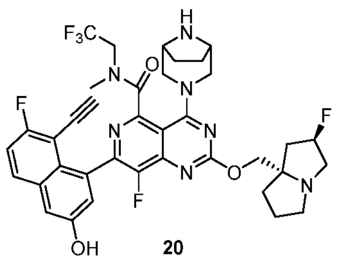

20

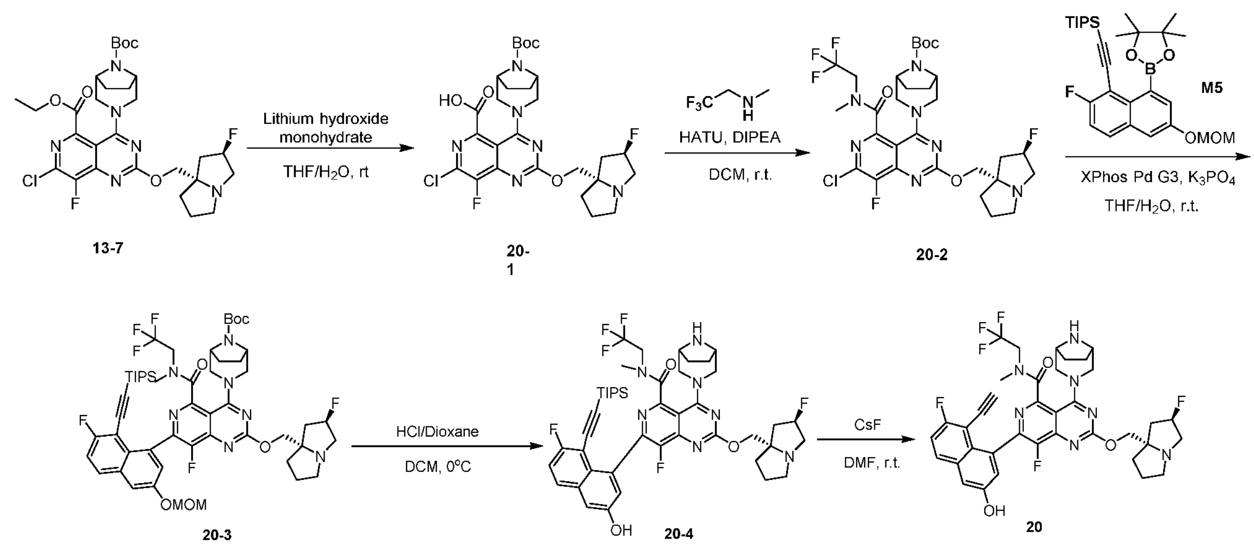


13-7 20-1 20-2 20-3 20-4 20

The synthesis of compound **20** can refer to the synthesis of compound **18** in Example 18, methylamine hydrochloride, the initial raw material in step 2, was replaced with *N*-methyl-*N*-2,2,2-trifluoromethylethylamine hydrochloride, and finally 28.5 mg of a light yellow solid was obtained, the yield of the last step was 53.11%. LC-MS (ESI, pos. ion) m/z: 740.3 $[M+H]^+$; $^1H$ NMR (400 MHz, $CD_3OD$): δ 7.88 (dd, *J* = 9.1, 5.8 Hz, 1H), 7.34 (dd, *J* = 15.9, 5.9 Hz, 2H), 7.24 (d, *J* = 2.2 Hz, 1H), 5.48 - 5.27 (d, *J* = 52.3 Hz, 1H)), 4.39 (m, 2H), 4.25 - 3.99 (m, 2H), 3.57 (m, 4H), 3.40 (s, 3H), 3.22 - 3.07 (m, 2H), 2.64 - 2.19 (m, 5H), 2.18 - 1.86 (m, 5H), 1.85 - 1.66 (m, 3H), 1.64 - 1.47 (m, 2H); $^{19}F$ NMR (376 MHz, $CD_3OD$) δ -70.26 (s, 3F), -111.51 - -111.56 (m, 1F), -137.67 (s, 1F), -173.90 (s, 1F).

**Example 21: Synthesis of Compound 21**

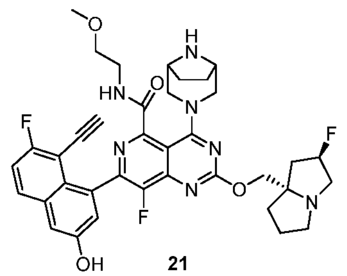

21

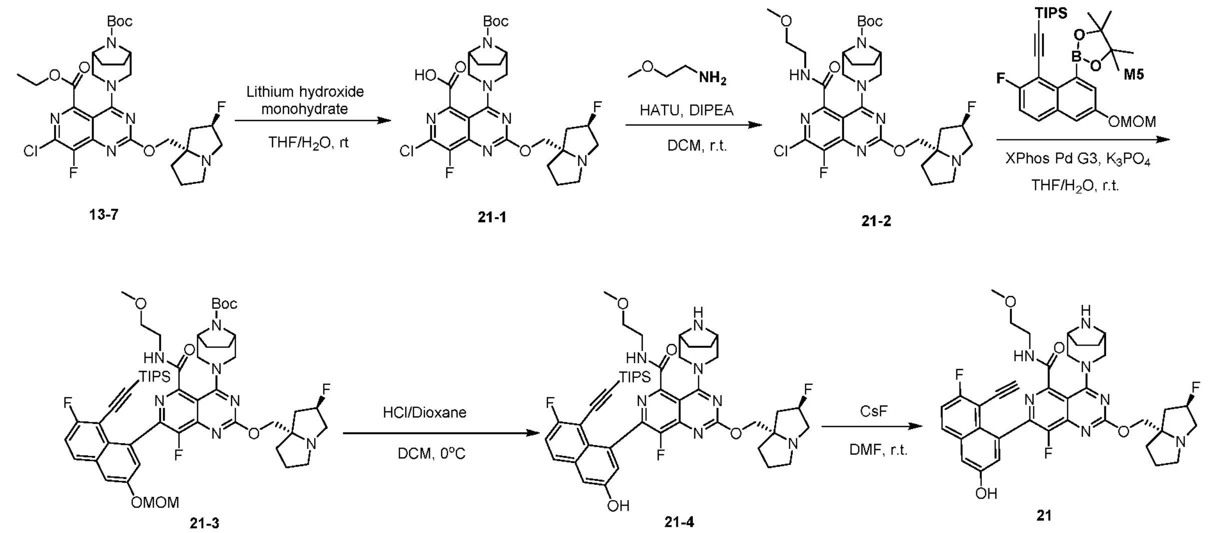


13-7 21-1 21-2 21-3 21-4 21

The synthesis of compound **21** can refer to the synthesis of compound **18** in Example 18, methylamine hydrochloride, the initial raw material in step 2, was replaced with 2-methoxyethylamine, and finally 13.0 mg of a light yellow solid was obtained, the yield of the last step was 35.33%. LC-MS (ESI, pos. ion) m/z: 702.3 $[M+H]^+$; $^1H$ NMR (400 MHz, $CD_3OD$): δ 7.87 (dd, *J* = 9.1, 5.7 Hz, 1H), 7.34 (dd, *J* = 14.9, 5.8 Hz, 2H), 7.27 (s, 1H), 5.31-5.47 (d, *J* = 53.4 Hz, 1H), 4.80-4.71 (m, 3H), 4.37 (m, 2H), 3.67 - 3.58 (m, 3H), 3.56 (s, 3H), 3.50 - 3.37 (m, 3H), 3.35 (s, 2H), 3.23-3.18 (m, 1H), 3.13 (m, 1H), 2.48 - 2.17 (m, 5H), 2.15 - 1.90 (m, 5H), 1.83 - 1.73 (m, 2H).

**Example 22: Synthesis of Compound 22**

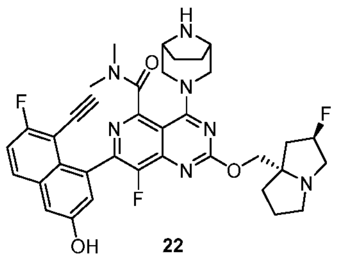

22

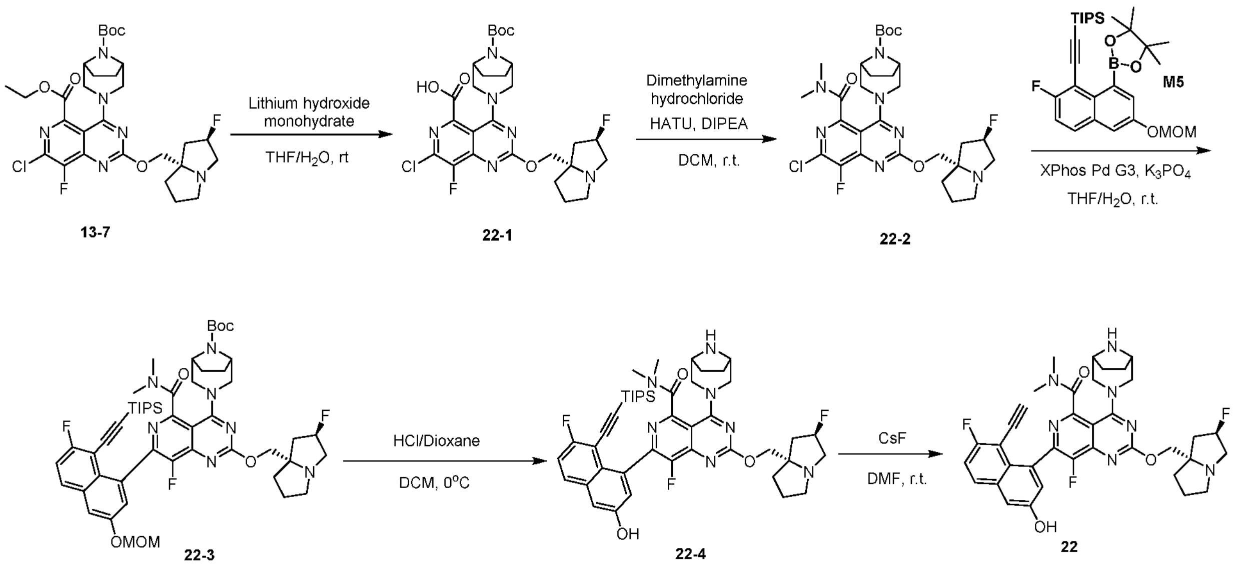


The synthesis of compound **22** can refer to the synthesis of compound **18** in Example 18, methylamine hydrochloride, the initial raw material in step 2, was replaced with dimethylamine hydrochloride, and finally 30.0 mg of a light yellow solid was obtained with a yield of 78.62%. LC-MS (ESI, pos. ion) m/z: 672.3 $[M+H]^+$; $^1H$ NMR (400MHz, $CD_3OD$): δ7.91-7.87 (m, 1H), 7.38-7.32 (m, 2H), 7.25-7.24 (m, 1H), 5.45-5.31 (m, 1H), 4.42-4.30 (m, 2H), 3.68 (br. s, 2H), 3.57-3.37 (m, 5H), 3.30 (s, 3H), 3.14 (s, 3H), 2.46-1.62 (m, 14H); $^{19}F$ NMR (400MHz, $CD_3OD$): δ -100.20, -111.64, - 173.74.

**Example 23: Synthesis of Compound 23**

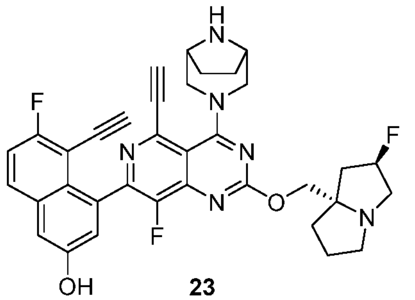

23

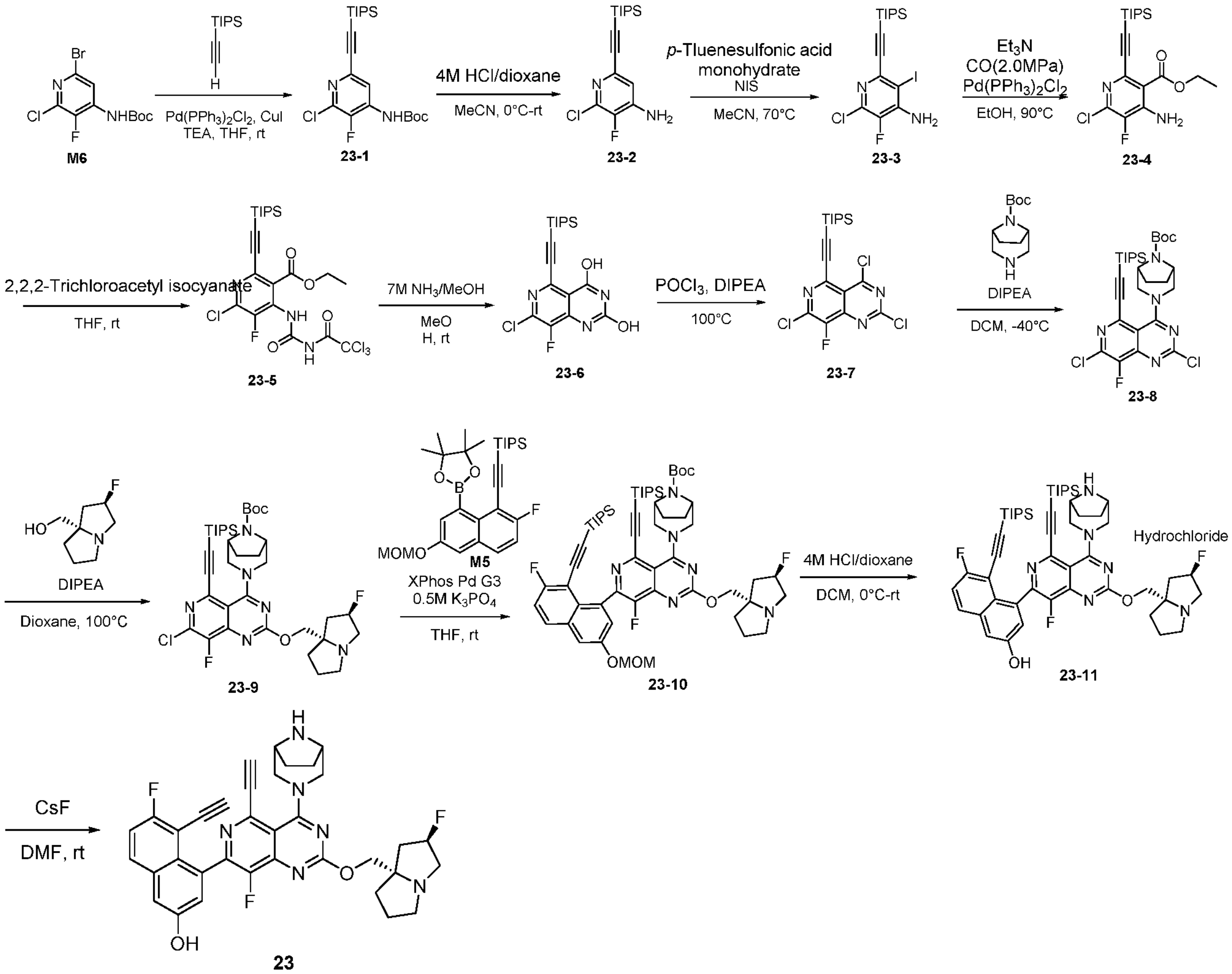


Step 1: Synthesis of Compound **23-1**

**M6** (1.0g, 3.07mmol), $Pd(PPh_3)_2Cl_2$ (0.43g, 0.61mmol) and CuI (0.12 g, 0.61mmol) were added to a 25 mL two-neck bottle, the bottle was replaced with $N_2$ three times, and then anhydrous THF (10 mL), TEA (0.93g, 9.21mmol) and triisopropylsilyl acetylene (0.73g, 3.99mmol) were added, the bottle was replaced with $N_2$ three times again, and the resulting mixture was stirred at room temperature for 4 h. Then saturated ammonium chloride (5 mL) was added and the reaction solution was extracted twice by EA (25 mL), the combined organic phase was dried and separated by silica gel column chromatography with PE/EA (v/v = 100/0 - 98/2) eluent to obtain 1.24g of yellow liquid, with a yield of 94.5%. $^1H$ NMR (400 MHz, $CDCl_3$): $\delta$ 8.23 (d, $J$ = 5.0 Hz, 1H), 7.07 (s, 1H), 1.53 (s, 9H), 1.17 - 1.13 (m, 21H).

Step 2: Synthesis of Compound **23-2**

Compound **23-1** (1.24 g, 2.90 mmol) and acetonitrile (20 mL) were added to a 50 mL single-neck bottle, the mixture was stirred, and then a solution of hydrochloric acid in 1,4-dioxane (3.63 mL, 14.5 mmol, 4 M) was slowly added

at 0°C, the resulting mixture was stirred for 18 h at room temperature. The precipitated yellow solid was filtered, the filter cake was washed with EA (3 mL) and dried, then 0.79 g of a white solid was obtained, with a yield of 83.2%. LC-MS (ESI, pos. ion) m/z: 327.3 $[M+H]^+$.

Step 3: Synthesis of Compound **23-3**

Compound **23-2** (0.79 g, 2.42 mmol), *p*-toluenesulfonic acid monohydrate (0.046 g, 0.24 mmol), NIS (0.98 g, 4.36 mmol) and acetonitrile (20 mL) were added to a 50 mL single-neck bottle, and the mixture was heated to 70°C and stirred for 6 h. Then the reaction was stopped, acetonitrile was spin-dried, and EA (30 mL) was added to dissolve, the organic phase was washed with sodium bisulfite (20 mL), then washed with saturated sodium chloride (20 mL) once again, and washed with 20 mL water again, and the organic phase was dried and concentrated to obtain 1.06 g of light brown liquid, with a yield of 96.9%. $^1H$ NMR (400 MHz, $CDCl_3$): δ 4.97 (s, 2H), 1.17 - 1.13 (m, 21H).

Step 4: Synthesis of Compound **23-4**

Compound **23-3** (1.06g, 2.34mmol), $Pd(PPh_3)_2Cl_2$ (0.33g, 0.47mmol), TEA (0.85g, 8.42mmol) and EtOH (20mL) were added to a 500 mL autoclave, the autoclave was replaced with $N_2$ and CO in sequence, then filled with CO to make the pressure inside the autoclave at 2 MPa, and the mixture was heated to 90 °C and stirred for 16 h. Then stop heating, the mixture was cooled to room temperature, the autoclave was released CO gas and opened, then silica gel was added, and the resulting mixture was separated by column chromatography with PE/DCM (v/v = 100/0 - 80/20) eluent to obtain 0.75 g of light yellow liquid, with a yield of 80.3%. $^1H$ NMR (400 MHz, $CDCl_3$): δ 6.14 (s, 2H), 4.44 (q, *J* = 7.1 Hz, 2H), 1.40 (t, *J* = 7.1 Hz, 3H), 1.17 - 1.13 (m, 21H).

Step 5: Synthesis of Compound **23-5**

2,2,2-Trichloroacetyl isocyanate (0.22 g, 1.18 mmol) was added to a solution of Compound **23-4** (0.365 g, 0.91 mmol) in THF (6 mL) in a 50mL single-neck bottle, the mixture was stirred at room temperature for 0.5 h, and then spin-dried and directly used for the next step, the yield was calculated as 100%.

Step 6: Synthesis of Compound **23-6**

Compound **23-5** (0.54 g, 0.92 mmol), methanol (6 mL) and methanol solution of ammonia (0.66 mL, 4.60 mmol, 7 M) were added to a 50 mL single-neck bottle, the mixture was stirred at room temperature for 0.5 h, then spin-dried, the obtained white solid was slurried by 10 mL PE/EA (v/v = 90/10) for 0.5 h, and 0.32 g of white solid was obtained after filtration, with a yield of 87.9%.

LC-MS (ESI, pos. ion) m/z: 396.2 $[M+H]^+$.

Step 7: Synthesis of Compound **23-7**

Compound **23-6** (0.200g, 0.51 mmol, purity 100%), DIPEA (0.34 mL, 2.04 mmol), toluene (5 mL) and phosphorus oxychloride (0.19 mL, 2.04 mmol) were added to a 50 mL single-neck bottle, and the mixture was heated to 100 °C and stirred for 2 h. Then stop heating, the resulting mixture was spin-dried and directly used for the next step.

Step 8: Synthesis of Compound **23-8**

DCM (8 mL) was added to the crude product Compound **23-7** (0.22 g, 0.51 mmol) obtained in the previous step, the mixture was cooled to -40°C, then DIPEA (0.33 g, 2.55 mmol) and *tert*-butyl 3,8-diazabicyclo[3.2.1]octane-8-carboxylate (0.097 g, 0.46 mmol) in DCM (2 mL) were added and the resulting mixture was stirred at -40 °C for 30 min. Then saturated ammonium chloride was added to quench the reaction, the organic phase was separated by column chromatography (EA/PE (v/v=1/20) to obtain 0.20 g of yellow viscous liquid, with a yield of 64.6%. LC-MS (ESI, pos. ion) m/z: 608.3 $[M+H]^+$.

Step 9: Synthesis of Compound **23-9**

Compound **23-8** (0.2 g, 0.33 mmol), ((2*R*,7a*S*)-2-fluorotetrahydro-1*H*-pyrrolizin-7a(5*H*)-yl)methanol (0.11g, 0.38 mmol) DIPEA (0.16 mL, 0.99 mmol) and 1,4-dioxane (2 mL) were added to a 25 mL single-neck bottle, the bottle was replaced with $N_2$ three times, and the mixture was reacted at 85°C for 12 h. After the reaction was stopped, the reaction solution was quenched with saturated ammonium chloride and extracted with EA, the organic phase was dried and

filtered over anhydrous sodium sulfate and concentrated, the resulting mixture was separated by silica gel column chromatography with DCM/MeOH (v/v = 100/0 - 90/10) eluent to obtain 130 mg of yellow solid product, with a yield of 54.09%. LC-MS (ESI, pos. ion) m/z: 731.7 $[M+H]^+$.

Step 10: Synthesis of Compound **23-10**

Compound **23-9** (0.13 g, 0.17 mmol), **M5** (0.14 g, 0.27 mmol), XPhos G3 (0.046 g, 0.054 mmol), anhydrous potassium phosphate (0.076g, 0.36mmol) and THF (2 mL) were added to a 25 mL single-neck bottle, the mixture was reacted at room temperature under $N_2$ for 24 h. After the reaction was stopped, the reaction solution was quenched with saturated ammonium chloride and extracted with EA, the resulting mixture was separated by silica gel column chromatography with DCM/MeOH (v/v = 100/0 - 98/02) as eluent to obtain 150 mg of red-black solid, with a yield of 78.03%. LC-MS (ESI, pos. ion) m/z: 541.5 $[M+2H]^{2+}$.

Step 11: Synthesis of Compound **23-11**

Compound **23-10** (0.15 g, 0.14 mmol) and DCM (1 mL) were added to a 25 mL single-neck bottle, and then HCl/dioxane (1 mL, 4 mmol, 4 M) was added dropwise at 0 °C, the mixture was stirred at room temperature for 1 h. Then stirring was stopped, the resulting mixture was concentrated to obtain 130 mg of a yellow solid. The yield was calculated as 100% and the yellow solid was directly used for the next step. LC-MS (ESI, pos. ion) m/z: 937.8 $[M+H]^+$.

Step 12: Synthesis of Compound **23**

Compound **23-11** (0.13g, 0.14mmol), cesium fluoride (0.32g, 2.1mmol) and DMF (1 mL) were added to a 25 mL single-neck bottle, and the mixture was stirred at room temperature for 12 h. After the reaction was stopped, the reaction solution was separated by a reverse silica gel column with $H_2O$/MeCN (v/v = 100/0 - 80/20) eluent to obtain 26 mg of a yellow solid. LC-MS (ESI, pos. ion) m/z: 625.3 $[M+H]^+$; $^1$H NMR (400 MHz, DMSO-$d_6$): $\delta$ 7.85 - 7.76 (m, 1H), 7.38 - 7.30 (m, 1H), 7.22 - 7.11 (m, 2H), 5.28 (d, *J* = 54.6 Hz, 1H), 4.72 (br.s, 1H), 4.17 - 4.08 (m, 2H), 4.05 - 3.99 (m, 1H), 3.70 (s, 1H), 3.13 - 3.05 (m, 3H), 3.03 - 2.97 (m, 2H), 2.92 - 2.70 (m, 5H), 2.15 - 1.96 (m, 4H), 1.90 - 1.71 (m, 4H), 1.60 - 1.48 (m, 2H).

**Example 24: Synthesis of Compound 24**

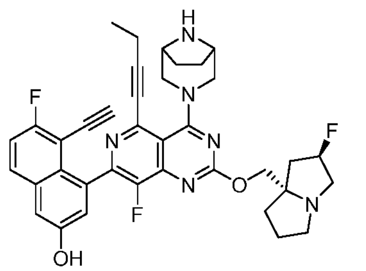

**24**

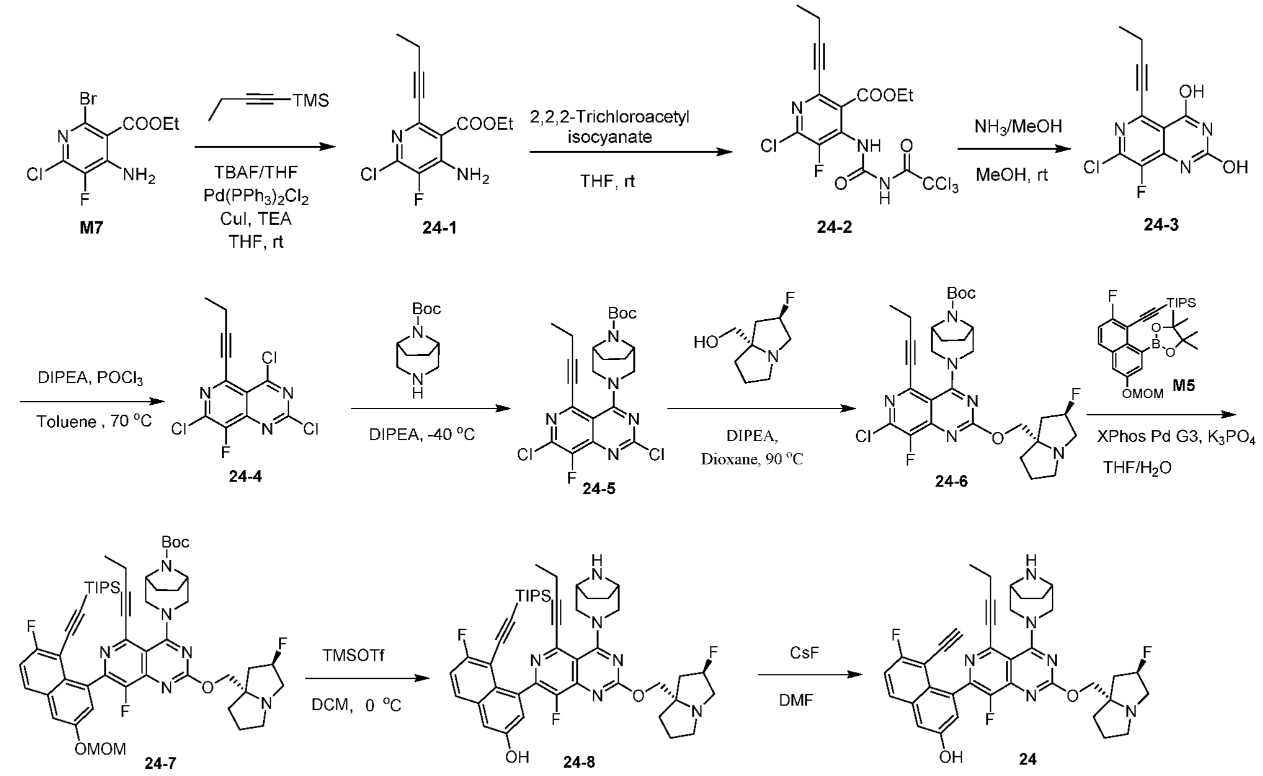


Step 1: Synthesis of Compound **24-1**

**M1** (705.1 mg, 2.37 mmol), (but-1-yn-1-yl)trimethylsilane (448.89 mg, 3.56 mmol), $Pd(PPh_3)_2Cl_2$ (332.70 mg, 0.47 mmol), CuI (90.27 mg, 0.47 mmol), TEA (1.64 mL, 11.85 mmol) and anhydrous THF (10 mL) were added to a 50 mL single-neck bottle, the bottle was replaced with $N_2$ three times, and then a solution of tetrabutyl ammonium fluoride in THF (2.84 mL, 2.84 mmol, 1 M) was added at 25°C and the mixture was stirred at room temperature 30 °C for 6 h. After the reaction was stopped, the reaction solution was filtered through a celite pad, the filtrate was dried over anhydrous sodium sulfate and spin-dried, the resulting mixture was purified by column chromatography with PE/DCM (v/v = 100/1-2/1) eluent to obtain 354 mg of a yellow solid 24-1, with a yield of 55.2%. LC-MS (ESI, pos. ion) m/z: 271.3 $[M+H]^+$; $^1H$ NMR (400 MHz, $CDCl_3$): δ 6.25 (s, 2H), 4.46 - 4.34 (m, 2H), 2.46 (q, *J* = 7.5 Hz, 2H), 1.46 - 1.37 (m, 3H), 1.25 (m, 3H).

Step 2: Synthesis of Compound **24-2**

2,2,2-Trichloroacetyl isocyanate (365 mg, 1.94 mmol) was added to a solution of Compound **24-1** (350 mg, 1.29 mmol) in anhydrous THF (6 mL) at a 50 mL single-neck bottle, the mixture was stirred at room temperature 30°C for 1 h. After the reaction was stopped, the reaction solution was spin-dried and the resulting mixture was directly used for the next step, the yield was calculated as 100%. LC-MS (ESI, pos. ion) m/z: 460.0 $[M+H]^+$.

Step 3: Synthesis of Compound **24-3**

Methanol (6 mL) was added to Compound **24-2** obtained in the previous step, the mixture was stirred to dissolve, then methanol solution of ammonia (1.84 mL, 12.9 mmol, 7 M) was added, the resulting mixture was stirred at room temperature 30 °C for 2 h. After the reaction was stopped, and the reaction solution was concentrated, the obtained crude product was slurried with MTBE (10 mL) for 0.5 h, then filtered and dried to obtain 320 mg of a white solid Compound **24-3**, the yield in steps 2 and 3 was 92.5%. LC-MS (ESI, pos. ion) m/z: 268.1 $[M+H]^+$.

Step 4: Synthesis of Compound **24-4**

Phosphorus oxychloride (1.02 mL, 11.5 mmol) and DIPEA (0.93 mL, 5.60 mmol) were added to a solution of Compound **24-3** (300 mg, 1.12 mmol) in anhydrous toluene (6 mL) in a 50 mL single-neck bottle, the mixture was heated to 70 °C and stirred for 1 h, after the reaction was stopped, and the reaction solution was cooled and concentrated, the resulting mixture was directly used for the next step, the yield was calculated as 100%.

Step 5: Synthesis of Compound **24-5**

Anhydrous dichloromethane (10 mL) was added to Compound **24-4** obtained in the previous step, the bottle was replaced with $N_2$ three times, and the mixture was stirred and cooled to -40°C, then DIPEA (0.93 mL, 5.6 mmol) and a solution of *tert*-butyl 3,8-diazabicyclo[3.2.1]octane-8-carboxylate (238 mg, 1.12 mmol) in DCM (2 mL) were added. The reaction was stopped after the mixture was stirred at -40°C for 0.5 h, then 20 mL saturated ammonium chloride was added to quench the reaction, then the mixture was returned to room temperature, and DCM (20 mL × 2) was added for extraction, the separated organic phase was dried over anhydrous sodium sulfate and concentrated, then purified by column chromatography with PE/EA (v/v = 9/1) eluent to obtain 496 mg of a yellow solid Compound **24-5**, with a two-step yield of 85.4%. LC-MS (ESI, pos. ion) m/z: 480.1 $[M+H]^+$.

Step 6: Synthesis of Compound **24-6**

Compound **24-5** (450 mg, 0.94 mmol), ((2*R*, 7a*S*)-2-fluorohexahydro-1*H*-pyrrolizin-7a-yl)methanol (299 mg, 1.88 mmol), DIPEA (0.47 mL, 2.82 mmol) and anhydrous 1,4-dioxane (4 mL) were added to a 50 mL single-neck bottle, the bottle was replaced with $N_2$ three times, and the mixture was heated to 90 °C and stirred for 12 h. The reaction solution was cooled to room temperature and 15 mL of saturated chlorine ammonium was added after the reaction was stopped, then extracted with EA (15 mL × 3), the combined organic phase was dried over anhydrous sodium sulfate and concentrated, and purified by column chromatography with PE/EA (v/v = 100/1-1/1) eluent to obtain 200 mg of a yellow foamy solid Compound **24-6**, with a yield of 35.4%. LC-MS (ESI, pos. ion) m/z: 603.5 $[M+H]^+$.

Step 7: Synthesis of Compound **24-7**

Compound **24-6** (180 mg, 0.30 mmol), (2-(2-fluoro-6-(methoxymethoxy)-8-(tetramethyl-1,3,2-dioxaborolan-2-yl)naphthalene-1-yl)ethynyl)tris(prop-2-yl)silane (307.5 mg, 0.60 mmol), Xphos Pd G3 (127 mg, 0.15 mmol), $K_3PO_4$ (191 mg, 0.90 mmol) and THF/$H_2O$ (6 mL/0.5 mL) were added to a 25 mL single-neck bottle, the bottle was replaced with $N_2$ three times, and the mixture was stirred at room temperature 30 °C for 14 h, then the reaction was stopped, the resulting mixture was filtered and added with 10 mL saturated ammonium chloride and extracted by EA (10 mL × 3), the combined organic phase was dried over anhydrous sodium sulfate, then concentrated and purified by column chromatography with DCM/MeOH (v/v = 100/0-40/1) eluent to obtain 140 mg of a tan solid **24-7**, with a yield of 49.2%. LC-MS (ESI, pos. ion) m/z: 954.4 $[M+H]^+$.

Step 8: Synthesis of Compound **24-8**

TMSOTf (0.08 mL, 0.45 mmol) was added to a solution of Compound **24-7** (140 mg, 0.15 mmol) in acetonitrile (5 mL) at 0°C in a 25 mL single-neck bottle, the mixture was stirred at 0°C for 0.5 h, then the reaction was stopped, the resulting mixture was filtered and added with 10 mL saturated sodium bicarbonate and extracted with DCM (10 mL × 3), the combined organic phase was dried over anhydrous sodium sulfate and concentrated, the resulting mixture was directly used for the next step, the yield was calculated as 100%, LC-MS (ESI, pos. ion) m/z: 809.4 $[M+H]^+$.

Step 9: Synthesis of Compound **24**

Compound **24-8** (110 mg, 0.14 mmol) obtained in the previous step, cesium fluoride (213 mg, 1.4 mmol) and DMF (1 mL) were added to a 25 mL single-neck bottle, the mixture was stirred at room temperature 30 °C for 6 h, then 8 mL of saturated brine was added after the reaction was stopped, the resulting mixture was extracted with DCM/MeOH (30 mL/2 mL), and the organic phase was washed with saturated brine (10 mL × 4). The organic phase was dried over anhydrous sodium sulfate and concentrated, the resulting mixture was separated and purified by thin-layer chromatography with DCM/MeOH/ammonia (v/v = 10/1/0.05) eluent to obtain 31 mg of a brown solid Compound 24, with a yield of 34.7%. LC-MS (ESI, pos. ion) m/z: 653.3 $[M+H]^+$; HRMS(ESI): 653.2835 $[M+H]^+$; $^1H$ NMR (400 MHz, $CD_3OD$): δ 7.87 (dd, *J* = 9.1, 5.7 Hz, 1H), 7.38 - 7.29 (m, 2H), 7.21 (s, 1H), 5.43 (d, *J* = 51.2 Hz, 1H), 4.72 - 4.53 (m, 2H), 4.53 - 4.40 (m, 3H), 4.14 - 3.91 (m, 2H), 3.91 - 3.74 (m, 3H), 3.68 - 3.59 (m, 1H), 3.58 - 3.41 (m, 3H), 3.27 - 3.16 (m, 1H), 2.59 (q, *J* = 7.5 Hz, 3H), 2.53 - 2.35 (m, 2H), 2.31 - 2.22 (m, 1H), 2.21 - 2.08 (m, 2H), 2.08 - 1.75 (m, 5H). $^{19}F$ NMR (376 MHz, $CD_3OD$): δ -111.505 (1F), -140.12 (1F), -173.78 (1F).

**Example 25: Synthesis of Compound 25**

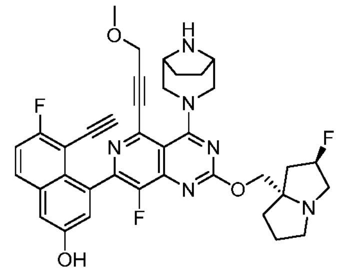

25

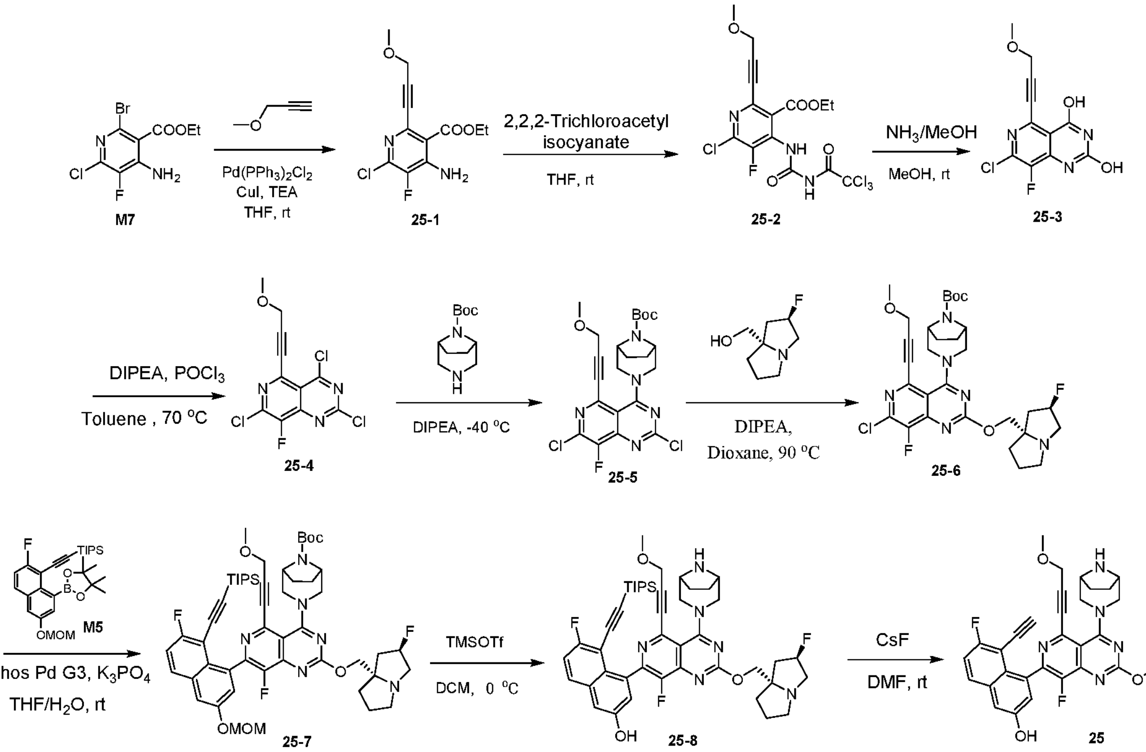


Step 1: Synthesis of Compound **25-1**

**M7** (1.2 g, 4.03 mmol), $Pd(PPh_3)_2Cl_2$ (0.566 g, 0.81 mmol) and CuI (0.153 g, 0.81 mmol) were added to a 100 mL double-neck bottle, the bottle was replaced with $N_2$ three times, and the mixture was added with methyl propargyl ether (0.51 mL, 6.04 mmol), TEA (1.22 g, 12.09 mmol) and anhydrous THF (20 mL), the bottle was replaced with $N_2$ three times again, the reaction solution was stirred at room temperature for 1 h. After the reaction was stopped, the mixture was filtered through a celite pad, and the filtrate was dried with anhydrous sodium sulfate and spin-dried, the resulting mixture was purified by column chromatography with PE/EA (v/v = 80/1-92/8) eluent to obtain 0.478 g of a dark yellow solid Compound **25-1**, with a yield of 41%. $^1H$ NMR (400 MHz, $CDCl_3$): δ 6.31 (s, 2H), 4.42 (q, *J* = 7.1 Hz, 2H), 4.34 (s, 2H), 3.44 (s, 3H), 1.43 (t, *J* = 7.1 Hz, 3H).

Step 2: Synthesis of Compound **25-2**

2,2,2-Trichloroacetyl isocyanate (0.30 mL, 2.5 mmol) was added to a solution of Compound **25-1** (0.478 g, 1.67 mmol) in anhydrous THF (15 mL) in a 50 mL single-neck bottle, the mixture was stirred at room temperature for 0.5 h, then the reaction was stopped, the resulting mixture was spin-dried and directly used for the next step, the yield was calculated as 100%.

Step 3: Synthesis of Compound **25-3**

Methanol (15 mL) was added to the Compound **25-2** obtained in the previous step, the mixture was stirred and added with methanol solution of ammonia (1.19 mL, 8.35 mmol, 7 M), then the resulting mixture was stirred at room

temperature for 0.5 h. After the reaction was stopped, the crude product after concentration was slurried by MTBE (10 mL) for 0.5 h, the resulting mixture was filtered and dried to obtain 394 mg of a yellow solid Compound **25-3**, with a two-step yield of 83%. LC-MS (ESI, neg. ion) m/z: 282.1 $[M-H]^-$;$^1H$ NMR (400 MHz, DMSO-$d_6$): δ 4.39 (s, 2H), 3.40 (s, 3H).

Step 4: Synthesis of Compound **25-4**

Phosphorus oxychloride (0.38 mL, 4.17 mmol) and DIPEA (1.15 mL, 6.95 mmol) were added to a solution of Compound **25-3** (394 mg, 1.39 mmol) in anhydrous toluene (10 mL) in a 50 mL single-neck bottle, the mixture was heated to 70 °C and stirred for 1 h, after the reaction was stopped, and the reaction solution was cooled and concentrated, the resulting mixture was directly used for the next step, the yield was calculated as 100%.

Step 5: Synthesis of Compound **25-5**

Anhydrous dichloromethane (8 mL) was added to Compound **25-4** (445 mg, 1.39mmol) obtained in the previous step, the bottle was replaced with $N_2$ three times, and the mixture was stirred and cooled to -40°C, and then DIPEA (1.15 mL, 6.95 mmol) and a solution of tert-butyl 3,8-diazabicyclo[3.2.1]octane-8-carboxylate (295 mg, 1.39 mmol) in DCM (2 mL) were added. The reaction was stopped after stirring for 0.5 h at -40°C, then 20 mL saturated ammonium chloride was added to quench the reaction, and DCM (20 mL × 2) was added for extraction at room temperature, the separated organic phase was dried over anhydrous sodium sulfate and concentrated, the resulting mixture was purified by column chromatography with PE/EA (v/v = 7/1) eluent to obtain 345 mg of a yellow solid Compound **25-5**, the yield in steps 4 and 5 was 50%. LC-MS (ESI, pos. ion) m/z: 496.1 $[M+H]^+$.

Step 6: Synthesis of Compound **25-6**

Compound **25-5** (345 mg, 0.70 mmol), ((2*R*, 7a*S*)-2-fluorohexahydro-1*H*-pyrrolizin-7a-yl)methanol (222.9 mg, 1.4 mmol), DIPEA (0.35 mL, 2.1 mmol) and anhydrous 1,4-dioxane (6 mL) were added to a 50 mL single-neck bottle, the bottle was replaced with $N_2$ three times, and the mixture was heated to 90 °C and stirred for 14 h. The mixture was cooled to room temperature after the reaction was stopped, and added with 15 mL of saturated chlorine ammonium, and extracted with EA (15 mL × 3), the combined organic phase was dried over anhydrous sodium sulfate and concentrated, the resulting mixture was purified by column chromatography with DCM/MeOH (v/v = 100/0-20/1) eluent to obtain 271 mg of yellow oil Compound **25-6**, with a yield of 62%. LC-MS (ESI, pos. ion) m/z: 619.3 $[M+H]^+$.

Step 7: Synthesis of Compound **25-7**

Compound **25-6** (271 mg, 0.44 mmol), **M5** (338.3 mg, 0.66 mmol, Anaiji Chemical), Xphos Pd G3 (74.49 mg, 0.088 mmol), $K_3PO_4$ (233.5 mg, 1.1 mmol) and THF (4 mL) were added to a 25 mL single-neck bottle, the bottle was replaced with $N_2$ three times, and the mixture was stirred at room temperature for 14 h, then the reaction was stopped, the reaction solution was filtered and added with 10 mL saturated ammonium chloride, then extracted with EA (10 mL × 3), the combined organic phase was dried over anhydrous sodium sulfate and concentrated, the resulting mixture was purified by column chromatography with DCM/MeOH (v/v = 100/0-80/1) eluent to obtain 210 mg of a yellow solid Compound **25-7**, with a yield of 49%. LC-MS (ESI, pos. ion) m/z: 969.5 $[M+H]^+$.

Step 8: Synthesis of Compound **25-8**

Compound **25-7** (150 mg, 0.15 mmol) and acetonitrile (3 mL) were added to a 25 mL single-neck bottle, the mixture was cooled to 0 °C, then TMSOTf (0.081 mL, 0.45 mmol) was added and the resulting mixture was stirred at 0 °C for 2 h, and filtered after the reaction was stopped, then 10 mL saturated sodium bicarbonate was added and extracted with DCM (10 mL × 3), the combined organic phase was dried over anhydrous sodium sulfate and concentrated, the resulting mixture was directly used for the next step, the yield was calculated as 100%, LC-MS (ESI, pos. ion) m/z: 825.9 $[M+H]^+$.

Step 9: Synthesis of Compound **25**

Compound **25-8** (127 mg, 0.15 mmol), cesium fluoride (228 mg, 1.5 mmol) and DMF (1 mL) were added to a 25 mL single-neck bottle, the mixture was stirred at room temperature for 12 h, then the reaction was stopped, to the mixture was added water with 8 mL, and solid was precipitated and then filtered, the precipitated solid was separated and purified by thin layer chromatography with DCM/MeOH (v/v = 7/1) eluent to obtain 21 mg of a red solid Compound **25**, with a yield of 19%. LC-MS (ESI, pos. ion) m/z: 669.3 $[M+H]^+$; RMS(ESI): 669.2806 $[M+H]^+$; $^1H$ NMR (400 MHz,

$CD_3OD$): δ 7.89 (dd, *J* = 9.1, 5.7 Hz, 1H), 7.42 - 7.30 (m, 2H), 7.24 (s, 1H), 5.45 (d, *J* = 53.2 Hz, 1H), 4.62 - 4.42 (m, 3H), 4.49 (s, 2H), 4.10 - 3.84 (m, 4H), 3.72 - 3.62 (m, 1H), 3.62 - 3.50 (m, 3H), 3.47 (s, 3H), 3.38 (s, 1H), 3.28 - 3.18 (m, 1H), 2.60 - 2.38 (m, 2H), 2.38 - 2.25 (m, 1H), 2.24 - 2.12 (m, 2H), 2.11 - 1.73 (m, 5H); $^{19}$F NMR (376 MHz, $CD_3OD$): δ -111.46 (1F), -138.63 (1F), -173.82 (1F).

**Example 26: Synthesis of Compound 26**

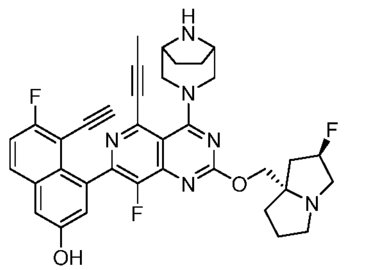

**26**

The synthesis of compound **26** can refer to the synthesis of compound **24** in Example 24, in step 1, the starting material 1-trimethylsilyl-1-butyne was replaced with 1-trimethylsilyl-1-propyne, and finally 15 mg of brown solid was obtained, and the yield of the last step was 27.31%. LC-MS (ESI, pos. ion) m/z: 639.3 $[M+H]^+$; HRMS(ESI): 639.2624 $[M+H]^+$; $^1$H NMR (400 MHz, $CD_3OD$): δ 7.87 (dd, *J* = 9.1, 5.7 Hz, 1H), 7.39 - 7.30 (m, 2H), 7.21 (s, 1H), 5.54 - 5.35 (m, 1H), 4.69 - 4.38 (m, 4H), 4.09 - 3.80 (m, 4H), 3.70 - 3.45 (m, 4H), 3.26 - 3.19 (m, 1H), 2.59 - 2.23 (m, 4H), 2.20 (s, 3H), 2.18 - 2.10 (m, 2H), 2.08 - 1.99 (m, 2H), 1.95 - 1.85 (m, 2H). $^{19}$F NMR (376 MHz, $CD_3OD$): δ -111.52 (1F), -140.12 (1F), -173.92 (1F).

**Example 27: Synthesis of Compound 27**

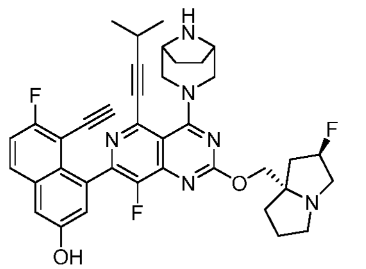

**27**

The synthesis of compound **27** can refer to the synthesis of compound **25** in Example 25, methyl propargyl ether, the initial raw material in step 1 was replaced with 3-methyl-1-butyne, and finally 10 mg of brown solid was obtained, the yield of the last step was 26.38%. LC-MS (ESI, pos. ion) m/z: 667.3 $[M+H]^+$; HRMS(ESI): 667.3028 $[M+H]^+$.

**Example 28: Synthesis of Compound 28**

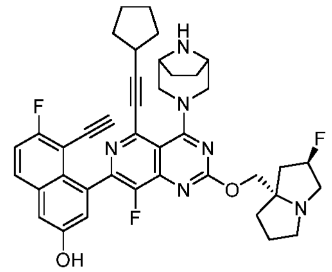

**28**

The synthesis of compound **28** can be referred to the synthesis of compound **25** in Example 25, methyl propargyl ether, the initial raw material in step 1 was replaced with cyclopentylacetylene, and finally 35 mg of brown solid was obtained, and the yield of the last step was 36%. LC-MS (ESI, pos. ion) m/z: 693.3 $[M+H]^+$; HRMS(ESI): 693.3199 $[M+H]^+$; $^1H$ NMR (599 MHz, $CD_3OD$): δ 7.90 - 7.84 (m, 1H), 7.37 - 7.30 (m, 2H), 7.22 (s, 1H), 5.37 (d, *J* = 53.4 Hz, 1H), 4.72 - 4.51 (m, 2H), 4.45 - 4.27 (m, 3H), 3.74 - 3.59 (m, 3H), 3.48 - 3.34 (m, 3H), 3.30 - 3.27 (m, 1H), 3.16 - 3.09 (m, 1H), 3.03 - 2.96 (m, 1H), 2.45 - 2.15 (m, 4H), 2.14 - 1.87 (m, 6H), 1.85 - 1.72 (m, 6H), 1.69 - 1.62 (m, 2H).

**Example 29: Synthesis of Compound 29**

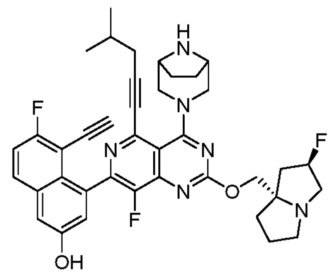

**29**

The synthesis of compound **29** can be referred to the synthesis of compound **25** in Example 25, methyl propargyl ether, the initial raw material in step 1 was replaced with 4-methyl-1-pentyne, and finally 110 mg of brown solid was obtained, and the yield of the last step was 79.6%. LC-MS (ESI, pos. ion) m/z: 681.3 $[M+H]^+$; HRMS(ESI): 681.3175 $[M+H]^+$; $^1H$ NMR (400 MHz, $CD_3OD$): δ 7.85 (dd, *J* = 9.0, 5.7 Hz, 1H), 7.37 - 7.28 (m, 2H), 7.22 (s, 1H), 5.31 (d, *J* = 54.0 Hz, 1H), 4.80 - 4.48 (m, 2H), 4.36 - 4.20 (m, 3H), 3.63 - 3.51 (m, 3H), 3.29 - 3.13 (m, 6H), 3.08 - 2.97 (m, 1H), 2.51 - 2.40 (m, 2H), 2.33 - 2.08 (m, 4H), 2.07 - 1.88 (m, 5H), 1.70 - 1.60 (m, 6H).

**Example 30: Synthesis of Compound 30**

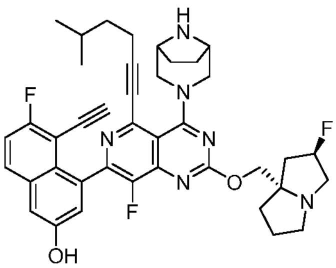

**30**

The synthesis of compound **30** can be referred to the synthesis of compound **25** in Example 25, methyl propargyl ether, the initial raw material in step 1 was replaced with 5-methyl-1-hexyne, and finally 33 mg of yellow solid was obtained, and the yield of the last step was 80%. LC-MS (ESI, pos. ion) m/z: 695.6 $[M+H]^+$; HRMS(ESI): 695.3311

$[M+H]^+$; $^1H$ NMR (400 MHz, $CDCl_3$): δ 7.70 - 7.58 (m, 1H), 7.37 - 7.27 (m, 1H), 7.21 - 7.10 (m, 2H), 5.42 - 5.19 (m, 1H), 4.41 - 4.17 (m, 3H), 4.14 - 3.81 (m, 2H), 3.75 - 3.15 (m, 7H), 3.12 - 2.81 (m, 2H), 2.80 - 2.68 (m, 1H), 2.51 - 2.38 (m, 3H), 2.37 - 2.10 (m, 7H), 2.09 - 1.88 (m, 9H).

**Example 31: Synthesis of Compound 31**

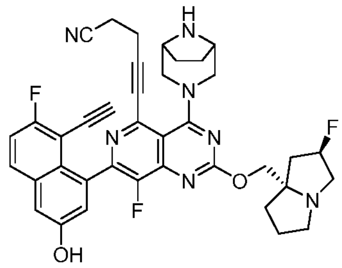

**31**

The synthesis of compound **31** can be referred to the synthesis of compound **25** in Example 25, methyl propargyl ether, the initial raw material in step 1 was replaced with 4-cyano-1-butyne, and finally 10 mg of brown solid was obtained. LC-MS (ESI, neg. ion) m/z: 676.3 $[M-H]^-$; HRMS(ESI): 678.2801 $[M+H]^+$.

**Example 32: Synthesis of Compound 32**

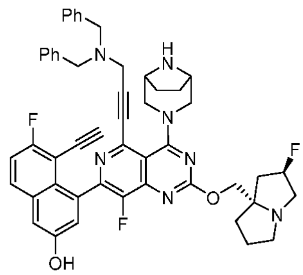

**32**

The synthesis of compound **32** can be referred to the synthesis of compound **25** in Example 25, methyl propargyl ether, the initial raw material in step 1 was replaced with *N,N*-dibenzylprop-2-yn-1-amine, and finally 35 mg of brown solid was obtained, and the yield of the last step was 29.7%. LC-MS (ESI, pos. ion) m/z: 834.7 $[M+H]^+$; HRMS(ESI): 834.371 $[M+H]^+$; $^1H$ NMR (400 MHz, $CD_3OD$): δ 7.89 (dd, *J* = 9.1, 5.8 Hz, 1H), 7.45 - 7.34 (m, 6H), 7.34 - 7.26 (m, 5H), 7.26 - 7.20 (m, 2H), 5.56 (d, *J* = 52.0 Hz, 1H), 4.69 - 4.44 (m, 3H), 4.21 - 3.93 (m, 5H), 3.92 - 3.77 (m, 3H), 3.75 (s, 4H), 3.68 (s, 2H), 3.51 - 3.46 (m, 1H), 3.45 - 3.37 (m, 1H), 2.70 - 2.42 (m, 3H), 2.36 - 2.27 (m, 2H), 2.21 - 1.94 (m, 5H).

**Example 33: Synthesis of Compound 33**

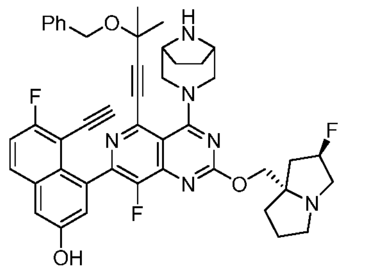

**33**

The synthesis of compound **33** can be referred to the synthesis of compound **25** in Example 25, methyl propargyl ether, the initial raw material in step 1 was replaced with (((2-methylbut-3-yn-2-yl)oxy)methyl)benzene, and finally 13 mg of brown solid was obtained, and the yield of the last step was 20.7%. LC-MS (ESI, pos. ion) m/z: 773.3 $[M+H]^+$; HRMS(ESI): 773.3415 $[M+H]^+$; $^1H$ NMR (400 MHz, $CD_3OD$): δ 7.91 (dd, *J* = 9.1, 5.7 Hz, 1H), 7.42 - 7.33 (m, 4H), 7.33 - 7.20 (m, 4H), 5.55 (d, *J* = 52.3 Hz, 1H), 4.73 - 4.59 (m, 5H), 4.07 - 3.86 (m, 4H), 3.85 - 3.74 (m, 3H), 3.71 - 3.57 (m, 1H), 3.48 - 3.35 (m, 2H), 2.75 - 2.36 (m, 5H), 2.34 - 2.11 (m, 5H), 2.08 - 1.75 (m, 6H).

**Example 34: Synthesis of Compound 34**

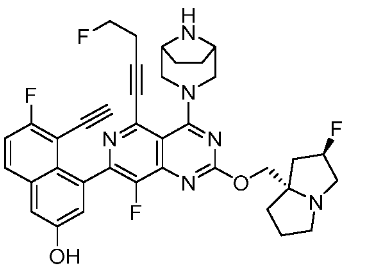

**34**

The synthesis of compound **34** can be referred to the synthesis of compound **25** in Example 25, methyl propargyl ether, the initial raw material in step 1 was replaced with 4-fluoro-butyne, and finally 11 mg of yellow brown solid was obtained. LC-MS (ESI, pos. ion) m/z: 671.3 $[M+H]^+$; HRMS(ESI): 671.2734 $[M+H]^+$= 671.2758.

**Example 35: Synthesis of Compound 35**

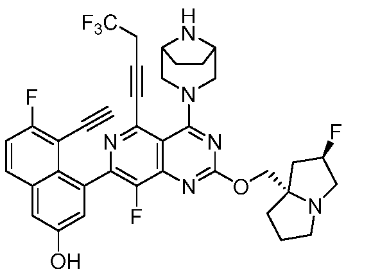

**35**

The synthesis of compound **35** can be referred to the synthesis of compound **25** in Example 25, methyl propargyl ether, the initial raw material in step 1 was replaced with 1-triisopropylsilyl-3-trifluoromethyl-propyne, and finally 9 mg of yellow solid was obtained. LC-MS (ESI, pos. ion) m/z: 707.4 $[M+H]^+$; HRMS(ESI): 707.2544 $[M+H]^+$.

**Example 36: Synthesis of Compound 36**

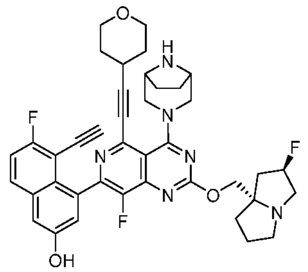

36

The synthesis of compound **36** can be referred to the synthesis of compound **25** in Example 25, methyl propargyl ether, the initial raw material in step 1 was replaced with 4-ethynyl-pyran, and finally 13 mg of brown solid was obtained. LC-MS (ESI, pos. ion) m/z: 709.4 $[M+H]^+$; HRMS(ESI): 709.3105 $[M+H]^+$.

**Example 37: Synthesis of Compound 37**

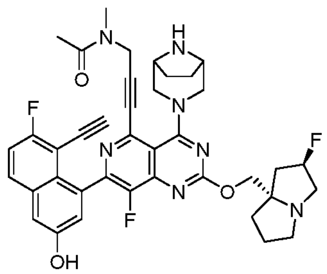

37

The synthesis of compound **37** can be referred to the synthesis of compound **25** in Example 25, methyl propargyl ether, the initial raw material in step 1 was replaced with *N*-methyl-*N*-(2-propynyl)acetamide, and finally 9 mg of yellow solid was obtained. LC-MS (ESI, pos. ion) m/z:710.3 $[M+H]^+$. HRMS(ESI): 710.3025 $[M+H]^+$= 710.3066.

**Example 38: Synthesis of Compound 38**

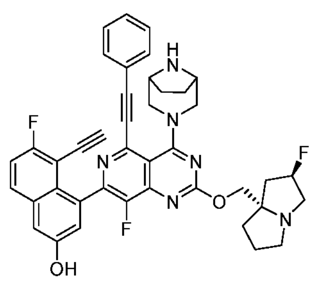

38

The synthesis of compound **38** can be referred to the synthesis of compound **25** in Example 25, methyl propargyl ether, the initial raw material in step 1 was replaced with phenylacetylene, and finally 45 mg of grey solid was obtained. LC-MS (ESI, pos. ion) m/z: 701.3 $[M+H]^+$; HRMS(ESI): 701.2839 $[M+H]^+$; $^1H$ NMR (400 MHz, $CD_3OD$): δ 7.90 (dd, *J* = 9.1, 5.7 Hz, 1H), 7.74 - 7.64 (m, 2H), 7.54 - 7.42 (m, 3H), 7.42 - 7.32 (m, 2H), 7.32 - 7.21 (m, 1H), 5.41 (d, *J* = 53.2 Hz, 1H), 4.69 - 4.50 (m, 3H), 4.51 - 4.37 (m, 2H), 4.13 - 3.94 (m, 1H), 3.81 - 3.71 (m, 2H), 3.58 - 3.39 (m, 3H), 3.39 - 3.36

(m, 1H), 3.22 - 3.12 (m, 1H), 2.52 - 2.19 (m, 4H), 2.18 - 1.96 (m, 4H), 1.91 - 1.81 (m, 2H); $^{19}$F NMR (376 MHz, MeOD): δ -111.40 (1F), -139.03 (1F), -173.74 (1F).

**Example 39: Synthesis of Compound 39**

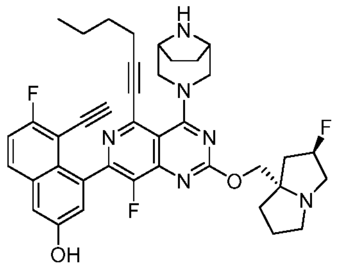

**39**

The synthesis of compound **39** can be referred to the synthesis of compound **25** in Example 25, methyl propargyl ether, the initial raw material in step 1 was replaced with 1-hexyne, and finally 40 mg of brown solid was obtained. LC-MS (ESI, pos. ion) m/z: 681.7 $[M+H]^+$; HRMS(ESI): 681.3140 $[M+H]^+$; $^1$H NMR (400 MHz, $CD_3OD$): δ 7.86 (dd, *J* = 9.1, 5.7 Hz, 1H), 7.37 - 7.29 (m, 2H), 7.21 (s, 1H), 5.35 (d, *J* = 53.6 Hz, 1H), 4.45 - 4.24 (m, 3H), 4.03 - 3.56 (m, 5H), 3.49 - 3.34 (m, 2H), 3.30 - 3.25 (m, 2H), 3.15 - 3.04 (m, 1H), 2.58 (t, *J* = 7.1 Hz, 2H), 2.47 - 2.14 (m, 4H), 2.11 - 1.87 (m, 4H), 1.69 - 1.61 (m, 3H), 1.55 - 1.43 (m, 3H), 0.95 (t, *J*= 7.3 Hz, 3H). $^{19}$F NMR (376 MHz, $CD_3OD$): δ -111.80 (1F), -140.25(1F), -173.70 (1F).

**Example 40: Synthesis of Compound 40**

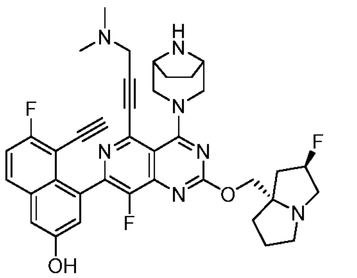

**40**

The synthesis of compound **40** can be referred to the synthesis of compound **25** in Example 25, methyl propargyl ether, the initial raw material in step 1 was replaced with 1-dimethylamino-2-propyne, and finally 11 mg of yellow solid was obtained. LC-MS (ESI, pos. ion) m/z: 682.3 $[M+H]^+$; HRMS(ESI): 682.3110 $[M+H]^+$.

**Example 41: Synthesis of Compound 41**

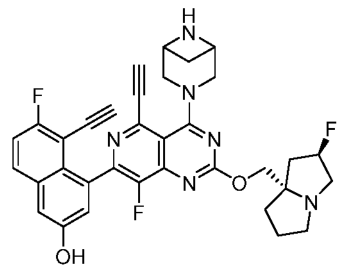

**41**

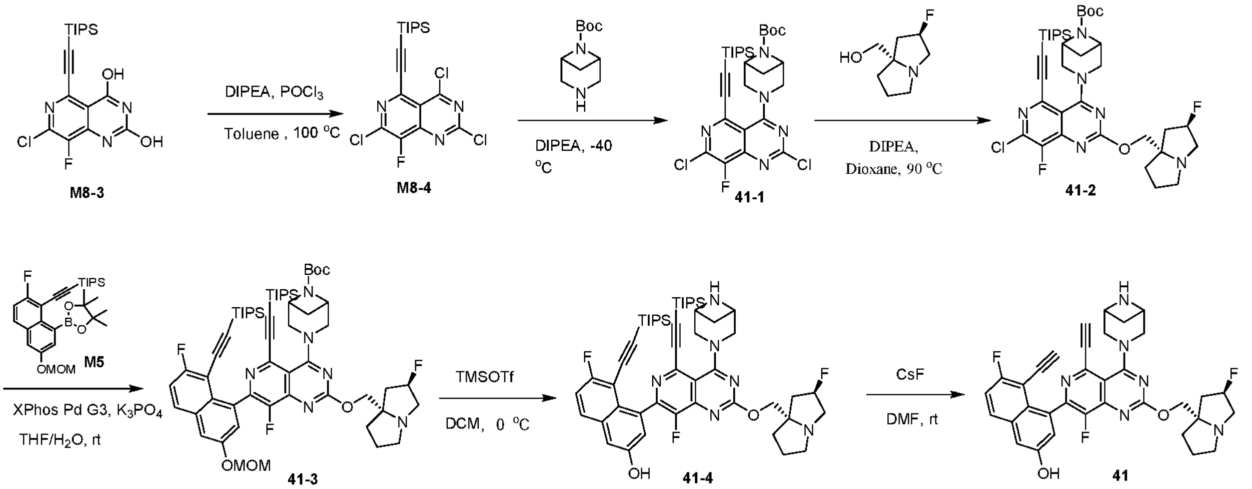


Step 1: Synthesis of Compound **M8-4**

Phosphorus oxychloride (0.46 mL, 5.04 mmol) and DIPEA (0.84 mL, 5.04 mmol) were added to a solution of **M8-3** (0.5 g, 1.26 mmol) in anhydrous toluene (5 mL) at a 25 mL single-neck bottle, then the mixture was heated to 100°C and stirred for 2 h, then the reaction was stopped, the resulting mixture was cooled and concentrated and used directly for the next step, the yield was calculated as 100%.

Step 2: Synthesis of Compound **41-1**

Anhydrous dichloromethane (10 mL) was added to the **M8-4** (0.55 g, 1.26 mmol) obtained in the previous step, the bottle was replaced with $N_2$ three times, and the mixture was stirred to dissolve, then cooled to -40 °C, and DIPEA (1.05 mL, 6.35 mmol) and a solution of *tert*-butyl 3,6-diazabicyclo[3.1.1]heptane-6-carboxylate (0.25 g, 1.26 mmol) in DCM (2 mL) were added. The resulting mixture was stirred at -40°C for 0.5 h, then the reaction was stopped, 10 mL saturated ammonium chloride was added to quench the reaction and the mixture was returned to room temperature, to the mixture was added with DCM (10 mL × 2) for extraction, the combined organic phase was dried and concentrated, and purified by column chromatography with PE/EA (v /v = 50/1-12/1) eluent to obtain 0.244 g of a yellow solid 41-1, the yield in steps 1 and 2 was 32%.LC-MS (ESI, pos. ion) m/z: 594.2 $[M+H]^+$.

Step 3: Synthesis of Compound **41-2**

Compound **41-1** (244 mg, 0.41 mmol), ((2*R*, 7a,*S*)-2-fluorohexahydro-1*H*-pyrrolizin-7a-yl)methanol (98 g, 9.86 mmol), DIPEA (0.20 mL, 1.23 mmol) and anhydrous dioxane (4 mL) were added to a 25 mL single-neck bottle, the bottle was replaced with $N_2$ three times, and the mixture was stirred at 90°C for 20 h. Then the reaction was stopped, the resulting mixture was cooled to room temperature, 10 mL of saturated ammonium chloride was added, and EA (10 mL × 3) was added for extraction, the combined organic phase was dried over anhydrous sodium sulfate and concentrated, and purified by column chromatography with DCM/MeOH (v/v = 100/1-30/1) eluent to obtain 233 mg of a yellow solid Compound **41-2**, the yield was 79%. LC-MS (ESI, pos. ion) m/z: 717.7 $[M+H]^+$.

Step 4: Synthesis of Compound **41-3**

Compound **41-2** (233 mg, 0.32 mmol), **M5** (250 mg, 0.48 mmol, Anaiji Chemical), Xphos Pd G3 (54 mg, 0.064 mmol), and $K_3PO_4$ (200 mg, 0.96mmol) and THF/$H_2O$ (2.5 mL/0.5 mL) were added to a 25 mL single-neck bottle, the bottle was replaced with $N_2$ three times, and the mixture was stirred at room temperature for 14 h, then the reaction was stopped, the resulting mixture was filtered, and 10 mL saturated ammonium chloride was added, and EA (10 mL × 3) was added for extraction, the combined organic phase was dried over anhydrous sodium sulfate and concentrated, and purified by column chromatography with DCM/MeOH (v/v = 100/0-80/1) eluent to obtain 140 mg of a yellow solid Compound **41-3**, with a yield of 40.4%. LC-MS (ESI, pos. ion) m/z: 1067.3 $[M+H]^+$.

Step 5: Synthesis of Compound **41-4**

Compound **41-3** (140 mg, 0.15 mmol) and DCM (3 mL) were added to a 25 mL single-neck bottle, the mixture was cooled to 0 °C, then TMSOTf (0.070 mL, 0.45 mmol) was added and the mixture was stirred at 0°C for 2 h, then the reaction was stopped, the resulting mixture was filterred and added with 10 mL saturated sodium bicarbonate, extracted with DCM (10 mL × 3), the combined organic phase was dried over anhydrous sodium sulfate and concentrated, and used directly for the next step, the yield was calculated as 100%, LC-MS (ESI, pos. ion) m/z: 923.8 $[M+H]^+$.

Step 6: Synthesis of Compound **41**

Compound **41-4** (120 mg, 0.13 mmol), cesium fluoride (300 mg, 1.95 mmol) and DMF (1 mL) were added to a 25 mL single-neck bottle, the mixture was stirred at room temperature for 12 h, then the reaction was stopped, 8 mL of water was added and solid was precipitated, then filtered, the precipitated solid was separated and purified by thin layer chromatography with DCM/MeOH (v/v = 7/1) eluent to obtain 19.5 mg of a brown-red solid Compound **41**, the total yield of steps 5 and 6 was 25%. LC-MS (ESI, pos. ion) m/z: 611.3 $[M+H]^+$; HRMS(ESI): 611.2398 $[M+H]^+$; $^1H$ NMR (400 MHz, $CD_3OD$): δ 7.88 (dd, *J* = 9.1, 5.8 Hz, 1H), 7.41 - 7.29 (m, 2H), 7.25 (s, 1H), 5.47 - 5.27 (m, 2H), 5.23 - 5.11 (m, 1H), 4.47 - 4.24 (m, 2H), 3.76 - 3.54 (m, 2H), 3.52 - 3.37 (m, 3H), 3.28 - 3.09 (m, 3H), 3.08 - 2.93 (m, 2H), 2.39 - 2.17 (m, 3H), 2.15 - 1.87 (m, 5H).

**Example 42: Synthesis of Compound 42**

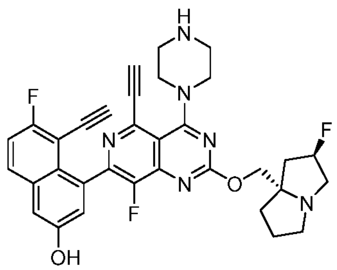

**42**

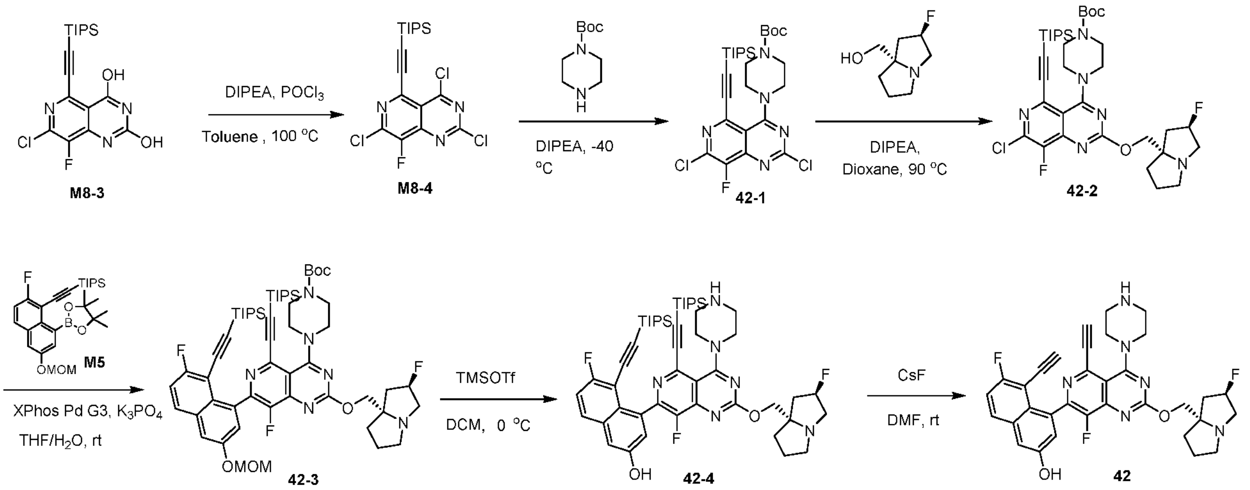


The synthesis of compound **42** can be referred to the synthesis of compound **41** in Example 41, *tert*-butyl 3,6-diazabicyclo[3.1.1]heptane-6-carboxylate, the initial raw material in step 2 was replaced with *tert*-butyl 1-piperazinecarboxylate, and finally 16 mg of yellow solid was obtained. LC-MS (ESI, pos. ion) m/z: 599.2 $[M+H]^+$; HRMS(ESI): 599.2376 $[M+H]^+$.

**Example 43: Synthesis of Compound 43**

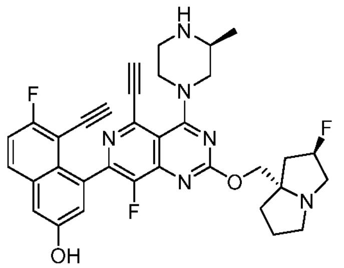

**43**

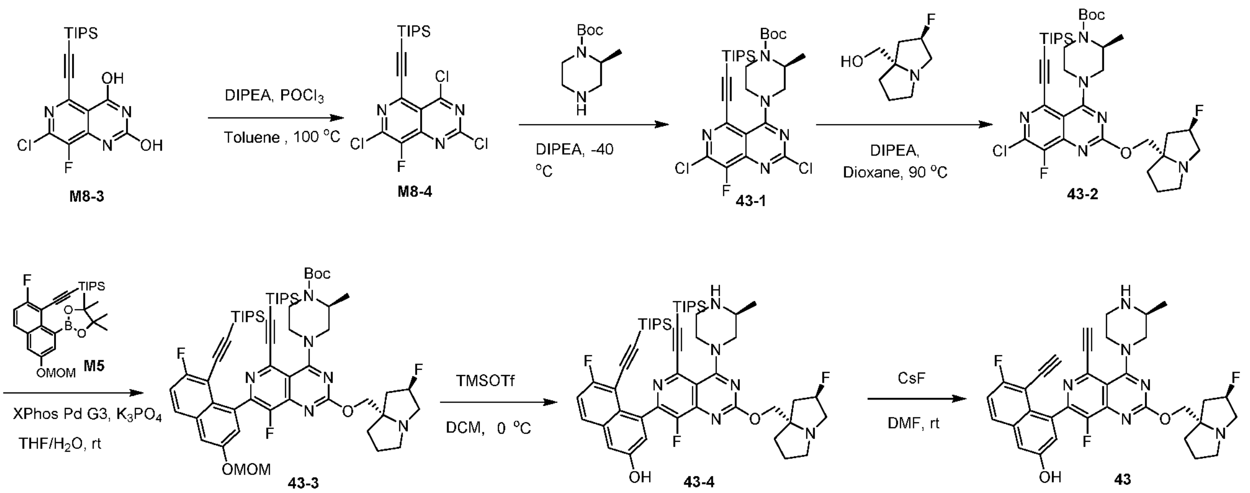


The synthesis of compound **43** can be referred to the synthesis of compound **41** in Example 41, *tert*-butyl 3,6-diazabicyclo[3.1.1]heptane-6-carboxylate, the initial raw material in step 2 was replaced with (*S*)-1-*tert*-butyl-2-methylpiperazine, and finally 7 mg of yellow solid was obtained. LC-MS (ESI, pos. ion) m/z: 613.3 $[M+H]^+$; HRMS(ESI): 613.2525 $[M+H]^+$.

**Example 44: Synthesis of Compound 44**

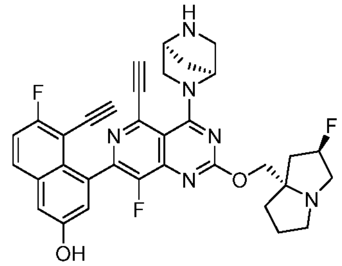

**44**

The synthesis of compound **44** can be referred to the synthesis of compound **41** in Example 41, *tert*-butyl 3,6-diazabicyclo[3.1.1]heptane-6-carboxylate, the initial raw material in step 2 was replaced with (1*S*,4*S*)-2-*tert*-butyl-2,5-diazabicyclo[2.2.1]heptane, and finally 10 mg of brown solid was obtained. LC-MS (ESI, pos. ion) m/z: 611.2 $[M+H]^+$; HRMS(ESI): 611.2363 $[M+H]^+$.

**Example 45: Synthesis of Compound 45**

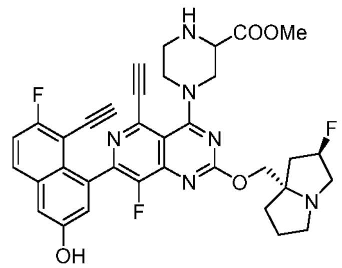

**45**

The synthesis of compound **45** can be referred to the synthesis of compound **41** in Example 41, *tert*-butyl 3,6-diazabicyclo[3.1.1]heptane-6-carboxylate, the initial raw material in step 2 was replaced with methyl *N*-*tert*-butyl-piperazine-2-carboxylate, and finally 8 mg of brown solid was obtained. LC-MS (ESI, pos. ion) m/z: 657.2 $[M+H]^+$; HRMS(ESI): 657.2417 $[M+H]^+$.

**Example 46: Synthesis of Compound 46**

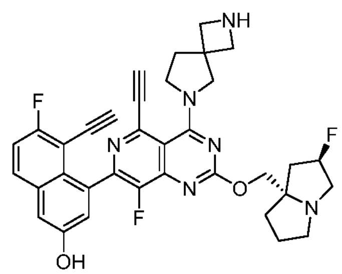

**46**

The synthesis of compound **46** can be referred to the synthesis of compound **41** in Example 41, *tert*-butyl 3,6-diazabicyclo[3.1.1]heptane-6-carboxylate, the initial raw material in step 2 was replaced with *tert*-butyl 2,6-diazaspiro[3.4]octane-2-carboxylate, and finally 7 mg of yellow solid was obtained. LC-MS (ESI, pos. ion) m/z: 625.5 $[M+H]^+$;

HRMS(ESI): 625.2530 [M+H]+.

**Example 47: Synthesis of Compound 47**

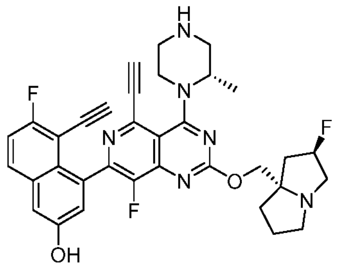

**47**

The synthesis of compound **47** can be referred to the synthesis of compound **41** in Example 41, *tert*-butyl 3,6-diazabicyclo[3.1.1]heptane-6-carboxylate, the initial raw material in step 2 was replaced with *tert*-butyl 2,6-diazaspiro[3.4]octane-2-carboxylate, and finally 10 mg of yellow solid was obtained. LC-MS (ESI, pos. ion) m/z: 613.3 [M+H]+; HRMS(ESI): 613.2522 [M+H]+.

**Example 48: Synthesis of Compound 48**

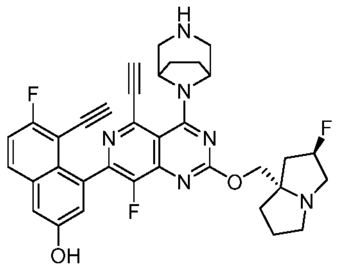

**48**

The synthesis of compound **48** can be referred to the synthesis of compound **41** in Example 41, *tert*-butyl 3,6-diazabicyclo[3.1.1]heptane-6-carboxylate, the initial raw material in step 2 was replaced with *tert*-butyl 3,8-diazabicyclo[3.2.1]octane-3-carboxylate, and finally 12 mg of yellow solid was obtained. LC-MS (ESI, pos. ion) m/z: 625.3 [M+H]+; HRMS(ESI): 625.2580 [M+H]+.

**Example 49: Synthesis of Compound 49**

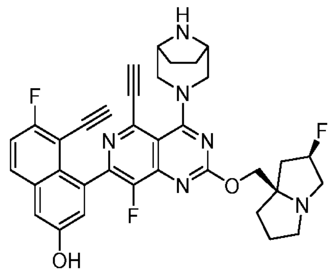

**49**

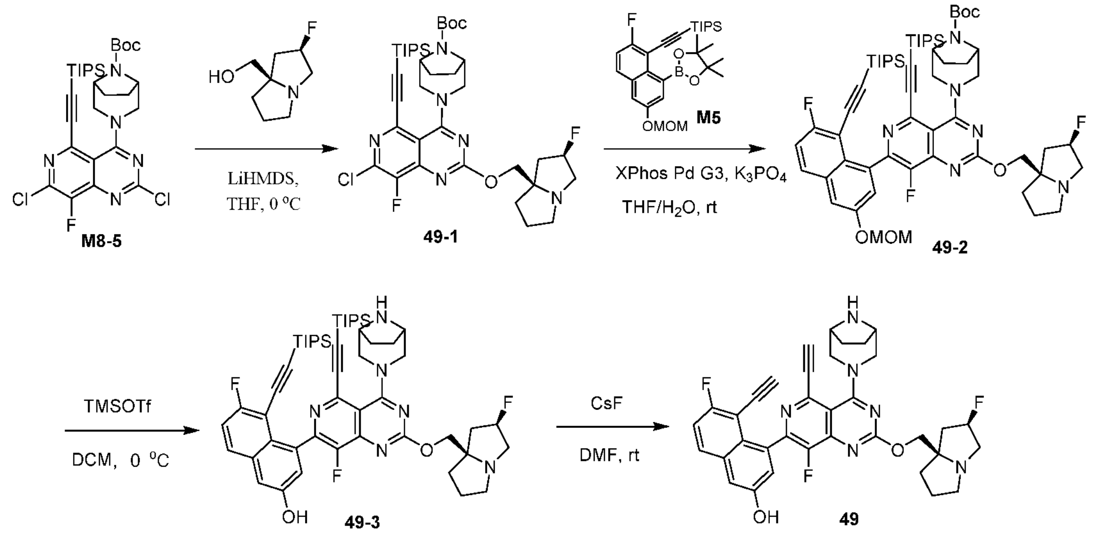


Step 1: Synthesis of Compound **49-1**

**M8-5** (220 mg, 0.36 mmol) and ((2*R*, 7a*S*)-2-fluorohexahydro-1*H*-pyrrolizin-7a-yl)methanol (69 mg, 0.43 mmol) were added to a 50 mL two-neck bottle, the bottle was replaced with $N_2$ three times, then anhydrous THF (5 mL) was added and the resulting mixture was cooled to 0°C, then a solution of lithium bistrimethylsilylamide in THF (1.2 mL, 1.2 mmol, 1 M) was added and the mixture was stirred at 0 °C for 12 h. Then the reaction was stopped, 20 mL saturated ammonium chloride was added and the mixture was added with EA (25 mL × 2) for extraction, the combined organic phase was dried and concentrated, and purified by column chromatography with DCM/MeOH (v/v = 100/0-98/2) eluent to obtain 150 mg of yellow oil Compound **49-1**, with a yield of 56%. LC-MS (ESI, pos. ion) m/z: 731.7 $[M+H]^+$.

Step 2: Synthesis of Compound **49-2**

Compound **49-1** (100 mg, 0.14 mmol), (2-(2-fluoro-6-(methoxymethoxy)-8-(tetramethyl-1,3,2-dioxaborolan-2-yl)naphthalene-1-yl)ethynyl)tris(prop-2-yl)silane (110 mg, 0.21 mmol), Xphos Pd G3 (24 mg, 0.028 mmol), $K_3PO_4$ (74 mg, 0.35 mmol), and THF (4mL) were added to a 25 mL single-neck bottle, the bottle was replaced with $N_2$ three times, and the mixture was stirred at room temperature for 12 h, then the reaction was stopped, and 10 mL saturated ammonium chloride was added, and EA (10 mL × 3) was added for extraction, the combined organic phase was dried over anhydrous sodium sulfate and concentrated, and purified by column chromatography with DCM/MeOH (v/v = 100/0-80/1) eluent to obtain 50 mg of a yellow solid Compound **49-2**, with a yield of 33%. LC-MS (ESI, pos. ion) m/z: 541.4 $[M+2H]^{2+}$.

Step 3: Synthesis of Compound **49-3**

Compound **49-2** (197 mg, 0.18 mmol) and DCM (4 mL) were added to a 25 mL single-neck bottle, the mixture was cooled to 0 °C, then TMSOTf (0.098 mL, 0.54 mmol) was added and the mixture was stirred at 0°C for 1 h, then the reaction was stopped, to the resulting mixture was added saturated sodium bicarbonate with 10 mL, extracted with DCM (10 mL × 3), the combined organic phase was dried over anhydrous sodium sulfate and concentrated, and used directly for the next step, the yield was calculated as 100%, LC-MS (ESI, pos. ion) m/z: 937.5 $[M+H]^+$.

Step 4: Synthesis of Compound **49**

Compound **49-3** (104 mg, 0.11 mmol), cesium fluoride (260 mg, 1.66 mmol) and DMF (1 mL) were added to a 25 mL single-neck bottle, the mixture was stirred at room temperature for 12 h, then the reaction was stopped, 8 mL of water was added and solid was precipitated, then filtered, the precipitated solid was separated and purified by thin layer chromatography with DCM/MeOH (v/v = 7/1) eluent to obtain 23 mg of a yellow solid Compound 49, with a yield of 33%. LC-MS (ESI, pos. ion) m/z: 625.3 $[M+H]^+$; HRMS(ESI): 625.2552 $[M+H]^+$; $^1H$ NMR (400 MHz, DMSO-$d_6$): δ 10.19 (br s, 1H), 8.07 - 7.89 (m, 1H), 7.55 - 7.35 (m, 2H), 7.23 (s, 1H), 5.49 - 5.23 (m, 1H), 4.60 - 4.05 (m, 4H), 3.90 - 3.75 (m, 2H), 3.70 - 3.46 (m, 3H), 2.99 - 2.53 (m, 3H), 2.37 - 2.15 (m, 2H), 2.03 - 1.77 (m, 6H), 1.71 - 1.43 (m, 5H).

**Example 50: Synthesis of Compound 50**

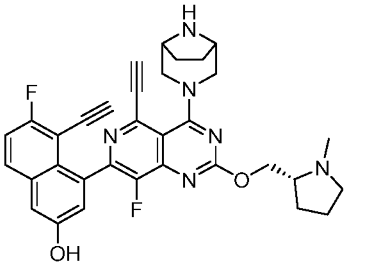

50

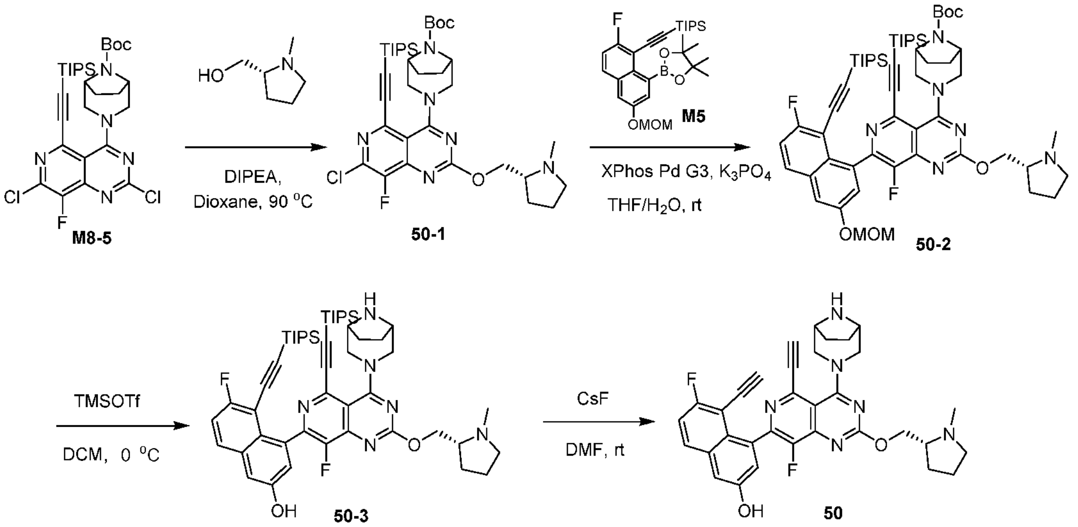


M8-5 50-1 50-2 50-3 50

Step 1: Synthesis of Compound **50-1**

Compound **50-1** (425 mg, 0.70 mmol), (R)-1-methyl-2-pyrrolidinemethanol (120 mg, 1.05 mmol), DIPEA (0.35 mL, 2.1 mmol) and anhydrous 1,4-dioxane (4 mL) were added to a 50 mL single-neck bottle, the bottle was replaced with $N_2$ three times, and the mixture was stirred at 90°C for 14 h, then the reaction was stopped, the resulting mixture was cooled to room temperature, added with 15 mL saturated ammonium chloride, and EA (15 mL × 3 ) was added for extraction, the combined organic phase was dried over anhydrous sodium sulfate and concentrated, and then purified by column chromatography with DCM/MeOH (v/v = 100/0-40/1) eluent to obtain 180 mg of yellow oil Compound **50-1**, with a yield of 37.5%. LC-MS (ESI, pos. ion) m/z: 687.7 $[M+H]^+$.

Step 2 to Step 4: Synthesis of Compound **50**

Steps 2 to 4 are synthesized with reference to steps 7 to 9 in Example **25.** Finally, 15 mg of a yellow solid was obtained, with a yield of 33%. LC-MS (ESI, pos. ion) m/z: 581.3 $[M+H]^+$; HRMS(ESI): 581.2465 $[M+H]^+$; $^1H$ NMR (400 MHz, $CD_3OD$): δ 7.89 (dd, *J* = 9.1, 5.7 Hz, 1H), 7.41 - 7.30 (m, 2H), 7.27 - 7.21 (m, 1H), 4.71 - 4.53 (m, 6H), 4.33 - 4.28 (m, 1H), 4.07 - 3.93 (m, 1H), 3.93 - 3.79 (m, 1H), 3.76 - 3.67 (m, 2H), 3.46 - 3.37 (m, 1H), 2.86 - 2.73 (m, 4H), 2.32 - 2.17 (m, 2H), 2.12 - 1.85 (m, 6H).

**Example 51: Synthesis of Compound 51**

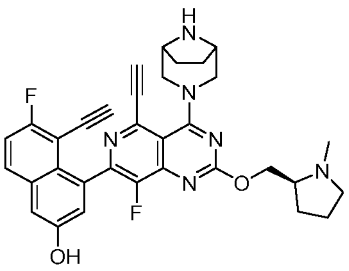

51

The synthesis of compound **51** can be referred to the synthesis of compound **50** in Example 50, (*R*)-1-methyl-2-pyrrolidinemethanol, the initial raw material in step 1 was replaced with (*S*)-1-methyl-2-pyrrolidinemethanol, and finally 15 mg of brown solid was obtained. LC-MS (ESI, pos. ion) m/z: 581.5 $[M+H]^+$; HRMS(ESI): 581.2466 $[M+H]^+$; $^1H$ NMR (400 MHz, $CD_3OD$): δ 7.93 - 7.82 (m, 1H), 7.44 - 7.28 (m, 2H), 7.25 (s, 1H), 4.69 - 4.46 (m, 3H), 4.42 - 4.24 (m, 1H), 4.11 - 3.76 (m, 2H), 3.76 - 3.57 (m, 2H), 3.24 - 3.11 (m, 1H), 3.05 - 2.82 (m, 1H), 2.83 - 2.54 (m, 4H), 2.29 - 2.08 (m, 2H), 2.06 - 1.73 (m, 6H), 1.69 - 1.49 (m, 2H).

**Example 52: Synthesis of Compound 52**

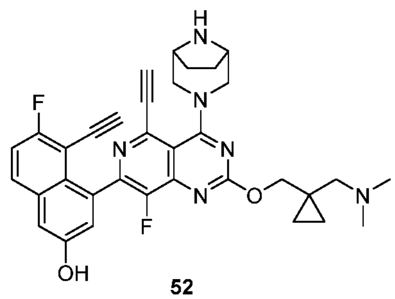

52

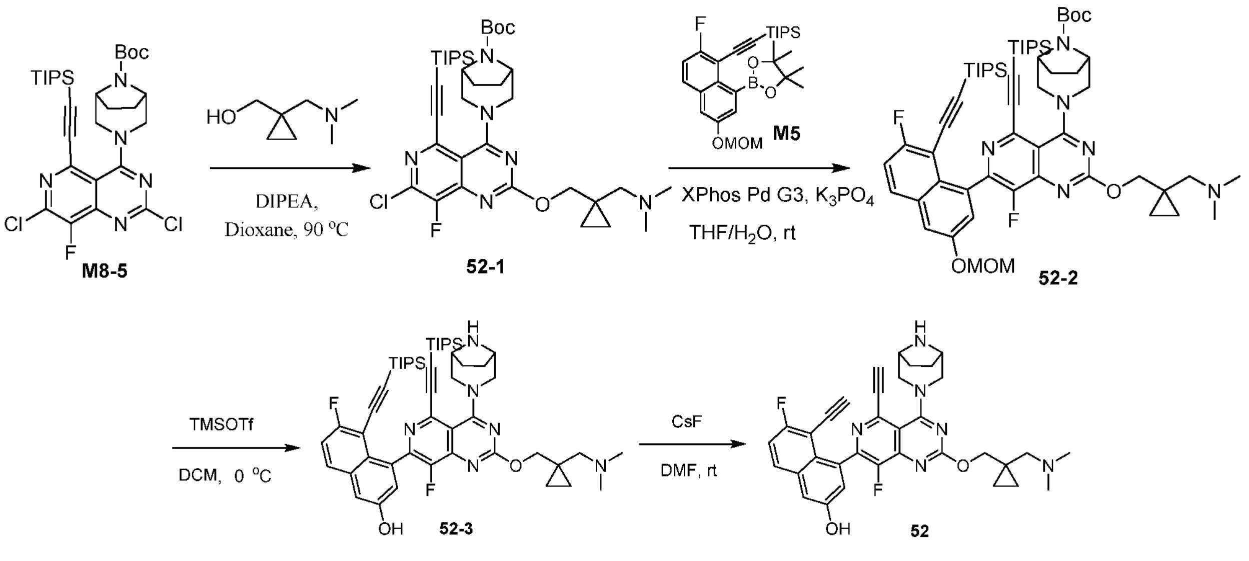


M8-5 52-1 M5 52-2 52-3 52

The synthesis of compound **52** can be referred to the synthesis of compound **50** in Example 50, (*R*)-1-methyl-2-pyrrolidinemethanol, the initial raw material in step 1 was replaced with (1-((dimethylamino)methyl)cyclopropyl)methanol, and finally 10 mg of yellow solid Compound **52** was obtained. LC-MS (ESI, pos. ion) m/z: 595.3 $[M+H]^+$; HRMS(ESI): 595.2620 $[M+H]^+$; $^1H$ NMR (400 MHz, $CD_3OD$): δ 7.90 (dd, *J* = 9.1, 5.7 Hz, 1H), 7.43 - 7.32 (m, 2H), 7.28 - 7.21 (m, 1H), 4.69 - 4.55 (m, 2H), 4.55 - 4.40 (m, 3H), 4.37 (m, 1H), 4.16 - 3.86 (m, 4H), 3.42 - 3.36 (m, 1H), 3.29 - 3.20 (m, 1H), 2.97 (s, 6H), 2.26 - 2.15 (m, 1H), 2.09 - 2.00 (m, 2H), 2.00 - 1.57 (m, 5H). $^{19}F$ NMR (376 MHz, $CD_3OD$): δ -111.41 (1F),

-138.91 (1F).

**Example 53: Synthesis of Compound 53**

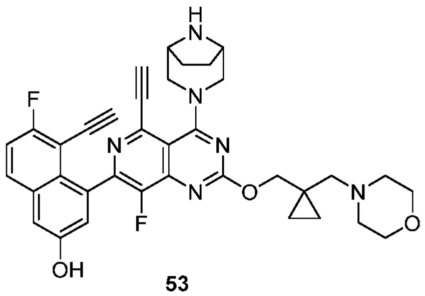

**53**

The synthesis of compound **53** can be referred to the synthesis of compound **49** in Example 49, ((2*R*, 7a,*S*)-2-fluorohexahydro-1*H*-pyrrolizin-7a-yl)methanol, the initial raw material in step 1 was replaced with (1-(morpholinylmethyl)cyclopropyl)methanol, and finally 40 mg of yellow solid Compound **53** was obtained. LC-MS (ESI, pos. ion) m/z: 637.6 $[M+H]^+$; HRMS(ESI): 637.2741 $[M+H]^+$; $^1H$ NMR (400 MHz, $CD_3OD$): δ 7.88 (dd, *J* = 9.1, 5.7 Hz, 1H), 7.40 - 7.29 (m, 2H), 7.28 - 7.20 (m, 1H), 4.70 - 4.50 (m, 1H), 4.45 (s, 2H), 4.27 (s, 1H), 4.05 - 3.88 (m, 1H), 3.86 - 3.72 (m, 1H), 3.72 - 3.56 (m, 6H), 2.61 - 2.38 (m, 6H), 2.10 - 1.92 (m, 1H), 1.87 - 1.69 (m, 3H), 1.66 - 1.48 (m, 2H), 0.78 - 0.65 (m, 2H), 0.57 - 0.42 (m, 2H). $^{19}F$ NMR (376 MHz, $CD_3OD$) δ -111.47, -138.46.

**Example 54: Synthesis of Compound 54**

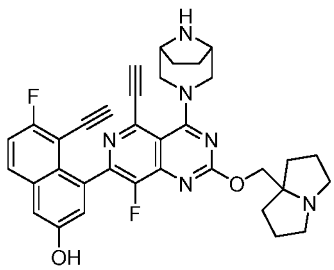

**54**

The synthesis of compound **54** can be referred to the synthesis of compound **49** in Example 49, ((2*R*, 7a*S*)-2-fluorohexahydro-1*H*-pyrrolizin-7a-yl)methanol, the initial raw material in step 1 was replaced with (tetrahydro-1*H*-pyrrolopyrrolidin-7a(5*H*)-yl)methanol, and finally 24 mg of yellow solid was obtained. LC-MS (ESI, pos. ion) m/z: 607.5 $[M+H]^+$; HRMS(ESI): 607.2625 $[M+H]^+$; $^1H$ NMR (400 MHz, $CD_3OD$): δ 7.88 (dd, *J* = 9.1, 5.8 Hz, 1H), 7.40 - 7.31 (m, 2H), 7.26 - 7.21 (m, 1H), 4.74 - 4.52 (m, 5H), 4.38 (s, 1H), 4.19 - 4.10 (m, 3H), 3.77 - 3.66 (m, 2H), 3.40 (s, 1H), 2.42 - 2.30 (m, 2H), 2.30 - 2.08 (m, 7H), 2.07 - 1.87 (m, 5H); $^{19}F$ NMR (376 MHz, $CD_3OD$) δ -111.46 (1F), -137.85 (1F).

**Example 55: Synthesis of Compound 55**

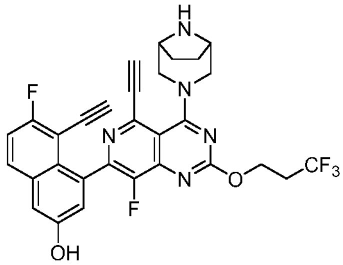

**55**

The synthesis of compound **55** can be referred to the synthesis of compound **49** in Example 49, ((2*R*, 7a*S*)-2-fluorohexahydro-1*H*-pyrrolizin-7a-yl)methanol, the initial raw material in step 1 was replaced with 3,3,3-trifluoro-1-propanol, and finally 20 mg of brown solid was obtained. LC-MS (ESI, pos. ion) m/z: 580.2 [M+H]$^+$; HRMS(ESI): 580.1782 [M+H]$^+$.

## Example 56: Synthesis of Compound 56

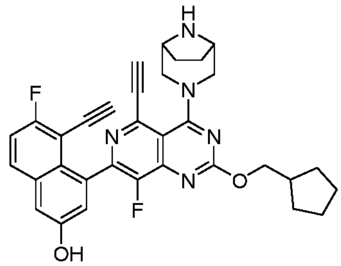

**56**

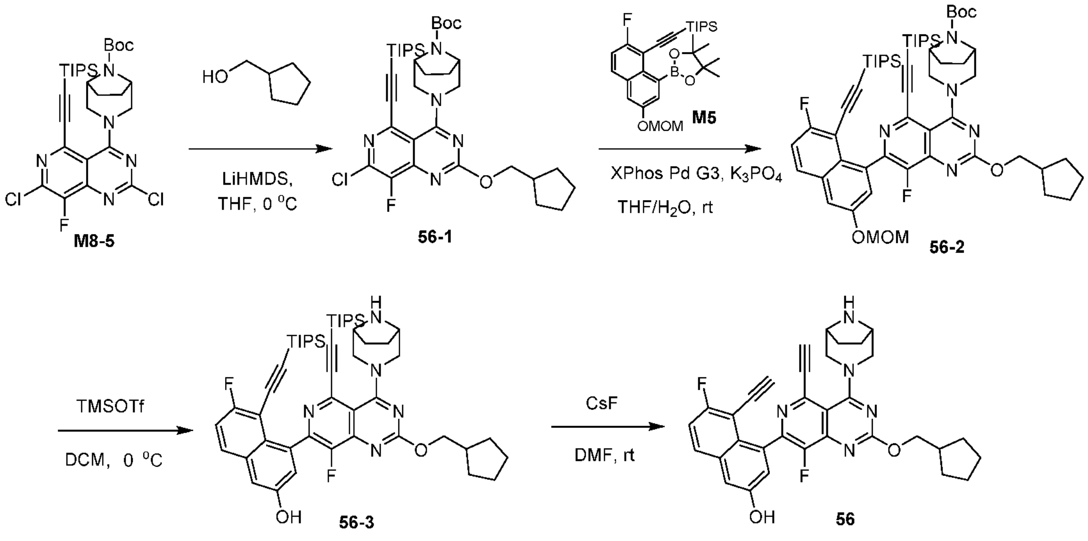


The synthesis of compound **56** can be referred to the synthesis of compound **49** in Example 49, ((2*R*, 7a*S*)-2-fluorohexahydro-1*H*-pyrrolizin-7a-yl)methanol, the initial raw material in step 1 was replaced with cyclohexylmethanol, and finally 12 mg of yellow solid was obtained. LC-MS (ESI, pos. ion) m/z: 566.2 [M+H]$^+$; HRMS(ESI) 566.2353 [M+H]$^+$.

## Example 57: Synthesis of Compound 57

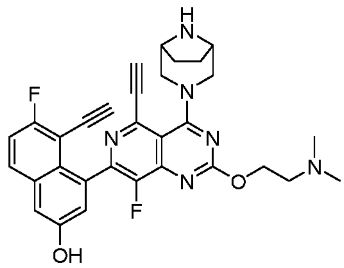

57

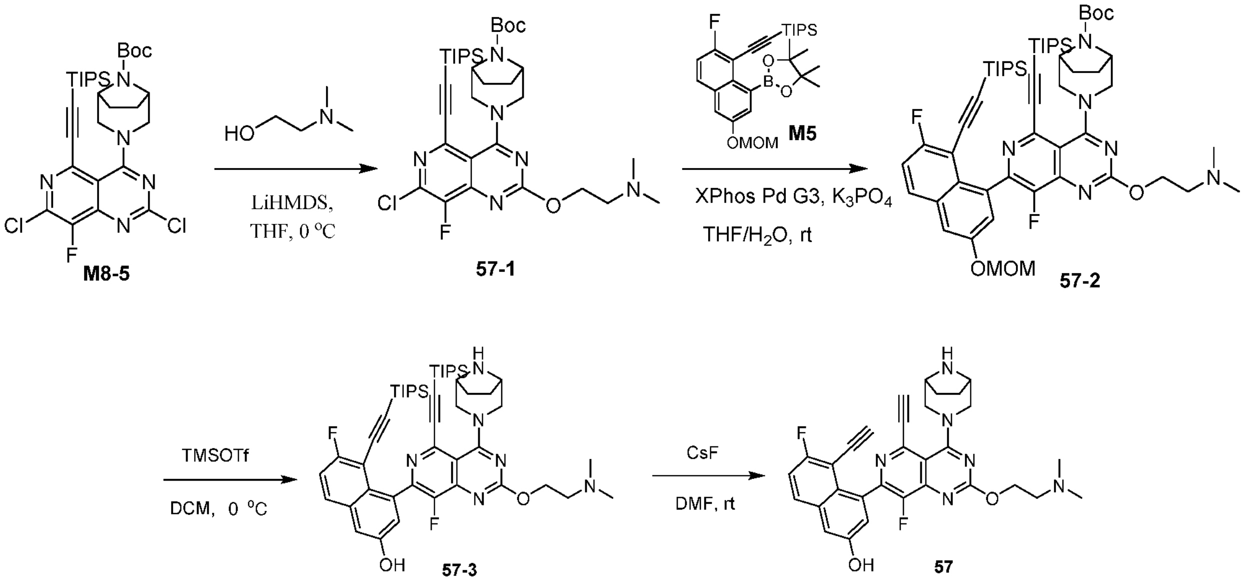


M8-5 57-1 57-2 57-3 57

The synthesis of compound **57** can be referred to the synthesis of compound **49** in Example 49, ((2*R*, 7a*S*)-2-fluorohexahydro-1*H*-pyrrolizin-7a-yl)methanol, the initial raw material in step 1 was replaced with 2-(dimethylamino)ethanol, and finally 16 mg of yellow solid was obtained. LC-MS (ESI, pos. ion) m/z: 555.2 $[M+H]^+$; HRMS(ESI): 555.2305 $[M+H]^+$.

**Example 58: Synthesis of Compound 58**

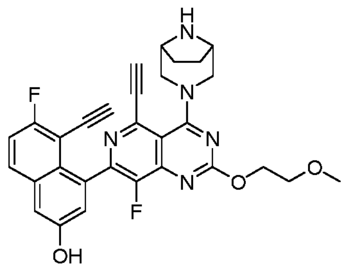

58

The synthesis of compound **58** can be referred to the synthesis of compound **49** in Example 49, ((2*R*, 7a*S*)-2-fluorohexahydro-1*H*-pyrrolizin-7a-yl)methanol, the initial raw material in step 1 was replaced with 2-methoxyethanol, and finally 15 mg of yellow solid was obtained. LC-MS (ESI, pos. ion) m/z: 542.2 $[M+H]^+$; HRMS(ESI): 542.1987 $[M+H]^+$.

**Example 59: Synthesis of Compound 59**

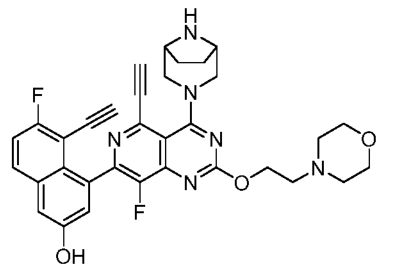

59

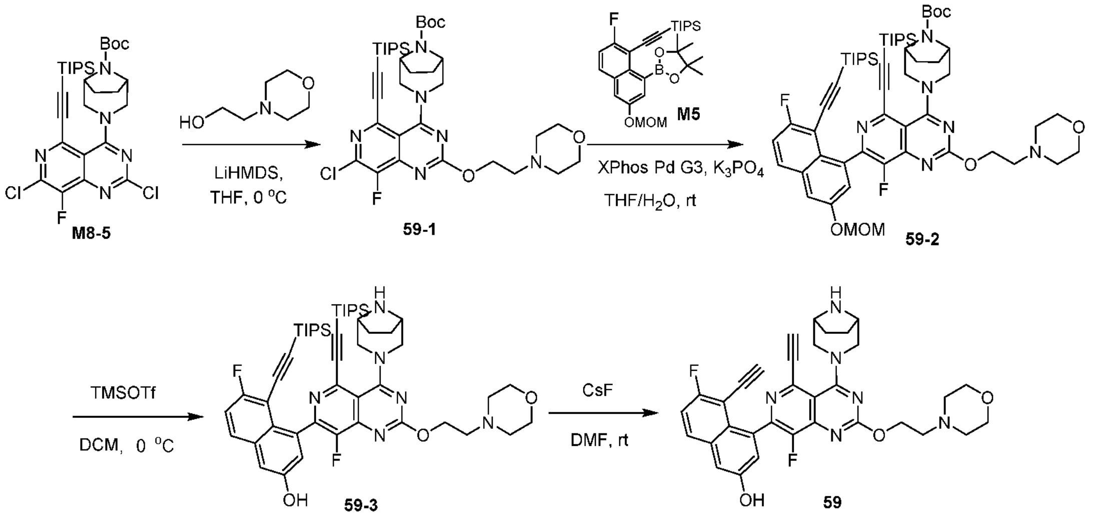


M8-5 59-1 59-2 59-3 59

The synthesis of compound **59** can be referred to the synthesis of compound **49** in Example 49, ((2*R*, 7a*S*)-2-fluorohexahydro-1*H*-pyrrolizin-7a-yl)methanol, the initial raw material in step 1 was replaced with 2-morpholinoethanol, and finally 25 mg of yellow solid was obtained. LC-MS (ESI, pos. ion) m/z: 597.2 $[M+H]^+$.

**Example 60: Synthesis of Compound 60**

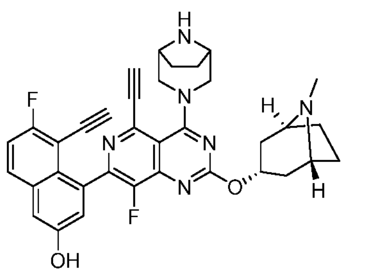

60

The synthesis of compound **60** can be referred to the synthesis of compound **50** in Example 50, (*R*)-1-methyl-2-pyrrolidinemethanol, the initial raw material in step 1 was replaced with Tropine, and finally 21 mg of yellow solid was obtained.

**Example 61: Synthesis of Compound 61**

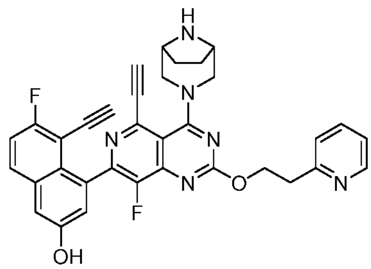

61

The synthesis of compound **61** can be referred to the synthesis of compound **50** in Example 50, (*R*)-1-methyl-2-pyrrolidinemethanol, the initial raw material in step 1 was replaced with 2-(2-hydroxyethyl)pyridine, and finally 14 mg of yellow solid was obtained. LC-MS (ESI, pos. ion) m/z: 589.2 $[M+H]^+$; HRMS(ESI): 589.2140 $[M+H]^+$.

**Example 62: Synthesis of Compound 62**

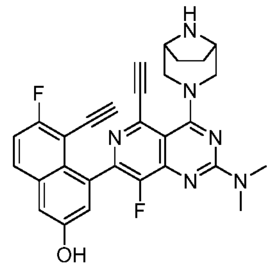

62

The synthesis of compound **62** can be referred to the synthesis of compound **50** in Example 50, (*R*)-1-methyl-2-pyrrolidinemethanol, the initial raw material in step 1 was replaced with dimethylamine hydrochloride, and the solvent 1,4-dioxane was replaced with tetrahydrofuran, the reaction temperature 90 °C was replaced with room temperature, and finally 42 mg of yellow solid was obtained. LC-MS (ESI, pos. ion) m/z: 511.2 $[M+H]^+$; HRMS(ESI): 511.2044 $[M+H]^+$; $^1H$ NMR (400 MHz, $CD_3OD$):$\delta$ 7.86 (dd, *J* = 9.1, 5.7 Hz, 1H), 7.36-7.26 (m, 2H), 7.20 (d, *J* = 2.3 Hz, 1H), 4.67-4.43 (m, 3H), 4.12 (d, *J* = 11.4 Hz, 2H), 4.06- 3.85 (m, 2H), 3.33 (s, 6H), 2.13 - 1.91 (m, 5H). $^{19}F$ NMR (376 MHz, $CD_3OD$): $\delta$ -111.68 (1F), -139.75 (1F).

**Example 63: Synthesis of Compound 63**

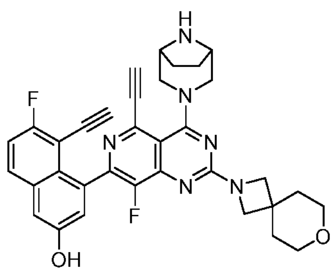

63

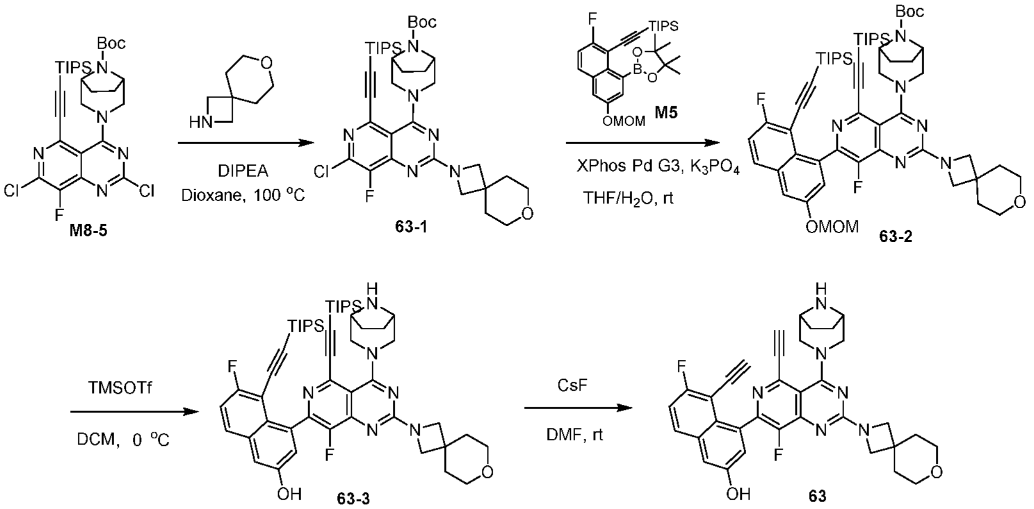


The synthesis of compound **63** can be referred to the synthesis of compound **50** in Example 50, (*R*)-1-methyl-2-pyrrolidinemethanol, the initial raw material in step 1 was replaced with 7-oxa-2-azaspiro[3.5]nonane, and finally 49 mg of light yellow solid was obtained. LC-MS (ESI, pos. ion) m/z: 593.6 [M+H]$^+$; HRMS(ESI): 593.2450 [M+H]$^+$.

**Example 64: Synthesis of Compound 64**

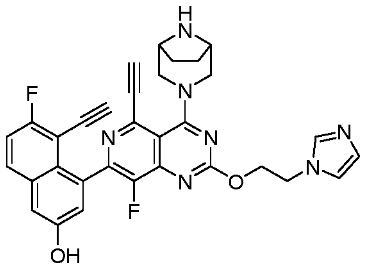

**64**

The synthesis of compound **64** can be referred to the synthesis of compound **50** in Example 50, (*R*)-1-methyl-2-pyrrolidinemethanol, the initial raw material in step 1 was replaced with 1-(2-hydroxyethyl)imidazole, and finally 16 mg of yellow solid was obtained. LC-MS (ESI, pos. ion) m/z: 578.2 [M+H]$^+$; HRMS(ESI): 578.2103 [M+H]$^+$.

**Example 65: Synthesis of Compound 65**

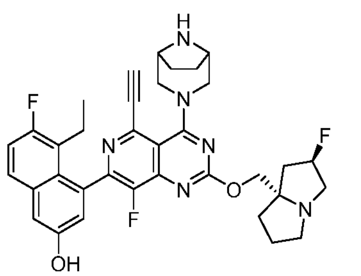

**65**

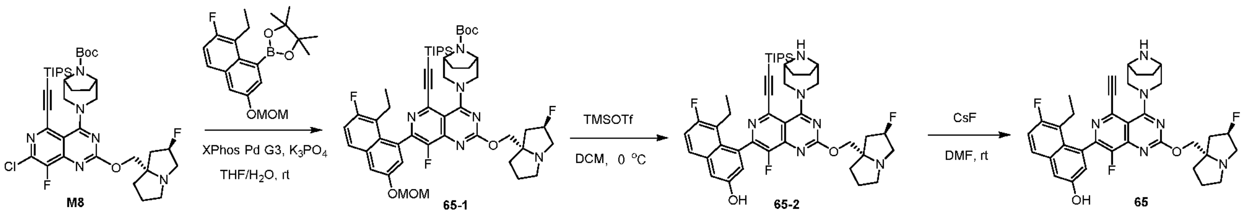


Step 1: Synthesis of Compound **65-1**

**M8** (300 mg, 0.41 mmol), 2-(8-ethyl-7-fluoro-3-(methoxymethoxy)naphthalen-1-yl)-4,4,5,5-tetramethyl-1,3,2-dioxaborolane (220 mg, 0.61 mmol), Xphos Pd G3 (100 mg, 0.12 mmol), $K_3PO_4$ (260 mg, 1.23 mmol) and THF /$H_2O$ (2.5 mL/0.5 mL) were added to a 25 mL single-neck bottle, the bottle was replaced with $N_2$ three times, and the mixture was stirred at room temperature for 14 h, then the reaction was stopped, to the resulting mixture was added saturated ammonium chloride with 10 mL, and extracted with DCM (10 mL × 3), the combined organic phase was dried and concentrated, and purified by column chromatography with DCM/MeOH (v/v = 100/0-100/1) eluent to obtain 330 mg of a brown solid Compound 65-1, with a yield of 86.6%. LC-MS (ESI, pos. ion) m/z: 929.9 $[M+H]^+$.

Step 2: Synthesis of Compound **65-2**

Compound **65-1** (330 mg, 0.36 mmol) and DCM (4 mL) were added to a 25 mL single-neck bottle, the mixture was cooled to 0 °C, then TMSOTf (0.19 mL, 1.08 mmol) was added and the mixture was stirred at 0°C for 2 h, then the reaction was stopped, to the resulting mixture was added with 10 mL saturated sodium bicarbonate, extracted with DCM (10 mL × 3), the combined organic phase was dried over anhydrous sodium sulfate and concentrated, and used directly for the next step, the yield was calculated as 100%, LC-MS (ESI, pos. ion) m/z: 785.4 $[M+H]^+$.

Step 3: Synthesis of Compound **65**

Compound **65-2** (280 mg, 0.36 mmol), cesium fluoride (820 mg, 5.4 mmol) and DMF (1.5 mL) were added to a 25 mL single-neck bottle, the mixture was stirred at room temperature for 12 h, then the reaction was stopped, 12 mL of water was added and solid was precipitated, then filtered, the precipitated solid was separated and purified by thin layer chromatography with DCM/MeOH/ammonia (v/v = 7/1/0.05) eluent to obtain 25 mg of a yellow solid Compound **65**, with a yield of 10%. LC-MS (ESI, pos. ion) m/z: 629.3 $[M+H]^+$; HRMS(ESI): 629.2832 $[M+H]^+$; $^1H$ NMR (400 MHz, $CD_3OD$): δ 7.72 - 7.60 (m, 1H), 7.36 - 7.22 (m, 2H), 7.12 (s, 1H), 5.45 - 5.20 (m, 1H), 4.66 - 4.50 (m, 4H), 4.36 - 4.21 (m, 2H), 3.69 - 3.46 (m, 3H), 3.24 - 3.10 (m, 2H), 3.09 - 2.97 (m, 2H), 2.89 - 2.68 (m, 2H), 2.25 - 2.13 (m, 3H), 2.09 - 2.01 (m, 3H), 1.84 - 1.71 (m, 2H), 1.68 - 1.55 (m, 2H), 1.01 - 0.86 (m, 3H).

**Example 66: Synthesis of Compound 66**

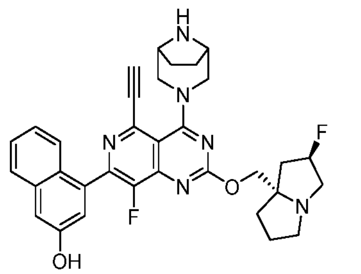

**66**

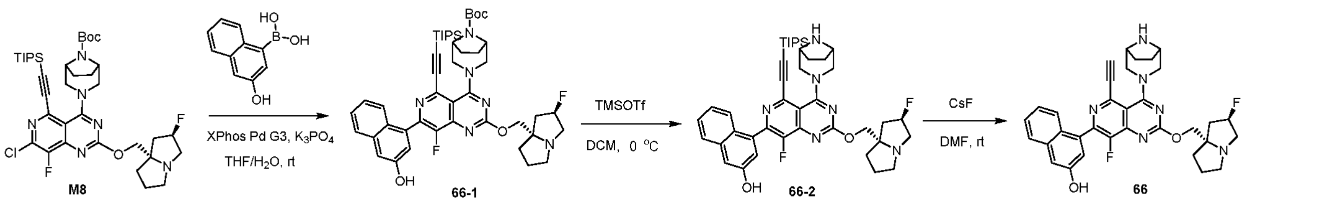


Step 1: Synthesis of Compound **66-1**

**M8** (300 mg, 0.41 mmol), (3-hydroxynaphthalen-1-yl)boronic acid (170 mg, 0.61 mmol), Xphos Pd G3 (69 mg, 0.082 mmol), $K_3PO_4$ (220 mg, 1.02 mmol) and $THF/H_2O$ (6 mL/2 mL) were added to a 25 mL single-neck bottle, the bottle was replaced with $N_2$ three times, and the mixture was stirred at room temperature for 4 h, then the reaction was stopped, to the mixture was added 10 mL saturated ammonium chloride, and DCM (10 mL × 3) was added for extraction, the combined organic phase was dried over anhydrous sodium sulfate and concentrated, and purified by column chromatography with DCM/MeOH (v/v = 100/0-80/1) eluent to obtain 310 mg of a brown solid Compound **66-1**, with a yield of 90%. LC-MS (ESI, pos. ion) m/z: 839.8 $[M+H]^+$; $^1H$ NMR (400 MHz, $CDCl_3$): δ 7.71 (d, *J*= 7.0 Hz, 1H), 7.63 (d,*J* = 8.2 Hz, 1H), 7.43 (d, *J*= 2.1 Hz, 1H), 7.35 (t, *J*= 7.4 Hz, 1H), 7.23 - 7.12 (m, 2H), 5.29 - 5.09 (m, 1H), 4.45 - 4.02 (m, 4H), 3.41 - 3.08 (m, 3H), 3.04 - 2.88 (m, 1H), 2.31 - 2.08 (m, 4H), 2.04 - 1.66 (m, 10H), 1.49 (s, 9H), 1.17 - 0.99 (m, 21H).

Step 2: Synthesis of Compound **66-2**

Compound **66-1** (310 mg, 0.37 mmol) and acetonitrile (5 mL) were added to a 25 mL single-neck bottle, the mixture was cooled to 0 °C, then TMSOTf (0.20 mL, 1.11 mmol) was added and the mixture was stirred at 0°C for 2 h, then the reaction was stopped, the resulting mixture was added with 10 mL saturated sodium bicarbonate, extracted with DCM (10 mL × 3), the combined organic phase was dried over anhydrous sodium sulfate and concentrated, and used directly for the next step, the yield was calculated as 100%, LC-MS (ESI, pos. ion) m/z: 739.4 $[M+H]^+$.

Step 3: Synthesis of Compound **66**

Compound **66-2** (272 mg, 0.37 mmol), cesium fluoride (837 mg, 5.55 mmol) and DMF (1 mL) were added to a 25 mL single-neck bottle, the mixture was stirred at room temperature for 12 h, then the reaction was stopped, 8 mL of water was added and solid was precipitated, then filtered, the precipitated solid was separated and purified by thin layer chromatography with DCM/MeOH/ammonia (v/v = 7/1/0.05) eluent to obtain 77 mg of a yellow solid Compound 66, with a yield of 36%. LC-MS (ESI, pos. ion) m/z: 583.6 $[M+H]^+$; HRMS(ESI): 583.2623 $[M+H]^+$; $^1H$ NMR (400 MHz, DMSO-$d_6$): δ 9.97 (s, 1H), 7.85 - 7.76 (m, 1H), 7.63 - 7.55 (m, 1H), 7.49 - 7.40 (m, 1H), 7.33 - 7.22 (m, 3H), 5.28 (d, *J* = 54.0 Hz, 1H), 4.79 (s, 1H), 4.22 - 3.96 (m, 3H), 3.90 - 3.41 (m, 5H), 3.16 - 2.94 (m, 4H), 2.87 - 2.75 (m, 1H), 2.15 - 1.95 (m, 3H), 1.90 - 1.70 (m, 4H), 1.62 - 1.45 (m, 3H); $^{19}F$ NMR (376 MHz, DMSO-$d_6$): δ -138.44 (1F), -172.14 (1F).

**Example 67: Synthesis of Compound 67**

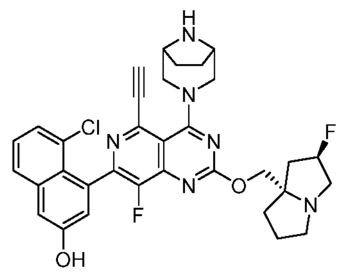

**67**

The synthesis of compound **67** can be referred to the synthesis of compound **65** in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with 2-(8-chloro-3-(methoxymethoxy)naphthalen-1-yl)-4,4,5,5-tetramethyl-1,3,2-dioxaborolane, and finally 60 mg of yellow solid Compound **67** was obtained. LC-MS (ESI, pos. ion) m/z: 617.3 $[M+H]^+$; HRMS(ESI): 617.2225

$[M+H]^+$; $^1H$ NMR (400 MHz, $CD_3OD$): δ 7.81 - 7.72 (m, 1H), 7.42 - 7.32 (m, 3H), 7.21 (s, 1H), 5.60 - 5.39 (m, 1H), 4.63 - 4.37 (m, 4H), 4.35 - 4.02 (m, 4H), 3.97 - 3.41 (m, 4H), 3.29 - 3.14 (m, 1H), 2.59 - 2.30 (m, 3H), 2.24 - 2.03 (m, 5H), 1.92 - 1.65 (m, 2H); $^{19}F$ NMR (376 MHz, $CD_3OD$): δ -138.46 (1F), -173.75 (1F).

**Example 68: Synthesis of Compound 68**

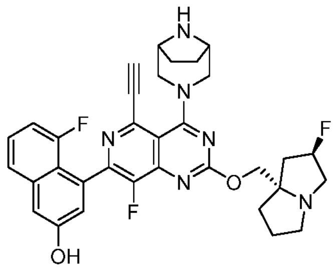

**68**

The synthesis of compound 68 can be referred to the synthesis of compound 65 in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with 2-(8-fluoro-3-(methoxymethoxy)naphthalen-1-yl)-4,4,5,5-tetramethyl-1,3,2-dioxaborolane, and finally 20 mg of brown solid Compound **68** was obtained. LC-MS (ESI, pos. ion) m/z: 601.6 $[M+H]^+$; $^1H$ NMR (400 MHz, $CD_3OD$): δ 7.65 - 7.56 (m, 1H), 7.46 - 7.37 (m, 1H), 7.37 - 7.31 (m, 1H), 7.28 - 7.16 (m, 1H), 6.98 - 6.89 (m, 1H), 5.40 (d, *J* = 53.4 Hz, 1H), 4.54 - 4.35 (m, 3H), 4.10 - 3.83 (m, 2H), 3.80 - 3.70 (m, 2H), 3.58 - 3.38 (m, 3H), 3.24 - 3.05 (m, 1H), 2.52 - 2.17 (m, 4H), 2.15 - 1.93 (m, 4H), 1.89 - 1.61 (m, 4H); 19F NMR(376 MHz, $CD_3OD$): δ -116.03 (1F), -139.77 (1F), -173.75 (1F).

**Example 69: Synthesis of Compound 69**

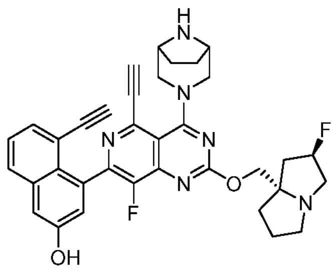

**69**

The synthesis of compound **69** can be referred to the synthesis of compound **65** in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with triisopropyl((6-(methoxymethoxy)-8-(4,4,5,5-tetramethyl-1,3,2-dioxaborolan-2-yl)naphthalen-1-yl)ethynyl)silane, and finally 24 mg of brown solid was obtained. LC-MS (ESI, pos. ion) m/z: 607.6 $[M+H]^+$; HRMS(ESI): 607.2615 $[M+H]^+$; $^1H$ NMR (400 MHz, $CD_3OD$): δ 7.89 - 7.78 (m, 1H), 7.58 - 7.48 (m, 1H), 7.46 - 7.37 (m, 1H), 7.37 - 7.28 (m, 1H), 7.25 - 7.14 (m, 1H), 5.37 (d, *J* = 53.4 Hz, 1H), 4.66 - 4.46 (m, 1H), 4.46 - 4.23 (m, 3H), 4.08 - 3.77 (m, 2H), 3.77 - 3.65 (m, 2H), 3.61 - 3.53 (m, 1H), 3.50 - 3.35 (m, 3H), 3.19 - 3.06 (m, 1H), 3.04 - 2.94 (m, 1H), 2.47 - 2.18 (m, 3H), 2.13 - 1.91 (m, 4H), 1.89 - 1.67 (m, 3H); $^{19}F$ NMR (376 MHz, $CD_3OD$): δ -138.29, -173.72.

**Example 70: Synthesis of Compound 70**

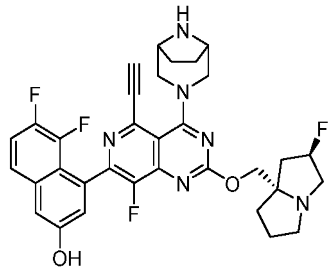

**70**

The synthesis of compound **70** can be referred to the synthesis of compound **65** in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with 2-(7,8-difluoro-3-(methoxymethoxy)naphthalen-1-yl)-4,4,5,5-tetramethyl-1,3,2-dioxaborolane, and finally yellow solid was obtained. LC-MS (ESI, pos. ion) m/z: 619.2 $[M+H]^+$; HRMS(ESI): 619.2430 $[M+H]^+$.

### Example 71: Synthesis of Compound 71

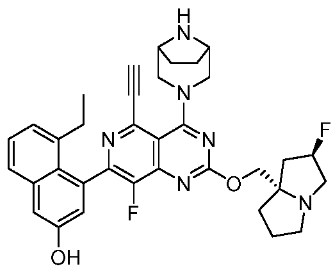

**71**

The synthesis of compound **71** can be referred to the synthesis of compound **65** in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with 2-(8-ethyl-3-(methoxymethoxy)naphthalen-1-yl)-4,4,5,5-tetramethyl-1,3,2-dioxaborolane, and finally 33 mg of yellow solid was obtained. LC-MS (ESI, pos. ion) m/z: 611.3 $[M+H]^+$; HRMS(ESI): 611.2921 $[M+H]^+$.

### Example 72: Synthesis of Compound 72

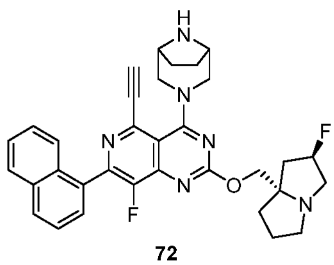

**72**

The synthesis of compound **72** can be referred to the synthesis of compound **66** in Example 66, (3-hydroxynaphthalen-1-yl)boronic acid, the initial raw material in step 1 was replaced with 1-naphthylboronic acid, and finally 10 mg of yellow solid was obtained. LC-MS (ESI, pos. ion) m/z: 567.6 $[M+H]^+$; HRMS(ESI): 567.2655 $[M+H]^+$; $^1H$ NMR (400 MHz, $CD_3OD$): $\delta$ 8.07 (d, $J$ = 8.0 Hz, 1H), 8.01 (d, $J$ = 7.9 Hz, 1H), 7.79 - 7.72 (m, 1H), 7.70 - 7.62 (m, 2H), 7.60 - 7.46 (m, 2H), 5.46 - 5.25 (m, 1H), 4.63 - 4.55 (m, 2H), 4.45 - 4.29 (m, 3H), 3.97 - 3.82 (m, 2H), 3.75 - 3.65 (m, 2H), 3.54 - 3.46 (m, 1H), 3.20 - 3.05 (m, 3H), 2.46 - 2.28 (m, 3H), 2.25 - 2.16 (m, 2H), 2.10 - 2.02 (m, 3H), 1.87 - 1.78 (m, 2H).

**Example 73: Synthesis of Compound 73**

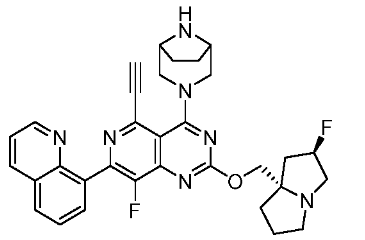

**73**

The synthesis of compound **73** can be referred to the synthesis of compound **66** in Example 66, (3-hydroxynaphthalen-1-yl)boronic acid, the initial raw material in step 1 was replaced with quinoline-8-boronic acid, and finally 30 mg of brown solid was obtained. LC-MS (ESI, pos. ion) m/z: 568.3 $[M+H]^+$; HRMS(ESI): 568.2626 $[M+H]^+$; $^1H$ NMR (400 MHz, $CD_3OD$): δ 8.88 - 8.78 (m, 1H), 8.55 - 8.47 (m, 1H), 8.22 - 8.15 (m, 1H), 8.05 - 7.98 (m, 1H), 7.87 - 7.76 (m, 1H), 7.67 - 7.57 (m, 1H), 5.59 - 5.37 (m, 1H), 4.76 - 4.47 (m, 4H), 4.39 - 4.29 (m, 1H), 4.20 - 3.83 (m, 4H), 3.81 - 3.49 (m, 3H), 3.29 - 3.22 (m, 1H), 2.63 - 2.28 (m, 3H), 2.28 - 2.13 (m, 2H), 2.13 - 1.81 (m, 5H); $^{19}F$ NMR (376 MHz, $CD_3OD$): δ -137.41 (1F), -173.90 (1F).

**Example 74: Synthesis of Compound 74**

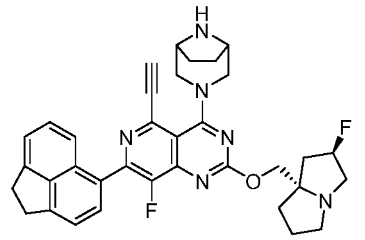

**74**

The synthesis of compound **74** can be referred to the synthesis of compound **66** in Example 66, (3-hydroxynaphthalen-1-yl)boronic acid, the initial raw material in step 1 was replaced with acenaphthene-5-boronic acid, and finally 4 mg of brownish yellow solid was obtained. LC-MS (ESI, pos. ion) m/z: 593.3 $[M+H]^+$; HRMS(ESI): 593.2825 $[M+H]^+$; $^1H$ NMR (400 MHz, $CD_3OD$): δ 7.77 - 7.68 (m, 1H), 7.63 - 7.56 (m, 1H), 7.53 - 7.44 (m, 2H), 7.43 - 7.33 (m, 1H), 5.53 - 5.30 (m, 1H), 4.82 - 4.73 (m, 1H), 4.65 - 4.38 (m, 5H), 4.05 - 3.88 (m, 2H), 3.88 - 3.75 (m, 2H), 3.68 - 3.57 (m, 1H), 3.53 - 3.43 (m, 4H), 3.27 - 3.14 (m, 2H), 2.46 - 2.35 (m, 1H), 2.27 - 2.12 (m, 3H), 2.08 - 1.97 (m, 3H), 1.93 - 1.76 (m, 3H).

**Example 75: Synthesis of Compound 75**

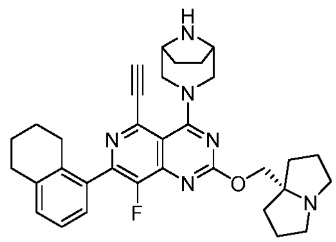

**75**

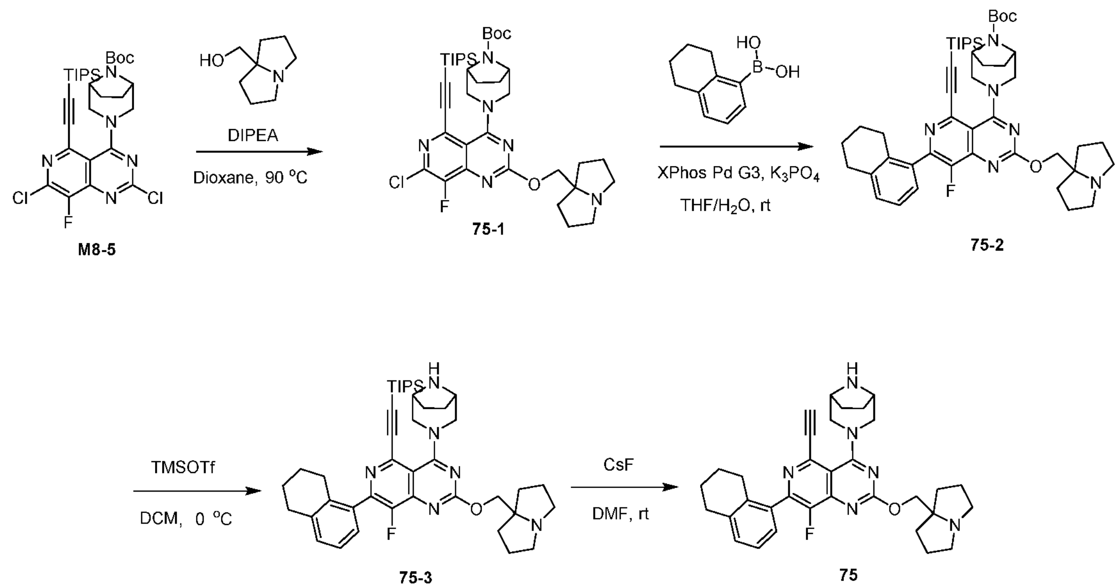


M8-5 75-1 75-2 75-3 75

Step 1: Synthesis of Compound **75-1**

The synthesis of compound **75-1** was prepared by referring to the synthesis of intermediate **M8.**

Steps 2 to 4: Synthesis of Compound **75**

The synthesis of compound **75** can be referred to the synthesis of compound **66** in Example 66, (3-hydroxynaphthalen-1-yl)boronic acid, the initial raw material in step 2 was replaced with (5,6,7,8-tetrahydronaphthalen-1-yl)boronic acid, and finally 45 mg of yellow solid was obtained. LC-MS (ESI, pos. ion) m/z: 553.3 $[M+H]^+$; HRMS(ESI): 553.3077 $[M+H]^+$; $^1H$ NMR (400 MHz, $CD_3OD$): δ 7.25 - 7.20 (m, 2H), 7.18 - 7.13 (m, 1H), 4.69 - 4.63 (m, 2H), 4.63 - 4.44 (m, 2H), 4.37 - 4.34 (m, 1H), 4.13 - 3.99 (m, 2H), 3.97 - 3.91 (m, 2H), 3.76 - 3.66 (m, 2H), 3.30 - 3.26 (m, 2H), 2.88 (t, *J* = 6.5 Hz, 2H), 2.59 (t, *J* = 6.1 Hz, 2H), 2.40 - 2.30 (m, 2H), 2.28 - 2.17 (m, 4H), 2.15 - 2.07 (m, 2H), 1.97 - 1.89 (m, 2H), 1.86 - 1.72 (m, 6H).

**Example 76: Synthesis of Compound 76**

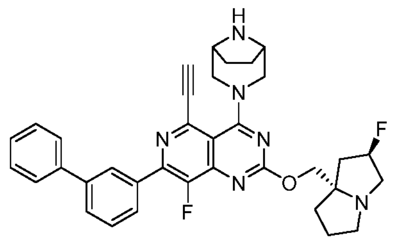

**76**

The synthesis of compound 76 can be referred to the synthesis of compound 66 in Example 66, (3-hydroxynaphthalen-1-yl)boronic acid, the initial raw material in step 1 was replaced with 3-biphenylboronic acid, and finally 40 mg of yellow solid was obtained. LC-MS (ESI, pos. ion) m/z: 593.2 $[M+H]^+$; HRMS(ESI): 593.2804 $[M+H]^+$; $^1H$ NMR (400 MHz, $CD_3OD$): δ 8.26 (s, 1H), 7.99 (d, *J* = 7.6 Hz, 1H), 7.77 (d, *J* = 7.7 Hz, 1H), 7.70 (d, *J* = 7.6 Hz, 2H), 7.65 - 7.58 (m, 1H), 7.54 - 7.43 (m, 2H), 7.43 - 7.34 (m, 1H), 5.39 (d, *J* = 53.7 Hz, 1H), 4.58 - 4.25 (m, 4H), 4.05 - 3.83 (m, 2H), 3.83 - 3.63 (m, 2H), 3.54 - 3.36 (m, 3H), 3.28 - 2.99 (m, 2H), 2.51 - 2.21 (m, 3H), 2.17 - 1.92 (m, 4H), 1.88 - 1.66 (m, 3H); $^{19}F$ NMR (376 MHz, $CD_3OD$): δ -142.24, -173.76.

**Example 77: Synthesis of Compound 77**

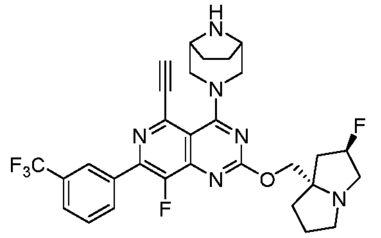

**77**

The synthesis of compound 77 can be referred to the synthesis of compound 66 in Example 66, (3-hydroxynaphthalen-1-yl)boronic acid, the initial raw material in step 1 was replaced with 3-(trifluoromethyl)benzeneboronic acid, and finally 10 mg of brown solid was obtained. LC-MS (ESI, pos. ion) m/z: 585.6 $[M+H]^+$; HRMS(ESI): 585.2402 $[M+H]^+$; $^1H$ NMR (400 MHz, $CD_3OD$): δ 8.42 - 8.31 (m, 2H), 7.85 (d, *J* = 7.8 Hz, 1H), 7.78 (t, *J* = 7.8 Hz, 1H), 5.53 (d, *J* = 52.4 Hz, 1H), 4.68 - 4.56 (m, 3H), 4.11 - 3.99 (m, 3H), 3.92 - 3.63 (m, 4H), 3.42 - 3.35 (m, 1H), 2.72 - 2.35 (m, 4H), 2.33 - 2.08 (m, 4H), 2.07 - 1.82 (m, 4H).

**Example 78: Synthesis of Compound 78**

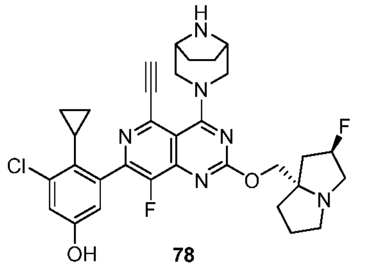

**78**

The synthesis of compound **78** can be referred to the synthesis of compound **65** in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with 2-(3-chloro-2-cyclopropyl-5-(methoxymethoxy)phenyl)-4,4,5,5-tetramethyl-1,3,2-dioxaborolane, and finally 50 mg of yellow solid was obtained. LC-MS (ESI, pos. ion) m/z: 607.2 $[M+H]^+$; HRMS(ESI): 607.2405 $[M+H]^+$;$^1H$ NMR (400 MHz, $CD_3OD$): δ 7.00 (d, *J* = 2.5 Hz, 1H), 6.84 (d, *J* = 2.4 Hz, 1H), 5.55 - 5.37 (m, 3H), 4.60 - 4.43 (m, 4H), 4.07 - 3.96 (m, 2H), 3.96 - 3.87 (m, 2H), 3.73 - 3.52 (m, 4H), 3.29 - 3.20 (m, 1H), 2.62 - 2.30 (m, 4H), 2.26 - 2.03 (m, 5H), 1.94 - 1.78 (m, 5H); $^{19}F$ NMR (376 MHz, $CD_3OD$): δ -138.29, -173.84.

**Example 79: Synthesis of Compound 79**

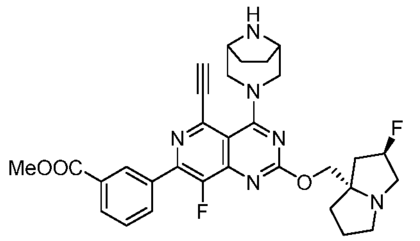

**79**

The synthesis of compound **79** can be referred to the synthesis of compound **66** in Example 66, (3-hydroxynaphthalen-1-yl)boronic acid, the initial raw material in step 1 was replaced with 3-methoxycarbonylphenylboronic acid, and finally 40 mg of yellow solid was obtained. LC-MS (ESI, pos. ion) m/z: 575.2 [M+H]$^+$. HRMS(ESI):calcd.for $C_{31}H_{33}F_2N_6O_3$ [M+H]$^+$=575.2582, found 575.2570. $^1$H NMR (400 MHz, $CD_3OD$): δ 8.66 (s, 1H), 8.25 (d, *J* = 7.5 Hz, 1H), 8.12 (d, *J* = 7.8 Hz, 1H), 7.65 (t, *J* = 7.8 Hz, 1H), 5.35 (d, *J* = 53.7 Hz, 1H), 4.37 - 4.25 (m, 3H), 3.96 (s, 3H), 3.93 - 3.74 (m, 2H), 3.67 - 3.59 (m, 2H), 3.42 - 3.33 (m, 2H), 3.29 - 3.26 (m, 1H), 3.12 - 3.02 (m, 1H), 2.43 - 2.12 (m, 4H), 2.11 - 1.86 (m, 4H), 1.82 - 1.57 (m, 4H); $^{19}$F NMR (376 MHz, $CD_3OD$) δ - 142.32 (1F), -173.70 (1F).

**Example 80: Synthesis of Compound 80**

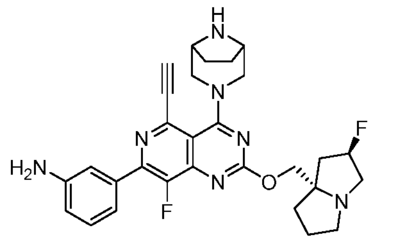

**80**

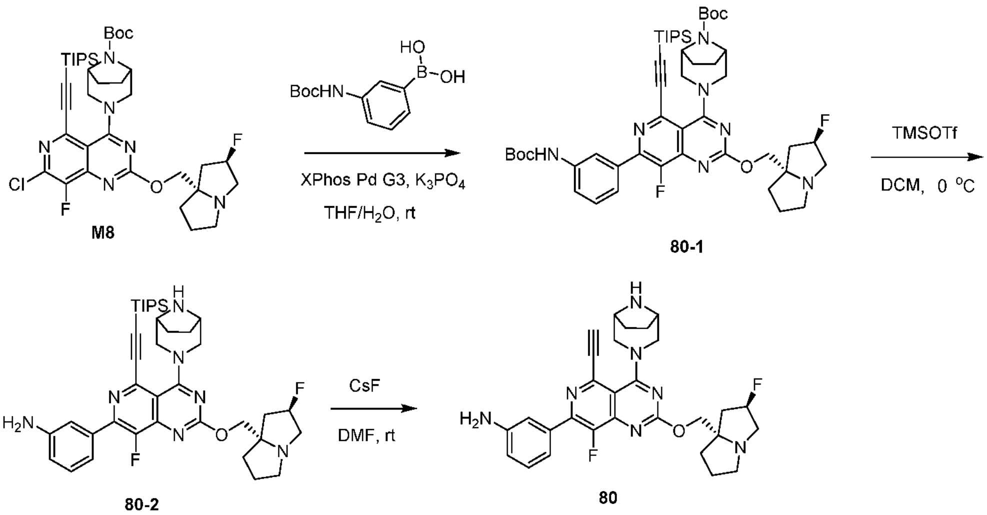


M8 80-1 80-2 80

The synthesis of compound **80** can be referred to the synthesis of compound **66** in Example 66, (3-hydroxynaphthalen-1-yl)boronic acid, the initial raw material in step 1 was replaced with 3-Boc-aminophenylboronic acid, and finally 15 mg of yellow solid was obtained. LC-MS (ESI, pos. ion) m/z: 532.5 [M+H]$^+$; HRMS(ESI): 532.2626 [M+H]$^+$; $^1$H NMR (400 MHz, $CD_3OD$): δ 7.40 (s, 1H), 7.32 (d, *J* = 6.9 Hz, 1H), 7.29 - 7.22 (m, 1H), 6.89 - 6.83 (m, 1H), 5.36 (d, *J* = 53.8 Hz, 1H), 4.39 - 4.27 (m, 3H), 3.96 - 3.77 (m, 2H), 3.69 - 3.59 (m, 2H), 3.46 - 3.34 (m, 2H), 3.32 - 3.24 (m, 1H), 3.14 - 3.05 (m, 1H), 2.47 - 2.13 (m, 4H), 2.12 - 1.88 (m, 4H), 1.82 - 1.60 (m, 4H); $^{19}$F NMR (376 MHz, $CD_3OD$) δ -142.02 (1F), -173.71 (1F).

**Example 81: Synthesis of Compound 81**

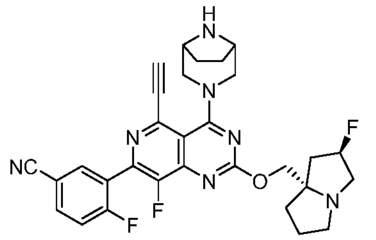

**81**

The synthesis of compound **81** can be referred to the synthesis of compound **66** in Example 66, (3-hydroxynaphthalen-1-yl)boronic acid, the initial raw material in step 1 was replaced with 5-cyano-2-fluorobenzeneboronic acid, and finally 51 mg of yellow solid was obtained. LC-MS (ESI, pos. ion) m/z: 560.2 $[M+H]^+$; HRMS(ESI): 560.2343 $[M+H]^+$; $^1H$ NMR (400 MHz, $CDCl_3$): δ 8.16 - 8.08 (m, 1H), 7.81 - 7.71 (m, 1H), 7.36 - 7.27 (m, 1H), 5.28 (d, *J* = 52.9 Hz, 1H), 4.33 - 4.23 (m, 1H), 4.21 - 4.12 (m, 1H), 4.01 - 3.79 (m, 2H), 3.71 - 3.62 (m, 2H), 3.58 (s, 1H), 3.40 - 3.13 (m, 3H), 3.06 - 2.93 (m, 1H), 2.37 - 2.09 (m, 4H), 2.02 - 1.82 (m, 4H), 1.78 - 1.47 (m, 4H); $^{19}F$ NMR (376 MHz, $CD_3OD$): δ -142.48, -134.63, -173.20.

**Example 82: Synthesis of Compound 82**

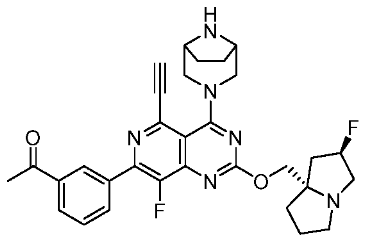

**82**

The synthesis of compound **82** can be referred to the synthesis of compound **66** in Example 66, (3-hydroxynaphthalen-1-yl)boronic acid, the initial raw material in step 1 was replaced with 3-acetylphenylboronic acid, and finally 30 mg of yellow solid was obtained. LC-MS (ESI, pos. ion) m/z: 559.3 $[M+H]^+$; HRMS(ESI): 559.2598 $[M+H]^+$; $^1H$ NMR (400 MHz, $CD_3OD$): δ 8.65 (s, 1H), 8.27 (d, *J* = 7.4 Hz, 1H), 8.15 (d, *J* = 7.8 Hz, 1H), 7.73 - 7.65 (m, 1H), 5.38 (d, *J* = 53.9 Hz, 1H), 4.44 - 4.30 (m, 3H), 3.99 - 3.79 (m, 2H), 3.76 - 3.61 (m, 2H), 3.52 - 3.35 (m, 3H), 3.18 - 3.04 (m, 1H), 2.70 (s, 3H), 2.47 - 2.16 (m, 4H), 2.13 - 1.89 (m, 4H), 1.85 - 1.58 (m, 4H); $^{19}F$ NMR (376 MHz, $CD_3OD$): δ -142.43, -173.77.

**Example 83: Synthesis of Compound 83**

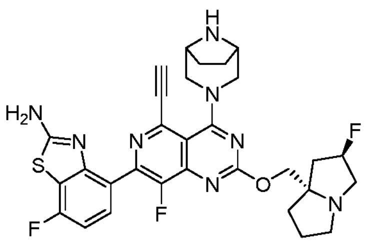

**83**

The synthesis of compound **83** can be referred to the synthesis of compound **66** in Example 66, (3-hydroxynaphthalen-1-yl)boronic acid, the initial raw material in step 1 was replaced with (2-((*tert*-butoxycarbonyl)amino)-7-fluoro-1,3-benzothiazol-4-yl)boronic acid, and finally 60 mg of yellow solid was obtained. LC-MS (ESI, pos. ion) m/z: 607.2 $[M+H]^+$; HRMS(ESI): 607.2164 $[M+H]^+$; $^1H$ NMR (400 MHz, $CD_3OD$): δ 7.56 (dd, *J* = 8.5, 5.5 Hz, 1H), 7.09 - 6.97 (m, 1H), 5.68 - 5.42 (m, 2H), 4.68 - 4.62 (m, 3H), 4.39 (s, 1H), 4.18 - 4.05 (m, 4H), 3.88 - 3.70 (m, 3H), 3.46 - 3.36 (m, 1H),

2.78 - 2.36 (m, 4H), 2.35 - 2.10 (m, 4H), 2.08 - 1.85 (m, 4H). $^{19}$F NMR (376 MHz, $CD_3OD$) δ -113.59, -136.96, -173.96.

**Example 84: Synthesis of Compound 84**

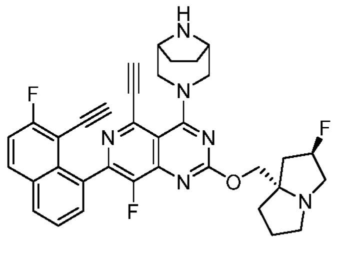

**84**

The synthesis of compound 84 can be referred to the synthesis of compound 65 in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with ((2-fluoro-8-(4,4,5,5-tetramethyl-1,3,2-dioxaborolane-2-yl)naphthalen-1-yl)ethynyl)triisopropylsilane, and finally 19 mg of yellow solid was obtained. LC-MS (ESI, pos. ion) m/z: 609.2 $[M+H]^+$; HRMS(ESI): 609.2593 $[M+H]^+$; $^1$H NMR (400 MHz, $CD_3OD$): δ 8.15 (dd, *J* = 8.7, 6.1 Hz, 2H), 7.69 (d, *J* = 7.2 Hz, 2H), 7.52 - 7.44 (m, 1H), 5.61 (d, *J* = 51.7 Hz, 1H), 4.80 - 4.67 (m, 4H), 4.41 (s, 1H), 4.33 - 4.16 (m, 4H), 4.12 - 3.78 (m, 3H), 3.61 - 3.53 (m, 1H), 3.52 - 3.41 (m, 1H), 2.87 - 2.44 (m, 3H), 2.41 - 2.18 (m, 3H), 2.17 - 1.90 (m, 4H); $^{19}$F NMR (376 MHz, $CD_3OD$): δ -106.64, -137.78, -173.92.

**Example 85: Synthesis of Compound 85**

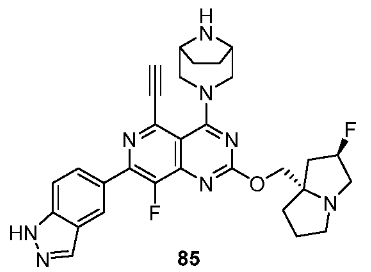

**85**

The synthesis of compound **85** can be referred to the synthesis of compound **65** in Example 65, 2-(8-ethyl-7-fluoro-3-(methoxymethoxy)naphthalen-1-yl)-4,4,5,5-tetramethyl-1,3,2-dioxaborolane, the initial raw material in step 1 was replaced with tert-butyl 5-(4,4,5,5-tetramethyl-1,3,2-dioxaborolane-2-yl)-1*H*-indazole-1-carboxylate, and finally 77 mg of yellow solid was obtained. LC-MS (ESI, pos. ion) m/z: 557.3 $[M+H]^+$; HRMS(ESI): 557.2550 $[M+H]^+$; $^1$H NMR (400 MHz, DMSO-$d_6$): δ 13.31 (s, 1H), 8.45 (s, 1H), 8.24 (s, 1H), 8.03 (d, *J* = 8.8 Hz, 1H), 7.69 (d, *J* = 8.8 Hz, 1H), 5.27 (d, *J* = 54.2 Hz, 1H), 4.81 (s, 1H), 4.22 - 3.98 (m, 3H), 3.61 - 3.48 (m, 5H), 3.17 - 2.95 (m, 4H), 2.91 - 2.74 (m, 1H), 2.20 - 1.93 (m, 3H), 1.92 - 1.65 (m, 3H), 1.65 - 1.40 (m, 4H); $^{19}$F NMR (376 MHz, DMSO-$d_6$): δ -142.14, -172.12.

**Example 86: Synthesis of Compound 86**

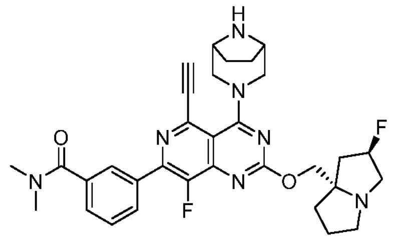

**86**

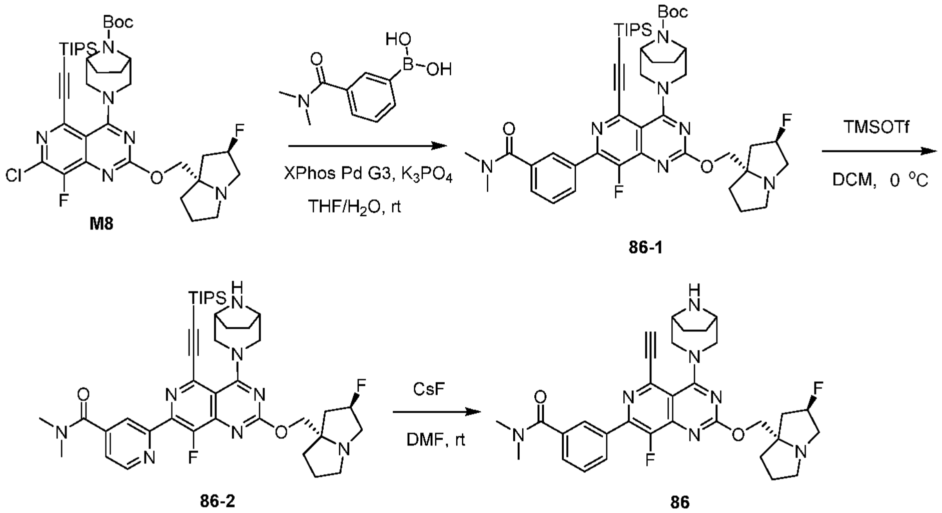


M8 86-1 86-2 86

The synthesis of compound **86** can be referred to the synthesis of compound **66** in Example 66, (3-hydroxynaphthalen-1-yl)boronic acid, the initial raw material in step 1 was replaced with 3-(dimethylaminocarbonyl)phenylboronic acid, and finally 10 mg of yellow solid was obtained. LC-MS (ESI, pos. ion) m/z: 588.3 $[M+H]^+$; HRMS(ESI): 588.2864 $[M+H]^+$; $^1H$ NMR (400 MHz, $CD_3OD$): δ 8.16 (d, *J* = 7.8 Hz, 1H), 8.12 (s, 1H), 7.69 - 7.63 (m, 1H), 7.59 (d, *J* = 7.7 Hz, 1H), 5.40 (d, *J* = 53.5 Hz, 1H), 4.47 - 4.29 (m, 3H), 4.05 - 3.87 (m, 2H), 3.80 - 3.72 (m, 2H), 3.57 - 3.35 (m, 4H), 3.17 (s, 3H), 3.08 (s, 3H), 2.51 - 2.18 (m, 4H), 2.16 - 1.92 (m, 4H), 1.87 - 1.64 (m, 4H); $^{19}F$ NMR (376 MHz, $CD_3OD$) δ -142.37, -173.79.

**Example 87: Synthesis of Compound 87**

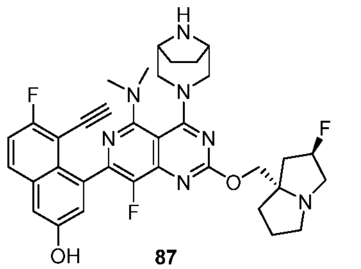

**87**

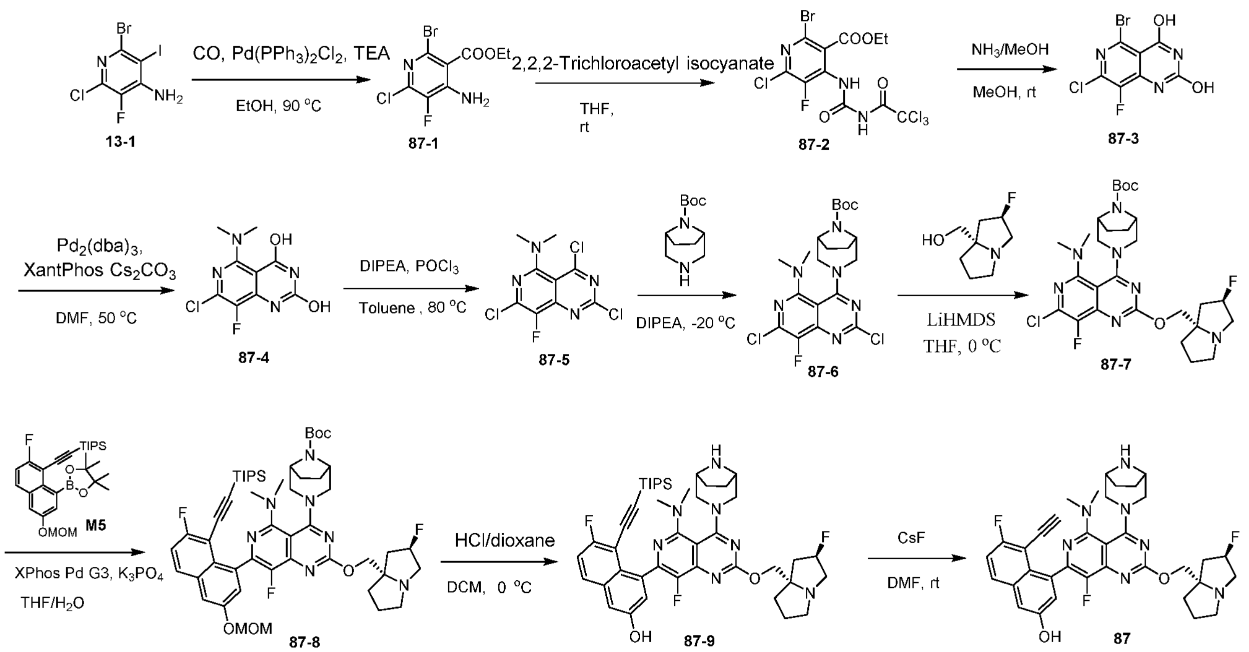


Step 1: Synthesis of Compound **87-1**

Compound **13-1** (10.0 g, 28 mmol), $Pd(PPh_3)_2Cl_2$ (3.93 mg, 6 mmol), TEA (20 mL, 140 mmol) and EtOH (100 mL) were added to a 500 mL autoclave, the autoclave was replaced with $N_2$ and CO in sequence, then filled with CO to make the pressure inside the autoclave at 2 MPa, and then the mixture was stirred at 80°C for 16 h. After the reaction was stopped, the resulting mixture was filtered through a celite pad, and the filtrate was dried with anhydrous sodium sulfate and spin-dried, and then purified by column chromatography with PE/EA (v/v = 100/1-100/2) eluent to obtain 3.47 g of a brownish yellow solid Compound 87-1, with a yield of 41%. LC-MS (ESI, pos. ion) m/z: 297.0 $[M+H]^+$.

Step 2: Synthesis of Compound **87-2**

Compound **87-1**(1.6 g, 5.38 mmol) and anhydrous THF (5 mL) were added to a 50 mL single-neck bottle, and stirred to dissolve under $N_2$, then 2,2,2-trichloroacetyl isocyanate (1.6 mL, 13.45 mmol) was added, the mixture was stirred at room temperature for 2 h, then the reaction was stopped, the resulting mixture was spin-dried and directly used for the next step, the yield was calculated as 100%.

Step 3: Synthesis of Compound **87-3**

Methanol (10 mL) was added to the Compound **87-2** obtained in the previous step, the mixture was stirred and added with methanol solution of ammonia (7.69 mL, 53.8 mmol, 7 M), then the resulting mixture was stirred at room temperature for 2 h, then solid was precipitated and filtered, the filter cake was dried to obtain 1.22 g of white solid Compound **87-3**, the yield in steps 1 and 2 was 77.04%. LC-MS (ESI, neg. ion) m/z: 292.0 $[M-H]^-$.

Step 4: Synthesis of Compound **87-4**

Compound **87-3** (250 mg, 0.85 mmol), cesium carbonate (830.0 mg, 2.55mmol), $d_2(dba)_3$ (125.0 mg, 0.14 mmol), XantPhos (162.0 mg, 0.28 mmol) and dimethylaminohydrochloride (105.0 mg, 1.29 mmol) were added to a 25 mL single-neck bottle, the bottle was replaced with $N_2$ three times, then DMF (4.0 mL) was added, and the mixture was stirred at 50 °C for 21 h. The reaction solution was diluted with DMF (10 mL), then filtered and the filter cake was rinsed with DMF (5 mL), the filtrate was concentrated to obtain 480 mg of crude product, the crude product was directly used for the next step, the yield was calculated at 100%. LC-MS (ESI, pos. ion) m/z: 259.2 $[M+H]^+$.

Step 5: Synthesis of Compound **87-5**

Compound **87-4** obtained in the previous step and anhydrous toluene (2 mL) were added to a 25 mL single-neck bottle, then phosphorus oxychloride (0.25 mL, 2.76 mmol) and DIPEA (0.70 mL, 4.25 mmol) were added, the

mixture was heated to 80 °C and stirred for 4.5 h. Then the reaction was stopped, the reaction solution was cooled and concentrated, and the resulting mixture was directly used for the next step, the yield was calculated as 100%.

Step 6: Synthesis of Compound **87-6**

Anhydrous dichloromethane (2 mL) was added to Compound **87-5** obtained in the previous step, the bottle was replaced with $N_2$ three times, and the mixture was stirred and cooled to -20°C, then DIPEA (0.70 mL, 4.25 mmol) and a solution of tert-butyl 3,8-diazabicyclo[3.2.1]octane-8-carboxylate (145 mg, 0.68 mmol) in DCM (2 mL) were added. The resulting mixture was stirred at -40°C for 0.5 h, then the reaction was stopped, 20 mL of saturated ammonium chloride was added to quench the reaction and the mixture was returned to room temperature, to the mixture was added with DCM (20 mL × 2) for extraction, the combined organic phase was dried over anhydrous sodium sulfate and concentrated, and purified by column chromatography with PE/EA (v/v =100/0- 94/6) eluent to obtain 50 mg of a yellow solid Compound 87-6, the yield in steps 4, 5 and 6 was 12%. LC-MS (ESI, pos. ion) m/z: 471.1 $[M+H]^+$.

Step 7: Synthesis of Compound **87-7**

Compound **24-5** (50 mg, 0.11 mmol), ((2R, 7a5)-2-fluorohexahydro-177-pyrrolizin-7a-yl)methanol (37 mg, 0.23 mmol) and anhydrous THF (2 mL) were added to a 10 mL single-neck bottle, the bottle was replaced with $N_2$ three times, then LiHMDS (0.44 mL, 0.44 mmol, 1 mol/L) was added dropwise, and the mixture was stirred in ice bath for 4.5 h. Saturated ammonium chloride (10 mL) and EA (15 mL) were added to the reaction solution for dilution, and the organic phase was dried over anhydrous sodium sulfate, then filtered and concentrated, the resulting mixture was purified by column chromatography with DCM/MeOH (v/v =100/0- 96/4) eluent to obtain 28.0 mg of brown foamy solid Compound **87-7**, with a yield of 44.4%. LC-MS (ESI, pos. ion) m/z: 594.5 $[M+H]^+$.

Step 8: Synthesis of Compound **87-8**

Compound **87-7** (28 mg, 0.05 mmol), **M5** (60 mg, 0.12 mmol), Xphos Pd G3 (16 mg, 0.02 mmol), $K_3PO_4$ (32 mg, 0.15 mmol) and THF/$H_2O$ (3 mL/0.5 mL) were added to a 25 mL single-neck bottle, the bottle was replaced with $N_2$ three times, and the mixture was stirred at 40 °C for 24 h, then the reaction was stopped, the reaction solution was filtered, and added with 10 mL of saturated ammonium chloride, and extracted with EA (10 mL × 3). The combined organic phase was dried over anhydrous sodium sulfate and concentrated, and the resulting mixture was purified by column chromatography with DCM/MeOH (v/v = 100/0-99/1) eluent to obtain 30 mg of tan solid Compound 87-8, with a yield of 67.4%. LC-MS (ESI, pos. ion) m/z: 944.5 $[M+H]^+$.

Step 9: Synthesis of Compound **87-9**

Compound **87-8** (30 mg, 0.037 mmol) and DCM (2 mL) were added to a 25 mL single-neck bottle, the mixture was cooled to 0°C, then hydrochloric acid (0.56 mL, 2.24 mmol, 4 M) was added dropwise, and the the reaction solution was stirred at room temperature 25 °C for 5 h, then the reaction was stopped, the mixture was concentrated, and used directly for the next step, the yield was calculated as 100%, LC-MS (ESI, pos. ion) m/z: 800.4 $[M+H]^+$.

Step 10: Synthesis of Compound **87**

Compound **87-9** (37.28 mg, 0.041 mmol) obtained in the previous step, cesium fluoride (93.42 mg, 0.61 mmol) and DMF (0.5 mL) were added to a 25 mL single-neck bottle, the mixture was stirred at 25 °C for 12 h, then the reaction was stopped, the reaction solution was added with 3 mL of saturated NaHCOs solution, then extracted with 2 × 10 mL mixed solution (DCM/MeOH (v/v) =20/1), and the combined organic phase was washed with 5 mL of saturated sodium chloride solution, the resulting mixture was dried over anhydrous sodium sulfate and concentrated, and the residue was purified by thin layer chromatography(DCM:MeOH=9: 1) to obtain 8 mg of yellow solid Compound **87**, with a yield of 30.32%. LC-MS (ESI, pos. ion) m/z: 644.3 $[M+H]^+$; HRMS(ESI): 644.3019 $[M+H]^+$.

**Example 88: Synthesis of Compound 88**

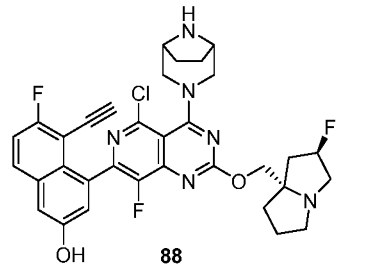

88

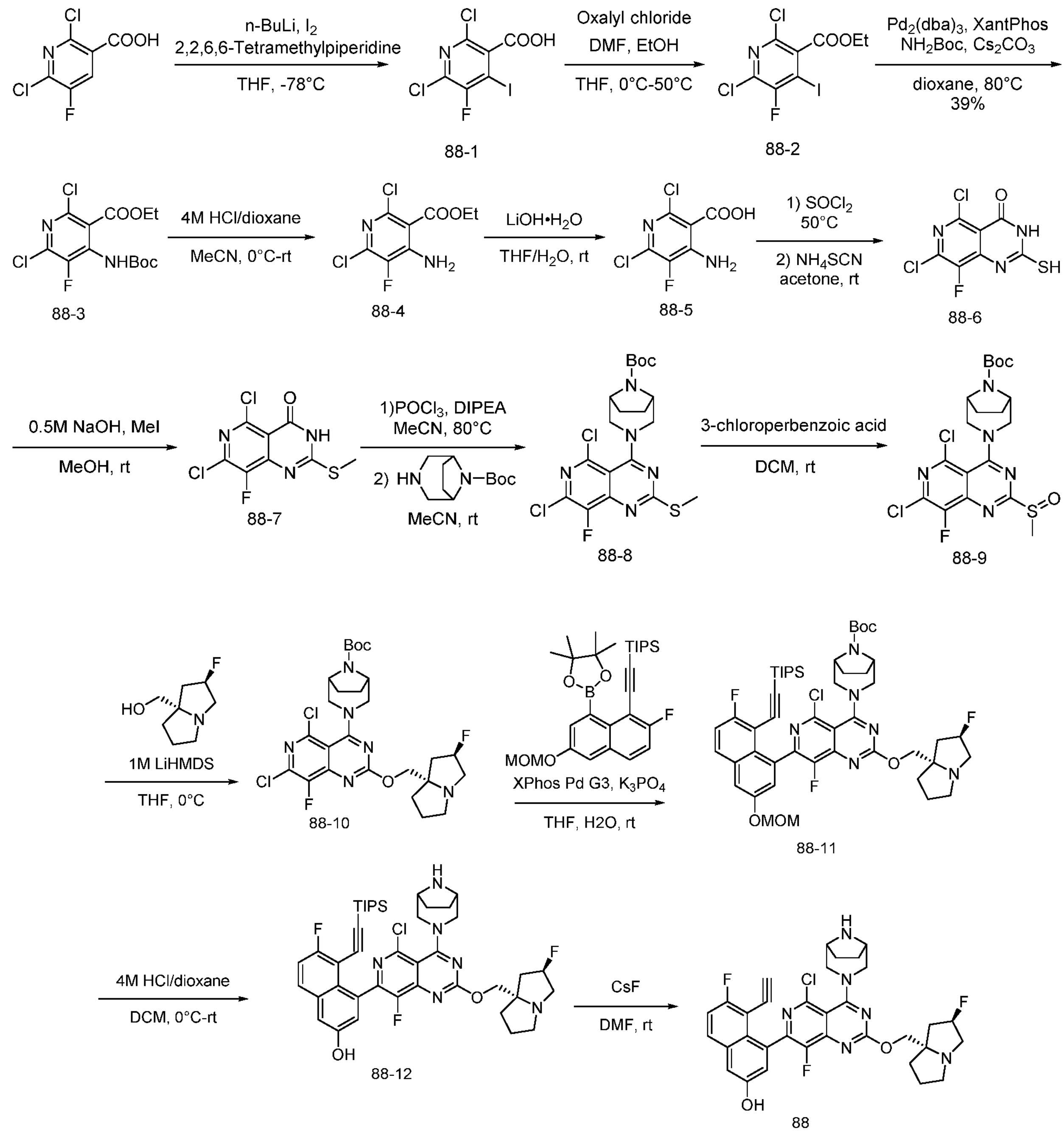


88-1, 88-2, 88-3, 88-4, 88-5, 88-6, 88-7, 88-8, 88-9, 88-10, 88-11, 88-12, 88

Step 1: Synthesis of Compound **88-1**

2,2,6,6-Tetramethylpiperidine (27.23 mL, 161.91 mmol) and THF (200 mL) were added to a 1000mL three-neck bottle, the bottle was replaced with $N_2$ three times, then the mixture was stirred at -78°C, a solution of n-BuLi in n-hexane (87.62 mL, 219.05 mmol 2.5mol/L) was slowly added dropwise, and the resulting mixture was stirred for 1h, then a solution of 2,6-dichloro-5-fluoropyridine-3-carboxylic acid (20g, 95.24mmol) in THF (50 mL) was added dropwise, and the resulting mixture was stirred for 3h, then a solution of I2 (36.26 g, 142.86 mmol) in THF (50 mL) was added dropwise, and the resulting mixture was stirred for 1h. 150 mL of saturated ammonium chloride solution was added to quench the reaction, 50 mL of EA was added, concentrated hydrochloric acid was added dropwise to adjust pH <4, then the resulting mixture was separated, the organic phase was washed with 50 mL of saturated sodium thiosulfate solution, then separated, concentrated hydrochloric acid was added dropwise to adjust pH <4, then the resulting mixture was separated, the organic phase was washed with 50 mL of saturated sodium chloride solution and concentrated under vacuum, and the fragment ions with shed-COOH were characterized by LC-MS calculated according to the yield of 100%. LC-MS (ESI, neg. ion) m/z: 290.0 $[M-COOH]^-$.

Step 2: Synthesis of Compound **88-2**

Compound **88-1** (31.99 g, 95.24 mmol) and THF (150 mL) were added to a 1000 mL single-neck bottle, and oxalyl chloride (16.12 mL, 190.50 mmol) and DMF (2.20 mL, 28.59 mmol) were added at 0°C, and the mixture was heated to 50°C and stirred for 18 h. Then stirring was stopped, the resulting mixture was concentrated and mixed with silica gel, and eluted with silica gel column chromatography (PE/EA(v/v) = 50/1) to obtain 26.13 g of yellow solid, with a yield of 75.39%. LC-MS (ESI, pos. ion) m/z: 364.0 $[M+H]^+$; $^1H$ NMR (400 MHz, $CDCl_3$): $\delta$ 4.49 (q, *J* = 7.1 Hz, 2H), 1.43 (t, *J* = 7.1 Hz, 3H).

Step 3: Synthesis of Compound **88-3**

Compound **88-2** (13 g, 35.72 mmol), *tert*-butyl carbamate (4.60 g, 39.29 mmol), tris(dibenzylacetone)palladium (1.64 g, 1.79 mmol), cesium carbonate (23.28 g, 71.44mmol), and 4,5-diphenylphosphine-9,9-dimethyloxyanthracene (2.07 g, 3.57 mmol) were added to a 500 mL three-neck bottle, the bottle was replaced with $N_2$ three times, then 1,4-dioxane (200 mL) was added, the resulting mixture was stirred at 80°C for 16 h. Then stirring was stopped, the resulting mixture was filtered through a celite pad and concentrated, mixed with silica gel and eluted with silica gel column chromatography (PE/EA(v/v) = 93/7) to obtain 5 g of a yellow solid, with a yield of 39.63%. LC-MS (ESI, neg. ion) m/z: 351.1 $[M-H]^-$; $^1H$ NMR (400 MHz, $CDCl_3$): $\delta$ 7.19 (s, 1H), 4.42 (q, *J* = 7.2 Hz, 2H), 1.51 (s, 9H), 1.42 (t, *J* = 7.2 Hz, 3H).

Step 4: Synthesis of Compound **88-4**

Compound **88-3** (5 g, 14.20 mmol) and ACN (80 mL) were added to a 1000 mL single-neck bottle, and HCl/dioxane solution (21.30 mL, 85.20 mmol, 4 M) was added dropwise at 0°C, and the resulting mixture was stirred at room temperature for 1.5 h. Then stirring was stopped, the reaction solution was concentrated, added with EA (100 mL), and adjusted pH at 8 with saturated sodium bicarbonate solution, then separated, and extracted with EA (80 mL $\times$ 2), the combined organic phase was concentrated to obtain about 4 g of a yellow solid, which was directly used for the next step, and the yield was calculated as 100%. LC-MS (ESI, pos. ion) m/z: 253.0 $[M+H]^+$. $^1H$ NMR (400 MHz, $CDCl_3$): $\delta$ 6.15 (s, 2H), 4.43 (q, *J* = 7.1 Hz, 2H), 1.42 (t, *J* = 7.1 Hz, 3H).

Step 5: Synthesis of Compound **88-5**

Compound **88-4** (5.4 g, 21.34 mmol), THF (70 mL), water (10 mL), and lithium hydroxide monohydrate (2.24 g, 53.35 mmol) were added to a 1000 mL single-neck bottle, the mixture was stirred at room temperature for 16 h. Then stirring was stopped, the reaction solution was concentrated, extracted with 50 mL of EA, the aqueous phase was added with concentrated hydrochloric acid to adjust pH=2, then extracted with EA (70 mL $\times$ 2), the combined organic phase was washed with 50 mL of saturated sodium chloride solution, then concentrated under vacuum to obtain about 3.5g of a yellow solid, with a yield of 72.89%. LC-MS (ESI, neg. ion) m/z: 223.0 $[M-H]^-$.

Step 6: Synthesis of Compound **88-6**

Compound **88-5** (3.5 g, 15.56 mmol) and thionyl chloride (100 mL, 1378.50 mmol) were added to a 250 mL single-neck bottle, the mixture was heated to 50 °C and stirred for 3 h. Then heating and stirring were stopped, the reaction solution was concentrated and added with acetone (20 mL) at room temperature to dissolve, and added with a solution of ammonium thiocyanate (3.55 g, 46.68 mmol) in acetone (30 mL) dropwise, the resulting mixture was stirred for 1 h. Then stirring was stopped, 30 mL of water was added, the mixture was concentrated and filtered, the filter cake was washed with 50ml of water, and the solid was vacuumed to obtain 3.54g of a yellow solid, with a yield of 85.50%. LC-MS (ESI, neg. ion) m/z: 264.0 $[M-H]^-$.

Step 7: Synthesis of Compound **88-7**

Compound **88-6** (3.54 g, 13.30 mmol), methanol (60 mL), NaOH solution (53.2 mL, 26.6 mmol, 0.5mol/L) and methyl iodide (1.66 mL, 26.6 mmol) were added to a 250 mL single-neck bottle, the mixture was stirred at room temperature for 3 h. To the reaction solution was added 100 mL of water, then concentrated hydrochloric acid was added dropwise to pH=4, the resulting mixture was filtered, the filter cake was washed with water (100 mL) and acetonitrile (50 mL), and vacuumed to obtain 2.49g of a yellow solid, with a yield of 66.84%. LC-MS (ESI, neg. ion) m/z: 278.0 $[M-H]^-$.

Step 8: Synthesis of Compound **88-8**

Compound **88-7** (2.49 g, 8.89 mmol), ACN (40 mL), phosphorus oxychloride (1.62 mL, 17.78 mmol), and ethyldiisopropylamine (2.94 mL, 17.78 mmol) were added to a 250 mL single-neck bottle, the mixture was stirred at 80°C for 2 h. Then stirring was stopped, the mixture was cooled, ethyldiisopropylamine (2.94 mL, 17.78 mmol) and tert-butyl 3,8-diazabicyclo[3.2.1]octane-8-carboxylate (3.77 g, 17.78 mmol) were added and the mixture was stirred at room temperature for 3 h. Then stirring was stopped, the reaction solution was concentrated and diluted with DCM (80 mL), then added with saturated ammonium chloride solution (100 mL), separated and extracted with DCM (100 mL), the combined organic phase was concentrated, mixed with silica gel, and eluted with silica gel column chromatography (PE/EA(v/v) = 20/1) to obtain about 3.41 g of a yellow solid, with a yield of 80.86%. LC-MS (ESI, pos. ion) m/z: 474.1 $[M+H]^+$; $^1H$ NMR (400 MHz, $CDCl_3$): δ 4.48 - 3.04 (m, 6H), 2.61 (s, 3H), 2.02 - 1.59 (m, 4H), 1.50 (s, 9H).

Step 9: Synthesis of Compound **88-9**

Compound **88-8** (200 mg, 0.42 mmol), 3-chloroperoxybenzoic acid (79.73 mg, 0.46 mmol) and DCM (8 mL) were added to a 100 mL single-neck bottle, the mixture was stirred at room temperature for 1 h. Then stirring was stopped, the reaction solution was concentrated and added with 20 mL of EA, and the organic phase was washed with saturated $NaHCO_3$ solution (30 mL) and saturated sodium chloride (30 mL) solution in sequence, and dried over anhydrous sodium sulfate, then concentrated under vacuum, the yield was calculated as 100%. LC-MS (ESI, pos. ion) m/z: 490.3 [M+H]+.

Step 10: Synthesis of Compound **88-10**

Compound **88-9** (1.28 g, 2.61 mmol) and ((2*R*,7a*S*)-2-fluorotetrahydro-1*H*-pyrrolizin-7a(5*H*)-yl)methanol (0.83 g, 5.22 mmol) were added to a 100 mL two-neck bottle, the bottle was replaced with $N_2$, then THF (20 mL) was added at 0°C, and a solution of LiHMDS (5.22 mL, 5.22 mmol, 1 mol/L) in THF was added and the mixture was stirred for 1 h. Then stirring was stopped, EA (50 mL) was added and the reaction was quenched with saturated ammonium chloride solution (50 mL), then separated, extracted with EA (50 mL), the combined organic phase was washed with saturated sodium chloride solution (30 mL), dried over anhydrous sodium sulfate and concentrated, and eluted by silica gel column chromatography (DCM/MeOH(v/v) = 50/1), the resulting mixture was slurried with MTBE (50 mL) to obtain 628 mg of a yellow solid, with a yield of 41.09%. LC-MS (ESI, pos. ion) m/z: 585.2 $[M+H]^+$.

Step 11: Synthesis of Compound **88-11**

Compound **88-10** (623 mg, 1.06 mmol), **M5** (814.94 mg, 1.59 mmol), XPhos Pd G3 (179.45 mg, 0.21 mmol) and potassium phosphate (562.52 mg, 2.65 mmol) were added to a 100 mL two-neck bottle, the bottle was replaced with $N_2$ three times, then THF (20 mL) and water (3 mL) were added, and the mixture was stirred at 35°C for 12 h. Then stirring was stopped, the mixture was filtered, and DCM (30 mL) was added, the organic phase was washed with saturated sodium chloride solution (30 mL), dried over anhydrous sodium sulfate and concentrated, then mixed with silica gel, eluted with silica gel column chromatography (DCM/MeOH (v/v) = 50/1) to obtain 330 mg of a yellow solid, with a yield of 33.15%. LC-MS (ESI, pos. ion) m/z: 935.7 $[M+H]^+$.

Step 12: Synthesis of Compound **88-12**

Compound **88-11** (200 mg, 0.21 mmol) and DCM (5 mL) were added to a 25 mL single-neck bottle, then HCl/dioxane (1.57 mL, 6.3 mmol, 4 M) was added at 0°C, and the mixture was stirred at room temperature for 4 h. Then stirring was stopped, the reaction solution was concentrated under vacuum to obtain 186 mg of a yellow solid, the yield was calculated as 100%. LC-MS (ESI, pos. ion) m/z: 791.3 $[M+H]^+$.

Step 13: Synthesis of Compound **88**

Compound **88-12** (186 mg, 0.21 mmol), cesium fluoride (478.49 mg, 3.15 mmol) and DMF (1 mL) were added to a 25 mL single-neck bottle, the bottle was replaced with $N_2$ three times, and the mixture was stirred at room temperature for 12 h. The reaction solution was sampled and tested by LC-MS, and showed that the raw material was completed, then stirring was stopped, the reaction solution was directly eluted with reversed-phase column ($H_2O$/MeCN (v/v) =1/1), and the obtained substance was purified by thin-layer chromatography chromatography (MeOH/H2O/NH4OH (v/v/v)=85/10/0.5) to obtain 30 mg of a yellow solid, with a yield of 22.88%. LC-MS (ESI, pos. ion) m/z: 635.5[M+H]$^+$; HRMS(ESI): 635.2125 [M+H]$^+$.

**Example 89: Synthesis of Compound 89**

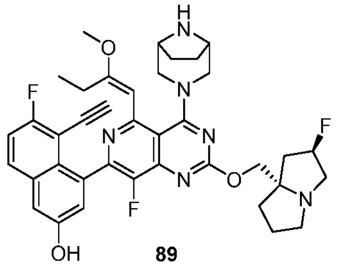

**89**

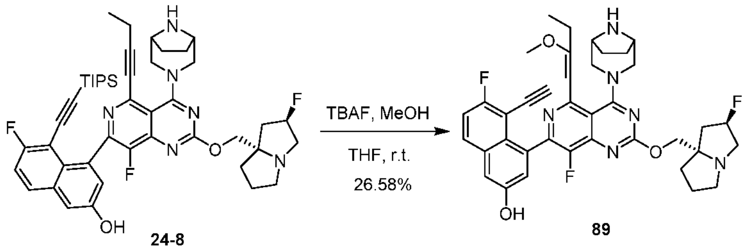


**24-8** **89**

Compound **24-8** (120 mg, 0.15 mmol) and THF (5 mL) were added to a 25 mL single-neck bottle, then TBAF (2.25 mL, 2.25 mmol) was added and the mixture was stirred at 30 °C for 6 h. To the reaction solution was added 10 mL of methanol and the mixture was reacted at 30 °C for 5 h. Then the reaction was stopped, the mixture was concentrated to remove solvent, the residue was dissolved by DCM (50 mL) and methanol (5 mL), and washed with saturated ammonium chloride solution (10mL × 2), then dried over anhydrous sodium sulfate, then filtered and concentrated under vacuum, the resulting mixture was separated by thin layer chromatography silica gel preparation plate (DCM/MeOH/35% ammonia (v/v/v) = 100/10/0.5) to obtain 27 mg of a brown solid, with a yield of 26.58%. LC-MS (ESI, pos. ion) m/z: 685.7[M+H]$^+$; HRMS(ESI): 685.2760 [M+H]$^+$; $^1$H NMR (400 MHz, $CD_3OD$): δ 8.08 (dd, *J* = 9.0, 5.7 Hz, 1H), 7.81 (d, *J* = 2.3 Hz, 1H), 7.52 - 7.42 (m, 2H), 5.56 (d, *J* = 51.8 Hz, 1H), 4.65 - 4.49 (m, 2H), 4.22 - 4.01 (m, 3H), 3.92 - 3.73 (m, 3H), 3.60-3.34 (m, 2H), 3.28 - 3.22 (m, 3H), 2.75 - 2.38 (m, 5H), 2.37 - 1.84 (m, 8H), 1.74 - 1.62 (m, 4H), 1.04 (t, *J* = 7.3 Hz, 3H). $^{19}$F NMR (376 MHz, $CD_3OD$) δ -80.06 (1F), -108.73 - -108.82 (1F), -152.57 (1F).

**Example 90: Synthesis of Compound 90**

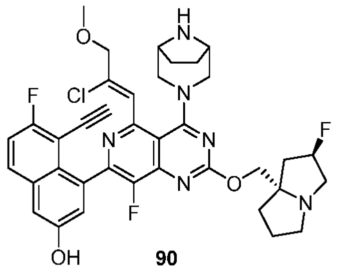

90

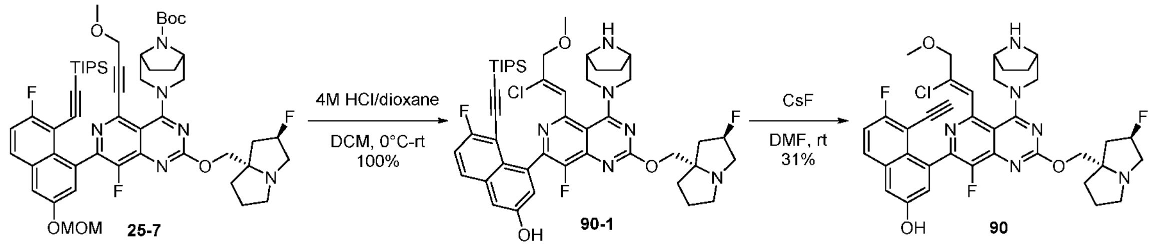


25-7 90-1 90

Step 1: Synthesis of Compound **90-1**

Compound **25-7** (36 mg, 0.037 mmol) and DCM (2 mL) were added to a 25 mL single-neck bottle, then HCl/dioxane solution (0.27 mL, 1.08 mmol, 4 M) was added at 0°C, and the mixture was stirred at room temperature for 2 h. Then stirring was stopped, the resulting mixture was concentrated under vacuum, and used directly for the next step, the yield was calculated as 100%. LC-MS (ESI, pos. ion) m/z: 861.4 $[M+H]^+$.

Step 2: Synthesis of Compound **90**

Compound **90-1** (34.6 mg, 0.036 mmol), cesium fluoride (82.03 mg, 0.54 mmol) and DMF (0.5 mL) were added to a 25 mL single-neck bottle, the bottle was replaced with $N_2$ three times, and the mixture was stirred at room temperature for 12 h. Then stirring was stopped, a solution of DCM/MeOH (v/v = 9/1, 10 mL) and 5 mL of saturated sodium chloride solution were added, then the mixture was separated, extracted with a solution of DCM/MeOH (v/v=9/1) (10 mL × 2), the combined organic phase was washed with saturated sodium chloride solution (10 mL), dried over anhydrous sodium sulfate, then concentrated, and separated and purified by thin layer chromatography chromatography (DCM/MeOH/$NH_4OH$ (v/v/v)=85/10/0.5) to obtain 8 mg of a red solid, with a yield of 31%. LC-MS (ESI, pos. ion) m/z: 705.5 $[M+H]^+$; HRMS(ESI): 705.2550 $[M+H]^+$.

**Example 91: Synthesis of Compound 91**

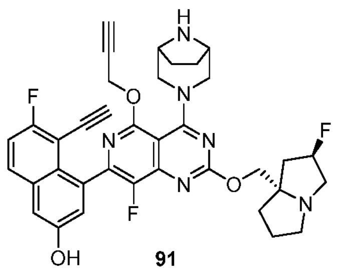

91

The synthesis of compound **91** can be referred to the synthesis of compound **9** in Example 9, 3,3,3-trifluoropropane-1-ol, the initial raw material in step 1 was replaced with 3-(triisopropylsilyl)propyl-2-yn-1-ol, and finally 7 mg of yellow solid Compound **91** was obtained. LC-MS (ESI, pos. ion) m/z: 655.5 $[M+H]^+$; HRMS(ESI): 655.2638 $[M+H]^+$.

**Example 92: Synthesis of Compound 94**

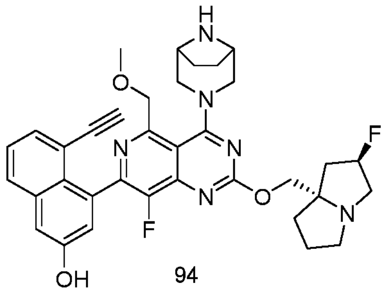

94

The synthesis of compound **94** can be referred to the synthesis of compound **12** in Example 12. LC-MS (ESI, pos. ion) m/z: 627.3 $[M+H]^+$.

**Example 93: Synthesis of Compound 101**

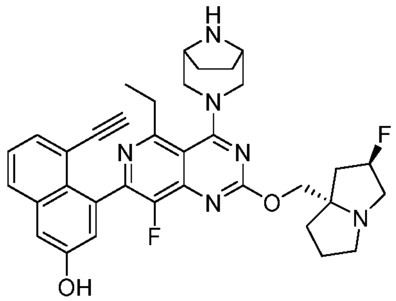

101

The synthesis of compound 101 can be referred to the synthesis of compound 1 in Example 1. LC-MS (ESI, pos. ion) m/z: 611.3 $[M+H]^+$.

**Example 94: Synthesis of Compound 103**

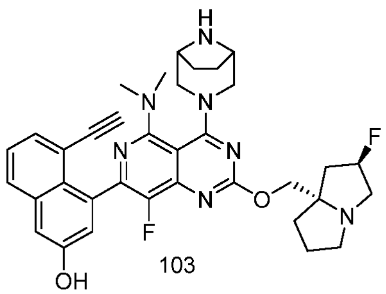

103

The synthesis of compound **103** can be referred to the synthesis of compound **87** in Example 87. LC-MS (ESI, pos. ion) m/z: 626.4 $[M+H]^+$.

**Example 95-97: Synthesis of Compounds 117, 118 and 119**

| Example | Compound | Synthesis process and structural characterization |
|---|---|---|
| Example **95** | 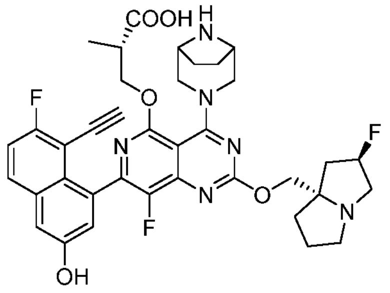 Compound 117 | Referring to the synthesis of compound 15 in Example 15. LC-MS (ESI, pos. ion) m/z: 703.3 $[M+H]^+$. |
| Example **96** | 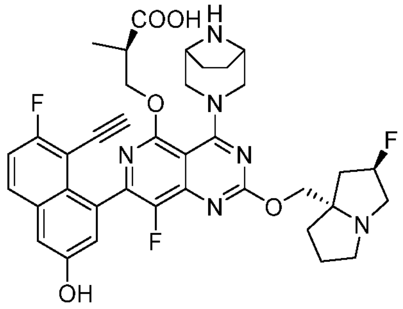 Compound 118 | Referring to the synthesis of compound 15 in Example 15. LC-MS (ESI, pos. ion) m/z: 703.3 $[M+H]^+$. |
| Example **97** | 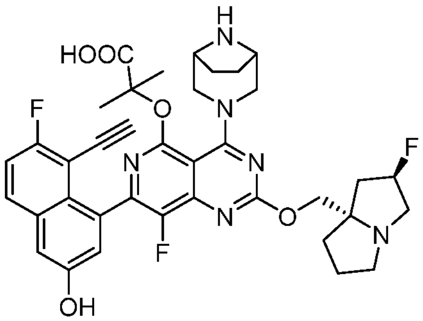 Compound 119 | Referring to the synthesis of compound 15 in Example 15. LC-MS (ESI, pos. ion) m/z: 703.3 $[M+H]^+$. |

**Example 98-100: Synthesis of Compounds 120, 121 and 122**

| Example | Compound | Synthesis process and structural characterization |
|---|---|---|
| Example **98** | 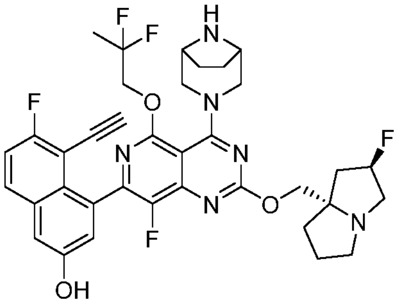 Compound 120 | Referring to the synthesis of compound 9 in Example 9. LC-MS (ESI, pos. ion) m/z: 695.3 $[M+H]^+$. |
| Example **99** | 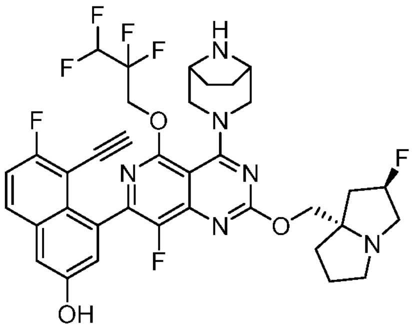 Compound 121 | Referring to the synthesis of compound 9 in Example 9. LC-MS (ESI, pos. ion) m/z: 731.4 $[M+H]^+$. |
| Example **100** | 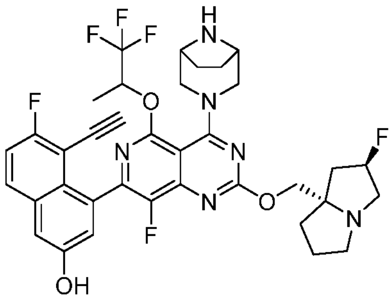 Compound 122 | Referring to the synthesis of compound 15 in Example 9. LC-MS (ESI, pos. ion) m/z: 713.5 $[M+H]^+$. |

**Example 101: Synthesis of Compound 126**

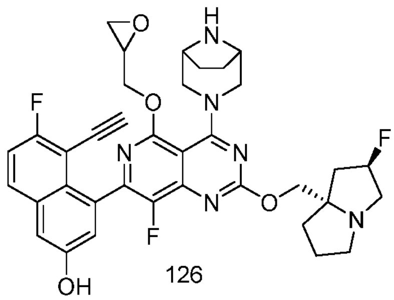

126

The synthesis of **compound 126** can be referred to the synthesis of compound **9** in Example 9. LC-MS (ESI,

pos. ion) m/z: 673.5 [M+H]+

**Example 102-105: Synthesis of Compounds 127, 128, 129 and 130**

| Example | Compound | Synthesis process and structural characterization |
|---|---|---|
| Example **102** | 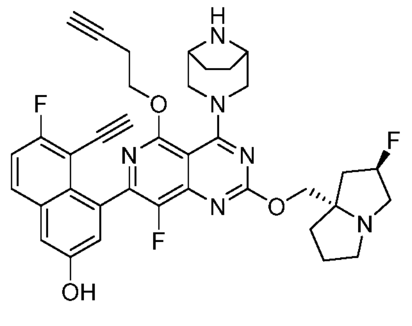 Compound 127 | Referring to the synthesis of compound 92 in Example 92. LC-MS (ESI, pos. ion) m/z: 669.3 [M+H]+. |
| Example **103** | 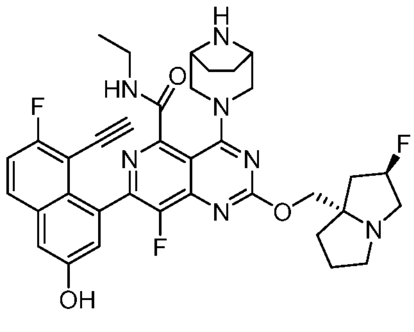 Compound 128 | Referring to the synthesis of compound 18 in Example 18. LC-MS (ESI, pos. ion) m/z: 672.5 [M+H]+. |
| Example **104** | 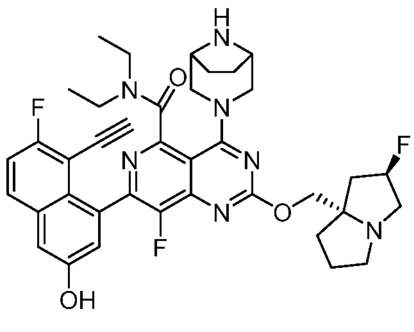 Compound 129 | Referring to the synthesis of compound 18 in Example 18. LC-MS (ESI, pos. ion) m/z: 700.3 [M+H]+. |

(continued)

| Example | Compound | Synthesis process and structural characterization |
|---|---|---|
| Example **105** | 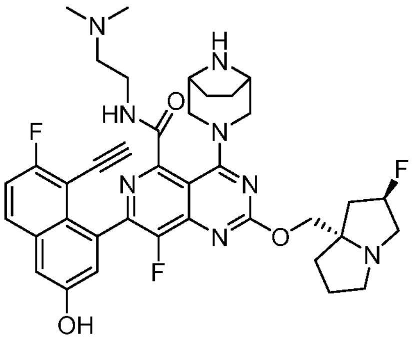 Compound 130 | Referring to the synthesis of compound 18 in Example 18. LC-MS (ESI, pos. ion) m/z: 715.3 $[M+H]^+$. |

**Embodiments of activity test**

**1. KRAS G12D/cRAF binding experiment**

Procedure:

(1) Compound was prepared with DMSO, and diluted by 3-fold gradient with DMSO.
(2) 0.1 μl of serial dilutions of the compound was added to a 384-well plate.
(3) 5 μl of Tag2-KRASG12D&GTP at a specific concentration was added to the 384-well plate, and the plate was centrifuged at 1000 rpm for 1 min.
(4) Then 5 μl of Tag1-cRAF at a specific concentration was added to the 384-well plate, and the plate was centrifuged at 1000 rpm for 1 min.
(5) The plate was incubated at 25 °C for 15 min.
(6) 10 μl of the mixture of anti-Tag1-$Tb^{3+}$ and anti-Tag2-XL665 was added to the 384-well plate.
(7) The plate was centrifuged at 1000 rpm for 1 min and incubated at 4 °C for 3 h.
(8) The 665/615 nm ratio was read by a microplate reader.
(9) Data analysis: The log value of the compound concentration was taken as the abscissa, and the 665/615 nm ratio was used as the ordinate, the data were analyzed by GraphPad Prism 8.0 software, and the IC50 was calculated, the specific experimental results are shown in Table 1.

**2. KRAS G12D/SOS1 binding experiment**

Procedure:

(1) Compound was prepared with DMSO, and diluted by 3-fold gradient with DMSO.
(2) 0.1 μl of serial dilutions of the compound was added to a 384-well plate.
(3) 5 μl of Tag2-KRASG12D&GTP at a specific concentration was added to the 384-well plate, and the plate was centrifuged at 1000 rpm for 1 min.
(4) Then 5 μl of Tag1-SOS1 at a specific concentration was added to the 384-well plate, and the plate was centrifuged at 1000 rpm for 1 min.
(5) The plate was incubated at 25 °C for 15 min.
(6) 10 μl of the mixture of anti-Tag1-Tb3+ and anti-Tag2-XL665 was added to the 384-well plate.
(7) The plate was centrifuged at 1000 rpm for 1 min and incubated at 4°C for 3 h.
(8) The 665/615 nm ratio was read by a microplate reader.
(9) Data analysis: The log value of the compound concentration was taken as the abscissa, and the 665/615 nm

ratio was used as the ordinate, the data were analyzed by GraphPad Prism 8.0 software, and the $IC_{50}$ was calculated, the specific experimental results are shown in Table 1.

**3. Inhibitory effect of compounds on intracellular pERK with In-cell Western method:**

Procedure:

(1) On the first day, AGS cells were resuscitated in a 1640 medium containing 10% fetal bovine serum;
(2) The next day, the cells were rinsed once with PBS, added with pre-warmed trypsin, and digested in a 5% COz incubator at 37 °C for 3 min; then an appropriate amount of 1640 complete culture medium (containing 10% fetal bovine serum) was added to terminate digestion and the mixture was transferred to a centrifuge tube, and then centrifuged at 1000 rpm for 5 min.
(3) The cells were resuspended with complete culture medium and counted.
(4) Cells were added with complete culture medium to an appropriate cell concentration, then the cells were added to a 96-well plate, and incubated overnight in a 5% $CO_2$ incubator at 37 °C.
(5) On the third day, 10× serial dilutions of the compound were prepared.
(6) 10 μl of the prepared 10× serial dilutions compound were added to the 96-well plate and incubated in a 5% $CO_2$ incubator at 37 °C for 3 h.
(7) Then the 96-well plate was taken out, the liquid was discarded, and 100 μl of 4% paraformaldehyde was added to each well to fix the cells for more than 30 min.
(8) The mixture was washed 3 times with a solution of 1% trizol-x100 in PBS for 5 min each time.
(9) Blocking solution was added and the mixture was shaken at room temperature at 300 rpm for 1 h.
(10) Then the blocking solution was discarded, primary antibody was added, and the cells were incubated at 4 °C overnight.
(11) On the fourth day, the 96-well plate was shaken at room temperature at 300 rpm for 30 min on a shaker.
(12) Then the primary antibody was discarded and the mixture was washed 3 times with TBST for 5 min each time.
(13) Then the TBST was discarded, and secondary antibody was added, the plate was shaken at room temperature at 300 rpm for 1 h with protecting from light.
(14) Then the secondary antibody discarded and the mixture was washed 3 times with TBST for 5 min each time.
(15) The 96-well plate was scanned with Odyssey CLx.
(16) Data analysis:

a)

Relative signal value = (signal value of the channel corresponding to the target protein / signal value of the corresponding channel of the reference protein) × 10000

b)

$$\text{Inhibition rate} = 100 - (\text{compound pore value/blank value}) \times 100$$

c) The log value of the compound concentration was used as the abscissa and the inhibition rate was used as the ordinate, the data were analyzed by GraphPad Prism 5.0 software, and the $IC_{50}$ was calculated, the specific experimental results are shown in Table 2.

Table 1. Competitive binding of compounds to KRAS G12D-GTP/cRAF and KRAS G12D-GTP/SOS1

| Compound | KRAS G12D-GTP & cRAF, HTRF, $IC_{50}$ (nM) | KRAS G12D-GTP & SOS1, HTRF, $IC_{50}$ (nM) |
|---|---|---|
| Compound 9 | 8.8 | 12.6 |
| Compound 10 | 8.7 | 13.9 |
| Compound 11 | 9.2 | 19.8 |

(continued)

| Compound | KRAS G12D-GTP & cRAF, HTRF, $IC_{50}$ (nM) | KRAS G12D-GTP & SOS1, HTRF, $IC_{50}$ (nM) |
|---|---|---|
| Compound 12 | 9.7 | 10.3 |
| Compound 13 | 5.1 | 11.3 |
| Compound 24 | 13.6 | 15.1 |
| Compound 25 | 15.0 | 16.8 |
| Compound 28 | 11.1 | 11.5 |
| Compound 29 | 14.7 | 15.9 |
| Compound 30 | 8.8 | 13.4 |
| Compound 31 | 24.6 | 34.6 |
| Compound 41 | 22.0 | 31.4 |
| Compound 43 | 16.3 | 24.1 |
| Compound 44 | 15.2 | 29.6 |
| Compound 49 | 6.9 | 15.7 |
| Compound 50 | 19.1 | 36.2 |
| Compound 51 | 11.1 | 20.8 |
| Compound 52 | 7.7 | 15.2 |
| Compound 53 | 4.3 | 14.6 |
| Compound 55 | 9.8 | 14.8 |
| Compound 56 | 18.5 | 15.7 |
| Compound 65 | 9.9 | 18.5 |
| Compound 66 | 5.9 | 11.7 |
| Compound 67 | 5.4 | 13.5 |
| Compound 68 | 6.5 | 16.6 |
| Compound 78 | 7.5 | 17.3 |
| Compound 84 | 20.8 | 29.6 |

As can be seen from Table 1, the compound of the present invention can effectively bind to KRAS G12D-GTP and hinder the binding of KRAS to cRAF and SOS 1 proteins.

Table 2. Inhibitory effect of compounds on intracellular pERK

| Compound | $IC_{50}$ (nM) |
|---|---|
| Compound 9 | 0.6 |
| Compound 23 | 0.6 |
| Compound 24 | 0.2 |
| Compound 25 | 0.8 |
| Compound 26 | 0.4 |
| Compound 29 | 1.0 |
| Compound 30 | 0.8 |
| Compound 39 | 0.5 |

(continued)

| Compound | $IC_{50}$ (nM) |
|---|---|
| Compound 49 | 0.6 |
| Compound 51 | 1.2 |
| Compound 52 | 1.5 |
| Compound 53 | 0.4 |
| Compound 65 | 0.4 |
| Compound 67 | 0.4 |
| Compound 68 | 0.8 |
| Compound 91 | 0.3 |

As can be seen from Table 2, the compounds of the present invention can effectively inhibit pERK downstream of KRAS.

**4. Stability evaluation of the compounds of the present invention in human and mouse liver microsomes**

Obj ective: To evaluate the stability of the compounds of the present invention in human and mouse liver microsomes by LC-MS/MS.

Brief experimental flow: The test compound and liver microsomes of human or mouse were co-incubated in 0.1 M potassium phosphate buffer (pH =7.4±0.1) at 37 °C, and the half-life of the compound was calculated by measuring the concentration of the test compound at different incubation times, and plotting the "relative content of the compound" against the "incubation time" in GraphPad Prism 5.01, and the intrinsic clearance was calculated. The experimental system is shown in Table A:

Table A Experimental system

| | |
|---|---|
| Test object | Compound of the present invention (dissolved with DMSO, diluted with acetonitrile) |
| Mouse liver microsomes (Purchased from BD (Becton, Dickinson and Company)) | Mixed individuals were tested at a final concentration of 0.5 mg/mL |
| Human liver microsomes (Purchased from BD (Becton, Dickinson and Company)) | Mixed individuals were tested at a final concentration of 0.5 mg/mL |
| Buffer | 0.1 M potassium phosphate buffer (pH=7.4±0.1) |
| The final concentration of the tested object | 1 μM |
| Final content of the organic solvent | 0.1% |
| Final reaction system | 30 μL buffer solution containing the compound of the present invention and human or mouse liver microsomes; 15 μL of NADPH buffer solution (concentration was 6 mM). |
| Test conditions | Time points: 0 min, 15 min, 30 min, 60 min<br>Temperature: 37 °C<br>pH: 7.4±0.1 |
| Number of resamples | 2 |
| Analytical Methods | LC/MS/MS, the internal standard was Propranolol |

The ratio of the sample peak area to the internal standard peak area was obtained by LC/MS/MS, and the content of the compounds at 0 min was regarded as 100%, and the relative content of the compounds at each time point was calculated. The half-life of the compound was calculated by plotting the "relative content of the compound" against the "incubation time", and the intrinsic clearance was calculated, in which the half-life and intrinsic clearance of the compound in human and mouse liver microsomes were determined in Table 3 and Table 4, respectively.

Table 3 Determination results of half-life and intrinsic clearance of compounds of the present invention in human liver microsomes

| Compound | Conc. (μM) | $T_{1/2}$ (min) | CLint (μL/min/mg) | CLHep (mL/min/kg) | CLin vivo (mL/min/kg) | ER |
|---|---|---|---|---|---|---|
| Compound 23 | 1.00 | 75.3 | 18.4 | 23.0 | 10.9 | 0.53 |
| Compound 39 | 1.00 | 91.1 | 15.2 | 19.0 | 9.9 | 0.48 |
| Compound 54 | 1.00 | 311.9 | 4.4 | 5.6 | 4.4 | 0.21 |
| Compound 67 | 1.00 | 119.4 | 11.6 | 14.5 | 8.5 | 0.41 |
| Remarks: Slow metabolism (ER<0.3), Moderate metabolism (0.3<ER<0.7), Fast metabolism (ER>0.7) | | | | | | |

The experimental results show that the above-mentioned compounds of the present invention are moderate metabolism or slow metabolism in human liver microsomes.

Table 4 Determination results of half-life and intrinsic clearance of compounds of the present invention in mouse liver microsomes

| Compound | Conc. (μM) | $T_{1/2}$ (min) | CLint (μL/min/mg) | CLHep (mL/min/kg) | CLin vivo (mL/min/kg) | ER |
|---|---|---|---|---|---|---|
| Compound 23 | 1.00 | 29.1 | 47.6 | 187.4 | 60.8 | 0.68 |
| Compound 39 | 1.00 | 102.3 | 13.5 | 53.4 | 33.5 | 0.37 |
| Compound 54 | 1.00 | 171.4 | 8.1 | 31.9 | 23.5 | 0.26 |
| Compound 67 | 1.00 | 30.6 | 45.3 | 178.5 | 59.8 | 0.66 |
| Remarks: Slow metabolism (ER<0.3), Moderate metabolism (0.3<ER<0.7), Fast metabolism (ER>0.7) | | | | | | |

The experimental results show that the compounds of the present invention are moderately metabolized or slow-metabolized in mouse liver microsomes.

**5. Pharmacokinetic evaluation of compounds of the present invention administered intravenously in mice**

The inventors performed a pharmacokinetic evaluation of the compounds of the present invention in mice. Among them, the detailed animal information shows in Table 2.

Table B Information table of the test animal

| Germline | Grade | Gender | Weight | Age | Source |
|---|---|---|---|---|---|
| BALB/c mice | SPF | Male | 18-30 g | 6-8 weeks | Hunan SJA Laboratory Animal Co., Ltd |

Experimental Method

The compound of the present invention was in the form of a solution of 5%DMSO+30%PEG400+65% Saline, 10%DMSO+10%Kolliphor HS15+ 80% Saline, 10%DMSO+89%(25%SBE-B-CD)+(2%HCl), 20%PEG400+80% sterilize water for injection or 10%DMA+10%HS15+30%PEG400+50% sterilize water for injection, the test animals were administered, and the animals were fasted for 12 h before administration, and water was free to drink. The test animals were administered intravenously at a dose of 2 mg/kg, blood (approximately 0.15 mL) was taken intravenously at the following time points after administration: 0.083 h, 0.25 h, 0.5 h, 1.0 h, 2.0 h, 5.0 h, 7.0 h, and 24 h, EDTA-K2 was pre-added as an anticoagulant in the blood collection tube, the blood samples were centrifuged at 12,000 rpm for 2 min, then plasma was collected and stored at -20 °C or -70 °C.

The plasma samples collected above were treated (after the frozen plasma thawed at room temperature,

vortexed for 15 s for mixing, 10-20 μL of plasma was taken and added with 120-150 μL of acetonitrile solution containing internal standard, the mixture was vortexed for 5 min for mixing, then centrifuged at 4,000 rpm for 5 min, 100 μL of supernatant was taken, and 120-150 μL of methanol/water (v/v = 1/1) was added to mix), and the compound concentration in plasma was analyzed by LC-MS/MS. The pharmacokinetic parameters of the compounds of the present invention for intravenous administration in mice are shown in Table 5.

Table 5 Pharmacokinetic parameters of the compound of the present invention for intravenous administration in mice

| Compound | $T_{max}$ (h) | $C_{max}$ (ng/mL) | $AUC_{last}$ (h*ng/mL) | $AUC_{INF}$ (h*ng/mL) | $T_{1/2}$ (h) | $MRT_{last}$ (h) | Cl (mL/min/kg) | $V_{ss}$ (L/kg) |
|---|---|---|---|---|---|---|---|---|
| Compound 23 | 0.083 | 3550 | 2140 | 2160 | 0.84 | 0.7 | 16 | 0.69 |
| Compound 67 | 0.083 | 4300 | 3210 | 3230 | 0.87 | 0.75 | 10 | 0.5 |

The results in Table 5 show that the compounds of the present invention have high Cmax and AUC values in mice, indicating that the compounds of the present invention have good druggability and have better clinical application prospects.

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. The appearances of the phrases throughout this specification are not necessarily referring to the same embodiment or example of the present disclosure. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure, the scope of the present invention is limited by the claims and equivalents.

## Claims

**1.** A compound having Formula (I) or a stereoisomer, a tautomer, an N-oxide, a solvate, a metabolite, a pharmaceutically acceptable salt or a prodrug thereof,

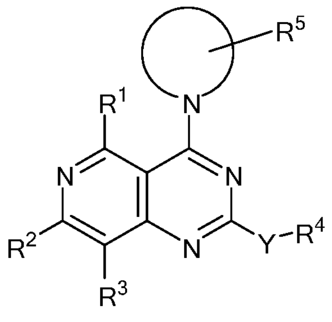

(I),

wherein,

$R^1$ is -F, -Br, -I, -CN, -SH, $-C(=O)R^{6a}$, $-C(=O)OR^{6a}$, $-C(=O)NR^6R^7$, $-NR^6C(=O)R^7$, $-NR^6R^7$, - $NR^6S(=O)_2R^7$, $C_{2-6}$ alkenyl, $C_{2-6}$ alkynyl, $C_{1-6}$ cyanoalkyl, $C_{1-6}$ hydroxyalkyl, $C_{2-6}$ haloalkenyl, $C_{2-6}$ haloalkynyl, $C_{1-6}$ alkoxy $C_{1-6}$ alkyl, $C_{1-6}$ carboxyalkyl, $C_{1-6}$ aminoalkyl, $C_{1-6}$ mercaptoalkyl or 3-6 membered heterocyclyl, wherein the $C_{2-6}$ alkenyl, $C_{2-6}$ alkynyl, $C_{1-6}$ cyanoalkyl, $C_{1-6}$ hydroxyalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, $-C(=O)OR^{6f}$, $-C(=O)NR^6R^7$, - $NR^6C(=O)R^7$, $-C_{1-6}$ alkyl $NR^6C(=O)R^7$, $-NR^{6f}C(=O)NR^6R^7$, $-C(=O)R^{6a}$, $-NR^6S(=O)_2R^7$, - $S(=O)_2NR^6R^7$, $-NR^{6f}S(=O)_2NR^6R^7$, $-NR^{6f}R^{7f}$, $-C_{1-6}$ alkyl $NR^{6f}R^{7f}$, $C_{1-6}$ alkyl, $C_{2-6}$ alkenyl, $C_{2-6}$ alkynyl, $C_{1-6}$ alkoxy, (6-12 mem-

bered aryl)-$C_{1-6}$ alkoxy, $C_{1-6}$ cyanoalkyl, $C_{1-6}$ hydroxyalkyl, $C_{1-6}$ haloalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl, 6-12 membered aryl, 5-12 membered heteroaryl, $C_{3-6}$ cycloalkyl and 3-6 membered heterocyclyl;

$R^2$ is 6-12 membered aryl or a 5-12 membered heteroaryl, wherein the 6-12 membered aryl and 5-12 membered heteroaryl are independently and optionally substituted with 1, 2, 3, 4, 5, 6 or 7 $R^8$;

each $R^8$ is independently -D, -OH, -F, -Cl, -Br, -I, -CN, -SH, $-CH^2C(=O)NR^{6b}R^{7b}$, $-C(=O)R^{6c}$, - $C(=O)OR^{6c}$, $-C(=O)NR^{6b}R^{7b}$, $-NR^{6b}C(=O)R^{7b}$, $-NR^{6b}R^{7b}$, $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl or 3-6 membered heterocyclyl, wherein the $C_{1-6}$ alkyl, $C_{1-6}$ alkylthio, $C_{2-6}$ alkenyl, $C_{2-6}$ alkynyl, $C_{2-6}$ hydroxyalkynyl, $C_{1-6}$ alkoxy, $C_{1-6}$ cyanoalkyl, $C_{1-6}$ hydroxyalkyl, $C_{1-6}$ haloalkyl, $C_{2-6}$ haloalkenyl, $C_{2-6}$ haloalkynyl, $C_{1-6}$ haloalkoxy, $C_{1-6}$ haloalkylthio, 6-12 membered aryl, 5-12 membered heteroaryl, $C_{3-6}$ cycloalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, $C_{1-6}$ alkyl, $C_{1-6}$ alkoxy, $C_{3-6}$ cycloalkyl or 3-6 membered heterocyclyl;

$R^3$ is -H, -D, -OH, -SH, -F, -Cl, -Br, -I, -CN or $C_{1-4}$ alkyl;

Y is a bond, O or S;

$R^4$ is -H, -D, $C_{1-6}$ alkyl, 3-10 membered heterocyclyl, -L-(3-10 membered heterocyclyl), -L-$C_{3-10}$ cycloalkyl, -L-(5-12 membered heteroaryl), -L-($C_{6-10}$ aryl), $-L-NR^6R^7$, $-NR^6R^7$, $-L-NHC(=NH)NH_2$ or $-L-C(=O)NR^6R^7$, wherein the $C_{1-6}$ alkyl, 3-10 membered heterocyclyl, -L-(3-10 membered heterocyclyl), -L-$C_{3-10}$ cycloalkyl, -L-(5-12 membered heteroaryl) and -L-($C_{6-10}$ aryl) are independently and optionally substituted with 1, 2, 3 or 4 substituents selected from -D, -OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-6}$ alkyl, $C_{1-6}$ alkoxy, $C_{1-6}$ haloalkyl, $C_{1-6}$ haloalkoxy, $C_{6-10}$ aryl $C_{1-6}$ alkyl, (5-12 membered heteroaryl)-$C_{1-6}$ alkyl, (3-6 membered heterocyclyl)-$C_{1-6}$ alkyl, $C_{3-6}$ cycloalkyl $C_{1-6}$ alkyl, $C_{3-6}$ cycloalkyl and 3-6 membered heterocyclyl, among the substituents, the $C_{1-6}$ alkyl, $C_{1-6}$ alkoxy, $C_{1-6}$ haloalkyl, $C_{1-6}$ haloalkoxy, $C_{6-10}$ aryl $C_{1-6}$ alkyl, (5-12 membered heteroaryl)-$C_{1-6}$ alkyl, (3-6 membered heterocyclyl)-$C_{1-6}$ alkyl, $C_{3-6}$ cycloalkyl $C_{1-6}$ alkyl, $C_{3-6}$ cycloalkyl and 3-6 membered heterocyclyl are independently and optionally substituted with 1, 2, 3 or 4 substituents selected from -D, -F, -Cl, -Br, -I, -OH, -CN, $NR^{6e}R^{7e}$, -C(=O)$C_{1-6}$ alkyl and $C_{1-6}$ alkyl;

L is $C_{1-6}$ alkylene;

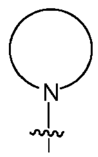

is a 5-10 membered heterocycle containing at least two ring N atoms;

$R^5$ is -H, -D, -OH, -SH, -F, -Cl, -Br, -I, -CN, -C(=O)H, $-C(=O)OR^{6a}$, $-C(=O)NR^6R^7$, $C_{1-6}$ alkyl, $C_{1-6}$ cyanoalkyl, $C_{1-6}$ hydroxyalkyl, $C_{1-6}$ haloalkyl, $C_{1-6}$ alkoxy, $C_{1-6}$ haloalkoxy or 5-6 membered heteroaryl;

each $R^{6a}$ and $R^{6c}$ is independently -H, -D or $C_{1-6}$ alkyl;

each $R^6$, $R^7$, $R^{6b}$, $R^{7b}$, $R^{6d}$, $R^{7d}$, $R^{6e}$, $R^{7e}$, $R^{6f}$ and $R^{7f}$ is independently -H, -D or $C_{1-6}$ alkyl, wherein the $C_{1-6}$ alkyl is optionally substituted with 1, 2, 3 or 4 substituents selected from -D, -OH, -F, -Cl, - Br, -I, -CN, -C(=O)H, -C(=O)OH, $-NR^{6g}R^{7g}$, $C_{1-6}$ alkoxy, 6-12 membered aryl, $C_{3-6}$ cycloalkyl and 3-6 membered heterocyclyl;

or $R^6$ and $R^7$, or $R^{6b}$ and $R^{7b}$, or $R^{6d}$ and $R^{7d}$, or $R^{6e}$ and $R^{7e}$, or $R^{6f}$ and $R^{7f}$, together with the N atoms to which they are attached, form 4-6 membered heterocycle, respectively, wherein the 4-6 membered heterocycle is optionally substituted with 1, 2, 3 or 4 substituents selected from -D, -OH, -F, -Cl, -Br, -I, -CN, $-NH_2$, $C_{1-6}$ alkyl, $C_{1-6}$ alkylamino, $C_{3-6}$ cycloalkyl, 3-6 membered heterocyclyl, $C_{1-6}$ alkoxy, $C_{1-6}$ cyanoalkyl, $C_{1-6}$ hydroxyalkyl, $C_{1-6}$ haloalkoxy or $C_{1-6}$ haloalkyl;

each $R^{6g}$ and $R^{7g}$ is independently -H, -D or $C_{1-6}$ alkyl.

**2.** The compound of claim 1, wherein, $R^1$ is -F, -Br, -I, -CN, -SH, $-C(=O)R^{6a}$, $-C(=O)OR^{6a}$, - $C(=O)NR^6R^7$, $-NR^6C(=O)R^7$, $-NR^6R^7$, $-NR^6S(=O)_2R^7$, $C_{2-4}$ alkenyl, $C_{2-4}$ alkynyl, $C_{1-4}$ cyanoalkyl, $C_{1-4}$ hydroxyalkyl, $C_{2-4}$ haloalkenyl, $C_{2-4}$ haloalkynyl, $C_{1-4}$ alkoxy $C_{1-4}$ alkyl, $C_{1-4}$ carboxyalkyl, $C_{1-4}$ aminoalkyl, $C_{1-4}$ mercaptoalkyl or 3-6 membered heterocyclyl, wherein the $C_{2-4}$ alkenyl, $C_{2-4}$ alkynyl, $C_{1-4}$ cyanoalkyl, $C_{1-4}$ hydroxyalkyl, $C_{1-4}$ alkoxy $C_{1-4}$ alkyl, $C_{1-4}$ carboxyalkyl, $C_{1-4}$ aminoalkyl, $C_{1-4}$ mercaptoalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, - $C(=O)OR^{6f}$, $-C(=O)NR^6R^7$, $-NR^6C(=O)R^7$, -$C_{1-4}$ alkyl $NR^6C(=O)R^7$, $-NR^{6f}C(=O)NR^6R^7$, $-C(=O)R^{6a}$, $-NR^6S(=O)_2R^7$, $-S(=O)_2NR^6R^7$, $-NR^{6f}S(=O)_2NR^6R^7$, $-NR^{6f}R^{7f}$, -$C_{1-4}$ alkyl $NR^{6f}R^{7f}$, $C_{1-4}$ alkyl, $C_{2-4}$ alkenyl, $C_{2-4}$ alkynyl, $C_{1-4}$ alkoxy,

(6-10 membered aryl)-$C_{1-4}$ alkoxy, $C_{1-4}$ cyanoalkyl, $C_{1-4}$ hydroxyalkyl, $C_{1-4}$ haloalkyl, $C_{1-4}$ alkoxy $C_{1-4}$ alkyl, $C_{1-4}$ carboxyalkyl, $C_{1-4}$ aminoalkyl, $C_{1-4}$ mercaptoalkyl, 6-10 membered aryl, 5-10 membered heteroaryl, $C_{3-6}$ cycloalkyl and 3-6 membered heterocyclyl;

each $R^{6a}$ is independently -H, -D, or $C_{1-4}$ alkyl;
each $R^6$, $R^7$, $R^{6f}$ and $R^{7f}$ is independently -H, -D or $C_{1-4}$ alkyl, wherein the $C_{1-4}$ alkyl is optionally substituted with 1, 2, 3 or 4 substituents selected from -D, -OH, -F, -Cl, -Br, -I, -CN, -C(=O)H, - C(=O)OH, $-NR^{6g}R^{7g}$, $C_{1-4}$ alkoxy, 6-10 membered aryl, $C_{3-6}$ cycloalkyl and 3-6 membered heterocyclyl;
or $R^6$ and $R^7$, or $R^{6f}$ and $R^{7f}$, together with the N atom to which they are attached respectively, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl;
each $R^{6g}$ and $R^{7g}$ is independently -H, -D or $C_{1-4}$ alkyl.

**3.** The compound of claim 1 or 2, wherein, $R^1$ is -F, -Br, -I, -CN, -SH, $-C(=O)R^{6a}$, $-C(=O)OR^{6a}$, $-C(=O)NR^6R^7$, $-NR^6C(=O)R^7$, $-NR^6R^7$, $-NR^6S(=O)_2R^7$, $-CH=CH_2$, $-CH=CHCH_3$, $-CH_2CH=CH_2$, - $CHFCH=CH_2$, -CH=CHF, -CH=CHCl, $-CH=CHCH_2F$, $-CH=CHCH_3$, -C=CH, $-C=CCH_3$, - $CH_2C=CH$, $-C=CCH_2F$, $-CH_2CN$, $-(CH_2)_2CN$, $-(CH_2)_3CN$, $-CH_2OH$, $-(CH_2)_2OH$, $-(CH_2)_3OH$, - $CH(OH)CH_3$, $-CH_2OCH_3$, $-(CH_2)_2OCH_3$, $-(CH_2)_2OCH_2CH_3$, $-CH_2OCH_2CH_3$, $-CH_2C(=O)OH$, - $(CH_2)_2C(=O)OH$, $-(CH_2)_3C(=O)OH$, $-CH_2NH_2$, $-(CH_2)_2NH_2$, $-CH_2SH$, $-(CH_2)_2SH$, oxiranyl, oxetanyl or pyrrolidinyl; wherein the $-CH=CH_2$, $-CH=CHCH_3$, $-CH_2CH=CH_2$, $-CHFCH=CH_2$, $-CH=CHCH_3$, -C=CH, $-C=CCH_3$, $-CH_2C\equiv CH$, $-CH_2CN$, $-(CH_2)_2CN$, $-(CH_2)_3CN$, -CHzOH, -(CHz)zOH, $-(CH_2)_3OH$, $-CH(OH)CH_3$, $-CH_2OCH_3$, $-(CH_2)_2OCH_3$, $-(CH_2)_2OCH_2CH_3$, $-CH_2OCH_2CH_3$, $-CH_2C(=O)OH$, - $(CH_2)_2C(=O)OH$, $-(CH_2)_3C(=O)OH$, $-CH_2NH_2$, $-(CH_2)_2NH_2$, $-CH_2SH$, $-(CH_2)SH$, oxiranyl, oxetanyl and pyrrolidinyl are independently and optionally substituted with 1, 2, 3 or 4 substituents selected from -D, -OH, -F, -Cl, -Br, -I, -CN, $-NH_2$, -C(=O)H, -C(=O)OH, $-C(=O)OR^{6f}$, $-C(=O)NR^6R^7$, - $NR^6C(=O)R^7$, $-CH_2NR^6C(=O)R^7$, $-NR^{6f}C(=O)NR^6R^7$, $-C(=O)R^{6a}$, $-NR^6S(=O)_2R^7$, $-S(=O)_2NR^6R^7$, - $NR^{6f}S(=O)_2NR^6R^7$, $-NR^{6f}R^{7f}$, $-CH_2NR^{6f}R^{7f}$, $-CH=CH_2$, $-CH=CHCH_3$, $-CH_2CH=CH_2$, -C=CH, - $C\equiv CCH_3$, $-CH_2C\equiv CH$, methyl, ethyl, n-propyl, isopropyl, methoxy, ethoxy, n-propoxy, isopropoxy, isobutoxy, phenylmethoxy, phenylethoxy, phenylpropoxy, naphthylmethoxy, $-CH_2CN$, $-(CH_2)_2CN$, - $(CH_2)_3CN$, $-CH_2OH$, $-(CH_2)_2OH$, $-(CH_2)_3OH$, $-CH(OH)CH_3$, $-CF_3$, $-CHF_2$, $-CH_2F$, $-(CH_2)_2F$, - $(CH_2)_2Cl$, $-CH_2CF_3$, $-CH_2OCH_3$, $-(CH_2)_2OCH_3$, $-(CH_2)_2OCH_2CH_3$, $-CH_2OCH_2CH_3$, $-CH_2C(=O)OH$, $-(CH_2)_2C(=O)OH$, $-(CH_2)_3C(=O)OH$, $-CH_2NH_2$, $-(CH_2)_2NH_2$, $-CH_2SH$, $-(CH_2)_2SH$, phenyl, naphthyl, pyridyl, pyrimidinyl, cyclopropyl, cyclobutyl, cyclopentyl, cyclohexyl, oxiranyl, oxetanyl, tetrahydropyranyl, azetidinyl and pyrrolidinyl;

each $R^{6a}$ is independently -H, -D, methyl, ethyl, *n*-propyl, isopropyl, *n*-butyl, isobutyl or *tert*-butyl;
each $R^6$, $R^7$, $R^{6f}$ and $R^{7f}$ is independently -H, -D, methyl, ethyl, *n*-propyl, isopropyl, *n*-butyl, isobutyl or *tert*-butyl, wherein the methyl, ethyl, *n*-propyl, isopropyl, *n*-butyl, isobutyl and *tert*-butyl are independently and optionally substituted with 1, 2, 3 or 4 substituents selected from -D, -OH, -F, -Cl, - Br, -I, -CN, -C(=O)H, -C(=O)OH, $-NR^{6g}R^{7g}$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl;
or $R^6$ and $R^7$, or $R^{6f}$ and $R^{7f}$, together with the N atom to which they are attached respectively, form pyrrolidine, piperazine, piperidine, morpholinyl, oxazolidine or imidazolidine, wherein the pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, - $NH_2$, methyl, ethyl, *n*-propyl, isopropyl, *n*-butyl, isobutyl, *tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl;
each $R^{6g}$ and $R^{7g}$ is independently -H, -D, methyl, ethyl, *n*-propyl, isopropyl, *n*-butyl, isobutyl or *tert*-butyl.

**4.** The compound of any one of claims 1 to 3, wherein, $R^2$ is one of the following substructures:

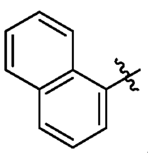, 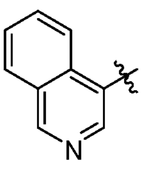, 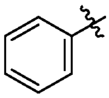, 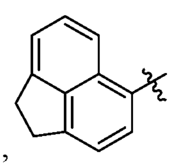, 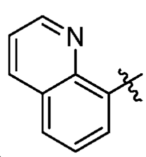, 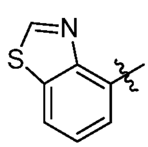, 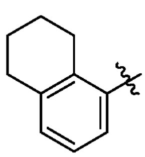, 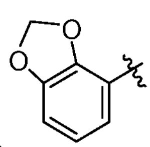,

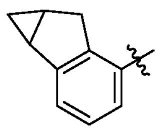, 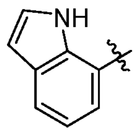, 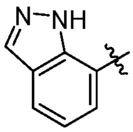, 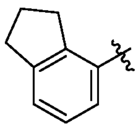, 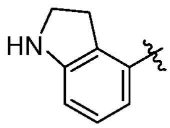, 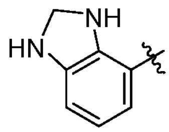, 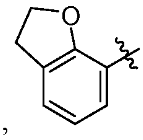, 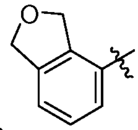,

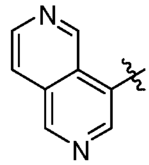, 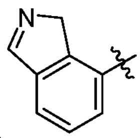, 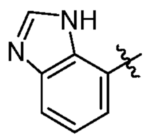, 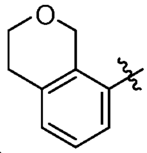, 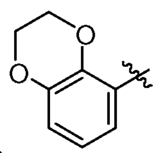 or 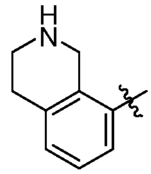,

wherein, each substructure of $R^2$ is independently and optionally substituted with 1, 2, 3, 4, 5, 6 or 7 $R^8$.

**5.** The compound of any one of claims 1 to 4, wherein,

each $R^8$ is independently -D, -OH, -F, -Cl, -Br, -I, -CN, -SH, $-CH^2C(=O)NR^{6b}R^{7b}$, $-C(=O)R^{6c}$, - $C(=O)OR^{6c}$, $-C(=O)NR^{6b}R^{7b}$, $-NR^{6b}C(=O)R^{7b}$, $-NR^{6b}R^{7b}$, $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl or 3-6 membered heterocyclyl, wherein the $C_{1-4}$ alkyl, $C_{1-4}$ alkylthio, $C_{2-4}$ alkenyl, $C_{2-4}$ alkynyl, $C_{2-4}$ hydroxyalkynyl, $C_{1-4}$ alkoxy, $C_{1-4}$ cyanoalkyl, $C_{1-4}$ hydroxyalkyl, $C_{1-4}$ haloalkyl, $C_{2-4}$ haloalkenyl, $C_{2-4}$ haloalkynyl, $C_{1-4}$ haloalkoxy, $C_{1-4}$ haloalkylthio, 6-10 membered aryl, 5-10 membered heteroaryl, $C_{3-6}$ cycloalkyl and 3-6 membered heterocyclyl are independently and optionally substituted with 1, 2, 3 or 4 substituents selected from -D, -OH, -F, -Cl, -Br, -I, -CN, $-NH_2$, -C(=O)H, -C(=O)OH, $C_{1-4}$ alkyl, $C_{1-4}$ alkoxy, $C_{3-6}$ cycloalkyl or 3-6 membered heterocyclyl;
each $R^{6c}$ is independently -H, -D or $C_{1-4}$ alkyl;
each $R^{6b}$ and $R^{7b}$ is independently -H, -D or $C_{1-4}$ alkyl, wherein the $C_{1-4}$ alkyl is independently substituted with 1, 2, 3 or 4 substituents selected from -D, -OH, -F, -Cl, -Br, -I, -CN, -C(=O)H, - C(=O)OH, $-NR^{6g}R^{7g}$, $C_{1-4}$ alkoxy, 6-10 membered aryl, $C_{3-6}$ cycloalkyl and 3-6 membered heterocyclyl;
or, $R^{6b}$ and $R^{7b}$, together with the N atom to which they are attached, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl.

**6.** The compound of any one of claims 1 to 5, wherein,

each $R^8$ is independently -D, -OH, -F, -Cl, -Br, -I, -CN, -SH, $-CH^2C(=O)NR^{6b}R^{7b}$, $-C(=O)R^{6c}$, - $C(=O)OR^{6c}$, $-C(=O)NR^{6b}R^{7b}$, $-NR^{6b}C(=O)R^{7b}$, $-NR^{6b}R^{7b}$, $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, $-CH=CHCH_3$, $-CH_2CH=CH_2$, -C=CH, - $C\equiv CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, $-CH(OH)CH_3$, $-(CH_2)_2F$, $-CH_2CHF_2$, - $CF_3$, $-CH_2CF_3$, $-CHF_2$, $-CH_2F$, $-(CH_2)_2Cl$, -CH=CHF, -CH=CH-Cl, $-CH=CHCH_2F$, $-C=CCH_2F$, - $C\equiv C(CH_2)_2F$, -C=CF, $-OCF_3$, $-OCHF_2$, $-OCH_2CHF_2$, $-OCH_2CF_3$, $-OCHClCHCl_2$, $-OCH_2CH_2F$, $-SCF_3$, $-SCH_2CF_3$, $-SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuranyl, piperidyl or piperazinyl, wherein the $-CH_3$, $-CH_2CH_3$, $-(CH_2)_2CH_3$, $-(CH_2)_3CH_3$, $-C(CH_3)_3$, $-CH(CH_3)_2$, $-SCH_3$, $-SCH_2CH_3$, $-CH=CH_2$, - $CH=CHCH_3$, $-CH_2CH=CH_2$, -C=CH, $-C=CCH_3$, $-CH_2C\equiv CH$, $-C\equiv CCH_2OH$, $-C\equiv C(CH_2)_2OH$, $-OCH_3$, $-OCH_2CH_3$, $-O(CH_2)_2CH_3$, $-CH_2CN$, $-(CH_2)_2CN$, $-(CH_2)_3CN$, $-CH_2OH$, $-(CH_2)_2OH$, $-(CH_2)_3OH$, - $CH(OH)CH_3$, $-(CH_2)_2F$, $-CH_2CHF_2$, $-CH_2CF_3$, $-CHF_2$, $-CH_2F$, $-(CH_2)_2Cl$, -CH=CHF, -CH=CHCl, - $CH=CHCH_2F$, $-C=CCH_2F$, $-C\equiv C(CH_2)_2F$, $-OCHF_2$, $-OCH_2CHF_2$, $-OCH_2CF_3$, $-OCHClCHCl_2$, - $OCH_2CH_2F$, $-SCH_2CF_3$, $-SCH_2CHF_2$, phenyl, naphthyl, pyridyl, pyrimidinyl, cyclopropyl, cyclobutyl, cyclopentyl, cyclohexyl, pyrrolidinyl, oxazolidinyl, tetrahydrofuranyl, piperidyl and piperazinyl are independently and optionally substituted with 1, 2, 3 or 4 substituents selected from -D, -OH, -F, -Cl, -Br, -I, -CN, $-NH_2$, -C(=O)H, -C(=O)OH, methyl, ethyl, n-propyl, isopropyl, methoxy, ethoxy, n-propoxy, isopropoxy, cyclopropyl, cyclobutyl, cyclopentyl, cyclohexyl, pyrrolidinyl, oxazolidinyl, tetrahydrofuranyl, piperidinyl and piperazinyl;
each $R^{6c}$ is independently -H, -D, methyl, ethyl, n-propyl, isopropyl, n-butyl, isobutyl or *tert*-butyl;
each $R^{6b}$ and $R^{7b}$ is independently -H, -D, methyl, ethyl, n-propyl, isopropyl, n-butyl, isobutyl or tert-butyl, wherein the methyl, ethyl, n-propyl, isopropyl, n-butyl, isobutyl and tert-butyl are independently and optionally substituted

with 1, 2, 3 or 4 substituents selected from -D, -OH, -F, -Cl, -Br, -I, -CN, -C(=O)H, -C(=O)OH, $-NR^{6g}R^{7g}$, methoxy, ethoxy, n-propoxy, isopropoxy, isobutoxy, phenyl, cyclopropyl, cyclobutyl, cyclopentyl, cyclohexyl, oxiranyl, oxetanyl, azetidinyl and pyrrolidinyl;

or, $R^{6b}$ and $R^{7b}$, together with the N atom to which they are attached respectively, form pyrrolidine, piperazine, piperidine, morpholinyl, oxazolidine or imidazolidine, wherein the pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, - $NH_2$, methyl, ethyl, n-propyl, isopropyl, n-butyl, isobutyl, tert-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

**7.** The compound of any one of claims 1 to 6, wherein,

$R^4$ is -H, -D, $C_{1-4}$ alkyl, 3-6 membered heterocyclyl, 7-10 membered heterocyclyl,

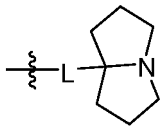,

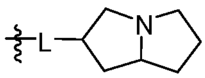, 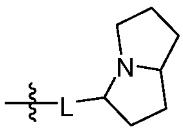, 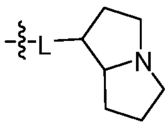, 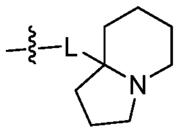,

-L-pyrrolidinyl, -L-morpholinyl, -L-oxetanyl, -L-oxypropyl, -L-tetrahydrofuranyl, -L-octahydroindolizinyl, -L-cyclopropyl, -L-cyclopentyl, -L-octahydropentadienyl, -L-octahydro-1*H*-indenyl, -L-decalinyl, -L-pyridyl, -L-pyrazolyl, -L-phenyl, $-L-NR^6R^7$, $-NR^6R^7$, $-L-NHC(=NH)NH_2$ or $-L-C(=O)NR^6R^7$, wherein the $C_{1-4}$ alkyl, 3-6 membered heterocyclyl, 7-10 membered heterocyclyl,

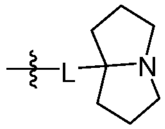, 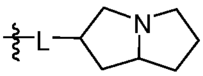,

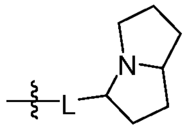, 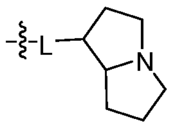, 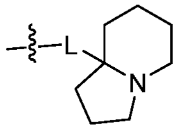,

-L-pyrrolidinyl, -L-morpholinyl, -L-oxetanyl, -L-oxypropyl, -L-tetrahydrofuranyl, -L-octahydroindolizinyl, -L-cyclopropyl, -L-cyclopentyl, -L-octahydropentadienyl, -L-octahydro-1*H*-indenyl, -L-decalinyl, -L-pyridyl, -L-pyrazolyl and -L-phenyl are independently and optionally substituted with 1, 2, 3 or 4 substituents selected from -D, - OH, -F, -Cl, -Br, -I, -CN, $-NR^{6d}R^{7d}$, $-C(=O)NR^{6d}R^{7d}$, $-CH_2NR^{6d}R^{7d}$, $-CH_2OC(=O)NR^{6d}R^{7d}$, $C_{1-4}$ alkyl, $C_{1-4}$ alkoxy, $C_{1-4}$ haloalkyl, $C_{1-4}$ haloalkoxy, phenyl $C_{1-4}$ alkyl, (5-6 membered heteroaryl)-$C_{1-4}$ alkyl, (3-6 membered heterocyclyl)-$C_{1-4}$ alkyl, $C_{3-6}$ cycloalkyl $C_{1-4}$ alkyl, $C_{3-6}$ cycloalkyl and 3-6 membered heterocyclyl, among the substituents, the $C_{1-4}$ alkyl, $C_{1-4}$ alkoxy, $C_{1-4}$ haloalkyl, $C_{1-4}$ haloalkoxy, phenyl $C_{1-4}$ alkyl, (5-6 membered heteroaryl)-$C_{1-4}$ alkyl, (3-6 membered heterocyclyl)-$C_{1-4}$ alkyl, $C_{3-6}$ cycloalkyl $C_{1-4}$ alkyl, $C_{3-6}$ cycloalkyl and 3-6 membered heterocyclyl are independently and optionally substituted with 1, 2, 3 or 4 substituents selected from -D, -F, -Cl, -Br, -I, -OH, -CN, $NR^{6e}R^{7e}$, $-C(=O)C_{1-4}$ alkyl and $C_{1-4}$ alkyl;

L is $C_{1-4}$ alkylene;

each $R^{6d}$, $R^{7d}$, $R^{6e}$ and $R^{7e}$ is independently -H, -D or $C_{1-4}$ alkyl, wherein the $C_{1-4}$ alkyl is optionally substituted with 1, 2, 3 or 4 substituents selected from -D, -OH, -F, -Cl, -Br, -I, -CN, - C(=O)H, -C(=O)OH, $-NR^{6g}R^{7g}$, $C_{1-4}$ alkoxy, 6-10 membered aryl, $C_{3-6}$ cycloalkyl and 3-6 membered heterocyclyl;

or $R^{6d}$ and $R^{7d}$, or $R^{6e}$ and $R^{7e}$, together with the N atom to which they are attached respectively, form 4-6 membered heterocycle, wherein the 4-6 membered heterocycle is optionally substituted with 1, 2, 3 or 4 substituents selected from -D, -OH, -F, -Cl, -Br, -I, -CN, $-NH_2$, $C_{1-4}$ alkyl, $C_{1-4}$ alkylamino, $C_{3-6}$ cycloalkyl, 3-6 membered heterocyclyl, $C_{1-4}$ alkoxy, $C_{1-4}$ cyanoalkyl, $C_{1-4}$ hydroxyalkyl, $C_{1-4}$ haloalkoxy or $C_{1-4}$ haloalkyl;

**8.** The compound of any one of claims 1 to 7, wherein,

$R^4$ is -H, -D, $-CH_3$, $-CH_2CH_3$, $-(CH_2)_2CH_3$, piperidinyl, piperazinyl, pyrrolidinyl, imidazolidinyl,

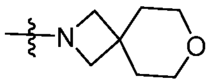, 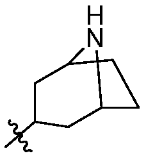, 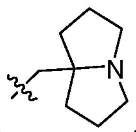, 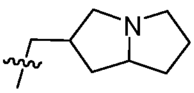, 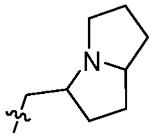, 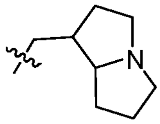, 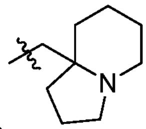,

$-CH_2$-pyrrolidinyl, $-CH_2$-morpholinyl, $-(CH_2)_2$-morpholinyl, $-CH_2$-oxetanyl, $-CH_2$-oxypropyl, $-CH_2$-tetrahydrofuranyl, $-CH_2$-octahydroindolizinyl, $-CH_2$-cyclopropyl, $-CH_2$-cyclopentyl, $-CH_2$-octahydropentadienyl, $-CH_2$-octahydro-1*H*-indenyl, $-CH_2$-decalinyl, $-CH_2$-pyridyl, $-(CH_2)_2$-pyridyl, $-CH_2$-pyrazolyl, $-(CH_2)_2$-pyrazolyl, $-CH_2$-phenyl, $-CH_2-NR^6R^7$, $-(CH_2)_2-NR^6R^7$, - $CH(CH_3)CH_2NR^6R^7$, $-NR^6R^7$, $-CH_2$-NHC(=NH)$NH_2$ or $-CH_2-C(=O)NR^6R^7$, wherein the $-CH_3$, - $CH_2CH_3$, $-(CH_2)_2CH_3$, piperidinyl, piperazinyl, pyrrolidinyl, imidazolidinyl,

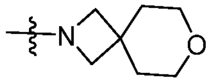,

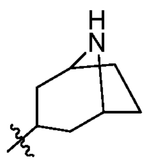, 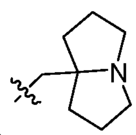, 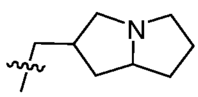, 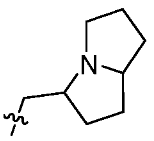, 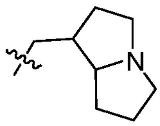, 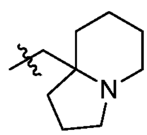,

$-CH_2$-pyrrolidinyl, - $CH_2$-morpholinyl, $-(CH_2)_2$-morpholinyl, $-CH_2$-oxetanyl, $-CH_2$-oxypropyl, $-CH_2$-tetrahydrofuranyl, - $CH_2$-octahydroindolizinyl, $-CH_2$-cyclopropyl, $-CH_2$-cyclopentyl, $-CH_2$-octahydropentadienyl, $-CH_2$-octahydro-1*H*-indenyl, $-CH_2$-decalinyl, $-CH_2$-pyridyl, $-(CH_2)_2$-pyridyl, $-CH_2$-pyrazolyl, $-(CH_2)_2$-pyrazolyl and $-CH_2$-phenyl are independently and optionally substituted with 1, 2, 3 or 4 substituents selected from -D, -OH, -F, -Cl, -Br, -I, -CN, $-NR^{6d}R^{7d}$, $-C(=O)NR^{6d}R^{7d}$, $-CH_2NR^{6d}R^{7d}$, - $CH_2OC(=O)NR^{6d}R^{7d}$, methyl, ethyl, n-propyl, isopropyl, n-butyl, isobutyl, tert-butyl, methoxy, ethoxy, isopropoxy, $-CHF_2$, $-CF_3$, $-OCF_3$, phenylmethyl, pyridylmethyl, pyrazolylmethyl, morpholinylmethyl, pyrrolidinylmethyl, piperazinylmethyl, azetidinylmethyl, piperidylmethyl, cyclopropylmethyl, cyclopentylmethyl, cyclohexylmethyl, cyclopentyl, cyclohexyl, morpholinyl, piperidinyl, pyrrolidinyl, piperazinyl or azetidinyl, among the substituents, the methyl, ethyl, *n*-propyl, isopropyl, *n*-butyl, isobutyl, *tert*-butyl, methoxy, ethoxy, isopropoxy, $-CHF_2$, phenylmethyl, pyridylmethyl, pyrazolylmethyl, morpholinylmethyl, pyrrolidinylmethyl, piperazinylmethyl, azetidinylmethyl, piperidinylmethyl, cyclopropylmethyl, cyclopentylmethyl, cyclohexylmethyl, cyclopentyl, cyclohexyl, morpholinyl, piperidinyl, pyrrolidinyl, piperazinyl and azetidinyl are independently and optionally substituted with 1, 2, 3 or 4 substituents selected from -D, -F, -Cl, -Br, -I, -OH, -CN, $-NH_2$, $-NHCH_3$, $-N(CH_3)_2$, $-NHCH_2CH_3$, $-C(=O)CH_3$, $-C(=O)CH_2CH_3$, methyl, ethyl, n-propyl and isopropyl;

each $R^{6d}$ and $R^{7d}$ is independently -H, -D, methyl, ethyl, n-propyl, isopropyl, n-butyl, isobutyl or tert-butyl, wherein the methyl, ethyl, n-propyl, isopropyl, n-butyl, isobutyl and tert-butyl are optionally substituted with 1, 2, 3 or 4 substituents selected from -D, -OH, -F, -Cl, -Br, -I, -CN, - C(=O)H, -C(=O)OH, $-NR^{6g}R^{7g}$, methoxy, ethoxy, n-propoxy, isopropoxy, isobutoxy, phenyl, cyclopropyl, cyclobutyl, cyclopentyl, cyclohexyl, oxiranyl, oxetanyl, azetidinyl and pyrrolidinyl;

or $R^{6d}$ and $R^{7d}$, together with the N atom to which they are attached, form azetidine, pyrrolidine, piperazine, piperidine, morpholinyl, oxazolidine or imidazolidine, wherein the azetidine, pyrrolidine, piperazine, piperidine, morpholinyl, oxazolidine and imidazolidine are independently and optionally substituted with 1, 2, 3 or 4 substituents selected from -D, -OH, -F, -Cl, -Br, -I, -CN, $-NH_2$, methyl, ethyl, *n*-propyl, isopropyl, *n*-butyl, isobutyl,

*tert*-butyl, methylamino, dimethylamino, ethylamino, cyclopropyl, cyclopentyl, pyrrolidinyl, methoxy, ethoxy, isopropoxy, cyanomethyl, hydroxymethyl, hydroxyethyl, trifluoromethoxy, monofluoromethyl, difluoromethyl, trifluoromethyl or 1,2-dichloroethyl.

**9.** The compound of any one of claims 1 to 8, wherein, substructures:

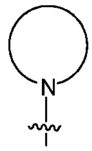

is one of the following

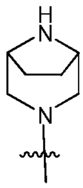 , 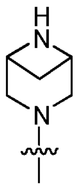 , 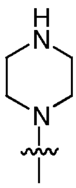 , 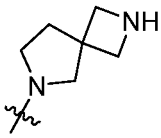 , 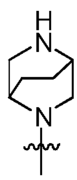 or 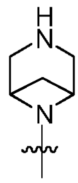 ;

$R^5$ is -H, -D, -OH, -SH, -F, -Cl, -Br, -I, -CN, -C(=O)H, $-C(=O)OCH_3$, $-C(=O)NH_2$, $-CH_3$, - $CH_2CH_3$, $C_{3-4}$ alkyl, -CHzOH, -(CHz)zOH, $-CH_2CN$, $-(CH_2)_2CN$, $-CF_3$, $-CHF_2$, $-CH_2F$, $-OCF_3$, $-OCH_3$ or $-OCH_2CH_3$.

**10.** The compound of any one of claims 1 to 9, wherein, $R^3$ is -H, -D, -OH, -SH, -F, -Cl, -Br, -I, -CN, methyl, ethyl, *n*-propyl or isopropyl.

**11.** The compound of any one of claims 1 to 10 having Formula (I-1), or a stereoisomer, a tautomer, an *N*-oxide, a solvate, a metabolite, a pharmaceutically acceptable salt or a prodrug thereof,

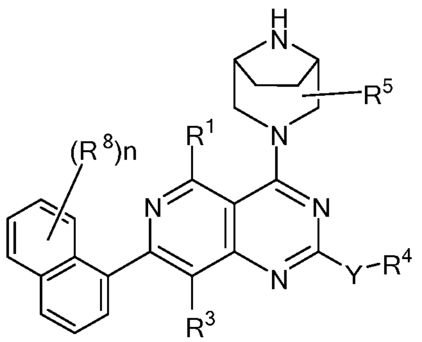

(I-1),

wherein, n is 1, 2, 3, 4, 5, 6 or 7;
wherein, each $R^1$, $R^3$, Y, $R^4$, $R^5$ and $R^8$ has definition as described in any one of claims 1 to 10.

**12.** The compound of any one of claims 1 and 4-11 having Formula (I-2), or a stereoisomer, a tautomer, an *N*-oxide, a solvate, a metabolite, a pharmaceutically acceptable salt or a prodrug thereof,

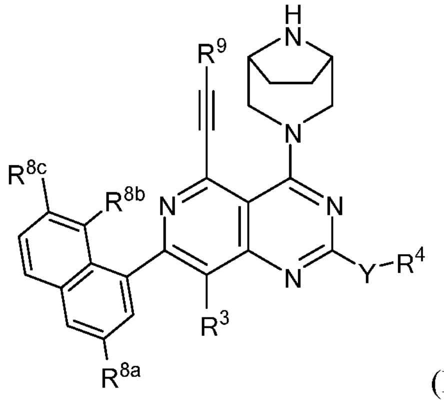

(I-2),

wherein, $R^9$ is -H, -D, -CN, $-NH_2$, -C(=O)H, -C(=O)OH, $-C(=O)OR^{6f}$, $-C(=O)NR^6R^7$, - $NR^6C(=O)R^7$, $-NR^{6f}C(=O)NR^6R^7$, $-C(=O)R^{6a}$, $-C(=O)OR^{6a}$, $-NR^6S(=O)_2R^7$, $-S(=O)_2NR^6R^7$, - $NR^{6f}S(=O)_2NR^6R^7$, $-NR^{6f}R^{7f}$, $-C_{1-6}$ alkyl $NR^6C(=O)R^7$, $-C_{1-6}$ alkyl $NR^{6f}R^{7f}$, $C_{1-6}$ alkyl, $C_{1-6}$ cyanoalkyl, $C_{1-6}$ hydroxyalkyl, $C_{1-6}$ haloalkyl, $C_{1-6}$ alkoxy $C_{1-6}$ alkyl, $C_{1-6}$ carboxyalkyl, $C_{1-6}$ aminoalkyl, $C_{1-6}$ mercaptoalkyl, 6-12 membered aryl, 5-12 membered heteroaryl, $C_{3-6}$ cycloalkyl and 3-6 membered heterocyclyl;
each $R^{6a}$ is independently -H, -D, or $C_{1-6}$ alkyl;
each $R^6$, $R^7$, $R^{6f}$ and $R^{7f}$ is independently -H, -D or $C_{1-6}$ alkyl, wherein the $C_{1-6}$ alkyl is optionally substituted with 1, 2, 3 or 4 substituents selected from -D, -OH, -F, -Cl, -Br, -I, -CN, -C(=O)H, - C(=O)OH, $-NR^{6g}R^{7g}$, $C_{1-6}$ alkoxy, 6-12 membered aryl, $C_{3-6}$ cycloalkyl and 3-6 membered heterocyclyl;
each $R^3$, Y, $R^4$, $R^5$, $R^{6g}$ and $R^{7g}$ has definition as described in any one of claims 1 and 4-11.
each $R^{8a}$, $R^{8b}$ and $R^{8c}$ has the same definition as $R^8$ described in any one of claims 1, 5 and 6.

**13.** The compound of claim 12, wherein, $R^9$ is -H, -D, -CN, $-NH_2$, -C(=O)H, -C(=O)OH, - $C(=O)OR^{6f}$, $-C(=O)NR^6R^7$, $-NR^6C(=O)R^7$, $-NR^{6f}C(=O)NR^6R^7$, $-C(=O)R^{6a}$, $-C(=O)OR^{6a}$, - $NR^6S(=O)_2R^7$, $-S(=O)_2NR^6R^7$, $-NR^{6f}S(=O)_2NR^6R^7$, $-NR^{6f}R^{7f}$, $-CH_2NR^6C(=O)R^7$, $-CH_2NR^{6f}R^{7f}$, $-CH_3$, $-CH_2CH_3$, $-(CH_2)_2CH_3$, $-(CH_2)_3CH_3$, $-C(CH_3)_3$, $-CH_2CH(CH_3)_2$, $-(CH_2)_2CH(CH_3)_2$, $-CH(CH_3)_2$, - $CH_2CN$, $-(CH_2)_2CN$, $-(CH_2)_3CN$, $-CH(CH_3)CN$, $-C(CH_3)_2CN$, $-CH_2OH$, $-(CH_2)_2OH$, $-(CH_2)_3OH$, - $CH(OH)CH_3$, $-CF_3$, $-CHF_2$, $-CH_2F$, $-(CH_2)_2F$, -(CHz)zCl, $-CH_2CF_3$, $-CH_2OCH_3$, $-(CH_2)_2OCH_3$, - $(CH_2)_2OCH_2CH_3$, $-CH_2OCH_2CH_3$, $-CH_2C(=O)OH$, $-(CH_2)_2C(=O)OH$, $-(CH_2)_3C(=O)OH$, $-CH_2NH_2$, $-(CH_2)_2NH_2$, $-CH_2SH$, $-(CH_2)_2SH$, phenyl, naphthyl, pyridyl, pyrimidinyl, cyclopropyl, cyclobutyl, cyclopentyl, cyclohexyl, oxiranyl, oxetanyl, tetrahydropyranyl, azetidinyl and pyrrolidinyl;

each $R^{6a}$ is independently -H, -D, methyl, ethyl, n-propyl, isopropyl, n-butyl, isobutyl or *tert*-butyl;
each $R^6$, $R^7$, $R^{6f}$ and $R^{7f}$ is independently -H, -D, methyl, ethyl, n-propyl, isopropyl, n-butyl, isobutyl or *tert-butyl,* wherein the methyl, ethyl, n-propyl, isopropyl, n-butyl, isobutyl and tert-butyl are independently and optionally substituted with 1, 2, 3 or 4 substituents selected from -D, -OH, -F, -Cl, -Br, -I, -CN, -C(=O)H, -C(=O)OH, $-NR^{6g}R^{7g}$, methoxy, ethoxy, n-propoxy, isopropoxy, isobutoxy, cyclopropyl, cyclobutyl, cyclopentyl, cyclohexyl, phenyl, oxiranyl, oxetanyl, azetidinyl and pyrrolidinyl.

**14.** A compound having the following structures, or a stereoisomer, a tautomer, an N-oxide, a solvate, a metabolite, a pharmaceutically acceptable salt or a prodrug thereof,

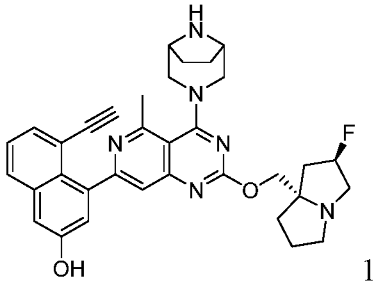

1,

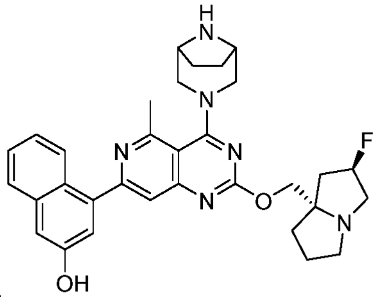

2,

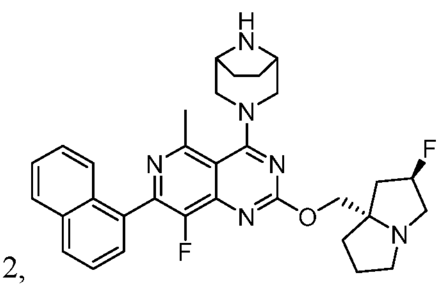

3,

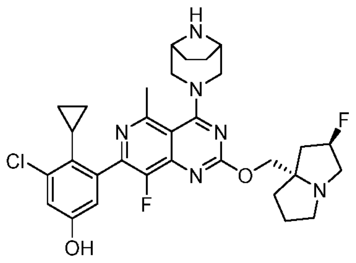 4, 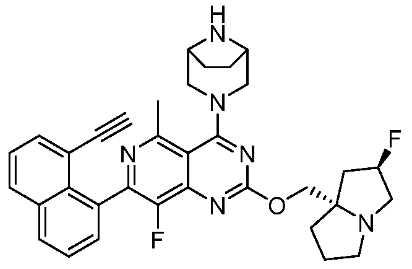 5, 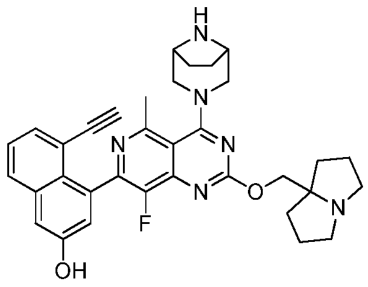 6,

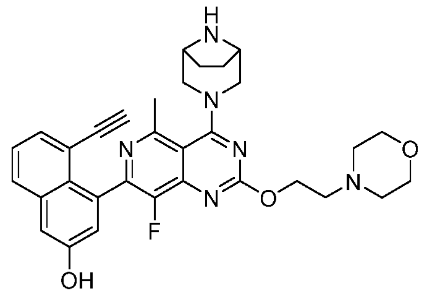 7, 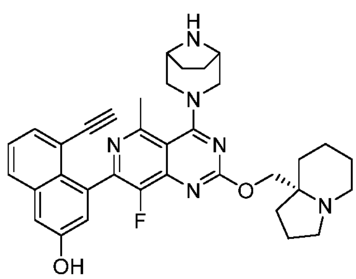 8, 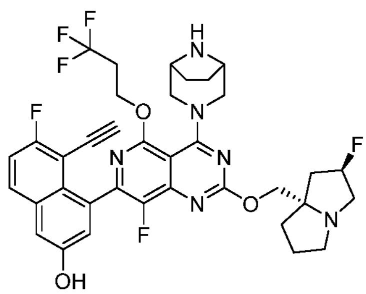 9,

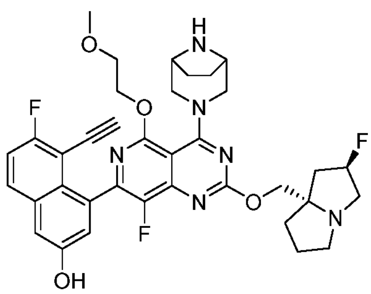 10, 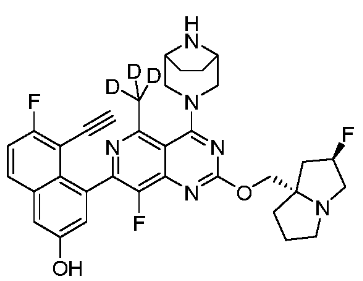 11, 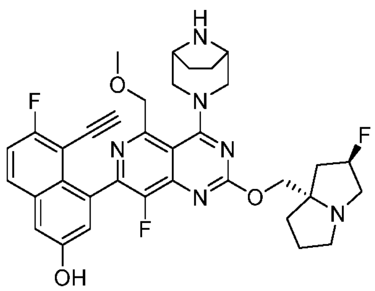 12,

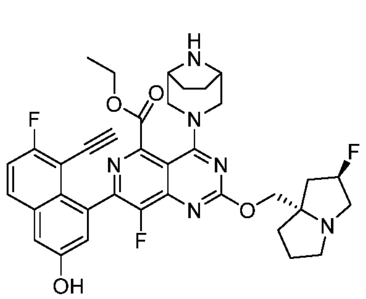 13, 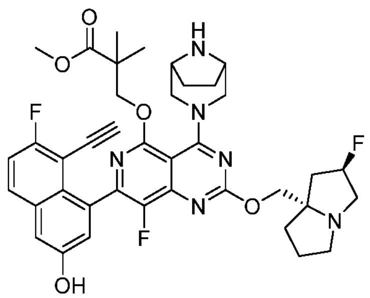 14, 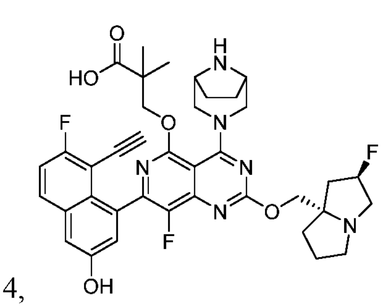 15,

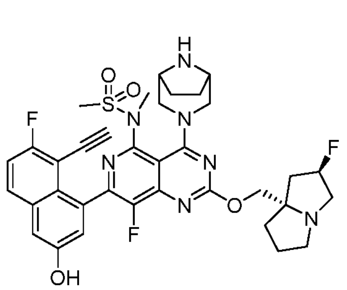 16, 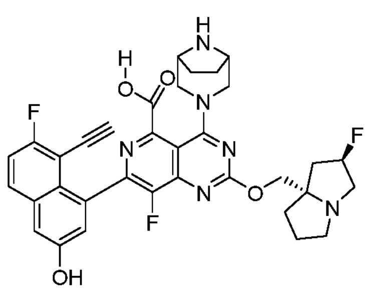 17, 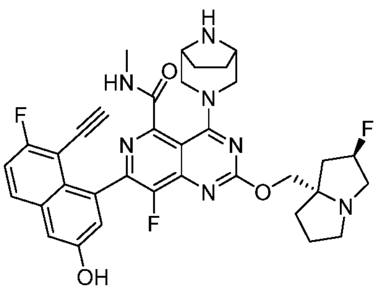 18,

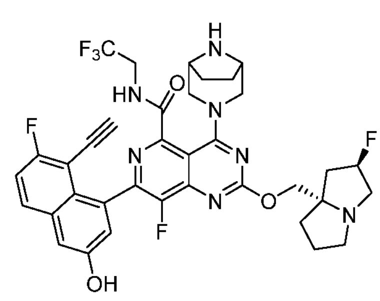 19, 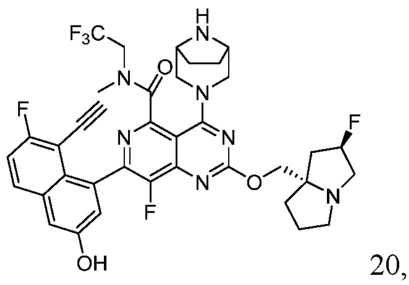 20, 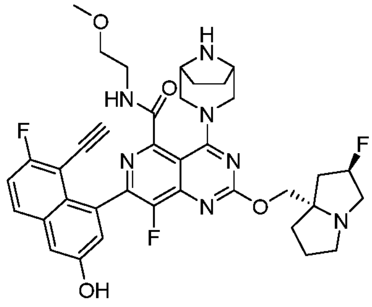 21,

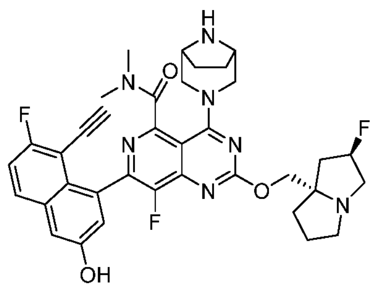 22, 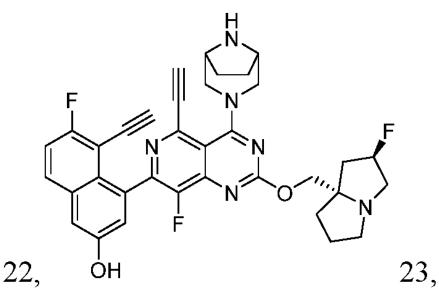 23, 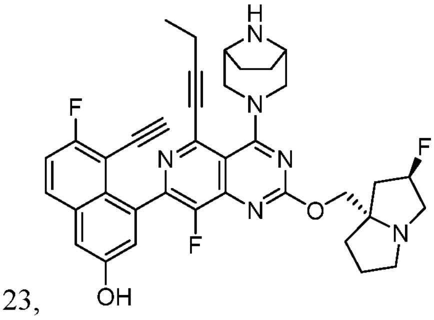 24,

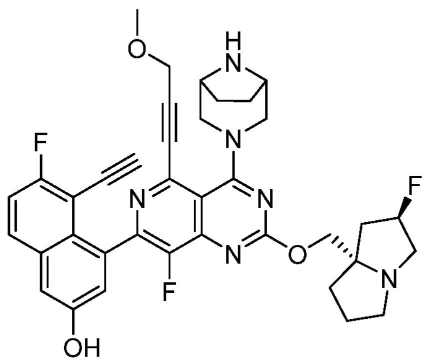 25, 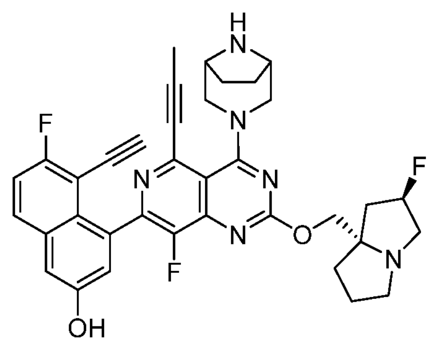 26,

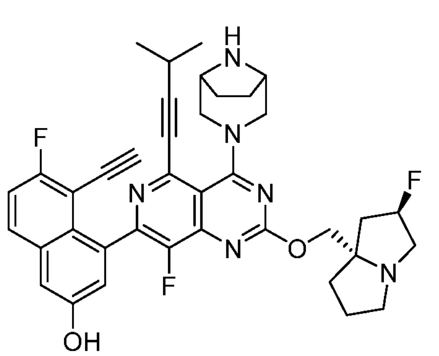 27, 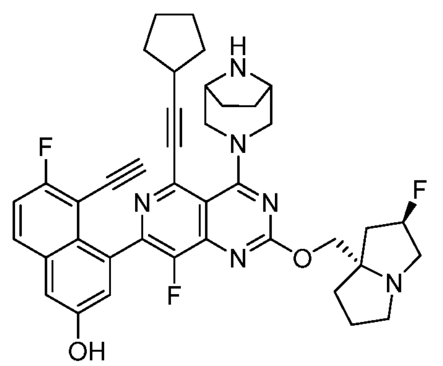 28,

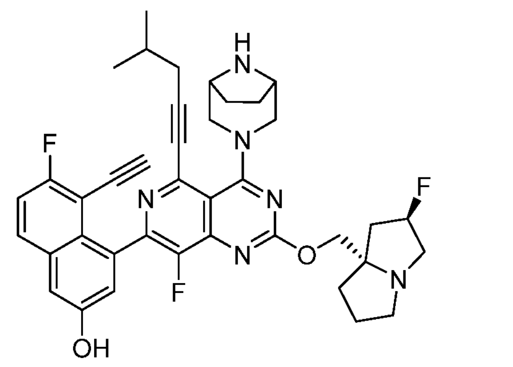

29,

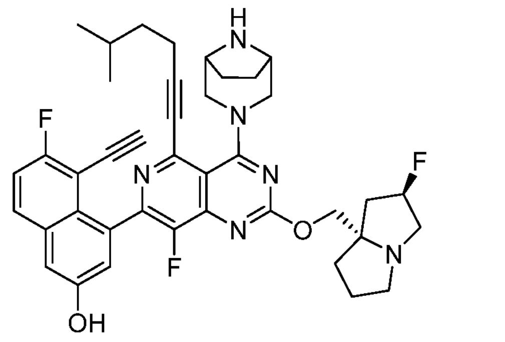

30,

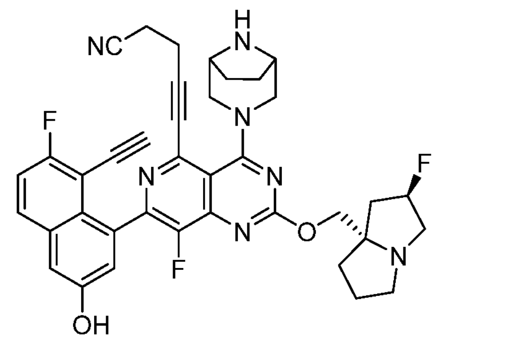

31,

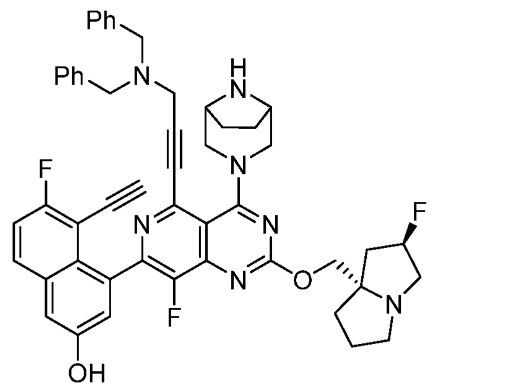

32,

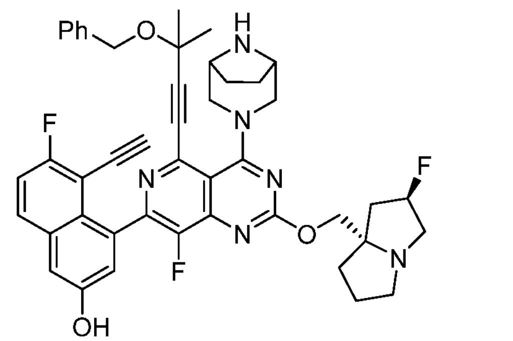

33,

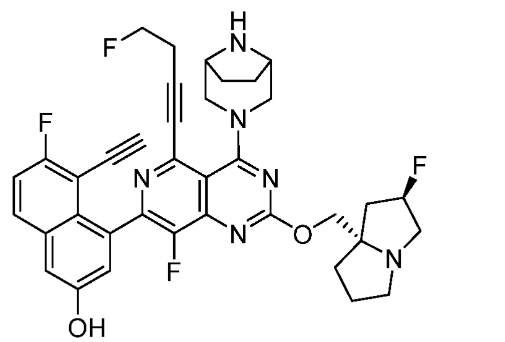

34,

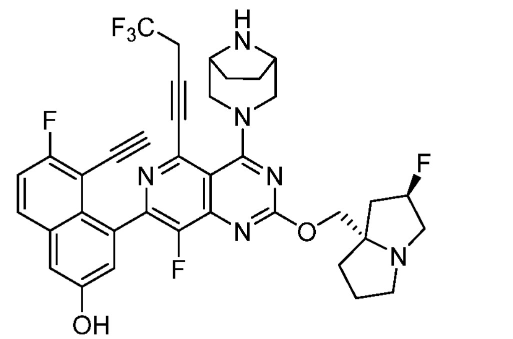

35,

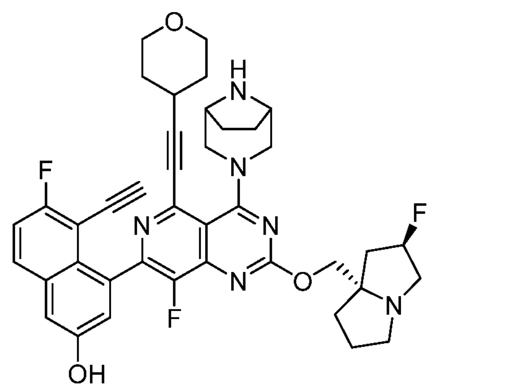

36,

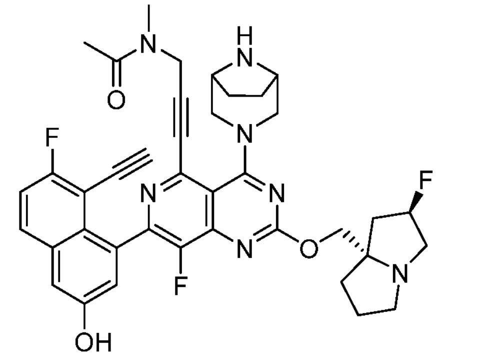

37,

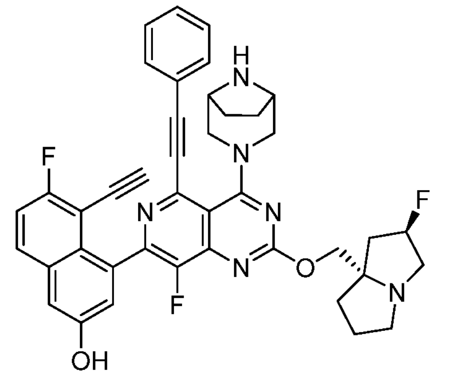

38,

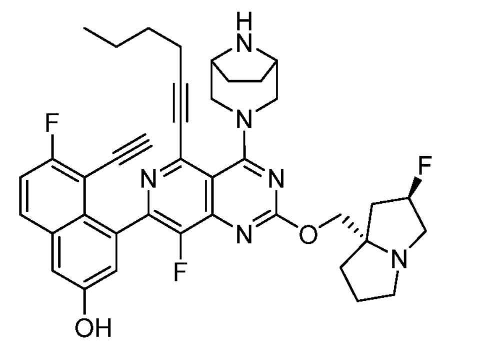

39,

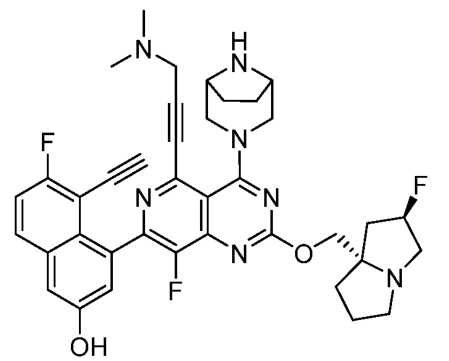

40,

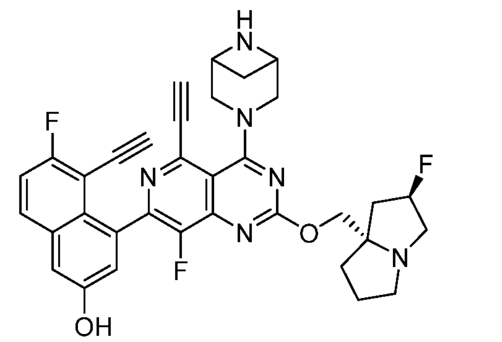

41,

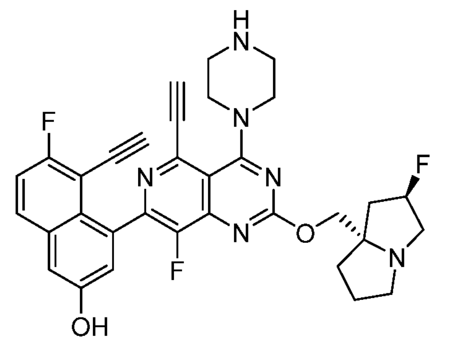

42,

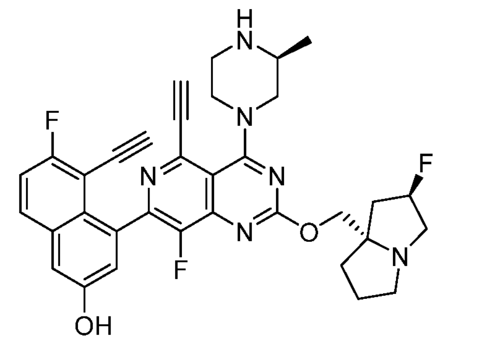

43,

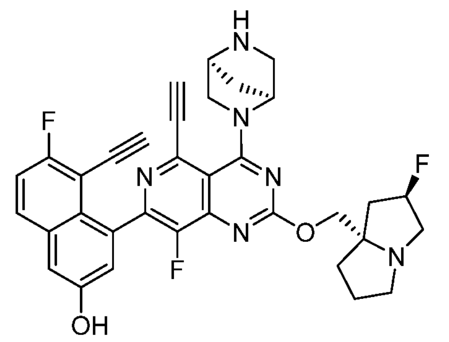

44,

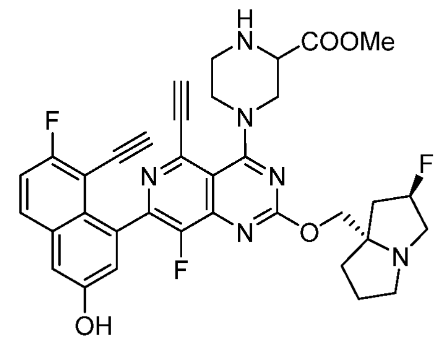
45,

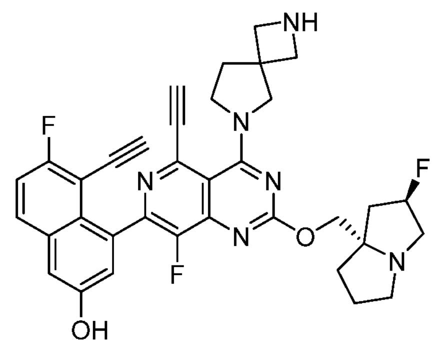
46,

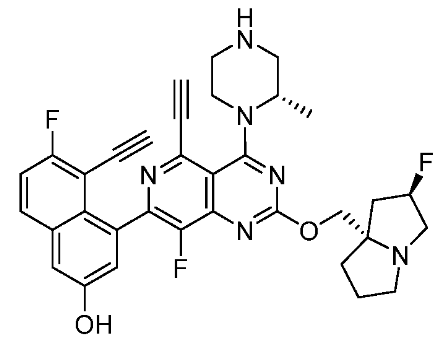
47,

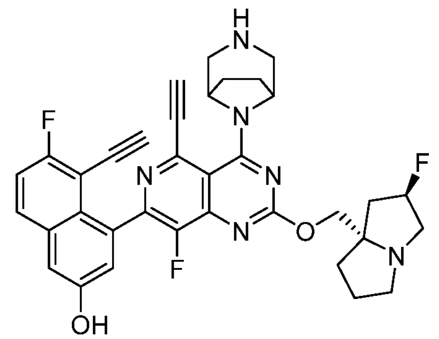
48,

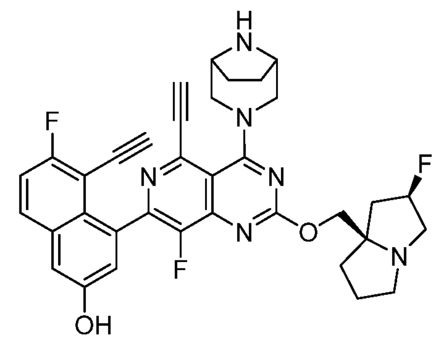
49,

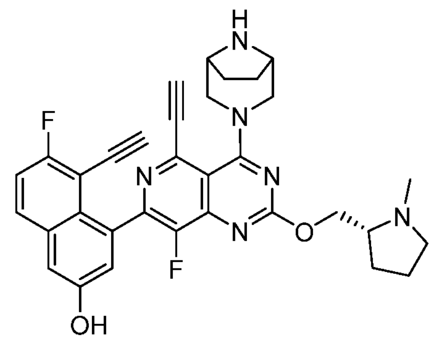
50,

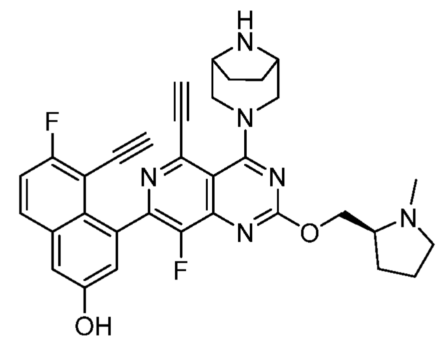
51,

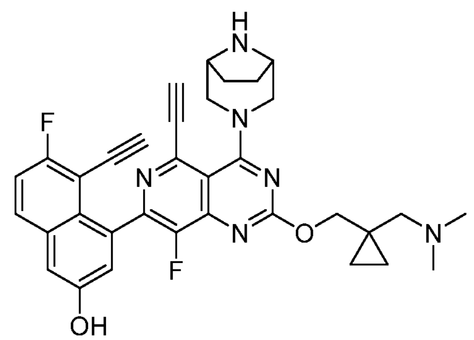
52,

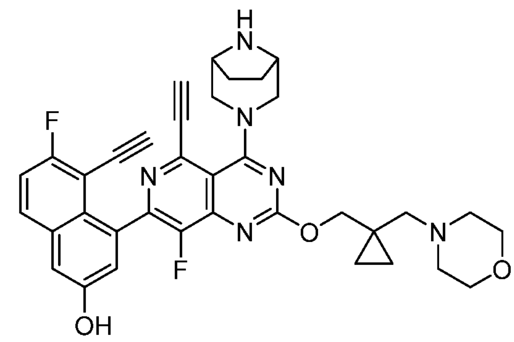
53,

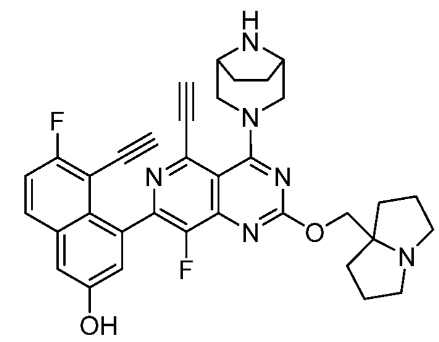
54,

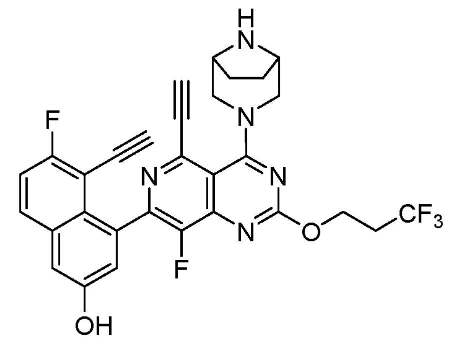
55,

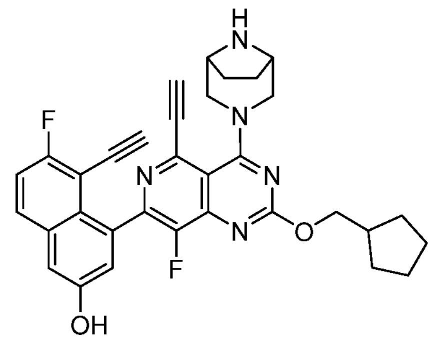
56,

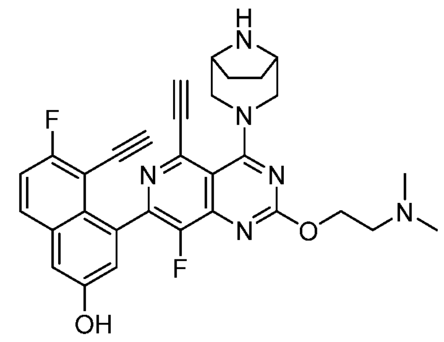
57,

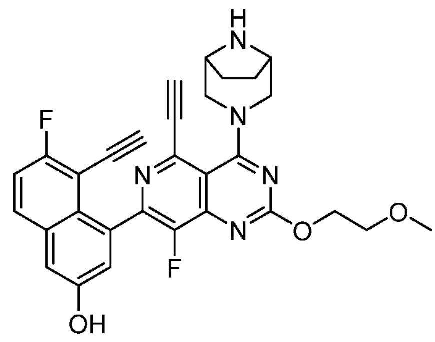
58,

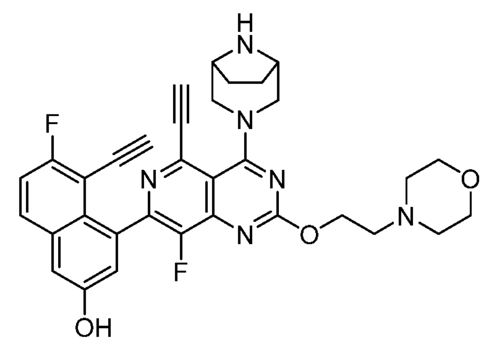
59,

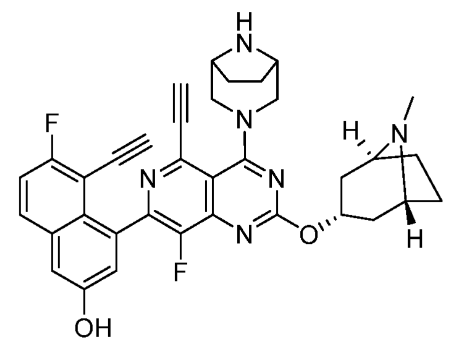
60,

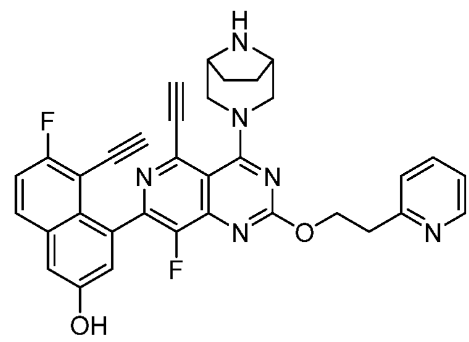

61,

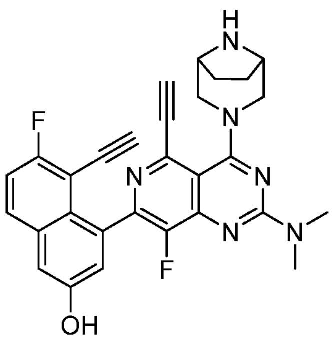

62,

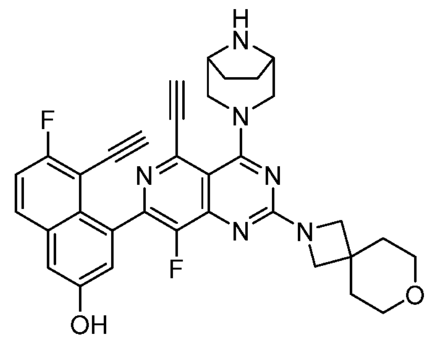

63,

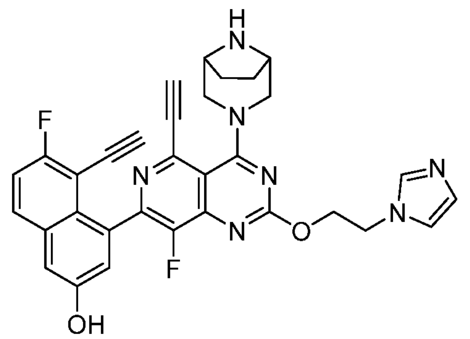

64,

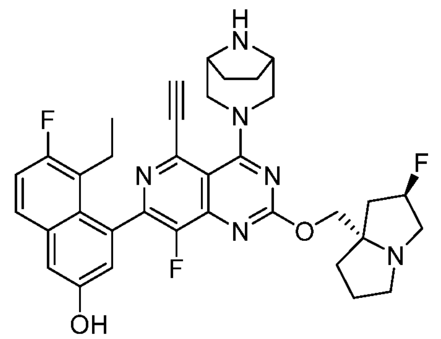

65,

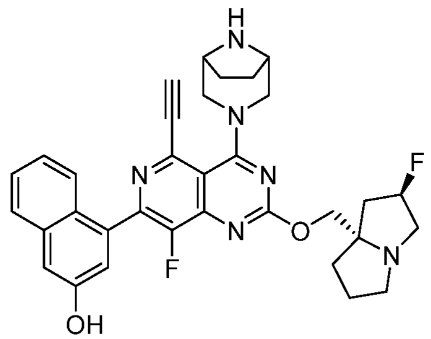

66,

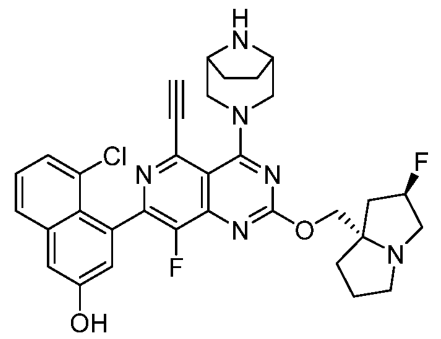

67,

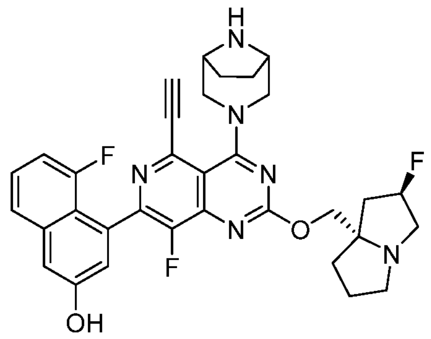

68,

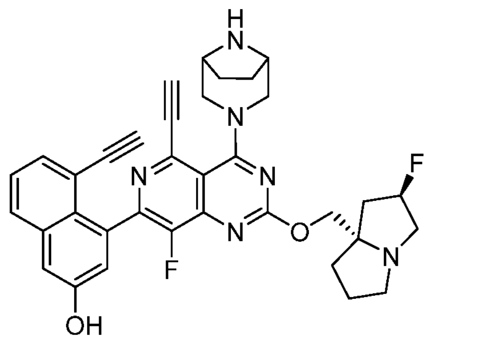
69,

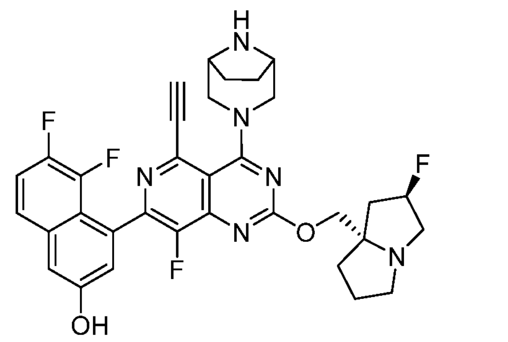
70,

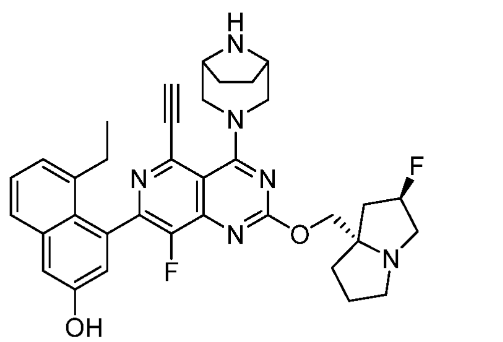
71,

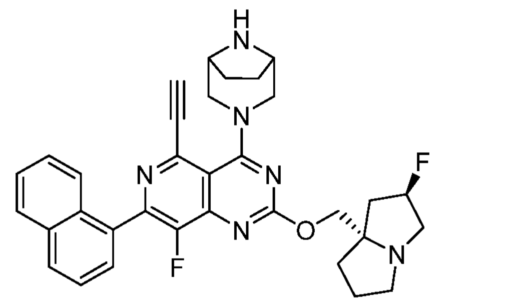
72,

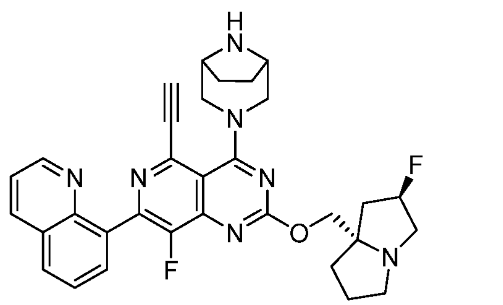
73,

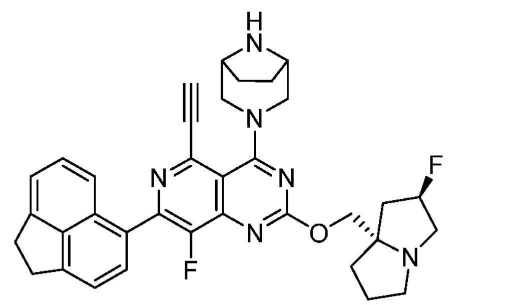
74,

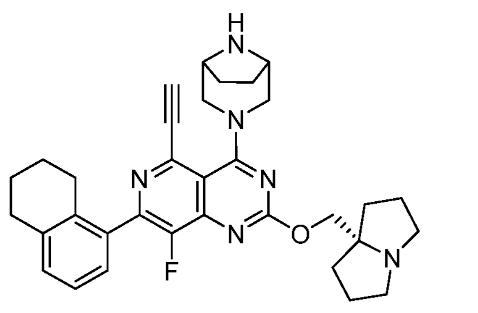
75,

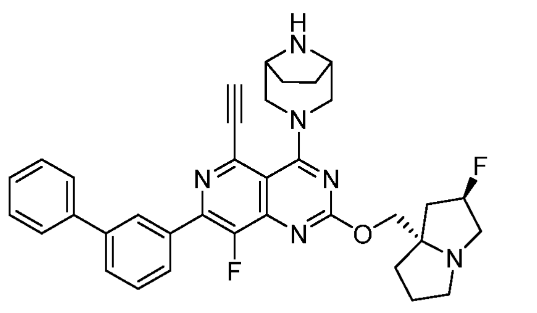
76,

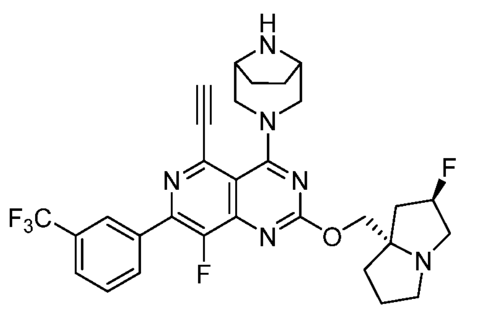
77,

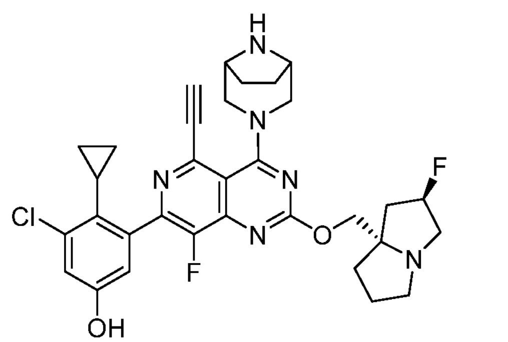
78,

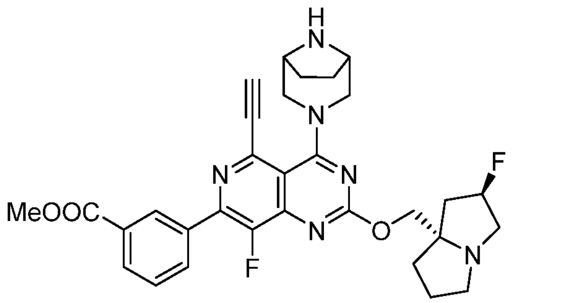 79,

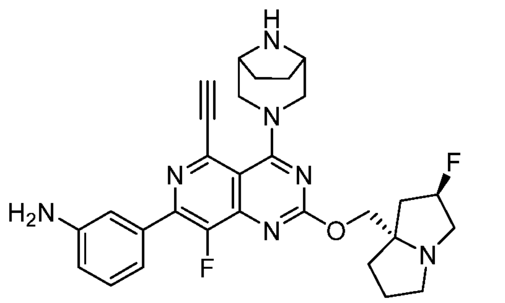 80,

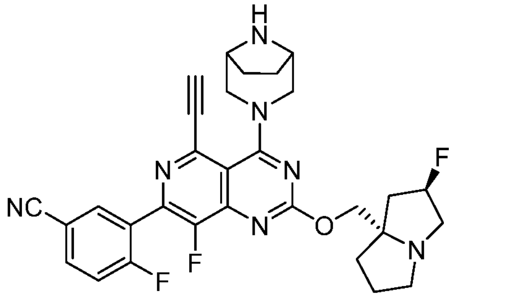 81,

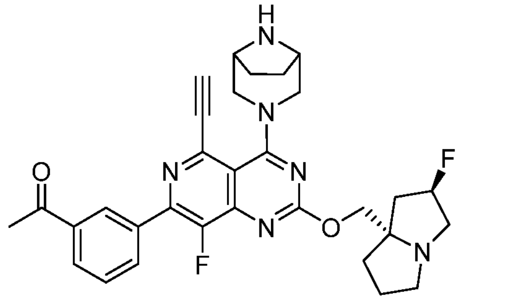 82,

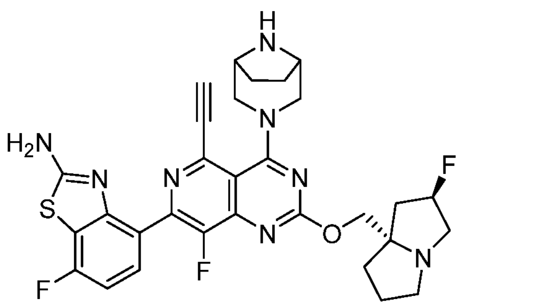 83,

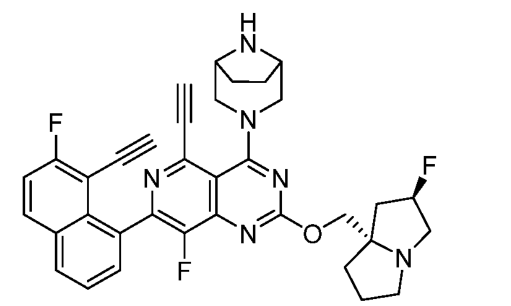 84,

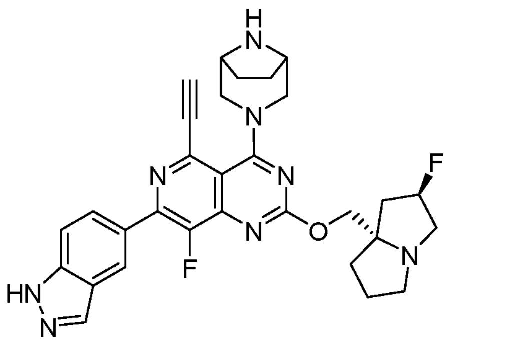 85,

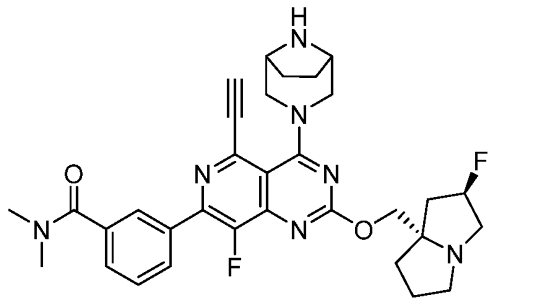 86,

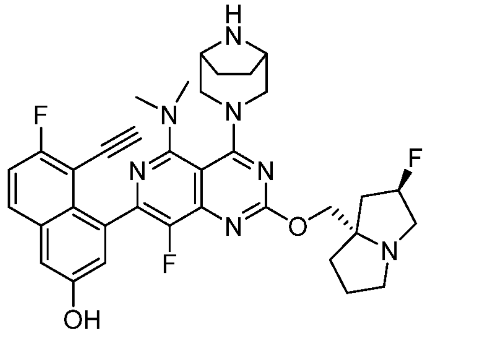 87,

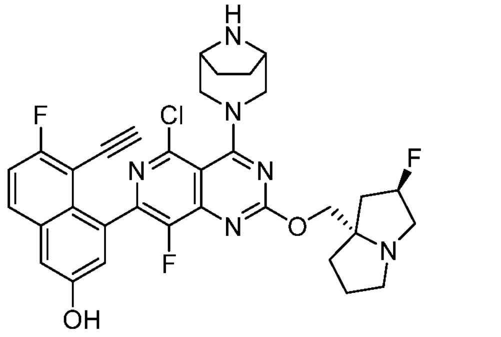 88,

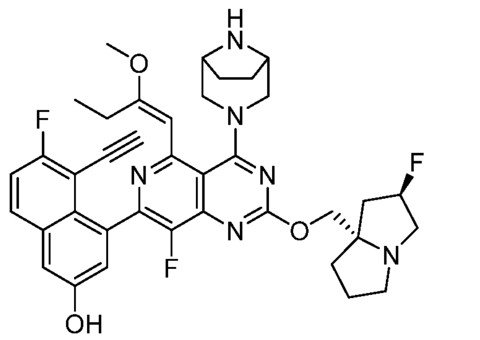 89,

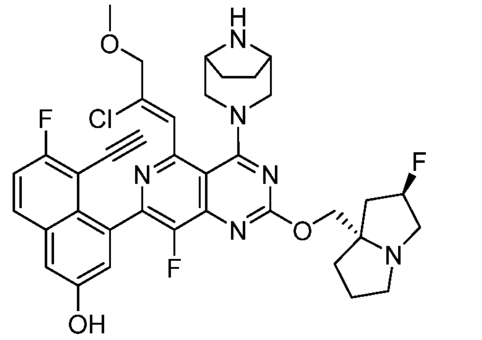 90,

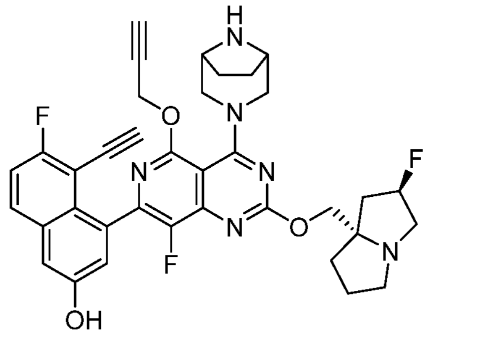 91,

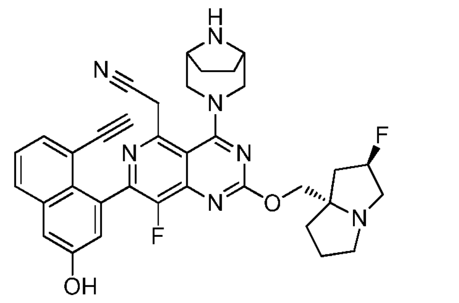 92,

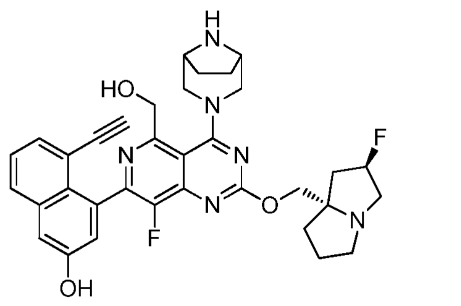 93,

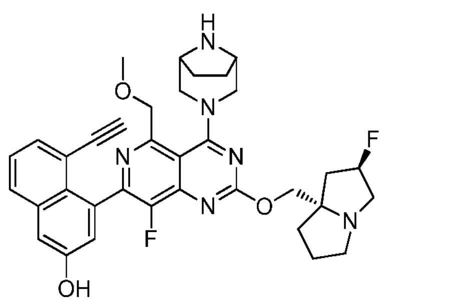 94,

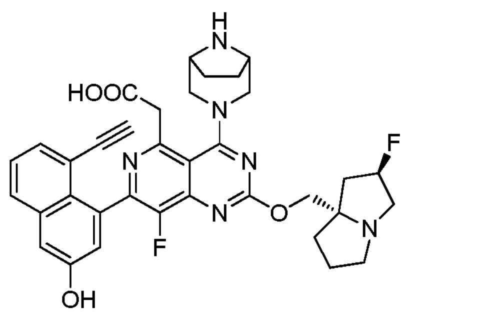 95,

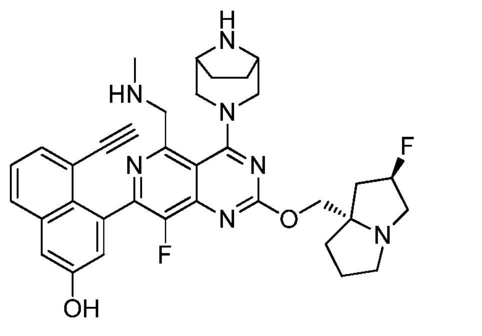 96,

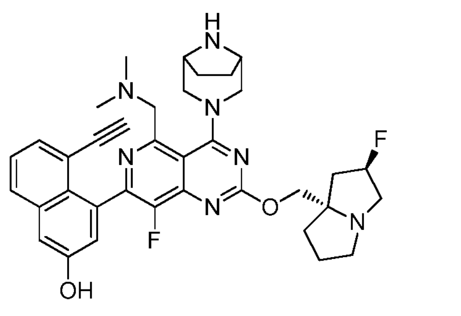 97,

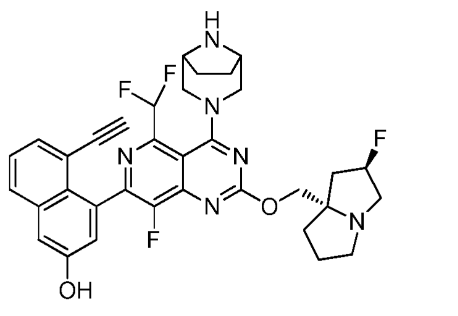 98,

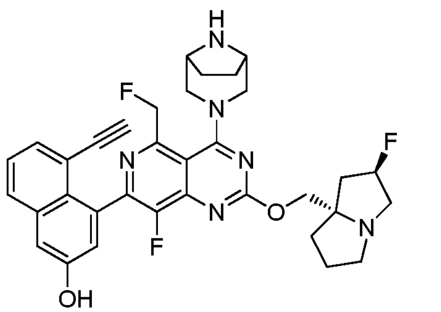 99,

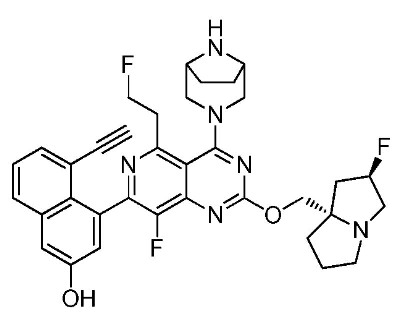 100,

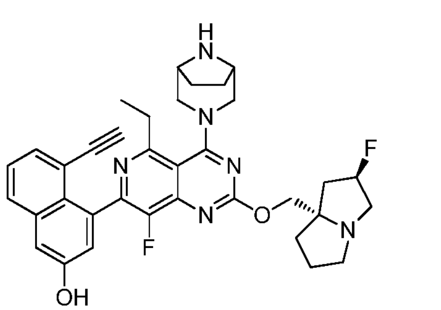 101,

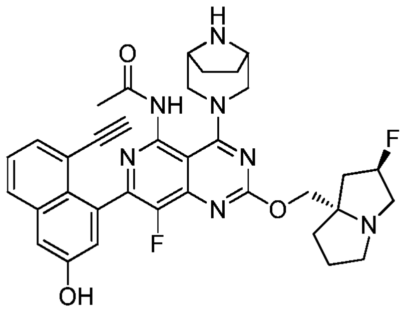 102,

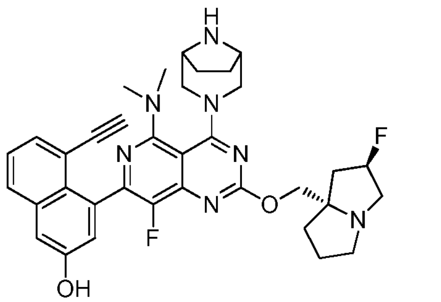 103,

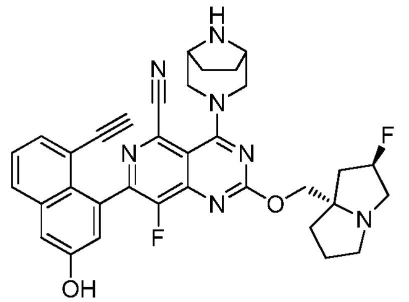 104,

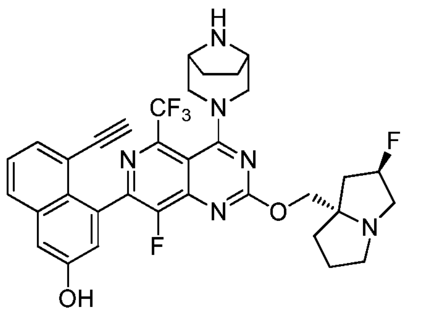 105.

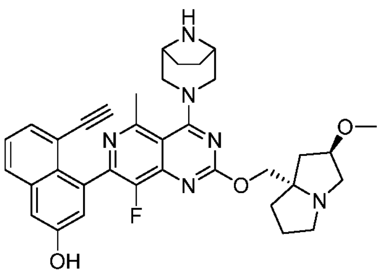 106.

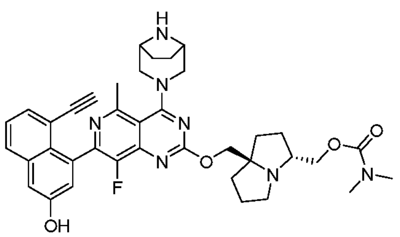 107,

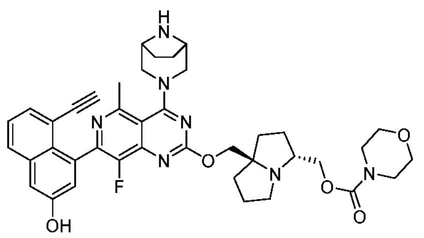 108,

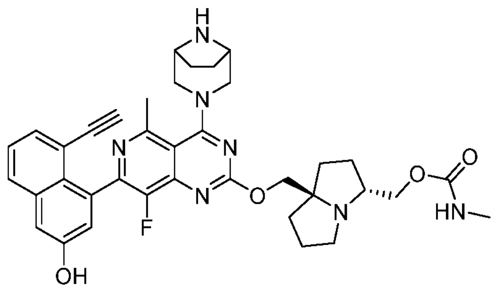
109,

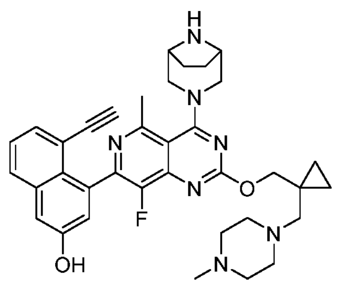
110,

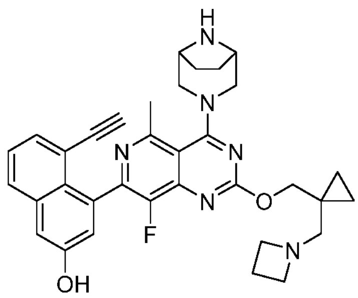
111,

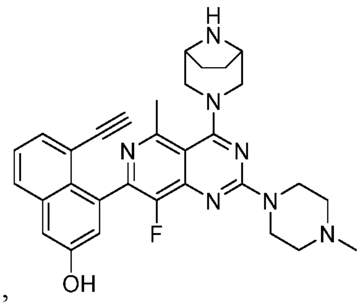
112,

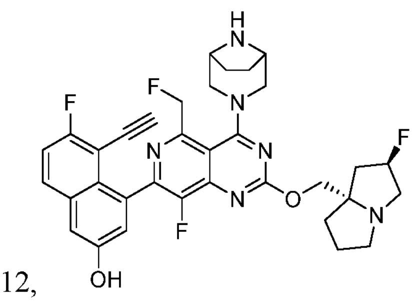
113,

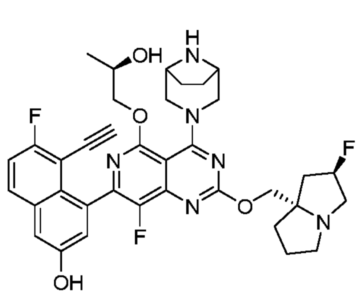
114,

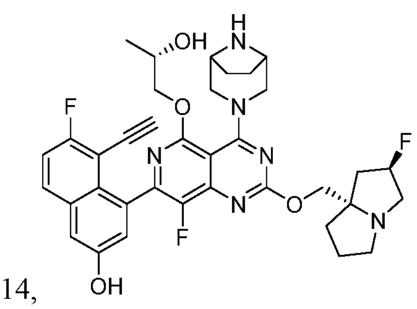
115,

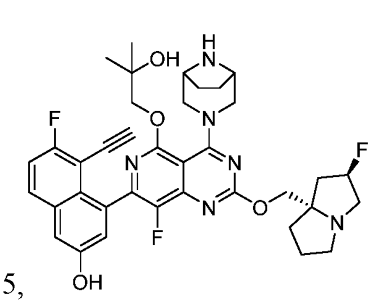
116,

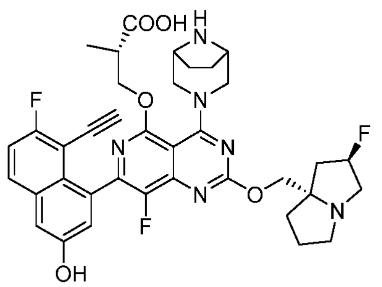
117,

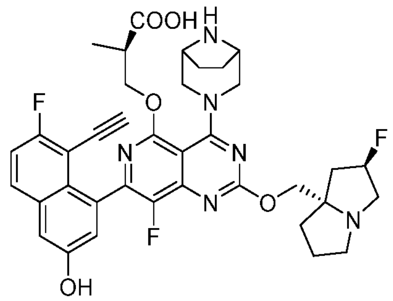
118,

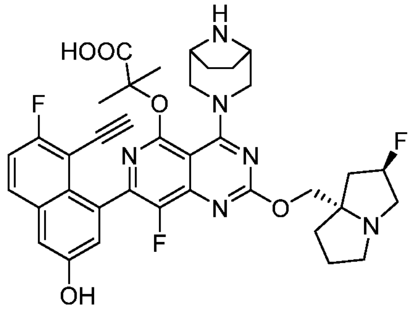
119,

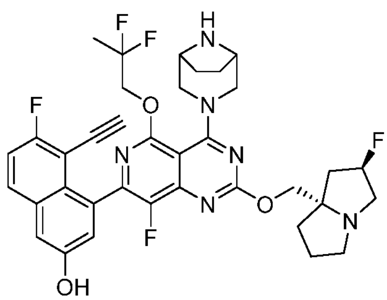
120,

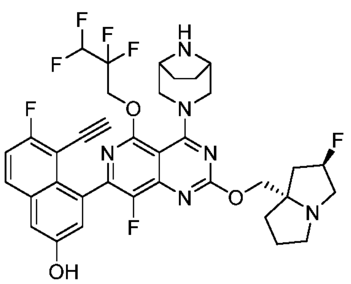 121, 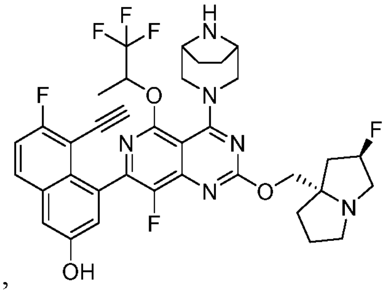 122, 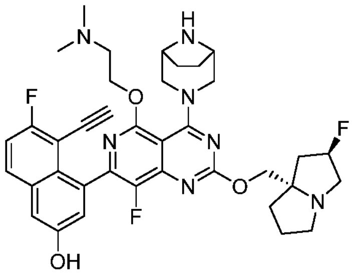 123,

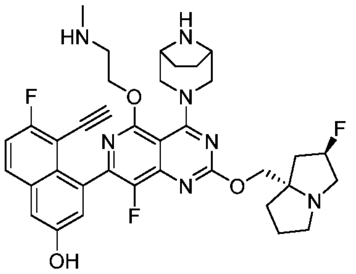 124, 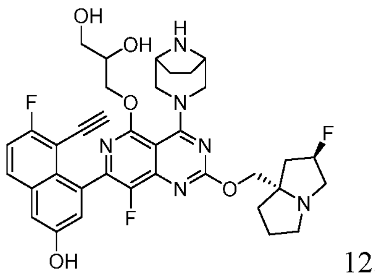 125, 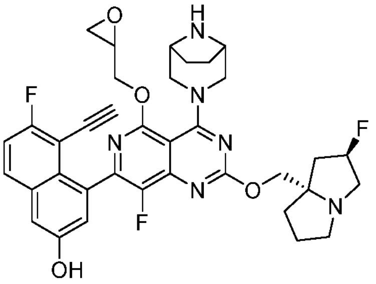 126,

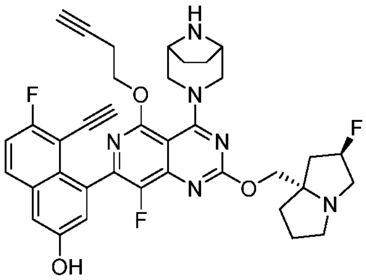 127, 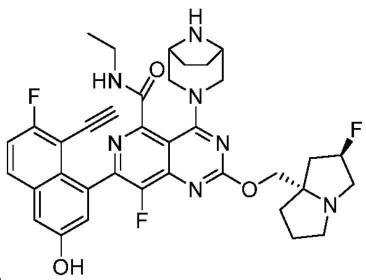 128, 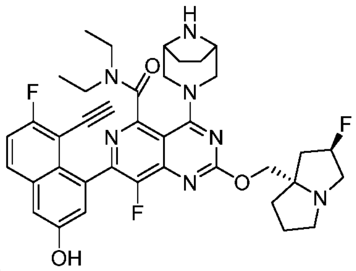 129

or 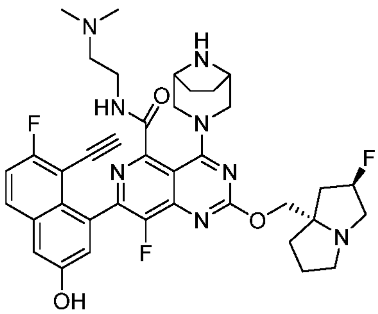 130.

**15.** A pharmaceutical composition comprising the compound of any one of claims 1-14; optionally, the pharmaceutical composition further comprises a pharmaceutically acceptable adjuvant.

**16.** Use of the compound of any one of claims 1-14 or the pharmaceutical composition of claim 15 in the manufacture of a medicament for preventing, treating or alleviating KRAS G12D related diseases.

**17.** The use of claim 16, wherein the KRAS G12D related disease is cancer.

**18.** The use of claim 17, wherein the cancer is non-small cell lung cancer, small cell lung cancer, colorectal cancer, rectal cancer, colon cancer, small intestine cancer, pancreatic cancer, uterine cancer, gastric cancer, esophageal cancer, prostate cancer, ovarian cancer, breast cancer, leukemia, melanoma, lymphoma or neuroma.

**INTERNATIONAL SEARCH REPORT**

International application No. **PCT/CN2022/125282**

**A. CLASSIFICATION OF SUBJECT MATTER**

C07D 471/00(2006.01)i; C07D 471/02(2006.01)i; C07D 471/04(2006.01)i; C07D 487/04(2006.01)i; C07D 519/00(2006.01)i; C07D 491/048(2006.01)i; A61K 31/435(2006.01)i; A61K 31/4375(2006.01)i; A61K 31/517(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

智能检索系统, CNTXT; ENXTX; CNABS; DWPI; SIPOABS; CNKI; Web of Science; STNext: KRAS G12D抑制剂, 嘧啶并吡啶, 癌症, 肿瘤, KRAS G12D inhibitors, pyrido[4, 3-d]pyrimidin, 基于式I化合物的结构检索, structural search based on formula I compound

**C. DOCUMENTS CONSIDERED TO BE RELEVANT**

| Category* | Citation of document, with indication, where appropriate, of the relevant passages | Relevant to claim No. |
|---|---|---|
| PX | WO 2022042630 A1 (INVENTISBIO CO., LTD. et al.) 03 March 2022 (2022-03-03)<br>claims 1-90, description, pages 273 to 295, table 1, and embodiment compound | 1-18 |
| PX | WO 2022002102 A1 (INVENTISBIO CO., LTD.) 06 January 2022 (2022-01-06)<br>claims 1-63, description, pages 138-153, table 1, and compounds 168 and 169 | 1-18 |
| PX | WO 2022199586 A1 (SUZHOU ZELGEN BIOPHARMACEUTICALS CO., LTD. et al.) 29 September 2022 (2022-09-29)<br>claims 1-15 | 1-18 |
| PX | WO 2022194191 A1 (GUANGDONG NEWOPP BIOPHARMACEUTICALS CO., LTD.) 22 September 2022 (2022-09-22)<br>claims 1-7, and description, embodiments 1-4 | 1-18 |
| PX | WO 2022184178 A1 (JACOBIO PHARMACEUTICALS CO., LTD.) 09 September 2022 (2022-09-09)<br>claims 1-42, description, embodiments 1-17, and table 13 | 1-18 |
| X | WO 2021041671 A1 (MIRATI THERAPEUTICS, INC. et al.) 04 March 2021 (2021-03-04)<br>claims 1-61, and description, embodiment, compounds 1-452 | 1-18 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 December 2022** | **12 January 2023** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/125282**

**C. DOCUMENTS CONSIDERED TO BE RELEVANT**

| Category* | Citation of document, with indication, where appropriate, of the relevant passages | Relevant to claim No. |
|---|---|---|
| X | WO 2020146613 A1 (MIRATI THERAPEUTICS, INC. et al.) 16 July 2020 (2020-07-16) claims 1-84 | 1-18 |
| A | CN 106488910 A (ARAXES PHARMA L.L.C.) 08 March 2017 (2017-03-08) claims 1-95, description, pages 40-133, table 1, and embodiment, compound | 1-18 |

Form PCT/ISA/210 (second sheet) (January 2015)

**INTERNATIONAL SEARCH REPORT**
**Information on patent family members**

International application No.
**PCT/CN2022/125282**

| Patent document cited in search report | Publication date (day/month/year) | Patent family member(s) | Publication date (day/month/year) |
|---|---|---|---|
| WO 2022042630 A1 | 03 March 2022 | None | |
| WO 2022002102 A1 | 06 January 2022 | None | |
| WO 2022199586 A1 | 29 September 2022 | None | |
| WO 2022194191 A1 | 22 September 2022 | None | |
| WO 2022184178 A1 | 09 September 2022 | None | |
| WO 2021041671 A1 | 04 March 2021 | IL 290845 A | 01 April 2022 |
| | | US 11453683 B1 | 27 September 2022 |
| | | EP 4021444 A1 | 06 July 2022 |
| | | BR 112022003543 A2 | 24 May 2022 |
| | | AU 2020337938 A1 | 17 March 2022 |
| | | CA 3148745 A1 | 04 March 2021 |
| | | CN 114615981 A | 10 June 2022 |
| | | CO 2022003782 A2 | 08 July 2022 |
| | | ZA 202202362 B | 28 September 2022 |
| | | TW 202122396 A | 16 June 2021 |
| | | JP 2022546043 A | 02 November 2022 |
| | | KR 20220071193 A | 31 May 2022 |
| | | CL 2022000447 A1 | 21 October 2022 |
| WO 2020146613 A1 | 16 July 2020 | WO 2021141628 A1 | 15 July 2021 |
| | | US 2020331911 A1 | 22 October 2020 |
| | | EP 4087573 A1 | 16 November 2022 |
| | | EP 3908283 A1 | 17 November 2021 |
| | | JP 2022517222 A | 07 March 2022 |
| CN 106488910 A | 08 March 2017 | NI 201600049 A | 20 May 2016 |
| | | NO 20160646 A1 | 19 April 2016 |
| | | EP 3055290 A1 | 17 August 2016 |
| | | CA 2926328 A1 | 16 April 2015 |
| | | BR 112016008016 A2 | 12 September 2017 |
| | | EA 201690752 A1 | 29 July 2016 |
| | | NZ 719076 A | 26 November 2021 |
| | | PH 12016500538 A1 | 13 June 2016 |
| | | EP 3636639 A1 | 15 April 2020 |
| | | AU 2014331794 A1 | 21 April 2016 |
| | | IL 244699 D0 | 21 April 2016 |
| | | SG 11201602662 Y A | 30 May 2016 |
| | | KR 20160076519 A | 30 June 2016 |
| | | MX 2016004360 A | 19 August 2016 |
| | | ZA 201602245 B | 25 September 2019 |
| | | UA 119971 C2 | 10 September 2019 |
| | | WO 2015054572 A1 | 16 April 2015 |
| | | JP 2016532656 A | 20 October 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111200645
- CN 202210090616
- CN 202210624652
- WO 20211041671 A


### Non-patent literature cited in the description


- *Nature,* 2013, vol. 503, 548-551
- McGraw-Hill Dictionary of Chemical Terms. McGraw-Hill Book Company, 1984
- **ELIEL, E. ; WILEN, S.** Stereochemistry of Organic Compounds. John Wiley & Sons, Inc, 1994
- *J. Pharmaceutical Sciences,* 1977, vol. 66, 1-19
- **REMINGTON: TROY et al.** Remington: The Science and Practice of Pharmacy. Lippincott Williams & Wilkins, 2005
- **SWARBRICK et al.** Encyclopedia of Pharmaceutical Technology. Marcel Dekker, 19881999